# EUROPEAN PATENT SPECIFICATION

(11) **EP 2 155 685 B1**
(45) Date of publication and mention of the grant of the patent: **16.03.2011**
(21) Application number: 08750215.9
(22) Date of filing: 08.05.2008
(51) Int. Cl.: C07D 231/12

(54) **PYRAZOLE DERIVATIVES AS P2X7 MODULATORS**
PYRAZOLDERIVATE ALS P2X7-MODULATOREN
DÉRIVÉS DU PYRAZOLE UTILISÉS COMME MODULATEURS DE P2X7

(30) Priority: 10.05.2007 GB 0709038; 02.05.2008 GB 0808123
(43) Date of publication of application: 24.02.2010
(73) Proprietor: Glaxo Group Limited, Greenford, Middlesex UB6 0NN (GB)
(72) Inventor: BESWICK, Paul John, 08960 Sant Just Desvern Barcelona (ES); CHAMBERS, Laura Jane, Harlow Essex CM19 5AW (GB); CHESSELL, Iain Patrick, Harlow Essex CM19 5AW (GB); DAVIES, David John, Stevenage Hertfordshire SG1 2NY (GB); DEMONT, Emmanuel Hubert, Stevenage Hertfordshire SG1 2NY (GB); GLEAVE, Robert James, Harlow Essex CM19 5AW (GB); LIVERMORE, David George Hubert, Harlow Essex CM19 5AW (GB); THEOBALD, Pamela Joan, Harlow Essex CM19 5AW (GB); VIMAL, Mythily, Harlow Essex CM19 5AW (GB); WALTER, Daryl Simon, Harlow Essex CM19 5AW (GB)
(74) Representative: Breen, Anthony Paul
(86) International application number: PCT/EP2008/055724
(87) International publication number: WO 2008/138876

(56) References cited:
- WO-A-2005/019182
- WO-A-2007/056091
- WO-A-2007/141267

## Description

The present invention relates to pyrazole derivatives which modulate P2X7 receptor function and are capable of antagonizing the effects of ATP at the P2X7 receptor ("P2X7 receptor antagonists"); to processes for their preparation; to pharmaceutical compositions containing them; and to the use of such compounds in therapy.

The P2X7 receptor is a ligand-gated ion-channel which is expressed in cells of the hematopoietic lineage, e.g. macrophages, microglia, mast cells, and lymphocytes (T and B) (see, for example, Collo, et al. Neuropharmacology, Vol.36, pp1277-1283 (1997)), and is activated by extracellular nucleotides, particularly adenosine triphosphate (ATP). Activation of P2X7 receptors has been implicated in giant cell formation, degranulation, cytolytic cell death, CD62L shedding, regulation of cell proliferation, and release of proinflammatory cytokines such as interleukin 1 (IL-1 β3) (e.g. Ferrari, et al., J. Immunol., Vol.176, pp3877-3883 (2006)), interleukin 18 (IL-18), and tumour necrosis factor (TNFα) (e.g. Hide, et al. Journal of Neurochemistry, Vol.75, pp965-972 (2000)). P2X7 receptors are also located on antigen presenting cells, keratinocytes, parotid cells, hepatocytes, erythrocytes, erythroleukaemic cells, monocytes, fibroblasts, bone marrow cells, neurones, and renal mesangial cells. Furthermore, the P2X7 receptor is expressed by presynaptic terminals in the central and peripheral nervous systems and has been shown to mediate glutamate release in glial cells (Anderson, C. et al. Drug. Dev. Res., Vol.50, page 92 (2000)).

The localisation of the P2X7 receptor to key cells of the immune system, coupled with its ability to release important inflammatory mediators from these cells suggests a potential role of P2X7 receptor antagonists in the treatment of a wide range of diseases including pain and neurodegenerative disorders. Recent preclinical *in vivo* studies have directly implicated the P2X7 receptor in both inflammatory and neuropathic pain (Dell'Antonio et al., Neurosci. Lett., Vo1.327, pp87-90 (2002),. Chessell, IP., et al., Pain, Vol.114, pp386-396 (2005), Honore et al., J. Pharmacol Exp Ther., Vol.319, p1376-1385 (2006)) while there is in vitro evidence that P2X7 receptors mediate microglial cell induced death of cortical neurons (Skaper, S.D., et al., Glia, Vol.54, p234-242 (2006)). In addition, up-regulation of the P2X7 receptor has been observed around β-amyloid plaques in a mouse model of Alzheimer's disease (Parvathenani, L. et al. J. Biol. Chem., Vol.278(15), pp13309-13317 (2003)). R.G. Jones and M.J. Mann, J. Am. Chem. Soc., 1953, 75, 4048-4052 discloses 4-pyrazole-N-benzylacetamide and syntheses of beta-aminoethylpyrazoles.

V.A. Zagorevskii et al., Khimiko-Farmatsevticheskii Zhurnal; 1989, 23(8), 966-971 (Russian) discloses pyrazole-4-carboxamides and -acetamides as antialcoholic agents, including 2-(3,5-dimethyl-1-pheny)-1*H*-pyrazol-4-yl)-*N-*(phenylmethyl)acetamide.

PCT/EP2007/055518 (Glaxo Group Limited), filed on 5 June 2007 and published on 13 December 2007 as WO 2007/141267 A1, discloses *N*-(phenylmethyl)-2-(1*H-*pyrazol-4-yl)acetamide derivatives which modulate P2X7 receptor function and are capable of antagonizing the effects of ATP at the P2X7 receptor (P2X7 receptor antagonists), and their use for the manufacture of a medicament for the treatment or prevention of pain, inflammation or a neurodegenerative disease. PCT/EP2007/055521 (Glaxo Group Limited), filed on 5 June 2007 and published on 13 December 2007 as WO 2007/141269 A1, discloses *N*-(phenylmethyl)-2-(4-isoxazolyl)acetamide derivatives which modulate P2X7 receptor function and are capable of antagonizing the effects of ATP at the P2X7 receptor.

The present invention provides compounds which modulate P2X7 receptor function and are capable of antagonizing the effects of ATP at the P2X7 receptor ("P2X7 receptor antagonists"). A first aspect of the invention provides a compound of formula (I) or a pharmaceutically acceptable salt thereof: 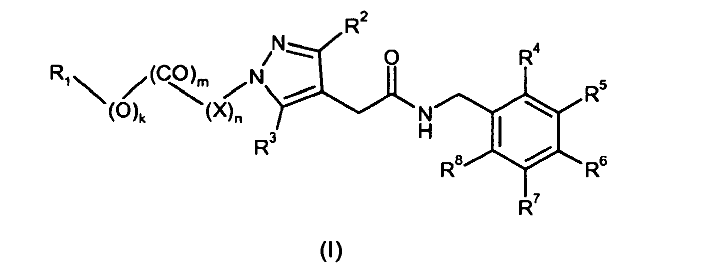 wherein:
R¹ represents NR⁹R¹⁰, C₁₋₆ alkyl or trifluoromethyl; or may represent hydrogen when k is 1;
or R¹ represents a C₆₋₁₀ aryl, a 5 to 6 membered or an 8 to 10 membered heteroaryl, a C₃₋₆ cycloalkyl or a 4 to 11 membered heterocyclyl; wherein any ring or ring system of said aryl, heteroaryl, cycloalkyl or heterocyclyl groups may be optionally substituted with 1 to 3 substituents, which may be the same or different, selected from the group consisting of halogen, hydroxyl, nitro, oxo, cyano, trifluoromethyl, C₁₋₆ alkyl, C₁₋₃alkoxy, C₁₋₃ alkoxyC₁₋₃ alkyl-, -COC₁₋₃ alkyl, -CONR¹¹R¹² and -NR¹¹R¹²; or R¹ represents a group selected from formula (II), (III), (IV), (V), (VI) and (VII): 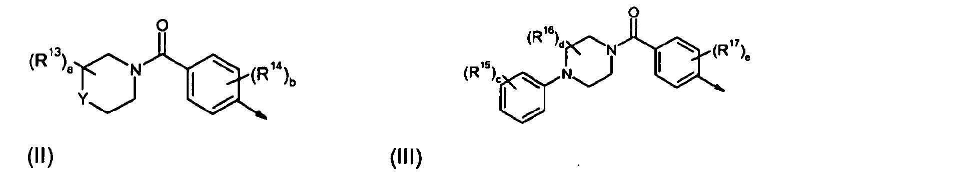 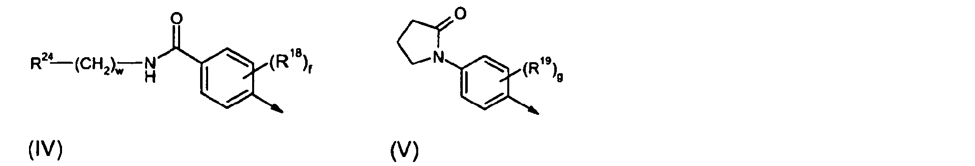 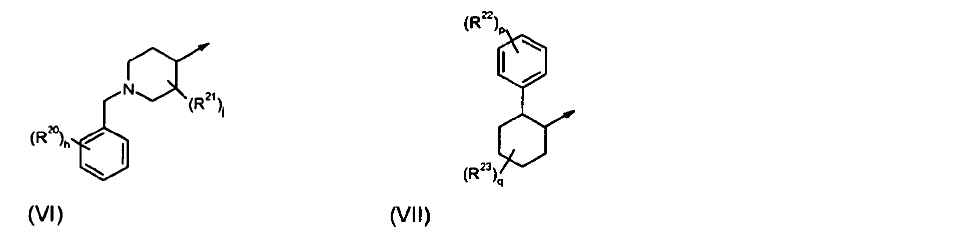 wherein:
Y represents CH₂, O or NR¹¹;
w represents 0, 1, 2 or 3;
a, b, c, d, e, f, g, h, j, p and q independently represent 0,1 or 2;
R¹³, R¹⁴, R¹⁵, R¹⁶, R¹⁷, R¹⁸, R¹⁹, R²⁰, R²¹, R²² and R²³ independently represent halogen, cyano, C₁₋₃ alkyl, C₁₋₃ alkoxy or trifluoromethyl;
R²⁴ represents NR¹¹R¹², -OH or -CONR¹¹R¹²;
or R²⁴ represents a C₃₋₆ cycloalkyl, a C₆₋₁₀ aryl, a 5 to 6 membered or an 8 to 10 membered heteroaryl, or a 4 to 11 membered heterocyclyl; wherein any ring or ring system of said aryl, heteroaryl, cycloalkyl or heterocyclyl groups may be optionally substituted with 1 to 3 substituents, which may be the same or different, selected from the group consisting of halogen, hydroxyl, nitro, oxo, cyano, trifluoromethyl, C₁₋₃ alkyl, C₁₋₃ alkoxy, C₁₋₃ alkoxyC₁₋₃ alkyl-, -COC₁₋₃ alkyl, -CONH₂ and -NR¹¹R¹²;
and wherein:
R² represents hydrogen, C₁₋₆ alkyl, C₁₋₄ alkoxy, trifluoromethyl, phenyl, a 5 to 6 membered monocyclic heteroaryl or a C₃₋₆ cycloalkyl;
R³ represents hydrogen, C₁₋₆ alkyl, C₁₋₄ alkoxy, trifluoromethyl, phenyl, a 5 to 6 membered monocyclic heteroaryl or C₃₋₆ cycloalkyl; or R³ represents halogen when R² is hydrogen;
R⁴ and R⁸ independently represent hydrogen, halogen, trifluoromethyl, C₁₋₆ alkyl, phenyl, phenyloxy, NO₂ or NH₂;
R⁵ and R⁷ independently represent hydrogen, halogen, trifluoromethyl, C₁₋₆ alkyl, phenyl, phenyloxy or NO₂;
R⁶ represents hydrogen, halogen, trifluoromethyl, trifluoromethoxy, C₁₋₆alkyl, C₃₋₆ cycloalkyl, phenyl, phenyloxy, NO₂ or cyano;
R⁹ and R¹⁰ independently represent hydrogen, C₁₋₄ alkyl, C₁₋₄ alkoxyC₁₋₄ alkyl-, -C₁₋₄ alkylOH or -C₁₋₃ alkylNR¹¹R¹²;
or a C₃₋₆ cycloalkyl or a 6 membered heterocyclyl, either ring of which may be optionally substituted with 1 to 3 substituents, which may be the same or different, selected from the group consisting of halogen, cyano, C₁₋₃ alkyl, C₁₋₃ alkoxy and trifluoromethyl;
R¹¹and R¹² independently represent hydrogen or C₁₋₃ alkyl;
k represents 0 or 1;
m represents 0 or 1;
n represents 0, 1, 2, 3 or 4;
X represents CR²⁵R²⁶; and
R²⁵ and R²⁶ at each occurrence independently represent hydrogen, fluorine or C₁₋₆ alkyl;
with the proviso that the compound of formula (I) is other than 2-(3,5-dimethyl-1-phenyl-1*H*-pyrazol-4-yl)-*N*-(phenylmethyl)acetamide or 2-(3,5-dimethyl-1-phenyl-1 *H-*pyrazol-4-yl)-*N*-[(2-fluorophenyl)methyl]acetamide.

As used herein, the term "alkyl" (when used as a group or as part of a group) refers to a straight or branched hydrocarbon chain containing the specified number of carbon atoms. For example, C₁₋₆ alkyl means a straight or branched hydrocarbon chain containing at least 1 and at most 6 carbon atoms. Examples of alkyl include, but are not limited to: methyl (Me), ethyl (Et), n-propyl, i-propyl, n-butyl, n-hexyl and i-hexyl.

As used herein, the term "alkoxy" (when used as a group or as part of a group) refers to an alkyl ether radical, wherein the term "alkyl" is defined above. Examples of alkoxy include, but are not limited to; methoxy, ethoxy, n-propoxy, i-propoxy, n-pentoxy and i-pentoxy.

The term 'cycloalkyl', unless otherwise stated (e.g. by virtue of a different specified number of carbon atoms), means a closed 3 to 8 membered non-aromatic ring, for example cyclopropyl, cyclobutyl, cyclopentyl, cyclohexyl, cycloheptyl or cyclooctyl.

The term 'halogen' is used herein to describe, unless otherwise stated, a group selected from fluorine, chlorine, bromine and iodine.

The term 'aryl' as used herein refers to a C₆₋₁₀ monocyclic or bicyclic hydrocarbon ring wherein at least one ring is aromatic. Examples of such groups include phenyl and naphthyl.

The term "heteroaryl" as used herein means a 5 to 6 membered monocyclic aromatic or a fused 8 to 10 membered bicyclic aromatic ring system containing 1 to 3 heteroatoms selected from oxygen, nitrogen and sulfur. Suitable examples of such monocyclic aromatic rings include thienyl, furanyl, pyrrolyl, triazolyl, imidazolyl, oxazolyl, thiazolyl, oxadiazolyl, isothiazolyl, isoxazolyl, thiadiazolyl, pyrazolyl, pyrimidyl, pyridazinyl, pyrazinyl and pyridyl. Suitable examples of such fused bicyclic aromatic ring systems include quinolinyl, isoquinolinyl, quinazolinyl, quinoxalinyl, cinnolinyl, naphthyridinyl, indolyl, indazolyl, pyrrolopyridinyl, benzofuranyl, benzothienyl, benzimidazolyl, benzoxazolyl, benzisoxazolyl, benzothiazolyl, benzisothiazolyl, benzoxadiazolyl, benzothiadiazolyl and the like. Heteroaryl groups, as described above, may be linked to the remainder of the molecule via a carbon atom or, when present, a suitable nitrogen atom except where otherwise indicated above.

The term "heterocyclyl" as used herein means a 4 to 7 membered monocyclic saturated or partially unsaturated ring containing 1 to 3 hetroatoms selected from oxygen, nitrogen or sulphur; or a 4 to 7 membered monocyclic saturated or partially unsaturated ring system containing 1 to 3 heteroatoms selected from oxygen or nitrogen fused to a benzene or monocyclic heteroaryl ring (referred to as fused rings); or a 7 membered bicyclic saturated ring system containing 1 nitrogen atom. Suitable examples of such monocyclic rings include azetidinyl, pyrrolidinyl, piperidinyl, oxypiperidinyl, piperazinyl, morpholinyl, thiomorpholinyl, diazepanyl, azepanyl, dihydroimidazolyl, tetrahydropyranyl, tetrahydrothiapyranyl and tetrahydrofuranyl. Suitable examples of such fused rings include dihydroindolyl, dihydroisoindolyl, tetrahydroquinolinyl, tetrahydrobenzazepinyl and tetrahydroisoquinolinyl. A suitable example of such a 7 membered bicyclic ring system is azabicylo[2.2.1]heptyl. Heterocyclyl groups, as described above, may be linked to the remainder of the molecule via a carbon atom or, when present, a suitable nitrogen atom except where otherwise indicated above.

It is to be understood that the present invention covers and discloses all possible combinations of particular, preferred, suitable, or other embodiments of groups or features (e.g., without limitation, of R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, X, Y, k, m, n, a, b, c, d, e, f, g, h, j, p, q, and/or w), e.g. all possible combinations of embodiments of different groups or features, which embodiments are described herein.

In certain embodiments of the invention, R represents NR⁹R¹⁰, C₁₋₆ alkyl or trifluoromethyl; or may represent hydrogen when k is 1; or R¹ represents a C₆₋₁₀ aryl, a 5 to 6 membered or an 8 to 10 membered heteroaryl, a C₃₋₆ cycloalkyl or a 4 to 11 membered heterocyclyl; wherein any ring or ring system of said aryl, heteroaryl, cycloalkyl or heterocyclyl groups may be optionally substituted with 1 to 3 substituents, which may be the same or different, selected from the group consisting of halogen, hydroxyl, nitro, oxo, cyano, trifluoromethyl, C₁₋₆ alkyl, C₁₋₃ alkoxy, C₁₋₃ alkoxyC₁₋₃ alkyl-, -COC₁₋₃ alkyl, -CONR¹¹R¹² and -NR¹¹R¹².

In a particular embodiment, R¹ represents NR⁹R¹⁰, C₁₋₆ alkyl or trifluoromethyl; or may represent hydrogen when k is 1; or R¹ represents a phenyl, a 5 to 6 membered or an 8 to 10 membered heteroaryl or a 4 to 11 membered heterocyclyl; wherein any ring or ring system of said phenyl, heteroaryl or heterocyclyl groups may be optionally substituted with 1 to 3 substituents, which may be the same or different, selected from the group consisting of halogen, C₁₋₆ alkyl, C₁₋₃ alkoxy, trifluoromethyl and -NR¹¹R¹².

In a particular embodiment, R¹ represents NR⁹R¹⁰, C₁₋₆ alkyl or trifluoromethyl; or may represent hydrogen when k is 1; or R¹ represents an optionally substituted group selected from phenyl, pyridinyl, pyrimidinyl, imidazolyl, pyrrolidinyl and pyrazolyl. The optional substituents, which may be the same or different, may be selected from the group consisting of halogen, C₁₋₆ alkyl, C₁₋₃ alkoxy, -CONH₂ and trifluoromethyl.

In an alternative particular embodiment of the invention, R¹ represents a group selected from formula (II), (III), (IV), (V), (VI) and (VII), in which the variables are as defined above.

In a particular embodiment, R¹ represents a group selected form formula (II), (III), (IV), (V), (VI) and (VII), in which a, b, c, d, e, f, g, h, j, p and q represent 0.

In one particular embodiment, R²⁴ represents NH₂, -OH or -CONH₂; or an unsubstituted phenyl, pyridinyl or tetrahydropyranyl; and w represents 1 or 2.

In one particular embodiment, R² represents hydrogen, C₁₋₆ alkyl (e.g. C₁₋₄ alkyl such as methyl, ethyl, n-propyl, i-propyl, or n-butyl) or trifluoromethyl.

In one particular embodiment, R³ represents hydrogen, C₁₋₆ alkyl (e.g. methyl, ethyl, n-propyl or i-propyl) or trifluoromethyl; or R³ represents halogen (e.g. bromine) when R² is hydrogen.

In one particular embodiment, both R² and R³ independently represent C₁₋₆ alkyl, e.g. C₁₋₄ alkyl such as methyl, ethyl, n-propyl, or i-propyl.

In a most particular embodiment, both R² and R³ represent methyl.

In one particular embodiment, R⁴ and R⁸ independently represent hydrogen, halogen or C₁₋₆ alkyl. In a more particular embodiment, R⁴ and R⁸ independently represent hydrogen, halogen or methyl. In a still more particular embodiment, one of R⁴ and R⁸, represents chlorine and the other represents hydrogen.

In one particular embodiment, R⁵ and R⁷ independently represent hydrogen, halogen or C₁₋₆ alkyl. In a more particular embodiment, R⁵ and R⁷ independently represent hydrogen, halogen or methyl. In a further embodiment, R⁵ and R⁷ represent hydrogen.

In one particular embodiment, R⁶ represents hydrogen or halogen. In a more particular embodiment, R⁶ represents halogen. In a still more particular embodiment, R⁶ represents fluorine.

Preferably, one of R⁴ and R⁸ represents chlorine and the other represents hydrogen, and R⁶ represents fluorine.

In one particular embodiment, R⁹ and R¹⁰ independently represent hydrogen, C₁₋₄ alkyl, C₁₋₄ alkoxy C₁₋₄alkyl- or -C₁₋₄ alkylOH.

In one particular embodiment, R¹¹ and R¹² independently represent hydrogen or methyl.

In one particular embodiment, n represents 0, 1 or 2 and R²⁵ and R²⁶ independently represent hydrogen or methyl. In a particular embodiment, k represents 0. In a particular embodiment m represents 0.

In one more particular embodiment, n represents 0, 1 or 2, R²⁵ and R²⁶ independently represent hydrogen or methyl, and k and m both represent 0.

In one particular embodiment of the invention there is provided a compound of formula (IA), or a pharmaceutically acceptable salt thereof: 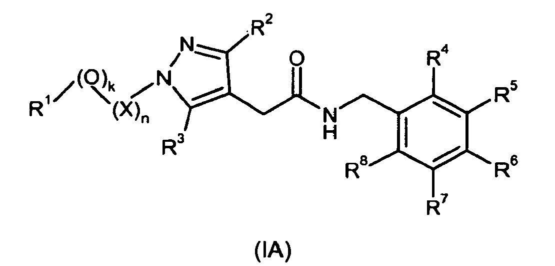 wherein:
R¹ represents NR⁹R¹⁰, C₁₋₆ alkyl or trifluoromethyl; or may represent hydrogen when k is 1;
or R¹ represents a C₆₋₁₀ aryl, a 5 to 6 membered or an 8 to 10 membered heteroaryl, a C₃₋₆ cycloalkyl or a 4 to 11 membered heterocyclyl; wherein any ring or ring system of said aryl, heteroaryl, cycloalkyl or heterocyclyl groups may be optionally substituted with 1 to 3 substituents, which may be the same or different, selected from the group consisting of halogen, hydroxyl, nitro, oxo, cyano, trifluoromethyl, C₁₋₆ alkyl, C₁₋₃alkoxy, C₁₋₃ alkoxyC₁₋₃ alkyl-, -COC₁₋₃ alkyl, -CONR¹¹R¹² and -NR¹¹R¹²;
R² and R³ independently represent hydrogen, methyl or trifluoromethyl;
R⁴ and R³ independently represent hydrogen, halogen or methyl;
R⁵ and R⁷ represent hydrogen;
R⁶ represents halogen;
R⁹ and R¹⁰ independently represent hydrogen, C₁₋₄ alkyl, C₁₋₄ alkoxyC₁₋₄ alkyl- or -C₁₋₄ alkylOH;
R¹¹ and R¹² independently represent hydrogen or methyl;
k represents 0 or 1;
n represents 0, 1 or 2;
X represents CR²⁵R²⁶; and
R²⁵ and R²⁶ at each occurrence independently represent hydrogen or methyl; with the proviso that the compound of formula (IA) is other than 2-(3,5-dimethyl-1-phenyl-1*H*-pyrazol-4-yl)-*N*-(phenylmethyl)acetamide or 2-(3,5-dimethyl-1-phenyl-1*H-*pyrazol-4-yl)-*N*-[(2-fluorophenyl)methyl]acetamide.

Particular compounds according to the invention include the compounds of E1-E183 as shown below, or a pharmaceutically acceptable salt thereof.

Therefore, a further particular aspect of the invention provides a compound or a pharmaceutically acceptable salt thereof, which is:
*N*-[(3,4-dichlorophenyl)methyl]-2-(3,5-dimethyl-1-phenyl-1*H*-pyrazol-4-yl)acetamide,
*N*-[(3,5-dichlorophenyl)methyl]-2-(3,5-dimethyl-1-phenyl-1*H*-pyrazol-4-yl)acetamide,
2-(3,5-dimethyl-1-phenyl-1*H*-pyrazol-4-yl)-*N*-({4-[(trifluoromethyl)oxy]phenyl}methyl)acetamide,
2-(3,5-dimethyl-1-phenyl-1*H*-pyrazol-4-yl)-*N*-{[2-fluoro-5-(trifluoromethyl)phenyl]methyl}acetamide,
2-(3,5-dimethyl-1-phenyl-1*H*-pyrazol-4-yl)-*N*-{[4-(trifluoromethyl)phenyl]methyl}acetamide,
2-(3,5-dimethyl-1-phenyl-1*H* pyrazol-4-yl)-*N*-{[4-fluoro-3-(trifluoromethyl)phenyl]methyl}acetamide,
*N*-[(3-chloro-4-fluorophenyl)methyl]-2-(3,5-dimethyl-l-phenyl-1*H*-pyrazol-4-yl)acetamide,
2-(3,5-dimethyl-1-phenyl-1 *H*-pyrazol-4-yl)-*N*-{[3-fluoro-5-(trifluoromethyl)phenyl]methyl}acetamide,
*N*-[(2-chloro-4-fluorophenyl)methyl]-2-(3,5-dimethyl-1-phenyl-1*H*-pyrazol-4-yl)acetamide,
*N*-(2-biphenylylmethyl)-2-(3,5-dimethyl-1-phenyl-1 *H*-pyrazol-4-yl)acetamide,
*N*-[(2,5-difluorophenyl)methyl]-2-(3,5-dimethyl-1-phenyl-1*H*-pyrazol-4-yl)acetamide,
*N*-[(3,4-difluorophenyl)methyl]-2-(3,5-dimethyl-1-phenyl-1*H*-pyrazol-4-yl)acetamide,
*N*-[(2-bromophenyl)methyl]-2-(3,5-dimethyl-1-phenyl-1*H*-pyrazol-4-yl)acetamide,
*N*-[(3-bromophenyl)methyl]-2-(3,5-dimethyl-1-phenyl-1*H*-pyrazol-4-yl)acetamide,
*N*-[(4-bromophenyl)methyl]-2-(3,5-dimethyl-1-phenyl-1*H*-pyrazol-4-yl)acetamide,
2-(3,5-dimethyl-1-phenyl-1*H*-pyrazol-4-yl)-N-[(2-nitrophenyl)methyl]acetamide,
2-(3,5-dimethyl-1-phenyl-1*H*-pyrazol-4-yl)-*N*-[(3-nitrophenyl)methyl]acetamide,
2-(3,5-dimethyl-1-phenyl-1*H*-pyrazol-4-yl)-*N*-[(4-nitrophenyl)methyl]acetamide,
*N*-[(3-chloro-2-methylphenyl)methyl]-2-(3,5-dimethyl-1-phenyl-1*H*-pyrazol-4-yl)acetamide,
2-(3,5-dimethyl-1-phenyl-1*H*-pyrazol-4-yl)-*N*-{[3-fluoro-4-(trifluoromethyl)phenyl]methyl}acetamide,
2-(3,5-dimethyl-1-phenyl-1*H*-pyrazol-4-yl)-*N*-[(2,3,4-trifluorophenyl)methyl]acetamide
*N*-[(2,3-difluoro-4-methylphenyl)methyl]-2-(3,5-dimethyl-1-phenyl-1*H*-pyrazol-4-yl)acetamide,
*N*-[(2,5-dimethylphenyl)methyl]-2-(3,5-dimethyl-1-phenyl-1*H*-pyrazol-4-yl)acetamide,
*N*-[(5-chloro-2-methylphenyl)methyl]-2-(3,5-dimethyl-1-phenyl-1*H*-pyrazol4-yl)acetamide,
*N*-[(2,6-difluorophenyl)methyl]-2-(3,5-dimethyl-1-phenyl-1*H*-pyrazol-4-yl)acetamide,
*N*-[(2-chloro-6-methylphenyl)methyl]-2-(3,5-dimethyl-1-phenyl-1*H*-pyrazol-4-yl)acetamide,
*N*-(4-biphenylylmethyl)-2-(3,5-dimethyl-1-phenyl-1*H*-pyrazol-4-yl)acetamide,
*N*-[(2,6-dimethylphenyl)methyl]-2-(3,5-dimethyl-1-phenyl-1*H*-pyrazol-4-yl)acetamide,
*N*-[(2-aminophenyl)methyl]-2-(3,5-dimethyl-1-phenyl-1*H*-pyrazol-4-yl)acetamide,
*N*-(3-biphenylylmethyl)-2-(3,5-dimethyl-1-phenyl-1*H*-pyrazol-4-yl)acetamide,
2-(3,5-dimethyl-1-phenyl-1*H*-pyrazol-4-yl)-*N*-[(5-fluoro-2-methylphenyl)methyl]acetamide,
*N*-{[3,5-bis(trifluoromethyl)phenyl]methyl}-2-(3,5-dimethyl-1-phenyl-1*H*-pyrazol-4-yl)acetamide,
*N*-[(4-cyanophenyl)methyl]-2-(3,5-dimethyl-1-phenyl-1*H*-pyrazol-4-yl)acetamide,
*N*-[(2-chlorophenyl)methyl]-2-(3,5-dimethyl-1-phenyl-1*H*-pyrazol-4-yl)acetamide,
*N*-[(2,3-difluorophenyl)methyl]-2-(3,5-dimethyl-1-phenyl-1*H*-pyrazol-4-yl)acetamide,
*N*-{[2-chloro-6-(phenyloxy)phenyl]methyl}-2-(3,5-dimethyl-1-phenyl-1*H*-pyrazol-4-yl)acetamide,
*N*-[(2,3-dichlorophenyl)methyl]-2-(3,5-dimethyl-1-phenyl-1*H*-pyrazol-4-yl)acetamide,
2-(3,5-dimethyl-1-phenyl-1*H* pyrazol-4-yl)-*N*-{[4-(phenyloxy)phenyl]methyl}acetamide,
*N*-[(2,4-dichlorophenyl)methyl]-2-(3,5-dimethyl-1-phenyl-1*H*-pyrazol-4-yl)acetamide,
*N*-[(2,3-dimethylphenyl)methyl]-2-(3,5-dimethyl-1-phenyl-1*H*-pyrazol-4-yl)acetamide,
*N*-[(2,6-dichlorophenyl)methyl]-2-(3,5-dimethyl-1-phenyl-1*H*-pyrazol-4-yl)acetamide,
*N*-[(2,5-dichlorophenyl)methyl]-2-(3,5-dimethyl-1-phenyl-1*H*-pyrazol-4-yl)acetamide,
*N*-[(2-chloro-6-fluorophenyl)methyl]-2-(3,5-dimethyl-1-phenyl-1*H*-pyrazol4-yl)acetamide,
*N*-{[2-chloro-5-(trifluoromethyl)phenyl]methyl}-2-(3,5-dimethyl-1-phenyl-1*H*-pyrazol-4-yl)acetamide,
2-(3,5-dimethyl-1-phenyl-1*H*-pyrazol-4-yl)-*N*-{[5-fluoro-2-(trifluoromethyl)phenyl]methyl}acetamide,
2-(3,5-dimethyl-1 -phenyl-1*H*-pyrazol-4-yl)-*N*-{[4-fluoro-2-(trifluoromethyl)phenyl]methyl}acetamide,
*N*-[(5-chloro-2-fluorophenyl)methyl]-2-(3,5-dimethyl-1-phenyl-1*H*-pyrazol-4-yl)acetamide,
*N*-[(2-chloro-4-fluorophenyl)methyl]-2-[1-(4-fluorophenyl-3,5-dimethyl-1*H*-pyrazol-4-yl]acetamide,
*N*-[(2-chloro-4-fluorophenyl)methyl]-2-{1-[2-(dimethylamino)ethyl]-3,5-dimethyl-1*H-*pyrazol-4-yl}acetamide,
*N*-[(2-chloro-4-fluorophenyl)methyl]-2-[1-(*H*-imidazol-4-ylmethyl)-3,5-dimethyl-1*H-*pyrazol-4-yl]acetamide,
*N*-[(2-chloro-4-fluorophenyl)methyl]-2-(1,3,5-trimethyl-1*H*-pyrazol-4-yl)acetamide,
*N*-[(2-chloro-4-fluorophenyl)methyl]-2-[3,5-dimethyl-1-(phenylmethyl)-1*H*-pyrazol-4-yl]acetamide,
*N*-[(2-chloro-4-fluorophenyl)methyl]-2-[1-(2,4-difluorophenyl)-3,5-dimethyl-1*H-*pyrazol-4-yl]acetamide,
*N*-[(2-chloro-4-fluorophenyl)methyl]-2-[1-(4-chlorophenyl)-3,5-dimethyl-1 *H*-pyrazol-4-yl]acetamide,
*N*-[(2-chloro-4-fluorophenyl)methyl]-2-{3,5-dimethyl-1-[4-(methyloxy)phenyl]-1*H-*pyrazol-4-yl}acetamide,
*N*-[(2-chloro-4-fluorophenyl)methyl]-2-[1-(3-fluorophenyl)-3,5-dimethyl-1*H*-pyrazol-4-yl]acetamide,
*N*-[(2-chloro-4-fluorophenyl)methyl]-2-[1-(3-chlorophenyl)-3,5-dimethyl-1*H*-pyrazol4-yl]acetamide,
*N*-[(2-chloro-4-fluorophenyl)methyl]-2-{3,5-dimethyl-1-[3-(methyloxy)phenyl]-1*H-*pyrazo)-4-yl}acetamide,
*N*-[(2-chloro-4-fluorophenyl)methyl]-2-[1-(2-fluorophenyl)-3,5-dimethyl-1*H*-pyrazol-4-yl]acetamide,
*N*-[(2-chloro-4-fluorophenyl)methyl]-2-[1-(2-chlorophenyl)3,5-dimethyl-1*H*-pyrazol-4-yl]acetamide,
*N*-[(2-chloro-4-fluorophenyl)methyl]-2-{3,5-dimethyl-1-[2-(methyloxy)phenyl]-1*H-*pyrazol-4-yl}acetamide,
*N*-[(2-chloro-4-fluorophenyl)methyl]-2-[3,5-dimethyl-1-(4-methylphenylyl)-1*H*-pyrazol-4-yl]acetamide,
*N*-[(2-chloro-4-fluorophenyl)methyl]-2-{1-[4-(ethyloxy)phenyl]-3,5-dimethyl-1*H-*pyrazol-4-yl}acetamide,
*N*-[(2-chloro-4-fluorophenyl)methyl]-2-[1-(5-cyano-2-pyridinyl)-3,5-dimethyl-1*H-*pyrazol-4-yl]acetamide,
*N*-[(2-chloro-4-fluorophenyl)methyl]-2-[1-(3,5-dichloro-2-pyridinyl)-3,5-dimethyl-1*H-*pyrazol-4-yl]acetamide,
*N*-[(2-chloro-4-fluorophenyl)methyl]-2-[3,5-dimethyl-1-(4-pyridinyl)-1*H*-pyrazol-4-yl]acetamide,
*N*-[(2-chloro-4-fluorophenyl)methyl]-2-{3,5-dimethyl-1-[6-methyl-4-(trifluoromethyl)-2-pyridinyl]-1*H*-pyrazol-4-yl}acetamide,
2-[1-(5-bromo-2-pyridinyl)-3,5-dimethyl-1*H*-pyrazol-4-yl]-*N*-[(2-chloro-4-fluorophenyl)methyl]acetamide,
*N*-[(2-chloro-4-fluorophenyl)methyl]-2-[1-(5-chloro-2-pyridinyl)-3,5-dimethyl-1*H-*pyrazol-4-yl]acetamide,
*N*-[(2-chloro-4-fluorophenyl)methyl]-2-[3,5-dimethyl-1-(1,3-thiazol-2-yl)1*H*-pyrazol-4-yl]acetamide,
*N*-[(2-chloro-4-fluorophenyl)methyl]-2-{3,5-dimethyl-1-[5-(trifluoromethyl)-2-pyridinyl]-1*H*-pyrazol-4-yl}acetamide,
-[(2-chloro-4-fluorophenyl)methyl]-2-{3,5-dimethyl-1-[3-(trifluoromethyl)-2-pyridinyl]-1*H*-pyrazol-4-yl}acetamide,
*N*-[(2-chloro-4-fluorophenyl)methyl]-2-[3,5-dimethyl-1-(3-pyridinyl)-1*H*-pyrazol-4-yl]acetamide,
*N*-[(2-chloro-4-fluorophenyl)methyl]-2-[1-(1,1-dimethylethyl)-3,5-dimethyl-1*H* pyrazol-4-yl]acetamide,
*N*-[(2-chloro-4-fluorophenyl)methyl]-2-[3,5-dimethyl-1-(2-pyridinyl)-1*H*-pyrazol-4-yl]acetamide,
*N*-[(2-chloro-4-fluorophenyl)methyl]-2-[1-(4-iodophenyl)-3,5-dimethyl-1*H*-pyrazol-4-yl]acetamide,
*N*-[(2-chloro-4-fluorophenyl)methyl]-2-[3,5-dimethyl-1-(4-pyridinylmethyl)-1*H*-pyrazol-4-yl]acetamide,
*N*-[(2-chloro-4-fluorophenyl)methyl]-2-[3,5-dimethyl-1-(6-methyl-3-pyridazinyl)-1*H-*pyrazol-4-yl]acetamide,
*N*-[(2-chloro-4-fluorophenyl)methyl]-2-[3,5-dimethyl-1-(2-pyrimidinyl)-1*H*-pyrazol-4-yl]acetamide,
*N*-[(2-chloro-4-fluorophenyl)methyl]-2-[1-(2,6-dimethyl-4-pyrimidinyl)-3,5-dimethyl-1*H-*pyrazol-4-yl]acetamide,
*N*-[(2-chloro-4-fluorophenyl)methyl]-2-{3,5-dimethyl-1-[4-(trifluoromethyl)-2-pyrimidinyl]-1*H*-pyrazol-4-yl}acetamide,
*N*-[(2-chloro-4-fluorophenyl)methyl]-2-[3,5-dimethyl-1-(2-phenylethyl)-1*H*-pyrazol-4-yl]acetamide,
*N*-[(2-chloro-4-fluorophenyl)methyl]-2-(1-cyclohexyl-3,5-dimethyl-1*H*-pyrazol-4-yl)acetamide,
*N*-[(2-chloro-4-fluorophenyl)methyl]-2-{1-[(2-chlorophenyl)methyl]-3,5-dimethyl-1*H-*pyrazol-4-yl}acetamide,
*N*-[(2-chloro-4-fluorophenyl)methyl]-2-[3,5-dimethyl-1-(2-methylpropyl)-1*H*-pyrazol-4-yl]acetamide,
*N*-[(2-chloro-4-fluorophenyl)methyl]-2-(1-cyclopentyl-3,5-dimethyl-1*H*-pyrazol-4-yl)acetamide,
*N*-[(2-chloro-4-fluorophenyl)methyl]-2-[3,5-dimethyl-1-(tetrahydro-2*H*-pyran-4-yl)-1*H-*pyrazol-4-yl]acetamide,
*N*-[(2-chloro-4-fluorophenyl)methyl]-2-{1-[(2,4-difluorophenyl)methyl]-3,5-dimethyl-1 *H*-pyrazol-4-yl}acetamide,
*N*-[(2-chloro-4-fluorophenyl)methyl]-2-{1-[(4-chlorophenyl)methyl]-3,5-dimethyl-1*H-*pyrazol-4-yl}acetamide,
*N*-[(2-chloro-4-fluorophenyl)methyl]-2-(3,5-dimethyl-1-{[4-(methyloxy)phenyl]methyl}-1 *H*-pyrazol-4-yl)acetamide,
*N*-[(2-chloro-4-fluorophenyl)methyl]-2-{3,5-dimethyl-1-[1-(phenylmethyl)-4-piperidinyl]-1*H*-pyrazol-4-yl}acetamide,
*N*-[(2-chloro-4-fluorophenyl)methyl]-2-(3,5-dimethyl-1-{[3-(methyloxy)phenyl]methyl}-1*H*-pyrazol-4-yl)acetamide,
*N*-[(2-chloro-4-fluorophenyl)methyl]-2-(3,5-dimethyl-1-{[2-(methyfoxy)phenyl]methyl}-1 *H*-pyrazol-4-yl)acetamide,
*N*-[(2-chloro-4-fluorophenyl)methyl]-2-[1-(2,2-dimethylpropyl)-3,5-dimethyl-1*H-*pyrazol-4-yl]acetamide,
*N*-[(2-chloro-4-fluorophenyl)methyl]-2-(1-{[2-(dimethylamino)phenyl]methyl}-3,5-dimethyl-1*H*-pyrazol-4-yl)acetamide,
*N*-[(2-chloro-4-fluorophenyl)methyl]-2-[3,5-dimethyl-1-(1-methyl-4-piperidinyl)-1*H-*pyrazol-4-yl]acetamide,
2-[1-(1-azabicyclo[2.2.1]hept-3-yl)-3,5-dimethyl-1*H*-pyrazol-4-yl]-*N*-[(2-chloro-4-fluorophenyl)methyl]acetamide,
*N*-[(2-chloro-4-fluorophenyl)methyl]-2-(3,5-dimethyl-1-{1-methyl-2-[4-(methyloxy)phenyl]ethyl}-1*H*-pyrazol-4-yl)acetamide,
*N*-[(2-chloro-4-fluorophenyl)methyl]-2-[3,5-dimethyl-1-(2,2,2-trifluoroethyl)-1*H-*pyrazol-4-yl]acetamide,
*N*-[(2-chloro-4-fluorophenyl)methyl]-2-(1-{2-[(2,6-dimethylphenyl)oxy]ethyl}-3,5-dimethyl-1*H*-pyrazol-4-yl)acetamide,
2-[1-(1,3-benzothiazol-2-ylmethyl)-3,5-dimethyl-1*H*-pyrazol-4-yl]-*N*-[(2-chloro-4-fluorophenyl)methyl]acetamide,
*N*-[(2-chloro-4-fluorophenyl)methyl]-2-[3,5-dimethyl-1-(2-phenylcyclohexyl)-1*H-*pyrazol-4-yl]acetamide,
*N*-[(2-chloro-4-fluorophenyl)methyl]-2-{3,5-dimethyl-1-[(4-methylphenyl)methyl]-1*H-*pyrazol-4-yl}acetamide,
2-(1-{[3,4-bis(methyloxy)phenyl]methyl}-3,5-dimethyl-1*H*-pyrazol-4-yl)-*N*-[(2-chloro-4-fluorophenyl)methyl]acetamide,
*N*-[(2-chloro-4-fluorophenyl)methyl]-2-[3,5-dimethyl-1-(1-methylethyl)1*H*-pyrazol-4-yl]acetamide,
*N*-[(2-chloro-4-fluorophenyl)methyl]-2-{3,5-dimethyl-1-[3-(phenyloxy)propyl]-1*H-*pyrazol-4-yl}acetamide,
*N*-[(2-chloro-4-fluorophenyl)methyl]-2-[1-(cyclohexylmethyl)-3,5-dimethyl-1*H*-pyrazol-4-yl]acetamide,
*N*-[(2-chloro-4-fluorophenyl)methyl]-2-(1-ethyl-3,5-dimethyl-1*H*-pyrazol-4-yl)acetamide,
*N*-[(2-chloro-4-fluorophenyl)methyl]-2-{3,5-dimethyl-1-[2-(phenyloxy)ethyl]-1*H-*pyrazol-4-yl}acetamide,
*N*-[(2-chloro-4-fluorophenyl)methyl]-2-[3,5-dimethyl-1-(1-methyl-2-phenylethyl)-1*H-*pyrazol-4-yl]acetamide,
*N*-[(2-chloro-4-fluorophenyl)methyl]-2-(1-{2-[(4-chloro-3-methylphenyl)oxy]ethyl}-3,5-dimethyl-1 *H*-pyrazol-4-yl )acetamide,
*N*-[(2-chloro-4-fluorophenyl)methyl]-2-{3,5-dimethyl-1-[4-(2-oxo-1-pyrrolidinyl)phenyl]-1 *H*-pyrazol-4-yl}acetamide,
*N*-[(2-chloro-4-fluorophenyl)methyl]-2-[1-(4-fluorophenyl)-1*H*-pyrazol-4-yl]acetamide,
*N*-[(2-chloro-4-fluorophenyl)methyl]-2-[1-(4-fluorophenyl)-5-(trifluoromethyl)-1*H-*pyrazol-4-yl]acetamide,
*N*-[(2-chloro-4-fluorophenyl)methyl]-2-[1-(2-methylphenyl)-1*H*-pyrazol-4-yl]acetamide,
*N*-[(2-chloro-4-fluorophenyl)methyl]-2-[1-(2-ethylphenyl)-1*H*-pyrazol-4-yl]acetamide,
*N*-[(2-chloro-4-fluorophenyl)methyl]-2-{3,5-dimethyl)-1-[4-(trifluoromethyl)phenyl]-1*H-*pyrazol-4-yl}acetamide,
*N*-[(2-chloro-4-fluorophenyl)methyl]-2-[3,5-dimethyl-1-(3-pyridinylmethyl)-1*H*-pyrazol-4-yl]acetamide,
*N*-[(2-chloro-4-fluorophenyl)methyl]-2-(5-methyl-1-phenyl-1*H*-pyrazol-4-yl)acetamide,
2-(3-butyl-5-methyl-1-phenyl-1*H*-pyrazol-4-yl)-*N*-[(2-chloro-4-fluorophenyl)methyl]acetamide,
*N*-[(2-chloro-4-fluorophenyl)methyl]-2-[3-methyl-5-(1-methylethyl)-1-phenyl-1*H-*pyrazol-4-yl]acetamide,
*N*-[(2-chloro-4-fluorophenyl)methyl]-2-[5-methyl-3-(1-methylethyl)-1-phenyl-1*H-*pyrazol-4-yl]acetamide,
*N*-[(2-chloro-4-fluorophenyl)methyl]-2-(3-methyl-1,5-diphenyl-1*H*-pyrazol-4-yl)acetamide,
*N*-[(2-chloro-4-fluorophenyl)methyl]-2-[1-(4-fluorophenyl)-5-methyl-1*H*-pyrazol-4-yl]acetamide,
Ethyl [4-(2-{[(2-chloro-4-fluorophenyl)methyl]amino}-2-oxoethyl)-3,5-dimethyl-1*H-*pyrazol-1-yl]acetate,
[4-(2-{[(2-chloro-4-fluorophenyl)methyl]amino}-2-oxoethyl)-3,5-dimethyl-1*H*-pyrazol-1-yl]acetic acid,
*N*-[(2-chloro-4-fluorophenyl)methyl]-2-{3,5-dimethyl-1-[2-(4-morpholinyl)-2-oxoethyl]-1 *H*-pyrazol-4-yl}acetamide,
*N*-[(2-chloro-4-fluorophenyl)methyl]-2-{3,5-dimethyl-1-[2-oxo-2-(1-piperidinyl)ethyl]-1 *H*-pyrazol-4-yl}acetamide,
2-[4-(2-{[(2-chloro-4-fluorophenyl)methyl]amino}-2-oxoethyl)3,5-dimethyl-1*H*-pyrazol-1-yl]-*N*,*N*-diethylacetamide,
*N*-[(2-chloro-4-fluorophenyl)methyl]-2{3,5-dimethyl-1-[2-(4-methyl-1-piperazinyl)-2-oxoethyl]-1*H*-pyrazol-4-yl}acetamide,
2-[4-(2-{[(2-chloro-4-fluorophenyl)methyl]amino}-2-oxoethyl)-3,5-dimethyl-1*H*-pyrazol-1-yl]-*N*-[2-(dimethylamino)ethyl]acetamide,
*N*-[(2-chloro-4-fluorophenyl)methyl]-2-[1-(2-hydroxyethyl)-3,5-dimethyl-1*H*-pyrazol-4-yl]acetamide,
*N*-[(2-chloro-4-fluorophenyl)methyl]-2-{3,5-dimethyl-1-[2-(4-morpholinyl)ethyl]-1*H-*pyrazol-4-yl}acetamide,
*N*-[(2-chloro-4-fluorophenyl)methyl]-2-{1-[2-(cyclohexylamino)ethyl]-3,5-dimethyl-1*H-*pyrazol-4-yl}acetamide,
*N*-[(2-chloro-4-fluorophenyl)methyl]-2-{1-[2-(diethylamino)ethyl]-3,5-dimethyl-1*H-*pyrazol-4-yl}acetamide,
*N*-[(2-chloro-4-fluorophenyl)methyl]-2-{3,5-dimethyl-1-[2-(tetrahydro-2*H*-pyran-4-ylamino)ethyl]-1*H*-pyrazol-4-yl}acetamide,
*N*-[(2-chloro-4-fluorophenyl)methyl]-2-{3,5-dimethyl-1-[2-(1-piperidinyl)ethyl]-1*H* pyrazol-4-yl}acetamide,
*N*-[(2-chloro-4-fluorophenyl)methyl]-2-{3,5-dimethyl-1-[2-(1-pyrrolidinyl)ethyl]-1*H-*pyrazol-4-yl}acetamide,
*N*-[(2-chloro-4-fluorophenyl)methyl]-2-(3,5-dimethyl-1-{2-[(2-methylpropyl)amino]ethyl}-1*H*-pyrazol-4-yl)acetamide,
*N*-[(2-chloro-4-fluorophenyl)methyl]-2-(1-{2-[(2-hydroxyethyl)amino]ethyl}-3,5-dimethyl-1 *H*-pyrazol-4-yl)acetamide,
*N*-[(2-chloro-4-fluorophenyl)methyl]-2-(1-{2-[(2-hydroxyethyl)(methyl)amino]ethyl}-3,5-dimethyl-1 *H*-pyrazol-4-yl)acetamide,
*N*-[(2-chloro-4-fluorophenyl)methyl]-2-[3,5-dimethyl-1-(2-{[2-(methyloxy)ethyl]amino}ethyl)-1*H*-pyrazol-4-yl]acetamide,
2-[1-(2-aminoethyl)-3,5-dimethyl-1*H*-pyrazol-4-yl]-*N*-[(2-chloro-4-fluorophenyl)methyl]acetamide,
*N*-[(2-chloro-4-fluorophenyl)methyl]-2{3,5-dimethyl-1-[2-(methyloxy)ethyl]-1*H-*pyrazol-4-yl}acetamide,
*N*-[(2-chloro-4-fluorophenyl)methyl]-2-(3-methyl-1-phenyl-1*H*-pyrazol-4-yl)acetamide,
*N*-[(2-chloro-4-fluorophenyl)methyl]-2-[3,5-dimethyl-1-(2-methyl-4-pyridinyl)-1*H-*pyrazol-4-yl]acetamide,
*N*-[(2-chloro-4-fluorophenyl)methyl]-2-[3,5-dimethyl-1-(6-methyl-3-pyridinyl)-1*H-*pyrazol-4-yl]acetamide,
*N*-[(2-chloro-4-fluorophenyl)methyl]-2-[1-(2,6-dimethyl-3-pyridinyl)-3,5-dimethyl-1*H-*pyrazol-4-yl]acetamide,
*N*-[(2-chloro-4-fluorophenyl)methyl]-2-[3,5-dimethyl-1-(4-methyl-3-pyridinyl)-1*H-*pyrazol-4-yl]acetamide,
*N*-[(2-chloro-4-fluorophenyl)methyl]-2-[3,5-dimethyl-1-(2-methyl-3-pyridinyl)-1*H-*pyrazol-4-yl]acetamide,
*N*-[(2-chloro-4-fluorophenyl)methyl]-2-[3,5-dimethyl-1-(5-methyl-3-pyridinyl)-1*H-*pyrazol-4-yl]acetamide,
*N*-[(2-chloro-4-fluorophenyl)methyl]-2-[3,5-dimethyl-1-(2-pyridinylmethyl)-1*H*-pyrazol-4-yl]acetamide,
*N*-[(2-chloro-4-fluorophenyl)methyl]-2-{3,5-dimethyl-1-[4-(1-piperidinylcarbonyl)phenyl]-1*H*-pyrazol-4-yl}acetamide,
*N*-[(2-chloro-4-fluorophenyl)methyl]-2-{3,5-dimethyl-1-[4-(4-morpholinylcarbonyl)phenyl]-1*H*-pyrazol-4-yl}acetamide,
*N*-[(2-chloro-4-fluorophenyl)methyl]-2-(3,5-dimethyl-1-{4-[(4-methyl-1-piperazinyl)carbonyl]phenyl}-1*H*-pyrazol-4-yl)acetamide,
4-[4-(2-{[(2-chloro-4-fluorophenyl)methyl]amino}-2-oxoethyl)-3,5-dimethyl-1*H*-pyrazol-1-yl]-*N*-[2-(dimethylamino)ethyl]benzamide,
4-[4-(2-{[(2-chloro-4-fluorophenyl)methyl]amino}-2-oxoethyl)-3,5-dimethy)-1*H*-pyrazol-1-yl]benzamide,
*N*-[(2-chloro-4-fluorophenyl)methyl]-2-(3,5-dimethyl-1-{4-[(4-phenyl-1-piperazinyl)carbonyl]phenyl}-1*H*-pyrazol-4-yl)acetamide,
4-[4-(2-{[(2-chloro-4-fluorophenyl)methyl]amino}-2-oxoethyl)-3,5-dimethyl-1*H*-pyrazol-1-yl]-*N*-(2-hydroxyethyl)benzamide,
4-[4-(2-{[(2-chloro-4-fluorophenyl)methyl]amino}-2-oxoethyl)-3,5-dimethyl-1*H*-pyrazol-1-yl]-*N*-(tetrahydro-2*H*-pyran-4-ylmethyl)benzamide,
4-[4-(2-{[(2-chloro-4-fluorophenyl)methyl]amino}-2-oxoethyl)-3,5-dimethyl-1*H*-pyrazol-1-yl]-*N*-(2-pyridinylmethyl)benzamide,
*N*-(2-amino-2-oxoethyl)-4-[4-(2-{[(2-chloro-4-fluorophenyl)methyl]amino}-2-oxoethyl)-3,5-dimethyl-1*H*-pyrazol-1-yl]benzamide,
*N*-[(3-chloro-2-methylphenyl)methyl]-2-[1-(2-hydroxyethyl)-3,5-dimethyl-1*H*-pyrazol-4-yl]acetamide,
*N*-[(3-chloro-2-methylphenyl)methyl]-2{3,5-dimethyl-1-[2-(4-morpholinyl)ethyl]-1*H-*pyrazol-4-yl}acetamide,
*N*-[(2-chloro-4-fluorophenyl)methyl]-2-[1-(2-hydroxy-2-methylpropyl)-3,5-dimethyl-1*H-*pyrazol-4-yl]acetamide,
2-[1-(2-hydroxy-2-methylpropyl)-3,5-dimethyl-1*H*-pyrazol-4-yl]-*N*-[(2,3,4-trifluorophenyl)methyl]acetamide,
*N*-[(3-chloro-2-methylphenyl)methyl]-2-[1-(2-hydroxy-2-methylpropyl)-3,5-dimethyl-1 *H*-pyrazol-4-yl]acetamide,
*N*-[(2-chloro-6-methylphenyl)methyl]-2-[1-(2-hydroxy-2-methylpropyl)-3,5-dimethyl-1 *H*-pyrazol-4-yl]acetamide,
*N*-[(2-chloro-4-fluorophenyl)methyl]-2-{1-[6-(ethyloxy)-2-pyridinyl]-1*H*-pyrazol-4-yl}acetamide,
*N*-[(2-chloro-4-fluorophenyl)methyl]-2-{3,5-dimethyl-1-[6-(methyloxy)-3-pyridinyl]-1*H-*pyrazol-4-yl}acetamide,
*N*-[(2-chloro-4-fluorophenyl)methyl]-2-{3,5-dimethyl-1-[5-(methyloxy)-3-pyridinyl]-1*H-*pyrazol-4-yl}acetamide,
*N*-[(2-chloro-4-fluorophenyl)methyl]-2{3,5-dimethyl-1-[2-(methyloxy)-3-pyridinyl]-1*H-*pyrazol-4-yl}acetamide,
*N*-[(2-chloro-4-fluorophenyl)methyl]-2-[1-(2-hydroxyethyl)-5-(trifluoromethyl)-1*H-*pyrazol-4-yl]acetamide,
*N*-[(2-chloro-4-fluorophenyl)methyl]-2-[1-(2-hydroxyethyl)-3-(trifluoromethyl)-1*H-*pyrazol-4-yl]acetamide,
*N*-[(2-chloro-4-fluorophenyl)methyl]-2-[1-{2-[(2-hydroxyethyl)amino]ethyl}-3-(trifluoromethyl)-1*H*-pyrazol4-yl]acetamide,
*N*-[(2-chloro-4-fluorophenyl)methyl]-2-[1-[2-(tetrahydro-2*H*-pyran-4-ylamino)ethyl]-3-(trifluoromethyl)-1 *H*-pyrazol-4-yl]acetamide,
*N*-[(2-chloro-4-fluorophenyl)methyl]-2-[1-[2-(dimethylamino)ethyl]-5-(trifluoromethyl)-1 *H*-pyrazol-4-yl]acetamide,
*N*-[(2-chloro-4-fluorophenyl)methyl]-2-[1-[2-(tetrahydro-2*H*-pyran-4-ylamino)ethyl]-5-(trifluoromethyl)-1*H*-pyrazol-4-yl]acetamide,
*N*-[(2-chloro-4-fluorophenyl)methyl]-2-[1-[2-(1-piperidinyl)ethyl]-5-(trifluoromethyl)-1*H-*pyrazol-4-yl]acetamide,
*N*-[(2-chloro-4-fluorophenyl)methyl]-2-[1-(2-{[2-(methyloxy)ethyl]amino}ethyl)-5-(trifluoromethyl)-1*H*-pyrazol-4-yl]acetamide,
2-[3,5-dimethyl-1-(2-pyrimidinyl)-1*H*-pyrazol-4-yl]-*N*-[(2,3,4-trifluorophenyl)methyl]acetamide,
2-[5-bromo-1-(4-fluorophenyl)-1*H*-pyrazol-4-yl]-1*H*-[(2-chloro-4-fluorophenyl)methyl]acetamide, or
*N*-[(2-chloro-4-fluorophenyl)methyl]-2-[5-ethyl-1-(4-fluorophenyl)-1*H*-pyrazol-4-yl]acetamide;
or a pharmaceutically acceptable salt thereof.

Antagonists of P2X7 may be useful in preventing, treating, or ameliorating a variety of pain states (e.g. neuropathic pain, chronic inflammatory pain, and visceral pain), inflammation and neurodegeneration, in particular Alzheimer's disease. P2X7 antagonists may also constitute useful therapeutic agents in the management of rheumatoid arthritis and inflammatory bowel disease.

Compounds of the present invention which modulate P2X7 receptor function and are capable of antagonizing the effects of ATP at the P2X7 receptor ("P2X7 receptor antagonists") may be competitive antagonists, inverse agonists or negative allosteric modulators of P2X7 receptor function.

Certain compounds of formula (I) may in some circumstances form acid or base addition salts thereof. It will be appreciated that for use in medicine the salts of the compounds of formula (I) should be pharmaceutically acceptable. Pharmaceutically acceptable salts include those described by Berge, Bighley and Monkhouse , J. Pharm. Sci., 1977, 66, 1-19.

The term "pharmaceutically acceptable salts" includes salts prepared from pharmaceutically acceptable bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Salts derived from pharmaceutically acceptable organic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, trishydroxylmethyl amino methane, tripropyl amine, tromethamine, and the like.

When a compound of the present invention is basic, in one embodiment a pharmaceutically acceptable salt is prepared from a pharmaceutically acceptable acid, such as an inorganic or organic acid. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like. In a particular embodiment, the pharmaceutically acceptable acid is benzenesulfonic, camphorsulfonic, ethanesulfonic, hydrobromic, hydrochloric, methanesulfonic, nitric, phosphoric, sulfuric, or p-toluenesulfonic acid.

Examples of pharmaceutically acceptable salts include the ammonium, calcium, magnesium, potassium, and sodium salts, and salts formed from maleic, fumaric, benzoic, ascorbic, pamoic, succinic, hydrochloric, sulfuric, bismethylenesalicylic, methanesulfonic, ethanedisulfonic, propionic, tartaric, salicylic, citric, gluconic, aspartic, stearic, palmitic, itaconic, glycolic, p-aminobenzoic, glutamic, benzenesulfonic, cyclohexylsulfamic, phosphoric and nitric acids.

The compounds of formula (I) or salts thereof may be prepared in crystalline or non-crystalline form, and, if crystalline, may optionally be solvated, e.g. as the hydrate. This invention includes within its scope solvates (e.g. hydrates), such as stoichiometric solvates (e.g. hydrates), as well as compounds or salts thereof containing variable amounts of solvent (e.g. water).

Certain compounds of formula (I) or salts thereof may be capable of existing in stereoisomeric forms (e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The subject invention also includes isotopically-labeled compounds, which are identical to those recited in formula (I), or salts thereof, but for the fact that one or more atoms are replaced by an atom having an atomic mass or mass number different from the atomic mass or mass number most commonly found in nature. Examples of isotopes that can be incorporated into compounds or salts of the invention include isotopes of hydrogen, carbon, nitrogen, oxygen, phosphorous, fluorine, iodine, and chlorine, such as 3H, 11C, 14C, 18F, 1231 and 1251.

Compounds of the present invention and pharmaceutically acceptable salts of said compounds that contain the aforementioned isotopes and/or other isotopes of other atoms are within the scope of the present invention. Isotopically-labeled compounds or salts of the present invention, for example those into which radioactive isotopes such as 3H, 14C are incorporated, are potentially useful in drug and/or substrate tissue distribution assays. Tritiated, i.e., 3H, and carbon-14, i.e., 14C, isotopes are for example optionally chosen for their (in some cases) ease of preparation and/or detectability. 11C and 8F isotopes are generally useful in PET (positron emission tomography), and 1251 isotopes are generally useful in SPECT (single photon emission computerized tomography). PET and SPECT are useful in brain imaging. Further, substitution with heavier isotopes such as deuterium, i.e., 2H, can sometimes afford certain effects resulting from greater metabolic stability, for example increased in vivo half-life or reduced dosage requirements and, hence, may be chosen in some circumstances. Isotopically labeled compounds of formula (I) or salts thereof and following of this invention are in one embodiment and in some cases prepared by carrying out the procedures disclosed in the Schemes and/or in the Examples below, by substituting a readily available isotopically labeled reagent for a non-isotopically labeled reagent.

A further particular aspect of the invention provides a compound of formula (I) or a pharmaceutically acceptable salt thereof which is not a radioactive isotopically labeled compound or salt. In a particular embodiment, the compound or salt is not an isotopically labeled compound or salt.

### PREPARATION OF COMPOUNDS

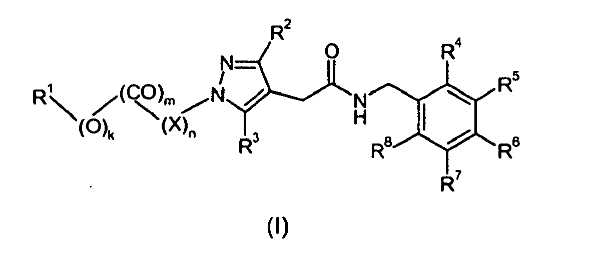

Compounds of formula (I), wherein the variables are defined above, and salts and solvates thereof may be prepared by the methodology described hereinafter, constituting a further aspect of this invention.

According to a further aspect of the invention, there is provided a process for preparing a compound of formula (I) or a pharmaceutically acceptable salt thereof which comprises:
(a) Coupling of a carboxylic acid of formula (2) (or an activated derivative thereof) with an amine of formula (3) (see Scheme 1), wherein R¹, R², R³, R⁴, R⁵, R⁶, R⁷, R⁸, X, k, m and n are as defined above. Compounds (2) and (3) are optionally protected. 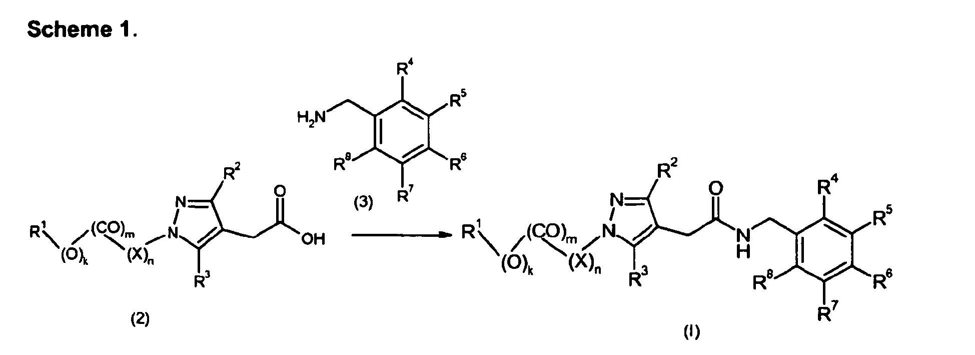 The coupling of an acid of formula (2) and an amine of formula (3) typically comprises the use of an activating agent or agents, such as water soluble carbodiimide (such as 1-ethyl-3-(3-dimethylaminopropyl)carbodiimide hydrochloride) or polymer-supported carbodiimide, 1-hydroxybenzotriazole (HOBT) and/or 1-Hydroxy-7-azabenzotriazole (HOAt), and optionally a suitable base such as a tertiary alkylamine (e.g. diisopropylethylamine, triethylamine) or pyridine, in a suitable solvent such as DMF (*N,N-*dimethylformamide) and e.g. at a suitable temperature e.g. between 0°C and room temperature. Alternatively the coupling of (2) and (3) may be accomplished by treatment with O-(7-Azabenzotriazol-1-yl)-N,N,N',N'-tetramethyluronium hexafluorophosphate and a suitable tertiary alkylamine such as diisopropylethylamine in a suitable solvent such as *N,N*-dimethylformamide e.g. at a suitable temperature such as room temperature. Where the compound of formula (2) is an activated derivative (e.g. acid chloride, mixed anhydride, active ester (e.g. O-acyl-isourea)), process (a) typically comprises treatment of said activated derivative with an amine (Ogliaruso, M.A.; Wolfe, J.F. in The Chemistry of Functional Groups (Ed. Patai, S.) Suppl.B: The Chemistry of Acid Derivatives, Pt. 1 (John Wiley and Sons, 1979), pp442-8; Beckwith, A.L.J. in The Chemistry of Functional Groups (Ed. Patai, S.) Suppl.B: The Chemistry of Amides (Ed. Zabricky, J.)(John Wiley and Sons, 1970), pp 73 ff.
(b) Reaction of a compound of formula (4) with a hydrazine of formula (5) (see Scheme 2), wherein R¹, R², R³, R⁴, R⁵, R⁶, R⁷, R⁸, X, k, m and n are as defined above and P¹ is a suitable oxygen protecting group. 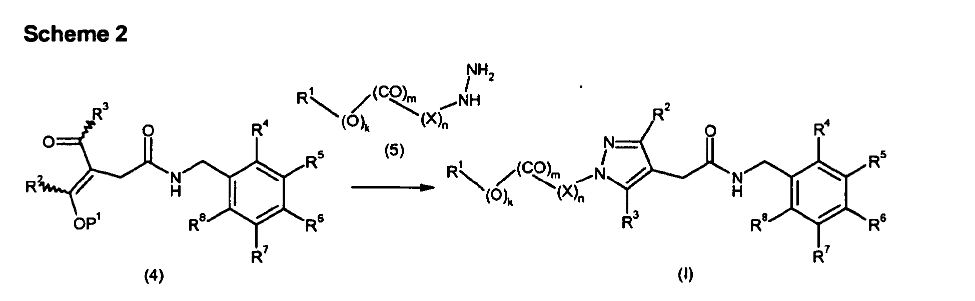 The reaction typically comprises treatment of a compound of formula (4) with a hydrazine of formula (5) and a suitable base such as sodium acetate in a suitable solvent such as ethanol, for example at a suitable temperature e.g. 110°C in a microwave oven.
   The protecting group P1 is typically a silicon-containing protecting group such as t-butyldimethylsilyl.
(c) Deprotecting a compound of formula (I) which is protected. Examples of protecting groups and the means for their removal can be found in T.W. Greene and P.G.M. Wuts 'Protective Groups in Organic Synthesis' (J.Wiley and Sons, 3rd Ed. 1999).
(d) Interconversion of compounds of formula (I) to other compounds of formula (I). Examples of conventional interconversion procedures include epimerisation, oxidation, reduction, alkylation, aromatic substitution, nucleophilic substitution, amide coupling, ester hydrolysis and halogenation (e.g. fluorination, chlorination, bromination, or iodination).

Representative methods for the preparation of compounds of formula (2) are shown in Schemes 3-8 below: 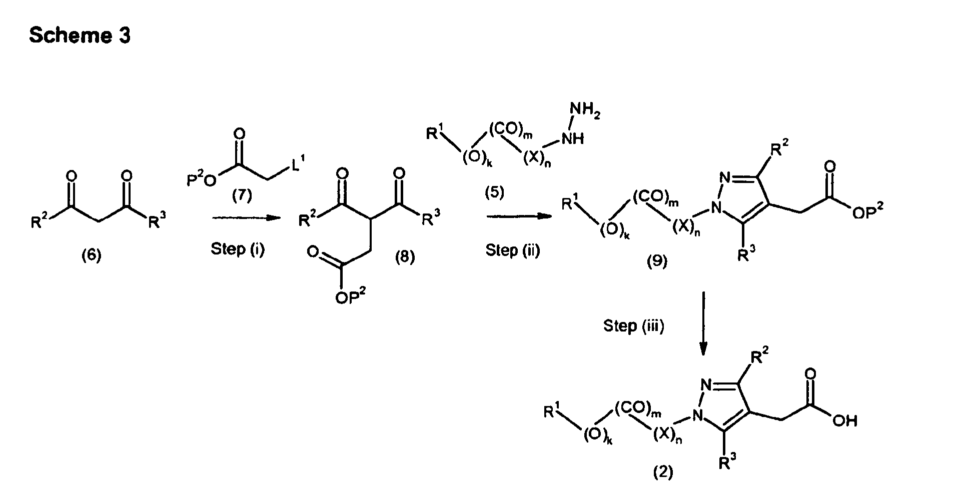 wherein R¹, R², R³, X, k, m and n are as defined above and P² represents a suitable protecting group such as C₁₋₆ alkyl, and L¹ represents a suitable leaving group such as a halogen atom (e.g. iodine, chlorine or bromine).

An analogous process has been described previously (US 4,146,721) starting from both the free base and salt (e.g. HCl) forms of the hydrazines (5).

Step (i) typically comprises the use of a suitable solvent such as a dioxane/water mixture and a suitable base such as potassium hydroxide at a suitable temperature e.g. between 0°C and room temperature.

Step (ii) typically comprises the use of the hydrazine (5) or a salt (e.g. HCl) thereof in a suitable solvent such as ethanol. If the free base is used then an acid such as acetic acid is also added to the mixture. Alternatively if the salt of hydrazine (5) is used then a salt such as sodium acetate is added to the mixture instead. The reaction mixtures are typically heated at a suitable temperature such as reflux temperature.

Step (iii) typically comprises a standard procedure for conversion of a carboxylic ester to an acid, such as use of an appropriate hydroxide salt (e.g. lithium hydroxide) in an appropriate solvent such as a mixture of tetrahydrofuran and water at a suitable temperature such as 50°C. 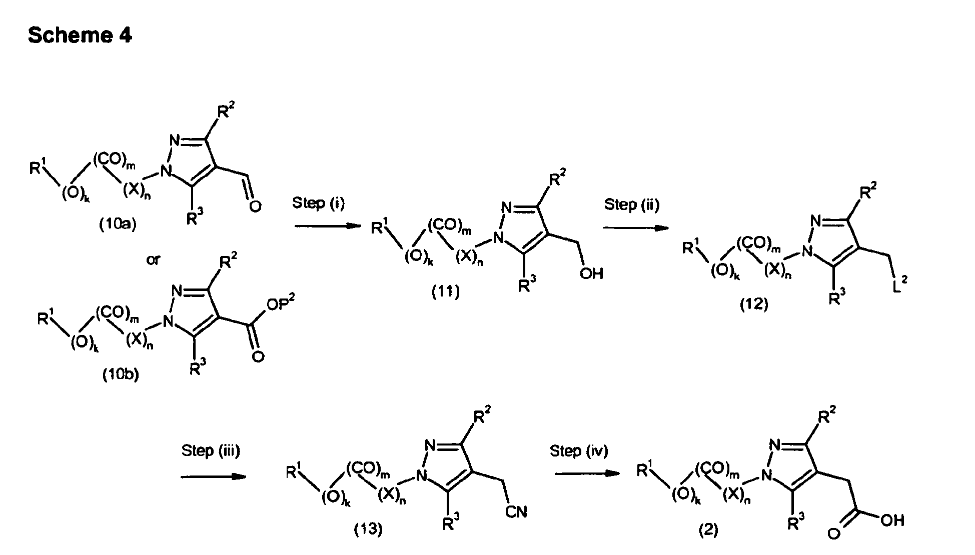 wherein R¹, R², R³, X, k, m and n are as defined above and L² represents a suitable leaving group such as a halogen atom (e.g. iodine, chlorine or bromine) and P² represents a suitable protecting group such as C₁₋₆ alkyl.

An analogous process has previously been described (see J. Heterocyclic Chem., 30, 997 (1993)).

Step (i) typically comprises the use of a suitable solvent such as ethanol (10a) or tetrahydrofuran (10b) and a suitable reducing agent such as sodium borohydride (10a) or lithium aluminium hydride (10b) at a suitable temperature e.g. between 0°C and room temperature.

Step (ii) typically comprises conversion of the hydroxyl group to a leaving group using a suitable reagent such as thionyl chloride (to prepare the chloro adduct for example) in a suitable solvent such as toluene at a suitable temperature such as reflux temperature.

Step (iii) typically comprises treatment with sodium cyanide in a suitable solvent such as dimethyl sulphoxide (DMSO) at a suitable temperature such as 70°C.

Step (iv) typically comprises a standard procedure for conversion of a nitrile to an acid, such as use of an appropriate hydroxide salt (e.g. potassium hydroxide) in an appropriate solvent such as ethanol at a suitable temperature such as reflux temperature. 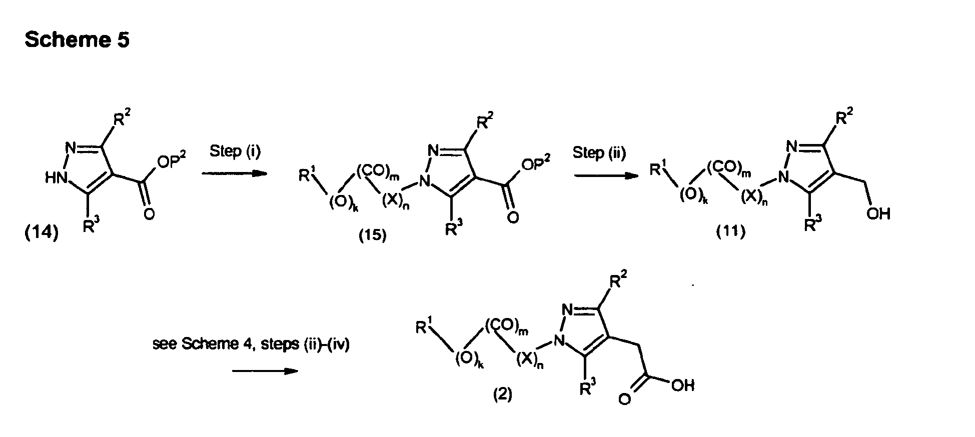 wherein R¹ is an optionally substituted C6-10 aryl or 5 to 10 membered heteroaryl group and R², R³, X, are as defined above, k=0, m=0 and n=0 and P² represents a suitable protecting group such as C₁₋₆alkyl.

Step (i) typically comprises treatment with an appropriate optionally substituted aryl or heteroaryl halide (e.g. bromide) and a suitable catalyst such as copper iodide and potassium phosphate (K3PO4) and N,N'-dimethylethylenediamine and a suitable solvent such as N-methylpyrrolidin-2-one at a suitable temperature such as 120°C in a microwave reactor. (See J.C.Antilla, J.M. Baskin, T.E. Barder, and S.L. Buchwald, J. Org. Chem., 2004, 69, 5578-5587).

Step (ii) typically comprises the use of a suitable solvent such as tetrahydrofuran and a suitable reducing agent such as lithium aluminium hydride at a suitable temperature e.g. between 0°C and room temperature.

Compounds of formula (11) can be converted to compounds of formula (2) by the processes outlined above in Scheme 4. 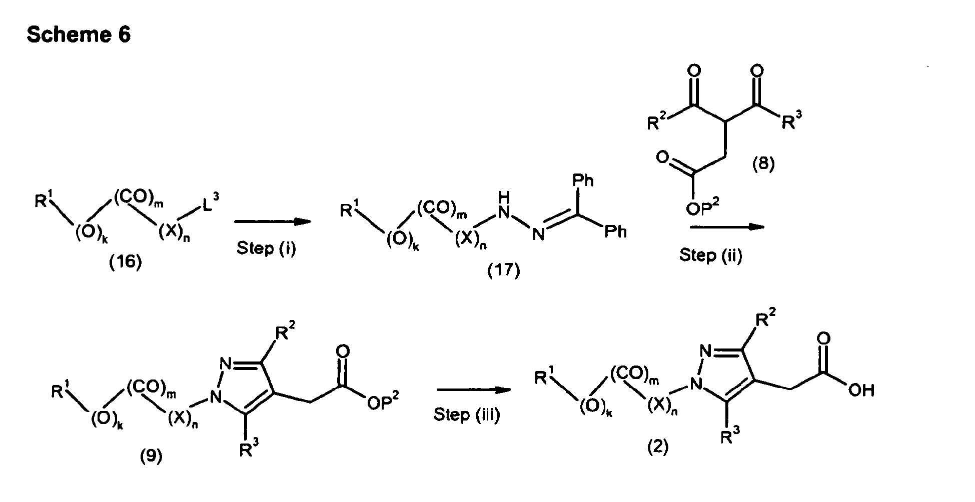 wherein R¹ is an optionally substituted C6-10 aryl or 5 to 10 membered heteroaryl group and R², R³, X, are as defined above, k=0, m=0 and n=0 and P² represents a suitable protecting group such as C₁₋₆ alkyl, and L³ represents a suitable leaving group such as a halogen atom (e.g. iodine, chlorine or bromine).

Step (i) typically comprises treatment with benzophenone hydrazone and a suitable catalyst such as Xantphos and palladium (II) acetate and a base such as sodium *t-*butoxide in a suitable solvent such as toluene at a suitable temperature such as 80°C. (See S. Wagaw, B.H. Yang, S.L. Buchwald, J.Am.Chem.Soc. 1999, 121(44), 10251-10263.

Step (ii) typically comprises treatment with a compound of formula (8) (prepared as described above in Scheme 3) and p-toluenesulphonic acid monohydrate in a suitable solvent such as ethanol at a suitable temperature such as reflux temperature.

Step (iii) typically comprises a standard procedure for conversion of a carboxylic ester to an acid, such as use of an appropriate hydroxide salt (e.g. lithium hydroxide) in an appropriate solvent such as a mixture of tetrahydrofuran and water at a suitable temperature such as 50°C. 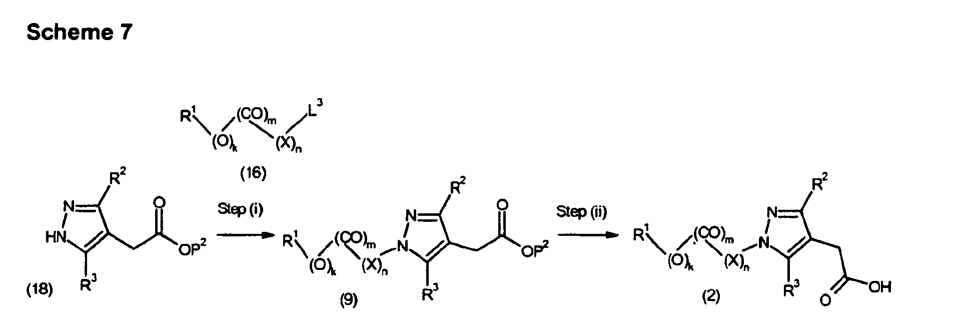 wherein R¹, R², R³, X, k, m and n are as defined above, P² represents a suitable protecting group such as C₁₋₆ alkyl and L³ represents a suitable leaving group such as a halogen atom (e.g. iodine, chlorine or bromine).

Step (i) typically comprises the use of a suitable base such as sodium hydride or cesium carbonate in a suitable solvent such as dimethylformamide at a suitable temperature e.g. between 0°C and room temperature.

Step (ii) typically comprises a standard procedure for conversion of a carboxylic ester to an acid, such as use of an appropriate hydroxide salt (e.g. lithium hydroxide) in an appropriate solvent such as a mixture of tetrahydrofuran and water at a suitable temperature such as 50°C. 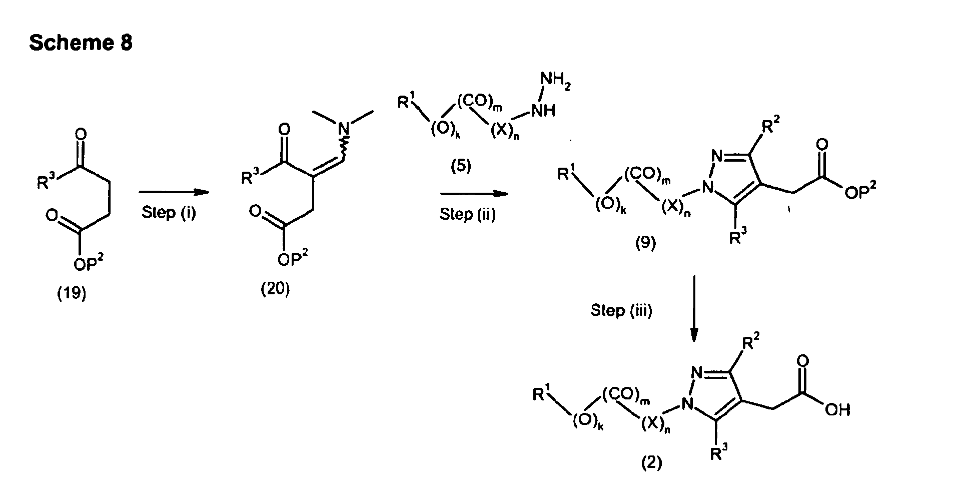 wherein R¹, R³, X, k, m and n are as defined above, R²=H and P² represents a suitable protecting group such as C₁₋₆ alkyl.

An analogous process has been described previously (US 4,146,721) Step (i) typically comprises treatment with dimethylformamide dimethyl acetal and acetic acid at a suitable temperature such as 120°C.

Step (ii) typically comprises the use of the hydrazine (5) or a salt (e.g. HCl) thereof in a suitable solvent such as ethanol. If the free base is used then a salt such as sodium acetate is also added to the mixture. Alternatively if the salt of hydrazine (5) is used then an acid such as acetic acid is added to the mixture. The reaction mixtures are typically heated at a suitable temperature such as reflux temperature.

Step (iii) typically comprises a standard procedure for conversion of a carboxylic ester to an acid, such as use of an appropriate hydroxide salt (e.g. sodium hydroxide) in an appropriate solvent such as methanol at a suitable temperature such as room temperature. 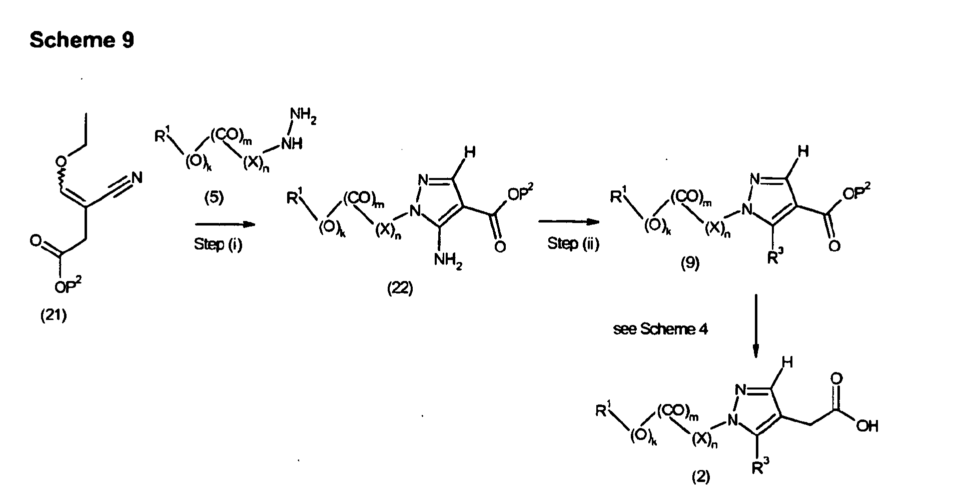 wherein R¹, X, k, m and n are as defined above, R³ = halogen and P² represents a suitable protecting group such as C₁₋₆ alkyl.

Step (i) typically comprises the use of the hydrazine (5) or a salt (e.g. HCl) thereof in a suitable solvent such as ethanol. If the hydrazine salt is used then a salt such as sodium acetate is also added to the mixture. The reaction mixtures are typically heated at a suitable temperature such as reflux temperature.

Step (ii) typically comprises a standard procedure for conversion of a heteroaryl amino group to a halogen, such as use of an appropriate diazotisation agent (e.g. isoamyl nitrite) in the presence of a halogen (e.g. bromine) in an appropriate solvent such as chloroform at a suitable temperature such as room temperature.

Compounds of the general formulae (3), (5), (6), (7), (10), (14), (16), (18), (19) and (21) are typically either available from commercial sources or can be prepared by a person skilled in the art using methods described in the chemical literature (or using analogous methods).

Pharmaceutically acceptable salts may for example be prepared conventionally by reaction (mixture) with the appropriate acid or acid derivative.

### CLINICAL INDICATIONS

It is believed that, as the compounds or pharmaceutically acceptable salts of the present invention modulate P2X7 receptor function and are capable of antagonizing the effects of ATP at the P2X7 receptor ("P2X7 receptor antagonists"), they may be useful in the treatment of pain, including acute pain, chronic pain, chronic articular pain, musculoskeletal pain, neuropathic pain, inflammatory pain, visceral pain, pain associated with cancer, pain associated with migraine, tension headache and cluster headaches, pain associated with functional bowel disorders, lower back and neck pain, pain associated with sprains and strains, sympathetically maintained pain; myositis, pain associated with influenza or other viral infections such as the common cold, pain associated with rheumatic fever, pain associated with myocardial ischemia, post operative pain, cancer chemotherapy, headache, toothache and dysmenorrhea.

Chronic articular pain conditions include rheumatoid arthritis, osteoarthritis, rheumatoid spondylitis, gouty arthritis and juvenile arthritis.

Pain associated with functional bowel disorders includes non-ulcer dyspepsia, non-cardiac chest pain and irritable bowel syndrome.

Neuropathic pain syndromes include: diabetic neuropathy, sciatica, non-specific lower back pain, trigeminal neuralgia, multiple sclerosis pain, fibromyalgia, HIV-related neuropathy, post-herpetic neuralgia, trigeminal neuralgia, and pain resulting from physical trauma, amputation, phantom limb syndrome, spinal surgery, cancer, toxins or chronic inflammatory conditions. In addition, neuropathic pain conditions include pain associated with normally non-painful sensations such as "pins and needles" (paraesthesias and dysesthesias), increased sensitivity to touch (hyperesthesia), painful sensation following innocuous stimulation (dynamic, static, thermal or cold allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

Other conditions which could potentially be treated by compounds or pharmaceutically acceptable salts of the present invention include fever, inflammation, immunological diseases, abnormal platelet function diseases (e.g. occlusive vascular diseases), impotence or erectile dysfunction; bone disease characterised by abnormal bone metabolism or resorbtion; hemodynamic side effects of non-steroidal anti-inflammatory drugs (NSAID's) and cyclooxygenase-2 (COX-2) inhibitors, cardiovascular diseases; neurodegenerative diseases and/or neurodegeneration, neurodegeneration following trauma, tinnitus, dependence on a dependence-inducing agent such as opiods (e.g. morphine), CNS depressants (e.g. ethanol), psychostimulants (e.g. cocaine) and nicotine; complications of Type I diabetes, kidney dysfunction, liver dysfunction (e.g. hepatitis, cirrhosis), gastrointestinal dysfunction (e.g. diarrhoea), colon cancer, overactive bladder and urge incontinence. Depression and alcoholism could potentially also be treated by compounds or pharmaceutically acceptable salts of the present invention.

Inflammatory conditions include skin conditions (e.g. sunburn, burns, eczema, dermatitis, allergic dermatitis, psoriasis), meningitis, ophthalmic diseases such as glaucoma, retinitis, retinopathies, uveitis and of acute injury to the eye tissue (e.g. conjunctivitis), inflammatory lung disorders (e.g. asthma, bronchitis, emphysema, allergic rhinitis, respiratory distress syndrome, pigeon fancier's disease, farmer's lung, chronic obstructive pulmonary disease (COPD), airways hyperresponsiveness); gastrointestinal tract disorders (e.g. aphthous ulcer, Crohn's disease, atopic gastritis, gastritis varialoforme, ulcerative colitis, coeliac disease, regional ileitis, irritable bowel syndrome, inflammatory bowel disease, gastrointestinal reflux disease); organ transplantation and other conditions with an inflammatory component such as vascular disease, migraine, periarteritis nodosa, thyroiditis, aplastic anaemia, Hodgkin's disease, sclerodoma, myaesthenia gravis, multiple sclerosis, sorcoidosis, nephrotic syndrome, Bechet's syndrome, gingivitis, myocardial ischemia, pyrexia, systemic lupus erythematosus, polymyositis, tendinitis, bursitis, and Sjogren's syndrome.

Immunological diseases include autoimmune diseases, immunological deficiency diseases or organ transplantation.

Bone diseases characterised by abnormal bone metabolism or resorbtion include osteoporosis (especially postmenopausal osteoporosis), hyper-calcemia, hyperparathyroidism, Paget's bone diseases, osteolysis, hypercalcemia of malignancy with or without bone metastases, rheumatoid arthritis, periodontitis, osteoarthritis, ostealgia, osteopenia, cancer cacchexia, calculosis, lithiasis (especially urolithiasis), solid carcinoma, gout and ankylosing spondylitis, tendinitis and bursitis. Cardiovascular diseases include hypertension or myocardiac ischemia; atherosclerosis; functional or organic venous insufficiency; varicose therapy; haemorrhoids; and shock states associated with a marked drop in arterial pressure (e.g. septic shock).

Neurodegenerative diseases include dementia, particularly degenerative dementia (including senile dementia, dementia with Lewy bodies, Alzheimer's disease, Pick's disease, Huntingdon's chorea, Parkinson's disease and Creutzfeldt-Jakob disease, Amyotrophic Lateral Sclerosis (ALS), motor neuron disease); vascular dementia (including multi-infarct dementia); as well as dementia associated with intracranial space occupying lesions; trauma; infections and related conditions (including HIV infection, meningitis and shingles); metabolism; toxins; anoxia and vitamin deficiency; and mild cognitive impairment associated with ageing, particularly Age Associated Memory Impairment.

The compounds of formula (I) or pharmaceutically acceptable salts may also be useful in the treatment of neuroprotection and in the treatment of neurodegeneration following trauma such as stroke, cardiac arrest, pulmonary bypass, traumatic brain injury, spinal cord injury or the like.

The compounds or pharmaceutically acceptable salts of the present invention may also be useful in the treatment of malignant cell growth and/or metastasis, and myoblastic leukaemia.

Complications of Type 1 diabetes include diabetic microangiopathy, diabetic retinopathy, diabetic nephropathy, macular degeneration, glaucoma, nephrotic syndrome, aplastic anaemia, uveitis, Kawasaki disease and sarcoidosis.

Kidney dysfunction includes nephritis, glomerulonephritis, particularly mesangial proliferative glomerulonephritis and nephritic syndrome.

It is to be understood that reference to treatment includes both treatment of established symptoms and prophylactic treatment, unless explicitly stated otherwise.

According to a further aspect of the invention, we therefore provide a compound of formula (I) or a pharmaceutically acceptable salt thereof for use in therapy and/or for use in human or veterinary medicine.

According to another aspect of the invention, we provide a compound of formula (I) or a pharmaceutically acceptable salt thereof for use in the treatment or prevention (e.g. treatment) of a condition which is mediated by P2X7 receptors, for example a condition or disease disclosed herein (in particular pain, inflammation or a neurodegenerative disease, more particularly pain such as inflammatory pain, neuropathic pain or visceral pain), e.g. in a mammal such as a human or rodent e.g. human or rat e.g. human.

According to a further aspect of the invention, we provide a method of treating a human or animal (e.g. rodent e.g. rat) subject, for example a human subject, suffering from a condition which is mediated by P2X7 receptors, for example a condition or disease disclosed herein (in particular pain, inflammation or a neurodegenerative disease, more particularly pain such as inflammatory pain, neuropathic pain or visceral pain), which comprises administering to said subject an effective amount of a compound of formula (I) or a pharmaceutical acceptable salt thereof.

According to a further aspect of the invention we provide a method of treating a human or animal (e.g. rodent e.g. rat) subject, for example a human subject, suffering from pain, inflammation, an immunological disease, a bone disease or a neurodegenerative disease (in particular pain, inflammation or a neurodegenerative disease, more particularly pain such as inflammatory pain, neuropathic pain or visceral pain), which method comprises administering to said subject an effective amount of a compound of formula (I) or a pharmaceutically acceptable salt thereof.

According to a yet further aspect of the invention we provide a method of treating a human or animal (e.g. rodent e.g. rat) subject, for example a human subject, suffering from inflammatory pain, neuropathic pain or visceral pain which method comprises administering to said subject an effective amount of a compound of formula (I) or a pharmaceutically acceptable salt thereof.

According to a further aspect of the invention we provide a method of treating a subject, for example a human subject, suffering from Alzheimer's disease which method comprises administering to said subject an effective amount of a compound of formula (I) or a pharmaceutically acceptable salt thereof.

According to another aspect of the invention, we provide the use of a compound of formula (I) or a pharmaceutically acceptable salt thereof for the manufacture of a medicament for the treatment or prevention (e.g. treatment) of a condition which is mediated by the action of P2X7 receptors, for example a condition or disease disclosed herein (in particular pain, inflammation or a neurodegenerative disease, more particularly pain such as inflammatory pain, neuropathic pain or visceral pain), e.g. in a mammal such as a human or rodent e.g. human or rat e.g. human.

According to another aspect of the invention we provide the use of a compound of formula (I) or a pharmaceutically acceptable salt thereof for the manufacture of a medicament for the treatment or prevention (e.g. treatment) of pain, inflammation, an immunological disease, a bone disease or a neurodegenerative disease (in particular pain, inflammation or a neurodegenerative disease, more particularly pain such as inflammatory pain, neuropathic pain or visceral pain), e.g. in a mammal such as a human or rodent e.g. human or rat e.g. human.

According to another aspect of the invention we provide the use of a compound of formula (I) or a pharmaceutically acceptable salt thereof for the manufacture of a medicament for the treatment or prevention (e.g. treatment) of inflammatory pain, neuropathic pain or visceral pain, e.g. in a mammal such as a human or rodent e.g. human or rat e.g. human.

In one aspect of the invention we provide the use of a compound of formula (I) or a pharmaceutically acceptable salt thereof for the manufacture of a medicament for the treatment or prevention (e.g. treatment) of Alzheimer's disease, e.g. in a mammal such as a human or rodent e.g. human or rat e.g. human.

In order to use a compound of formula (I) or a pharmaceutically acceptable salt thereof for the treatment of humans and/or other mammals, it is normally formulated in accordance with standard pharmaceutical practice as a pharmaceutical composition. Therefore in another aspect of the invention there is provided a pharmaceutical composition comprising a compound of formula (I), or a pharmaceutically acceptable salt thereof, adapted for use in human or veterinary medicine.

In order to use a compound of formula (I) or a pharmaceutically acceptable salt thereof in therapy, it will normally be formulated into a pharmaceutical composition in accordance with standard pharmaceutical practice. The present invention also provides a pharmaceutical composition, which comprises a compound of formula (I), or a pharmaceutically acceptable salt thereof, and optionally a pharmaceutically acceptable carrier or excipient.

The pharmaceutical composition may be for use in a method of treatment or in a use or in a treatment or prevention, as described herein.

A pharmaceutical composition of the invention, which may be prepared by admixture, for example at ambient temperature and atmospheric pressure, is usually adapted for oral, parenteral or rectal administration. As such, the pharmaceutical composition may be in the form of tablets, capsules, oral liquid preparations, powders, granules, lozenges, reconstitutable powders, injectable or infusable solutions or suspensions or suppositories. Orally administrable compositions are generally preferred.

Tablets and capsules for oral administration may be in unit dose form, and may contain excipients, such as binding agents, fillers, tabletting lubricants, disintegrants and/or acceptable wetting agents. The tablets may be coated, e.g. according to methods known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are for example prepared utilising a compound of the invention or pharmaceutically acceptable salt thereof and a sterile vehicle. In one particular embodiment, the compound or salt, depending on the vehicle and concentration used, is either suspended or dissolved in the vehicle. In preparing solutions, the compound or salt can be dissolved for injection and filter sterilised before filling into a suitable vial or ampoule and sealing. In one embodiment, adjuvant(s) such as a local anaesthetic, a preservative and/or a buffering agent is / are dissolved in the vehicle. To enhance the stability, the composition can for example be frozen after filling into the vial and the water removed under vacuum. Parenteral suspensions are typically prepared in substantially the same manner, except that the compound or salt is typically suspended in the vehicle instead of being dissolved, and sterilization is not usually readily accomplished by filtration. The compound or salt can be sterilised e.g. by exposure to ethylene oxide before suspension in a sterile vehicle. In a particular embodiment, a surfactant or wetting agent is included in the composition to facilitate uniform distribution of the compound or salt of the invention.

In one embodiment, the composition contains from 0.1% to 99% by weight, in particular from 10 to 60% by weight, of the active material (the compound or pharmaceutically acceptable salt of the invention), e.g. depending on the method of administration.

The dose of the compound or pharmaceutically acceptable salt thereof used in the treatment or prevention (e.g. treatment) of the aforementioned disorders / diseases / conditions may vary in the usual way with the seriousness of the disorders, the weight of the sufferer, and/or other similar factors. However, as a general guide, in one embodiment a suitable unit dose of 0.05 to 1000 mg, for example 0.05 to 200 mg, such as 20 to 40 mg, of the compound or pharmaceutically acceptable salt of the invention (measured as the compound), may be used. In one embodiment, such a unit dose is for administration once a day e.g. to a mammal such as a human; alternatively such a unit dose may be for administration more than once (e.g. twice) a day e.g. to a mammal such as a human. Such therapy may extend for a number of weeks or months.

### COMBINATIONS

Compounds of formula (I) or salts thereof may be used in combination with other therapeutic agents, for example medicaments which are or may be useful in the treatment of the above mentioned disorders.

Suitable examples of other such therapeutic agents may include a β2-agonist (also known as β2 adrenoceptor agonists; e.g. formoterol) and/or a corticosteroid (e.g. budesonide, fluticasone (e.g. as propionate or furoate esters), mometasone (e.g. as furoate), beclomethasone (e.g. as 17-propionate or 17,21-dipropionate esters), ciclesonide, triamcinolone (e.g. as acetonide), flunisolide, rofleponide and butixocort (e.g. as propionate ester), for the treatment of respiratory disorders (such as asthma and chronic obstructive pulmonary disease (COPD)) as described in WO 2007/008155 and WO 2007/008157.

A further therapeutic agent may include a 3-hydroxy-3-methylglutaryl coenzyme A (HMG CoA) reductase inhibitor (e.g. atorvastatin, fluvastatin, lovastatin, pravastatin, rosuvastatin, and simvastatin) for the treatment of cardiovascular disorders (such as atherosclerosis) as described in WO 2006/083214.

A further therapeutic agent may include a non-steroid anti-inflammatory drug (NSAID; e.g. ibuprofen, naproxen, aspirin, celecoxib, diclofenac, etodolac, fenoprofen, indomethacin, ketoprofen, ketoralac, oxaprozin, nabumetone, sulindac, tolmetin, rofecoxib, valdecoxib, lumaricoxib, meloxicam, etoricoxiband and parecoxib) for the treatment of an inflammatory disease or disorder (such as rheumatoid arthritis or osteoarthritis) as described in WO 2005/025571.

A further therapeutic agent may include a tumour necrosis factor α (TNFα) inhibitor (e.g. Etanercept or an anti- TNFα antibody such as Infliximab and Adalimumab) for the treatment of an inflammatory disease or disorder (such as rheumatoid arthritis or osteoarthritis) as described in WO 2004/105798.

A further therapeutic agent may include 2-hydroxy-5- [[4-[(2- pyridinylamino) sulfonyl] phenyl] azo] benzoic acid (sulfasalazine) for the treatment of an inflammatory disease or disorder (such as rheumatoid arthritis) as described in WO 2004/105797.

A further therapeutic agent may include N-[4-[[(2, 4-diamino-6-pteridinyl) methyl] methylamino] benzoyl]- L-glutamic acid (methotrexate) for the treatment of an inflammatory disease or disorder (such as rheumatoid arthritis) as described in WO 2004/105796.

A further therapeutic agent may include an inhibitor of pro TNFα convertase enzyme (TACE) for the treatment of an inflammatory disease or disorder (such as rheumatoid arthritis) as described in WO 2004/073704.

A further therapeutic agent may include:
a) sulfasalazine;
b) a statin, such as atorvastatin, lovastatin, pravastatin, simvastatin, fluvastatin, cerivastatin, crilvastatin, dalvastatin, rosuvastatin, tenivastatin, fluindostatin, velostatin, dalvastatin, nisvastatin, bervastatin, pitavastatin, rivastatin, glenvastatin, eptastatin, tenivastatin, flurastatin, rosuvastatin or itavastatin;
c) a glucocorticoid agent, such as dexamethasone, methylprednisolone, prednisolone, prednisone and hydrocortisone;
d) an inhibitor of p38 kinase;
e) an anti-IL-6-receptor antibody;
f) anakinra;
g) an anti-IL-1 monoclonal antibody;
h) an inhibitor of JAK3 protein tyrosine kinase;
i) an anti-macrophage colony stimulation factor (M-CSF) monoclonal antibody; or
j) an anti-CD20 monoclonal antibody, such as rituximab, PRO70769, HuMax-CD20 (Genmab AJS), AME-133 (Applied Molecular Evolution), or hA20 (Immunomedics, Inc.)
for the treatment of an IL-1 mediated disease (such as rheumatoid arthritis) as described in WO 2006/003517.

When the compounds are used in combination with other therapeutic agents, the compounds may be administered either sequentially or simultaneously by any convenient route.

The invention thus provides, in a further aspect, a combination comprising a compound of formula (I) or a pharmaceutically acceptable salt thereof together with a further therapeutic agent or agents, e.g. as described herein.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a pharmaceutically acceptable salt thereof is used in combination with a second therapeutic agent active against the same disease state the dose of each compound may differ from that when the compound is used alone.

The following Descriptions and Examples illustrate the preparation of compounds of the invention but are not intended to be limiting.

### EXAMPLES

The general methods (a)-(d), along with the synthetic methods outlined in Schemes 1-9 above, for the preparation of compounds of the present invention are further illustrated by the following examples.

### Example 1 N-[(3,4-dichlorophenyl)methyl]-2-(3,5-dimethyl-1-phenyl-1H-pyrazol-4-yl)acetamide (E1)

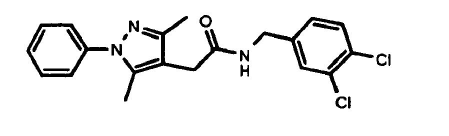

Pol-carbodiimide (-0.06 mmol) was added to a Robbins block using a 3.8 resin loader. To this was added a solution of HOAt (0.01 mmol) in a 1:1 mixture of tetrahydrofuran and dichloromethane (0.8 ml) followed by (3,5-dimethyl-1-phenyl-1*H-*pyrazol-4-yl)acetic acid (0.03 mmol, prepared as described in US 4,146,721 or purchased from commercial sources) in dichloromethane (0.25 ml). Lastly [(3,4-dichlorophenyl)methyl]amine (0.04 mmol) in dichloromethane (0.25 ml) was added and the Robbins block was sealed and shaken for 36 hours. Pol-isocyanate and trisamine resins were added to the mixture which was then left to shake overnight. The mixture was loaded onto an SCX column and washed twice with dichloromethane. Eluting with 0.5M ammonia in methanol and evaporation of the solvent gave the crude product. This was then loaded onto a 96-well silica block which had been pre-washed with methanol and eluted with a 1:1 dichloromethane:methanol mixture. Evaporation of the solvent and oven drying gave the title compound as product (3.45 mg, 0.009 mmol). LC/MS [M+H]⁺ = 388.00, retention time = 2.78 minutes.

### Examples 2-11

In a manner analogous to that described for Example 1 above the compounds tabulated below (Table 1) were prepared using the appropriate amines, all of which are available from commercial sources or can be prepared using routes described previously in the chemical literature, instead of the [(3,4-dichlorophenyl)methyl] amine used in the above procedure.

**Table 1**

| Example no. | Chemical name | [M+H]⁺ | Retention time (mins) |
|---|---|---|---|
| E2 | 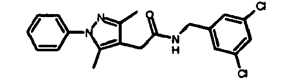 | 388.01 | 2.26 |
| | *N-*[(3,5-dichlorophenyl)methyl]-2-(3,5-dimethyl-1-phenyl-1*H*-pyrazo)-4-y)acetamide | | |
| E3 | 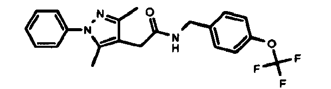 | 404.04 | 2.20 |
| | 2-(3,5-dimethyl-1-phenyl-1*H-*pyrazol-4-yl)-*N*-({4-[(trifluoromethyl)oxy]phenyl}methyl)acetamide | | |
| E4 | 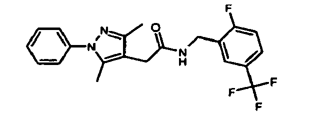 | 406.15 | 2.27 |
| | 2-(3,5-dimethyl-1-phenyl-1*H*-pyrazol-4-yl)-*N*-{[2-fluoro-5-(trifluoromethyl)phenyl]methyl}acetamide | | |
| E5 | 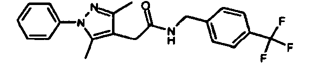 | 388.05 | 2.29 |
| | 2-(3,5-dimethy)-1-pheny)-1*H-*pyrazol-4-yl)-*N-*{[4-(trifluoromethyl)phenyl]methyl}acetamide | | |
| E6 | 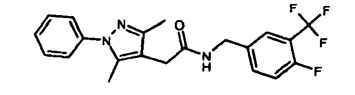 | 406.05 | 2.20 |
| | 2-(3,5-dimethyl-1-phenyl-1*H-*pyrazol-4-yl)-*N-*{[4-fluoro-3-(trifluoromethyl)phenyl]methyl}acetamide | | |
| E7 | 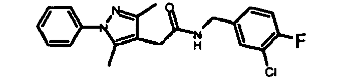 | 372.03 | 2.17 |
| | *N-*[(3-chloro-4-fluorophenyl)methyl]-2-(3,5-dimethyl-1-phenyl-1*H-*pyrazol-4-yl)acetamide | | |
| E8 | 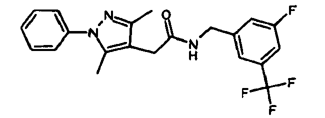 | 406.2 | 2.31 |
| | 2-(3,5-dimethyl-1-pheny)-1*H-*pyrazol-4-y)-*N-*{[3-fluoro-5-(trifluoromethyl)phenyl]methyl}acetamide | | |
| E9 | 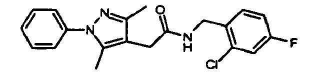 | 372.05 | 2.81 |
| | *N-*[(2-chloro-4-fluorophenyl)methyl]-2-(3,5-dimethyl-1-phenyl-1*H-*pyrazol-4-yl)acetamide | | |
| E10 | 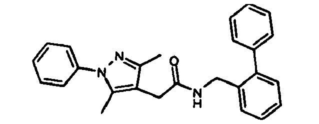 | 396.10 | 2.27 |
| | *N-*(2-biphenylylmethyl)-2-(3,5-dimethyl-1 -phenyl-1*H-*pyrazol-4-yl)acetamide | | |
| E11 | 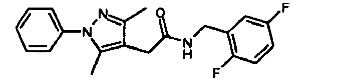 | 356.06 | 2.06 |
| | *N-*[(2,5-difluorophenyl)methyl]-2-(3,5-dimethyl-1-phenyl-1*H-*pyrazol-4-yl)acetamide | | |

### Example 12 N-[(3,4-difluorophenyl)methyl]-2-(3,5-dimethyl-1-phenyl-1H-pyrazol-4-yl)acetamide (E12)

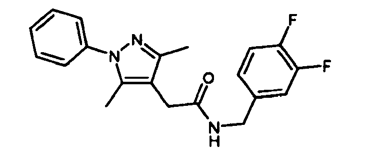

(3,5-dimethyl-1-phenyl-1*H-*pyrazol-4-yl)acetic acid (0.100 g, 0.43 mmol, prepared as described in US 4,146,721 or purchased from commercial sources) was added to a test-tube and to this was added water soluble carbodiimide (0.200 g, 1.04 mmol), 1-hydroxybenzotriazole (0.141 g, 1.04 mmol), N-ethyl morpholine (0.332 ml, 2.61 mmol), [(3,4-difluorophenyl)methyl]amine (0.149 g, 1.04 mmol), and dichloromethane (10 ml). The mixture was stirred at room temperature for 3-4 hrs and then washed sequentially with 2M aqueous hydrogen chloride and saturated aqueous sodium hydrogen carbonate, then filtered through a phase separator and the organic phase was then evaporated to give the crude product. The crude material was purified by mass-directed automated HPLC to give the title compound as product (0.052 g). LC/MS [M+H]⁺ = 356, retention time = 2.74 minutes.

### Examples 13-47

In a manner analogous to that described for Example 12 above the compounds tabulated below (Table 2) were prepared by substituting the appropriate amines, all of which are available from commercial sources or can be prepared using routes described previously in the chemical literature, for the [(3,4-difluorophenyl)methyl] amine used in the above procedure.

**Table 2**

| Example no. | Chemical name | [M+H]⁺ | Retention time (mins) |
|---|---|---|---|
| E13 | 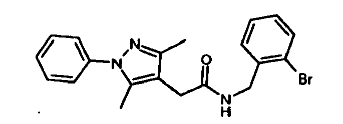 | 400.2 | 2.80 |
| | *N-*[(2-bromophenyl)methyl]-2-(3,5-dimethyl-1-phenyl-1*H-*pyrazol-4-yl)acetamide | | |
| E14 | 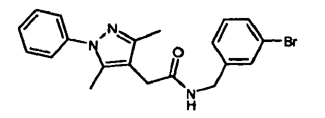 | 400.2 | 2.84 |
| | *N-*[(3-bromophenyl)methyl]-2-(3,5-dimethyl-1-phenyl-1*H*-pyrazol-4-yl)acetamide | | |
| E15 | 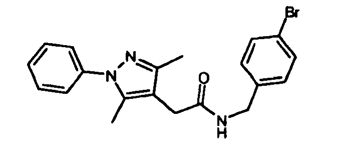 | 400.2 | 2.85 |
| | *N-*[(4-bromophenyl)methyl]-2-(3,5-dimethyl-1-phenyl-1*H-*pyrazol-4-yl)acetamide | | |
| E16 | 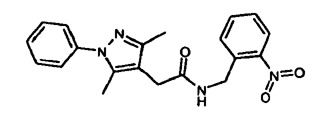 | 365.3 | 2.61 |
| | 2-(3,5-dimethyl-1-phenyl-1*H*-pyrazol-4-yl)-*N-*[(2-nitrophenyl)methyl]acetamide | | |
| E17 | 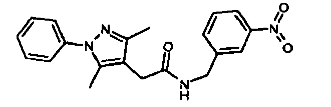 | 365.3 | 2.62 |
| | 2-(3,5-dimethyl-1-phenyl-1*H-*pyrazol-4-yl)-*N-*[(3-nitrophenyl)methyl]acetamide | | |
| E18 | 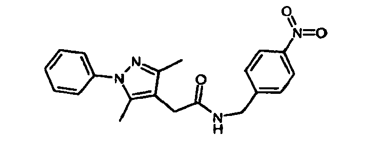 | 365.3 | 2.62 |
| | 2-(3,5-dimethyl-1-phenyl-1*H-*pyrazol-4-yl)-*N-*[(4-nitrophenyl)methyl]acetamide | | |
| E19 | 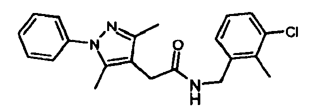 | 368 | 2.95 |
| | *N-*[(3-chloro-2-methylphenyl)methyl]-2-(3,5-dimethyl-1-phenyl-1*H*-pyrazol-4-yl)acetamide | | |
| E20 | 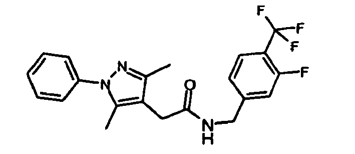 | 406 | 2.99 |
| | 2-(3,5-dimethyl-1-phenyl-1*H-*pyrazol-4-yl)-*N-*[3-fluoro-4-(trifluoromethyl)phenyl]methyl}acetamide | | |
| E21 | 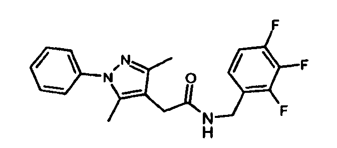 | 374 | 2.81 |
| | 2-(3,5-dimethyl-1-phenyl-1*H-*pyrazol-4-yl)-*N-*[(2,3,4-trifluorophenyl)methyl]acetamide | | |
| E22 | 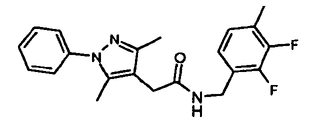 | 370 | 2.88 |
| | *N-*[(2,3-difluoro-4-methylphenyl)methyl]-2-(3,5-dimethyl-1-phenyl-1*H-*pyrazol-4-yl)acetamide | | |
| E23 | 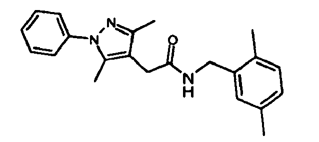 | 348 | 2.89 |
| | *N-*[(2,5-dimethyl)phenyl)methyl]-2-(3,5-dimethyl-1-phenyl-1*H-*pyrazol-4-yl)acetamide | | |
| E24 | 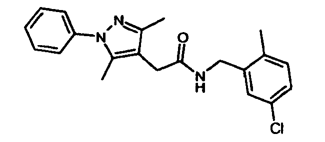 | 368 | 2.95 |
| | *N-*[(5-chloro-2-methylphenyl)methyl]-2-(3,5-dimethyl-1-phenyl-1*H*-pyrazol-4-yl)acetamide | | |
| E25 | 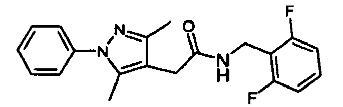 | 356 | 2.64 |
| | *N-*[(2,6-difluorophenyl)methyl]-2-(3,5-dimethyl-1-phenyl-1*H*-pyrazol-4-yl)acetamide | | |
| E26 | 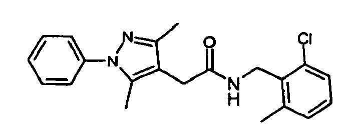 | 368 | 2.88 |
| | *N-*[(2-chloro-6-methylphenyl)methyl]-2-(3,5-dimethyl-1-phenyl-1*H-*pyrazol-4-yl)acetamide | | |
| E27 | 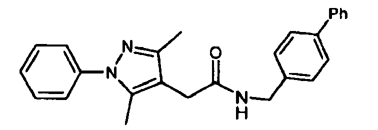 | 396 | 3.08 |
| | *N-*(4-biphenylylmethyl)-2-(3,5-dimethyl-1-phenyl-1*H-*pyrazol-4-yl)acetamide | | |
| E28 | 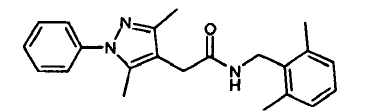 | 348 | 2.81 |
| | *N-*[(2,6-dimethylphenyl)methyl]-2-(3,5-dimethyl-1-phenyl-1*H-*pyrazol-4-yl)acetamide | | |
| E29 | 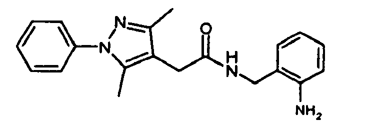 | 335.27 | 2.03 |
| | *N-*[(2-aminophenyl)methyl]-2-(3,5-dimethyl-1-phenyl-1*H-*pyrazol-4-yl)acetamide | | |
| E30 | 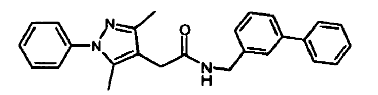 | 396.29 | 3.07 |
| | *N-*(3-biphenylylmethyl)-2-(3,5-dimethyl-1-phenyl-1*H-*pyrazol-4-yl)acetamide | | |
| E31 | 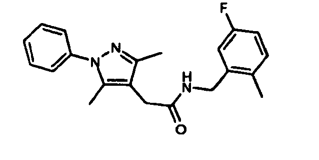 | 352.1 | 2.77 |
| | 2-(3,5-dimethyl-1-phenyl-1*H-*pyrazol-4-yl)-*N-*[(5-fluoro-2-methylphenyl)methyl]acetamide | | |
| E32 | 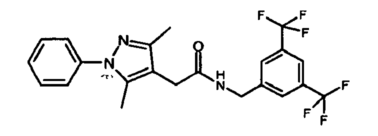 | 456.24 | 3.17 |
| | *N-*{[3,5-bis(trifluoromethyl)phenyl]methyl}-2-(3,5-dimethyl-1-phenyl-1*H-*pyrazol-4-yl)acetamide | | |
| E33 | 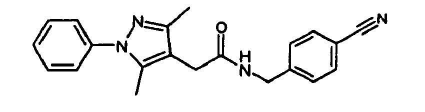 | 345.28 | 2.48 |
| | *N-*[(4-cyanophenyl)methyl]-2-(3,5-dimethyl-1-phenyl-1*H-*pyrazol-4-yl)acetamide | | |
| E34 | 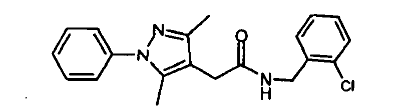 | 354.21 | 2.75 |
| | *N-*[(2-chlorophenyl)methyl]-2-(3,5-dimethyl-1-phenyl-1*H-*pyrazol-4-yl)acetamide | | |
| E35 | 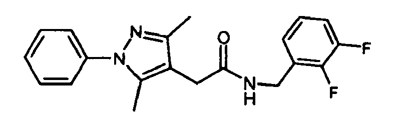 | 356.25 | 2.68 |
| | *N-*[(2,3-difluorophenyl)methyl]-2-(3,5-dimethyl-1-phenyl-1*H*-pyrazol-4-yl)acetamide | | |
| E36 | 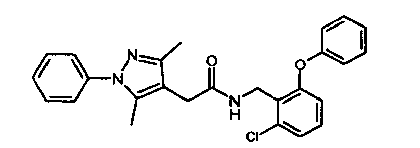 | 446.25 | 3.20 |
| | *N-*{[2-chloro-6-(phenyloxy)phenyl]methyl}-2-(3,5-dimethyl-1-phenyl-1*H-*pyrazol-4-yl)acetamide | | |
| E37 | 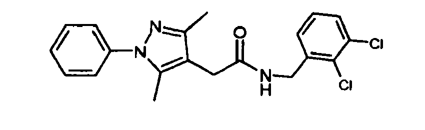 | 388.2 | 2.93 |
| | *N-*[(2,3-dichlorophenyl)methyl]-2-(3,5-dimethyl-1-phenyl-1*H-*pyrazol-4-yl)acetamide | | |
| E38 | 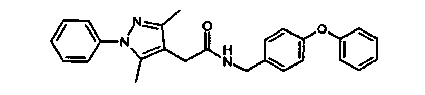 | 412.3 | 3.08 |
| | 2-(3,5-dimethyl-1-phenyl-1*H-*pyrazol-4-yl)-*N-*{[4-(phenyloxy)phenyl]methyl}acetamide | | |
| E39 | 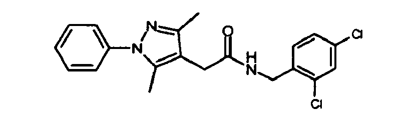 | 388.2 | 2.98 |
| | *N-*[(2,4-dichlorophenyl)methyl]-2-(3,5-dimethyl-1-phenyl-1*H-*pyrazol-4-yl)acetamide | | |
| E40 | 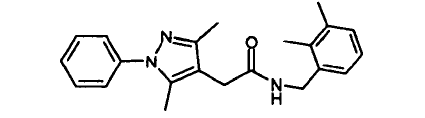 | 348.3 | 2.83 |
| | *N-*[(2,3-dimethylphenyl)methyl]-2-(3,5-dimethyl-1-phenyl-1*H-*pyrazol-4-yl)acetamide | | |
| E41 | 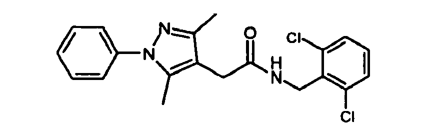 | 388.2 | 2.84 |
| | *N-*[(2,6-dichlorophenyl)methyl]-2-(3,5-dimethyl-1-phenyl-1*H-*pyrazol-4-yl)acetamide | | |
| E42 | 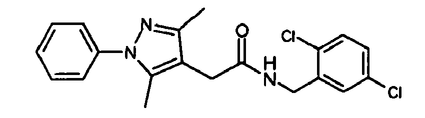 | 388.2 | 2.95 |
| | *N-*[(2,5-dichlorophenyl)methyl]-2-(3,5-dimethyl-1-phenyl-1*H-*pyrazol-4-yl)acetamide | | |
| E43 | 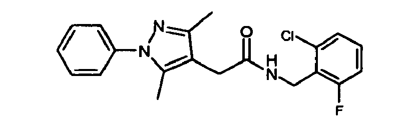 | 372.22 | 2.73 |
| | *N-*[(2-chloro-6-fluorophenyl)methyl]-2-(3,5-dimethyl-1-phenyl-1*H-*pyrazol-4-yl)acetamide | | |
| E44 | 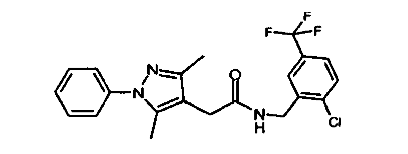 | 422.18 | 3.05 |
| | *N*-{[2-chloro-5-(trifluoromethyl)phenyl]methyl}-2-(3,5-dimethyl-1-phenyl-1*H-*pyrazol-4-yl)acetamide | | |
| E45 | 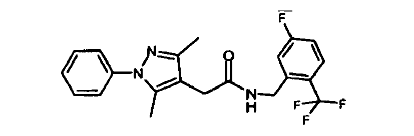 | 406.21 | 2.94 |
| | 2-(3,5-dimethyl-1-phenyl-1*H-*pyrazol-4-yl)-*N-*([5-fluoro-2-(trifluoromethyl)phenyl]methyl}acetamide | | |
| E46 | 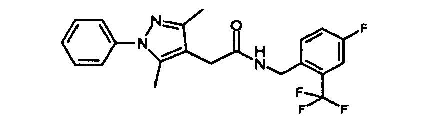 | 406.2 | 2.93 |
| | 2-(3,5-dimethyl-1-phenyl-1*H-*pyrazol-4-yl)-*N-*{[4-fluoro-2-(trifluoromethyl)phenyl]methyl}acetamide | | |
| E47 | 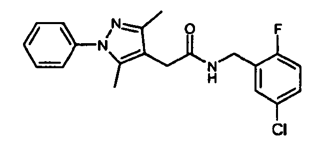 | 380 | 2.66 |
| | *N-*[(5-chloro-2-fluorophenyl)methyl]-2-(3,5-dimethyl-1-phenyl-1*H-*pyrazol-4-yl)acetamide | | |

### Example 48 N-[(2-chloro-4-fluorophenyl)methyl]-2-[1-(4-fluorophenyl)-3,5-dimethyl-1H-pyrazol-4-yl]acetamide (E48)

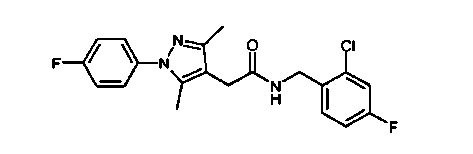

[1-(4-fluorophenyl)-3,5-dimethyl-1*H-*pyrazol-4-yl]acetic acid (0.600 g, 0.75 mmol) was dissolved in dimethylformamide (2 ml) and to this was added water soluble carbodiimide (0.187 g, 0.98 mmol), 1-hydroxybenzotriazole (0.132 g, 0.98 mmol), N-ethyl morpholine (0.286 ml, 2.25 mmol), and dichloromethane (2 ml). The mixture was stirred at room temperature for 10 minutes and then [(2-chloro-4-fluorophenyl)methyl]amine (0.120 g, 0.75 mmol) was added and the mixture was left to stir overnight at room temperature under an atmosphere of argon. The mixture was evaporated and then partitioned between dichloromethane and saturated aqueous sodium hydrogen carbonate. The mixture was stirred for 10 minutes then filtered through a phase separator and the organic phase was evaporated to give the crude product. The crude material was purified by mass-directed automated HPLC to give the title compound as product as an off-white solid (0.061 g). LC/MS [M+H]⁺= 390, retention time = 2.87 minutes.

[1-(4-Fluorophenyl)-3,5-dimethyl-1*H-*pyrazol-4-yl]acetic acid was obtained by hydrolysis of the corresponding ester (ethyl [1-(4-fluorophenyl)-3,5-dimethyl-1*H-*pyrazol-4-yl]acetate). Ethyl [1-(4-fluorophenyl)-3,5-dimethyl-1*H-*pyrazol-4-yl]acetate was prepared by the reaction of ethyl 3-acetyl-4-oxopentanoate with (4-fluorophenyl)hydrazine. Appropriate methodologies to prepare these or analgous compounds have been described previously (e.g. US 4, 146, 721).

### Examples 49-81

In a manner analogous to that described for Example 48 above the compounds tabulated below (Table 3) were prepared by substituting the appropriate hydrazines (or a salt thereof e.g. HCl salt), all of which are available from commercial sources or can be prepared using routes described previously in the chemical literature, for the (4-fluorophenyl)hydrazine used in the above procedure (synthesis of ester intermediate).

**Table 3**

| Example no. | Chemical name | [M+H]⁺ | Retention time (mins) |
|---|---|---|---|
| E49 | 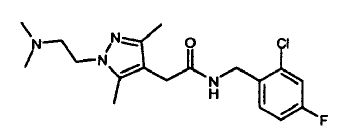 | 367 | 1.62 |
| | *N-*[(2-chloro-4-fluorophenyl)methyl)-2-{1-[2-(dimethylamino)ethyl]-3,5-dimethyl-1*H-*pyrazol-4-yl}acetamide | | |
| E50 | 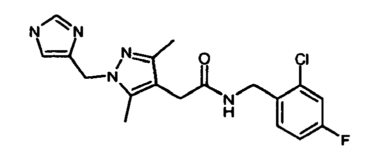 | 376 | 1.62 |
| | *N-*[(2-chloro-4-fluorophenyl)methyl]-2-[1-(1*H-*imidazol-4-ylmethyl)-3,5-dimethyl-1*H-*pyrazol-4-yl]acetamide | | |
| E51 | 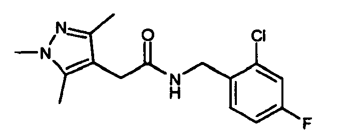 | 310 | 2.14 |
| | *N-*[(2-chloro-4-fluorophenyl)methyl]-2-(1,3,5-trimethyl-1*H-*pyrazol-4-yl)acetamide | | |
| E52 | 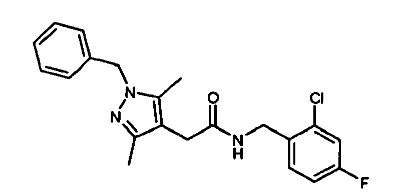 | 386 | 3.08 |
| | *N-*[(2-chloro-4-fluorophenyl)methyl]-2-[3,5-dimethyl-1-(phenylmethyl)-1*H-*pyrazol-4-yl]acetamide | | |
| E53 | 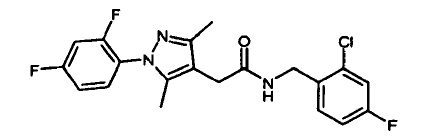 | 408 | 2.82 |
| | *N-*[(2-chloro-4-fluorophenyl)methyl]-2-[1-(2,4-difluorophenyl)-3,5-dimethyl-1*H-*pyrazol-4-yl]acetamide | | |
| E54 | 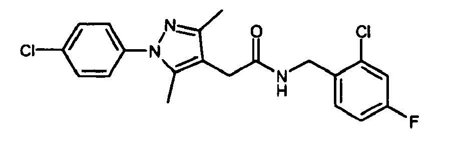 | 406 | 3.06 |
| | *N-*[(2-chloro-4-fluorophenyl)methyl]-2-[1-(4-chlorophenyl)-3,5-dimethyl-1*H-*pyrazol-4-yl]acetamide | | |
| E55 | 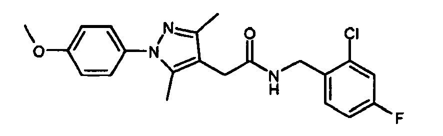 | 402 | 2.82 |
| | *N-*[(2-chloro-4-fluorophenyl)methyl]-2-{3,5-dimethyl-1-[4-(methyloxy)phenyl]-1*H-*pyrazol-4-yl}acetamide | | |
| E56 | 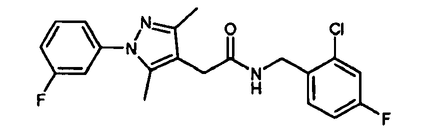 | 390 | 2.92 |
| | *N-*[(2-chloro-4-fluorophenyl)methyl]-2-[1-(3-fluorophenyl)-3,5-dimethyl-1*H-*pyrazol-4-yl]acetamide | | |
| E57 | 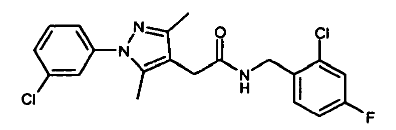 | 406 | 3.08 |
| | *N-*[(2-chloro-4-fluorophenyl)methyl]-2-[1-(3-chlorophenyl)-3,5-dimethyl-1*H-*pyrazol-4-yl]acetamide | | |
| E58 | 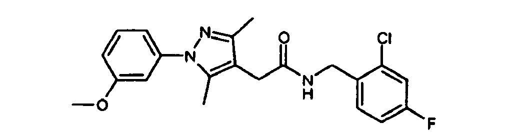 | 402 | 2.87 |
| | *N*-[(2-chloro-4-fluorophenyl)methyl]-2-{3,5-dimethyl-1-[3-(methyloxy)phenyl]-1*H-*pyrazol-4-yl}acetamide | | |
| E59 | 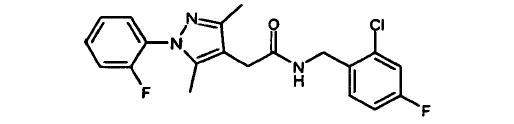 | 390 | 2.81 |
| | *N-*[(2-chloro-4-fluorophenyl)methyl]-2-[1-(2-fluorophenyl)-3,5-dimethyl-1*H-*pyrazol-4-yl]acetamide | | |
| E60 | 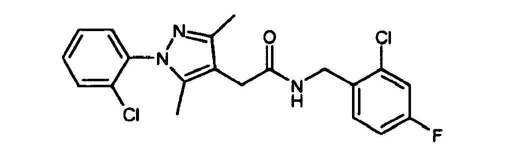 | 406 | 2.89 |
| | *N-*[(2-chloro-4-fluorophenyl)methyl]-2-[1-(2-chlorophenyl)-3,5-dimethyl-1*H-*pyrazol-4-yl]acetamide | | |
| E61 | 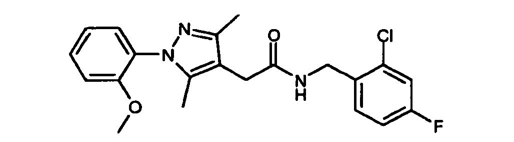 | 402 | 2.74 |
| | *N-*[(2-chloro-4-fluorophenyl)methyl]-2-{3,5-dimethyl-1-[2-(methyloxy)phenyl]-1*H-*pyrazol-4-yl}acetamide | | |
| E62 | 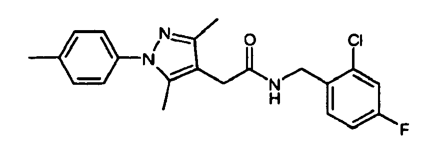 | 386 | 2.98 |
| | *N-*[(2-chloro-4-fluorophenyl)methyl]-2-[3,5-dimethyl-1-(4-methylphenyl)-1*H-*pyrazol-4-yl]acetamide | | |
| E63 | 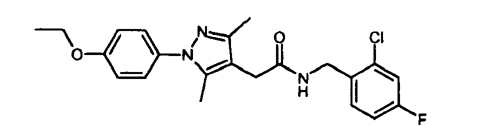 | 416 | 2.99 |
| | *N-*[(2-chloro-4-fluorophenyl)methyl]-2-{1-[4-(ethyloxy)phenyl]-3,5-dimethyl-1*H-*pyrazol-4-yl}acetamide | | |
| E64 | 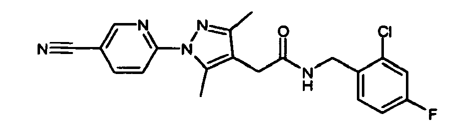 | 399 | 3.00 |
| | *N-*[(2-chloro-4-fluorophenyl)methyl]-2-[1-(5-cyano-2-pyridinyl)-3,5-dimethyl-1*H*-pyrazol-4-yl]acetamide | | |
| E65 | 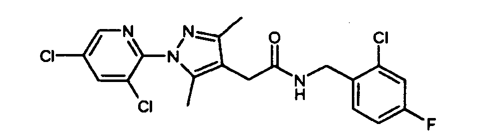 | 442 | 2.97 |
| | *N-*[(2-chloro-4-fluorophenyl)methyl]-2-[1-(3,5-dichloro-2-pyridinyl)-3,5-dimethyl-1*H-*pyrazol-4-yl]acetamide | | |
| E66 | 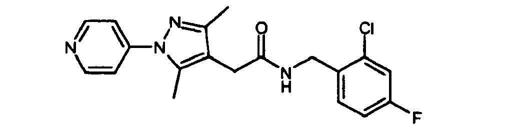 | 374 | 1.93 |
| | *N-*[(2-chloro-4-fluorophenyl)methyl]-2-[3,5-dimethyl-1-(4-pyridinyl)-1*H-*pyrazol-4-yl]acetamide | | |
| E67 | 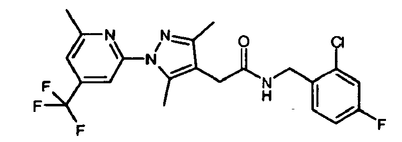 | 456 | 3.54 |
| | *N-*[(2-chloro-4-fluorophenyl)methyl]-2-{3,5-dimethyl-1-[6-methyl-4-(trifluoromethyl)-2-pyridinyl]-1*H-*pyrazol-4-yl}acetamide | | |
| E68 | 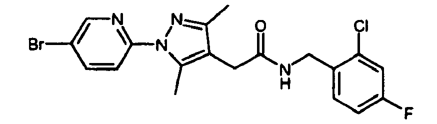 | 451 | 3.25 |
| | 2-[1-(5-bromo-2-pyridinyl)-3,5-dimethyl-1*H-*pyrazol-4-yl]-*N-*[(2-chloro-4-fluorophenyl)methyl]acetamide | | |
| E69 | 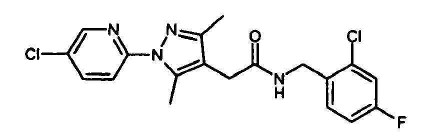 | 407 | 3.01 |
| | *N-*[(2-chloro-4-fluorophenyl)methyl]-2-[1-(5-chloro-2-pyridinyl)-3,5-dimethyl-1*H-*pyrazol-4-yl]acetamide | | |
| E70 | 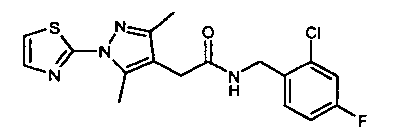 | 379 | 2.80 |
| | *N-*[(2-chloro-4-fluorophenyl)methyl]-2-[3,5-dimethyl-1-(1,3-thiazol-2-yl)-1*H-*pyrazol-4-yl]acetamide | | |
| E71 | 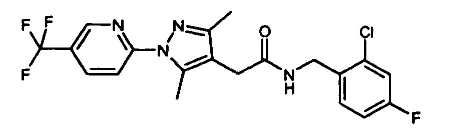 | 441 | 3.21 |
| | *N-*[(2-chloro-4-fluorophenyl)methyl]-2-(3,5-dimethyl-1-[5-(trifluoromethyl)-2-pyridinyl]-1*H-* pyrazol-4-yl}acetamide | | |
| E72 | 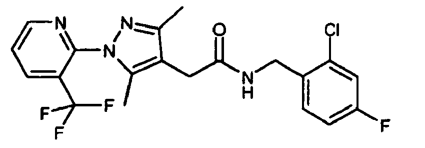 | 442 | 2.82 |
| | *N-*[(2-chloro-4-fluorophenyl)methyl]-2-{3,5-dimethyl-1-[3-(trifluoromethyl)-2-pyridinyl]-1*H*-pyrazol-4-yl}acetamide | | |
| E73 | 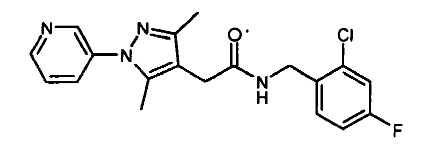 | 374 | 2.34 |
| | *N-*[(2-chloro-4-fluorophenyl)methyl]-2-[3,5-dimethyl-1-(3-pyridinyl)-1*H-*pyrazol-4-yl]acetamide | | |
| E74 | 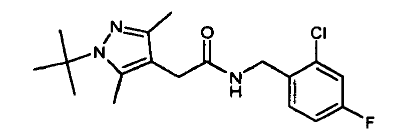 | 352 | 2.66 |
| | *N-*[(2-chloro-4-fluorophenyl)methyl]-2-[1-(1,1-dimethylethyl)-3,5-dimethyl-1*H-*pyrazol-4-yl]acetamide | | |
| E75 | 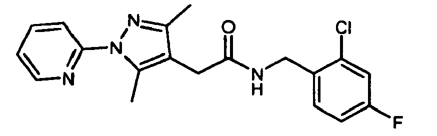 | 373.2 | 2.68 |
| | *N-*[(2-chloro-4-fluorophenyl)methyl]-2-[3,5-dimethyl-1-(2-pyridinyl)-1*H-*pyrazol-4-yqacetamide | | |
| E76 | 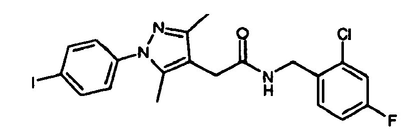 | 498.0 | 3.22 |
| | *N-*[(2-chloro-4-fluorophenyl)methyl]-2-[1-(4-iodophenyl)-3,5-dimethyl-1*H-*pyrazol-4-yl]acetamide | | |
| E77 | 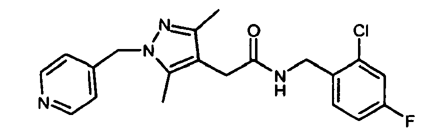 | 387 | 1.71 |
| | *N-*[(2-chloro-4-fluorophenyl)methyl]-2-[3,5-dimethyl-1-(4-pyridinylmethyl)-1*H-*pyrazol-4-yl]acetamide | | |
| E78 | 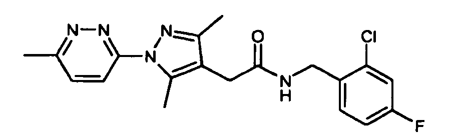 | 388 | 2.48 |
| | *N-*[(2-chloro-4-fluorophenyl)methyl]-2-[3,5-dimethyl-1-(6-methyl-3-pyridazinyl)-1*H-*pyrazol-4-yl]acetamide | | |
| E79 | 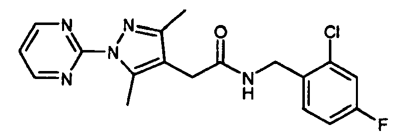 | 374 | 2.34 |
| | *N-*[(2-chloro-4-fluorophenyl)methyl]-2-[3,5-dimethyl-1-(2-pyrimidinyl)-1*H-*pyrazol-4-yl]acetamide | | |
| E80 | 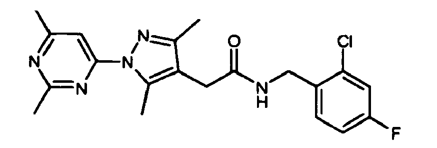 | 402 | 2.52 |
| | *N-*[(2-chloro-4-fluorophenyl)methyl]-2-[1-(2,6-dimethyl-4-pyrimidinyl)-3,5-dimethyl-1*H-*pyrazol-4-yl]acetamide | | |
| E81 | 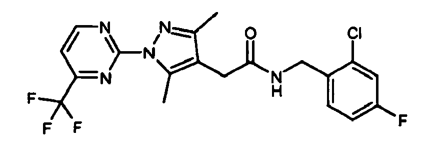 | 442 | 2.84 |
| | *N-*[(2-chloro-4-fluorophenyl)methyl]-2-{3,5-dimethyl-1-[4-(trifluoromethyl)-2-pyr)midiny)]-1*H-*pyrazol-4-yl}acetamide | | |

### Example 82 N-[(2-chloro-4-fluorophenyl)methyl]-2-[3,5-dimethyl-1-(2-phenylethyl)-1H-pyrazol-4-yl]acetamide (E82)

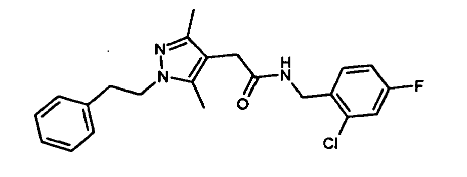

[3,5-dimethyl-1-(2-phenylethyl)-1*H-*pyrazol-4-yl]acetic acid (0.63 mmol) was dissolved in dimethylformamide (2 ml) and to this was added O-(7-Azabenzotriazol-1-yl)-N,N,N',N'-tetramethyluronium hexafluorophosphate (HATU, 0.63 mmol) and diisopropylethylamine (DIPEA, 1.26 mmol). The mixture was left to stand at room temperature for 15-20 minutes and then [(2-chloro-4-fluorophenyl)methyl]amine (0.63 mmol) in dimethylformamide (1 ml) was added. The mixture was left to stand overnight at room temperature and then the solvents were evaporated *in-vacuo.* The residue was partitioned between dichloromethane and saturated aqueous sodium hydrogen carbonate. The mixture was then filtered through a phase separator and the organic phase was evaporated to give the crude product. The crude material was purified by mass-directed automated HPLC to give the title compound as product after lyophilisation of the combined product fractions. LC/MS [M+H]⁺ = 399, retention time =3.16 minutes.

[3,5-Dimethyl-1-(2-phenylethyl)-1*H-*pyrazol-4-yl]acetic acid was obtained by hydrolysis of the corresponding ester (i.e. ethyl [3,5-dimethyl-l-(2-phenylethyl)-1*H-*pyrazol-4-yl]acetate). Ethyl [3,5-dimethyl-1-(2-phenylethyl)-1*H-*pyrazol-4-yl]acetate was prepared by the reaction of ethyl 3-acetyl-4-oxopentanoate with (2-phenylethyl)hydrazine. Appropriate methodologies to prepare these or analgous compounds have been described previously (e.g. US 4, 146, 721).

### Examples 83-111

In a manner analogous to that described for Example 82 above the compounds tabulated below (Table 4) were prepared by substituting the appropriate hydrazines (or a salt thereof e.g. HCl salt), all of which are available from commercial sources or can be prepared using routes described previously in the chemical literature, for the (2-phenylethyl)hydrazine used in the above procedure (synthesis of ester intermediate).

**Table 4**

| Example no. | Chemical name | [M+H]⁺ | Retention time (mins) |
|---|---|---|---|
| E83 | 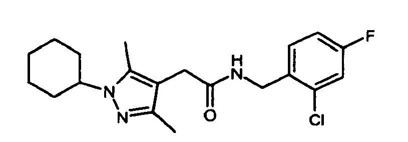 | 378 | 2.88 |
| | *N-*[(2-chloro-4-fluorophenyl)methyl]-2-(1-cyclohexyl-3,5-dimethyl-1*H-*pyrazol-4-yl)acetamide | | |
| E84 | 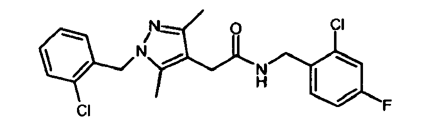 | 420 | 3.25 |
| | *N-*[(2-chloro-4-fluorophenyl)methyl]-2-{1-[(2-chlorophenyl)methyl]-3,5-dimethyl-1*H-*pyrazol-4-yl}acetamide | | |
| E85 | 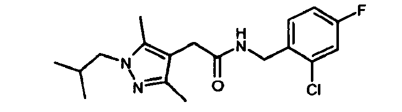 | 352 | 2.97 |
| | *N-*[(2-chloro-4-fluorophenyl)methyl]-2-[3,5-dimethyl-1-(2-methylpropyl)-1*H-*pyrazol-4-yl]acetamide | | |
| E86 | 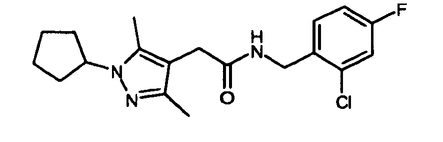 | 364 | 3.03 |
| | *N-*[(2-chloro-4-fluorophenyl)methyl]-2-(1-cyclopentyl-3,5-dimethyl-1*H-*pyrazol-4-yl)acetamide | | |
| E87 | 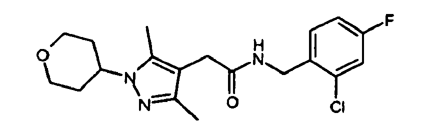 | 379 | 2.67 |
| | *N-*[(2-chloro-4-fluorophenyl)methyl]-2-[3,5-dimethyl-1-(tetrahydro-2*H-*pyran-4-yl)-1*H-*pyrazol-4-yl]acetamide | | |
| E88 | 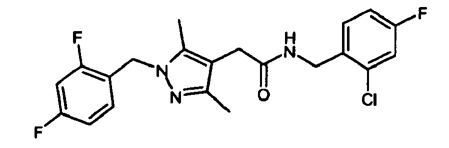 | 422 | 3.15 |
| | *N-*[(2-chloro-4-fluorophenyl)methyl]-2-{1-[(2,4-difluorophenyl)methyl]-3,5-dimethyl-1*H-*pyrazol-4-yl}acetamide | | |
| E89 | 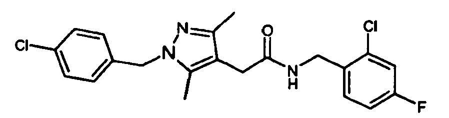 | 420 | 3.25 |
| | *N-*[(2-chloro-4-fluorophenyl)methyl]-2-{1-[(4-chlorophenyl)methyl]-3,5-dimethyl-1*H-*pyrazol-4-yl}acetamide | | |
| E90 | 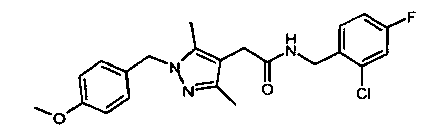 | 416 | 3.06 |
| | *N-*[(2-chloro-4-fluorophenyl)methyl]-2-(3,5-dimethyl-1-{[4-(methyloxy)phenyl]methyl}-1*H-*pyrazol-4-yl)acetamide | | |
| E91 | 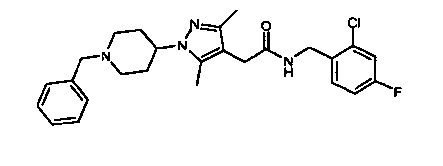 | 469 | 2.4 |
| | *N-*[(2-chloro-4-fluorophenyl)methyl]-2-{3,5-dimethyl-1-[1-(phenylmethyl)-4-piperidinyl]-1*H-*pyrazol-4-yl}acetamide | | |
| E92 | 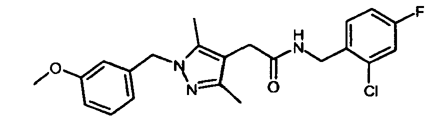 | 416 | 3.05 |
| | *N-*[(2-chloro-4-fluorophenyl)methyl]-2-(3,5-dimethyl-1-{[3-(methyloxy)phenyl]methyl}-1*H-*pyrazol-4-yl)acetamide | | |
| E93 | 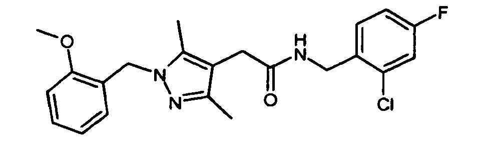 | 416 | 3.14 |
| | *N-*[(2-chloro-4-fluorophenyl)methyl]-2-(3,5-dimethyl-1-{[2-(methyloxy)phenyl]methyl}-1*H-*pyrazol-4-yl)acetamide | | |
| E94 | 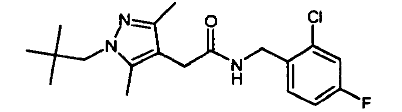 | 365 | 3.11 |
| | N-[(2-chloro-4-fluorophenyl)methyl]-2-[1-(2,2-dimethylpropyl)-3,5-dimethyl-1*H-*pyrazol-4-yl]acetamide | | |
| E95 | 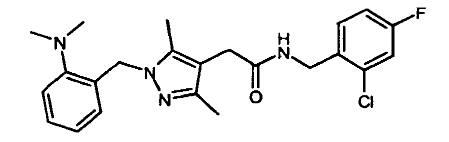 | 429 | 2.89 |
| | *N-*[(2-chloro-4-fluorophenyl)methyl]-2-(1-{[2-(dimethylamino)phenyl]methyl}-3,5-dimethyl-1*H-*pyrazol-4-yl)acetamide | | |
| E96 | 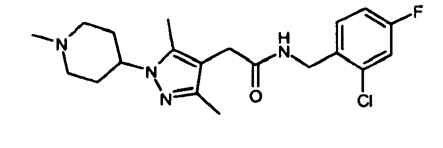 | 393 | 2.13 |
| | *N-*[(2-chloro-4-fluorophenyl)methyl]-2-[3,5-dimethyl-1-(1-methyl-4-piperidinyl)-1*H-*pyrazol-4-yl]acetamide | | |
| E97 | 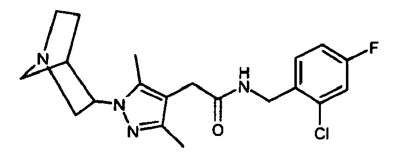 | 391 | 2.14 |
| | 2-[1-(1-azabicyclo[2.2.1]hept-3-yl)-3,5-dimethyl-1*H-*pyrazol-4-yl]-*N-*[(2-chloro-4-fluorophenyl)methyl]acetamide | | |
| E98 | 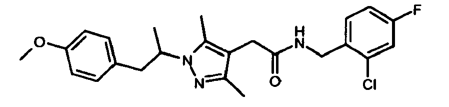 | 444 | 3.19 |
| | *N-*[(2-chloro-4-fluorophenyl)methyl]-2-(3,5-dimethyl-1-{1-methyl-2-[4-(methyloxy)phenyl]ethyl}-1*H-*pyrazol-4-yl)acetamide | | |
| E99 | 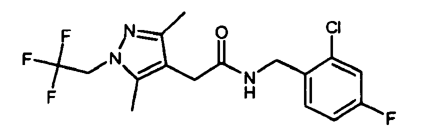 | 378 | 2.87 |
| | *N-*[(2-chloro-4-fluorophenyl)methyl]-2-[3,5-dimethyl-1-(2,2,2-trifluoroethyl)-1*H-*pyrazol-4-yl]acetamide | | |
| E100 | 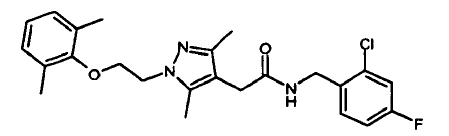 | 444 | 3.31 |
| | *N-*[(2-chloro-4-fluorophenyl)methyl]-2-(1-{2-[(2,6-dimethylphenyl)oxy]ethyl}-3,5-dimethyl-1*H-*pyrazol-4-yl)acetamide | | |
| E101 | 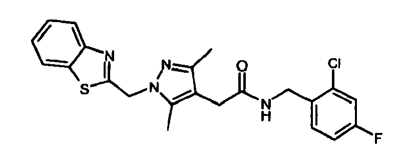 | 443 | 3.05 |
| | 2-[1-(1,3-benzothiazol-2-ylmethyl)-3,5-dimethyl-1*H-*pyrazol-4-yl]-*N-*[(2-chloro-4-fluorophenyl)methyl]acetamide | | |
| E102 | 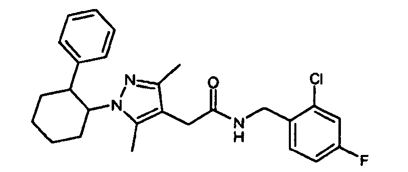 | 454 | 3.78 |
| | *N-*[(2-chloro-4-fluorophenyl)methyl]-2-[3,5-dimethyl-1-(2-phenylcyclohexyl)-1*H-*pyrazol-4-yl]acetamide | | |
| E103 | 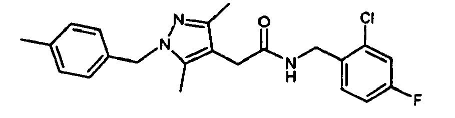 | 400 | 3.17 |
| | *N-*[(2-chloro-4-fluorophenyl)methyl]-2-{3,5-dimethyl-1-[(4-methylphenyl)methyl]-1*H-*pyrazol-4-yl}acetamide | | |
| E104 | 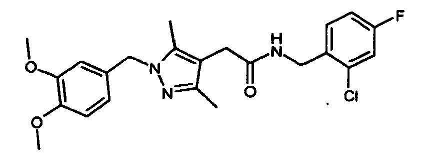 | 446 | 2.9 |
| | 2-(1-{[3,4-bis(methyloxy)phenyl]methyl}-3,5-dimethyl-1*H-*pyrazol-4-yl)-*N-*[(2-chloro-4-fluorophenyl)methyl]acetamide | | |
| E105 | 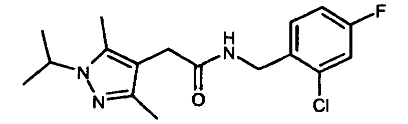 | 338 | 2.78 |
| | *N-*[(2-chloro-4-fluorophenyl)methyl]-2-[3,5-dimethyl-1-(1-methylethyl)-1*H-*pyrazol-4-yl]acetamide | | |
| E106 | 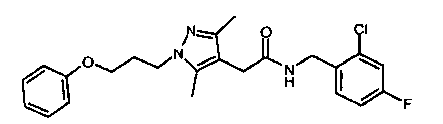 | 430 | 3.2 |
| | *N-*[(2-chloro-4-fluorophenyl)methyl]-2-{3,5-dimethyl-1-[3-(phenyloxy)propyl]-1*H-*pyrazol-4-yl}acetamide | | |
| E107 | 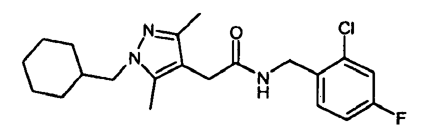 | 392 | 3.26 |
| | *N-*[(2-chloro-4-fluorophenyl)methyl]-2-[1-(cyclohexylmethyl)-3,5-dimethyl-1*H-*pyrazol-4-yl]acetamide | | |
| E108 | 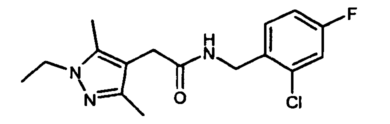 | 324 | 2.68 |
| | *N-*[(2-chloro-4-fluorophenyl)methyl]-2-(1-ethyl-3,5-dimethyl-1*H-*pyrazol-4-yl)acetamide | | |
| E109 | 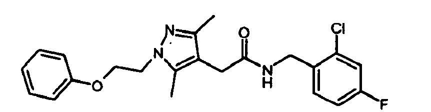 | 416 | 3.12 |
| | *N-*[(2-chloro-4-fluorophenyl)methyl]-2-{3,5-dimethyl-1-[2-(phenyloxy)ethyl]-1*H-*pyrazol-4-yl}acetamide | | |
| E110 | 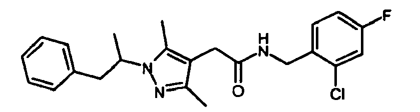 | 414 | 3.21 |
| | *N-*[(2-chloro-4-fluorophenyl)methyl]-2-[3,5-dimethyl-1-(1-methyl-2-phenylethyl)-1*H-*pyrazol-4-yl]acetamide | | |
| E111 | 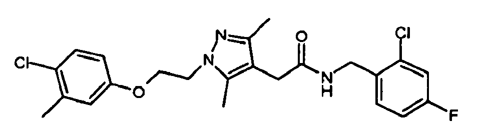 | 465 | 3.4 |
| | *N-*[(2-chloro-4-fluorophenyl)methyl]-2-(1-{2-[(4-chloro-3-methylphenyl)oxy]ethyl}-3,5-dimethyl-1*H-*pyrazol-4-yl)acetamide | | |

### Example 112 N-[(2-chloro-4-fluorophenyl)methyl]-2-{3,5-dimethyl-1-[4-(2-oxo-1-pyrrolidinyl)phenyl]-1H-pyrazol-4-yl}acetamide (E112)

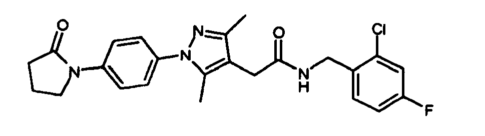

*N-*[(2-chloro-4-fluorophenyl)methyl]-2-[1-(4-iodophenyl)-3,5-dimethyl-1*H-*pyrazol-4-yl]acetamide (0.05 g, 0.1 mmol), which was prepared as described above (Example E76), was dissolved in toluene (3 ml) and to this was added tris(dibenzylideneacetone)dipalladium (0) (0.001 g, 0.001 mmol), 2-pyrrolidinone (0.010 g, 0.12 mmol), Xantphos^{™} (0.006 g) and cesium carbonate (0.049 g, 0.15 mmol). The mixture was heated at reflux temperature for 2 hrs then cooled and diluted with ethyl acetate. The mixture was washed with water and then the organic phase was dried over anhydrous magnesium sulphate and the solvents evaporated. The material thus obtained was purified by mass-directed automated HPLC to give the title compound as product after lyophilisation of the combined product fractions. LC/MS [M+H]⁺= 455.2, retention time = 2.62 minutes.

### Example 113 N-[(2-chloro-4-fluorophenyl)methyl]-2-[1-(4-fluorophenyl-1H-pyrazol-4-yl]acetamide (E113)

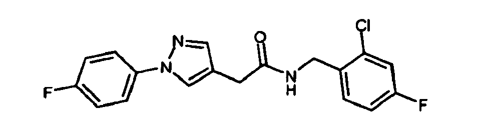

*N-*[(2-chloro-4-fluorophenyl)methyl]-2-[1-(4-fluorophenyl)-1*H**-***pyrazol-4-yl]acetamide (E113) was prepared in an analogous manner to that described above for example 48 but using [1-(4-fluorophenyl)-1*H-*pyrazol-4-yl]acetic acid in the place of [1-(4-fluorophenyl)-3,5-dimethyl-1*H-*pyrazol-4-yl]acetic acid. LC/MS [M+H]⁺ = 362.1, retention time = 2.93 minutes.

[1-(4-Fluorophenyl)-1*H-*pyrazol-4-yl]acetic acid was prepared from ethyl 1-(4-fluorophenyl)-1*H-*pyrazole-4-carboxylate by the procedure outlined in Scheme 5 and using analogous methodology to that described in J.Heterocyclic Chem., 30, 997 (1993). Ethyl 1-(4-fluorophenyl)-1*H-*pyrazole-4-carboxylate was prepared as follows (*c.f*. J.Org.Chem., 2004, 69, 5578-5587): Ethyl 1*H-*pyrazole-4-carboxylate (0.5 g, 3.6 mmol) was dissolved in *N-*methyl pyrrolidinone (10 ml) and treated with 1-fluoro-4-iodobenzene (0.5 ml, 4.3 mmol), copper (I) iodide (0.035g, 0.18 mmol), N,N'-dimethylethylenediamine (0.08 ml, 0.72 mmol), and potassium phosphate (1.61 g, 7.6 mmol). The mixture was heated in a sealed tube in a microwave reactor at 120°C for 1 hr then cooled and filtered, washed with ethyl acetate and evaporated *in vacuo.* The residue was purified by automated flash silica-gel chromatography (Flashmaster II) eluting with a 0-30% gradient of ethyl acetate in hexane. This entire process was repeated twice more and the combined fractions from the 3 experiments were combined and evaporated to give ethyl 1-(4-fluorophenyl)-1*H-*pyrazole-4-carboxylate (1g, 4.3 mmol) as a white solid. LC/MS [M+H]⁺ = 235.2, retention time = 2.98 minutes.

### Example 114 N-[(2-chloro-4-fluorophenyl)methyl]-2-[1-(4-fluoropheny)-5-(trifluoromethyl)-1H-pyrazol-4-yl]acetamide (E114)

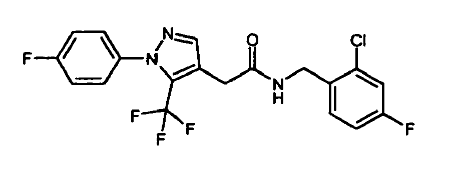

*N-*[(2-chloro-4-fluorophenyl)methyl]-2-[1-(4-fluorophenyl)-5-(trifluoromethyl)-1*H-*pyrazol-4-yl]acetamide (E114) was prepared in an analogous manner to that described above for example 48 but using [1-(4-fluorophenyl)-5-(trifluoromethyl)-1*H-*pyrazol-4-yl]acetic acid in the place of [1-(4-fluorophenyl)-3,5-dimethyl-1*H-*pyrazol-4-yl]acetic acid. LC/MS [M+H]⁺ = 430, retention time =3.16 minutes.

[1-(4-Fluorophenyl)-5-(trifluoromethyl)-1*H-*pyrazol-4-yl]acetic acid used above was prepared from commercially available ethyl 1-(4-fluorophenyl)-5-(trifluoromethyl)-1*H-*pyrazole-4-carboxylate by the procedure outlined in Scheme 5 and using analogous methodology to that described in *J.Heterocyclic Chem.,* **30**, 997 (1993).

### Example 115 N-[(2-chloro-4-fluorophenyl)methyl]-2-[1-(2-methylphenyl)-1H-pyrazol-4-yl]acetamide (E115)

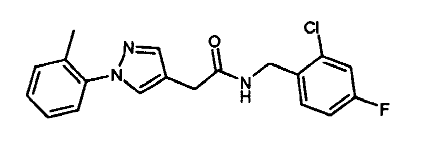

*N-*[(2-chloro-4-fluorophenyl)methyl]-2-[1-(2-methylphenyl)-1*H-*pyrazol-4-yl]acetamide (E115) was prepared in an analogous manner to that described above for example 48 but using [1-(2-methy)phenyl)-1*H-*pyrazol-4-yl]acetic acid in the place of [1-(4-fluorophenyl)-3,5-dimethyl-1*H-*pyrazol-4-yl]acetic acid. LC/MS [M+H]⁺ = 358, retention time = 2.86 minutes.

[[1-(2-Methylphenyl)-1*H-*pyrazol-4-yl]acetic acid used above was prepared from ethyl 1-(2-methylphenyl)-1*H*-pyrazole-4-carboxylate by the procedure outlined in Scheme 5 and using analogous methodology to that described in J.Heterocyclic Chem., 30, 997 (1993). Ethyl 1-(2-methylphenyl)-1*H-*pyrazole-4-carboxylate was prepared as follows (*c.f*. J.Org.Chem., 2004, 69, 5578-5587): Ethyl 1*H-*pyrazole-4-carboxylate (1.0 g, 7.14 mmol) was dissolved in *N-*methyl pyrrolidinone (NMP,10 ml) and treated with 1-iodo-2-methylbenzene (2.73 ml, 21.43 mmol), copper (I) iodide (0.068 g, 0.36 mmol), N,N'-dimethylethylenediamine (0.152 ml, 1.43 mmol), and potassium carbonate (2.07 g, 15 mmol). The mixture was heated in a sealed tube in a microwave reactor at 180°C for 3 hrs then cooled. An additional quantity (as above) of copper (I) iodide and N,N'-dimethylethylenediamine were added and the mixture was heated in the same manner as before for a further 1 hr. The mixture was loaded onto a C18 cartridge and washed with water to remove the NMP and then with methanol. The methanol fraction was evaporated *in vacuo* and then purified by flash column chromatography, eluting with a 9:1 mixture of petroleum ether (60/80) and ethyl acetate, to give ethyl 1-(2-methylphenyl)-1*H-*pyrazole-4-carboxylate (0.619 g, 2.69 mmol) after evaporation of the solvents. LC/MS [M+H]⁺ = 231, retention time = 2.81 minutes.

### Example 116 N-[(2-chloro-4-fluorophenyl)methyl]-2-[1-(2-ethylpheny)-1H-pyrazol-4-yl]acetamide (E116)

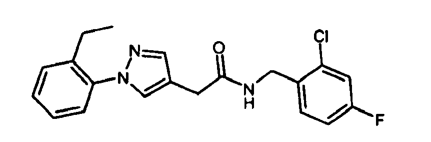

*N-*[(2-chloro-4-fluorophenyl)methyl]-2-[1-(2-ethylphenyl)-1*H*-pyrazol-4-yl]acetamide (E116) was prepared in an analogous manner to that described above for example 115 but using [1-(2-ethylphenyl)-1*H*-pyrazol-4-yl]acetic acid in the place of [1-(2-methylphenyl)-1*H*-pyrazol-4-yl]acetic acid. LC/MS [M+H]⁺ = 245, retention time = 3.01 minutes.

### Example 117 N-[(2-chloro-4-fluorophenyl)methyl]-2-{3,5-dimethyl-1-[4-(trifluoromethyl)phenyl]-1H-pyrazol-4-yl}acetamide (E117)

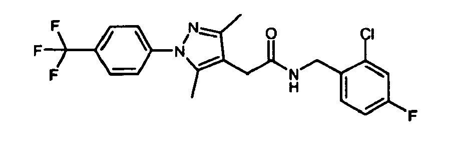

Crude (3Z)-3-acetyl-*N-*[(2-chloro-4-fluorophenyl)methyl]-4-{[(1,1-dimethylethyl)-(dimethyl)silyl]oxy}-3-pentenamide (-0.29 g, -0.5 mmol, prepared as described below) was dissolved in ethanol (1.5 ml) and treated with [4-(trifluoromethyl)phenyl]hydrazine (0.088 g, 0.5 mmol) and glacial acetic acid (0.029 ml, 0.5 mmol). The mixture was heated in a sealed tube in a microwave reactor at 110°C for 45 minutes. The mixture was then cooled and the solvent evaporated. The residue was purified by mass-directed automated HPLC to give the title compound as product (0.088 g, 0.2 mmol) after concentration of the combined product fractions. LC/MS [M+H]⁺ = 440, retention time = 3.21 minutes.

### (3Z)-3-acetyl-N-[(2-chloro-4-fluorophenyl)methyl]-4-{[(1,1-dimethylethyl)-(dimethyl)silyl]oxy}-3-pentenamide used in the above procedure was prepared using the following sequence of reactions:

(i) Phenylmethyl 3-acetyl-4-oxopentanoate (1.3 g, 5.24 mmol) (a preparation of which can be found in US 4,146,721), was dissolved in dichloromethane (5 ml) and cooled to 0°C in an ice-bath under an atmosphere of argon. The mixture was treated with (1,1-dimethylethyl)(dimethyl)silyl trifluoromethanesulfonate (1.8 ml, 7.85 mmol) and 2,6-lutidine (1.83 ml, 15.72 mmol) and stirred at 0°C for 10 minutes then left to warm to room temperature and stirred for an additional 30 minutes. The mixture was then treated with saturated aqueous ammonium chloride and filtered through a phase separator. The aqueous layer was extracted with further aliquots of dichloromethane and then the combined organic layers were concentrated to give a light yellow oil (2.7 g) which was used without purification in the next step.
(ii) The crude material obtained in step (i) was dissolved in ethanol (30 ml) and treated with 10% palladium on carbon under a flow of argon. The mixture was then hydrogenated at room temperature for -48 hrs. The mixture was flushed with argon, filtered and evaporated to give a light yellow oil (2.0 g). This material was used without further purification in the next step.
(iii) The crude material obtained in step (ii) was dissolved in dichloromethane (15 ml) and treated with water soluble carbodiimide (1.31 g, 6.81 mmol), 1-hydroxybenzotriazole (0.920 g, 6.81 mmol), N-ethyl morpholine (2.0 ml, 15.72 mmol) and [(3,4-difluorophenyl)methyl]amine (1.09 g, 6.81 mmol). The mixture was stirred at room temperature for 10 minutes and then left to stand overnight. Saturated aqueous sodium hydrogen carbonate was added to the mixture, stirred for 10 minutes, then filtered through a phase separator and the organic phase was then evaporated to give the crude product ((3*Z*)-3-acetyl-*N-*[(2-chloro-4-fluorophenyl)methyl]-4-{[(1,1-dimethylethyl)-(dimethyl)silyl]oxy}-3-pentenamide) as an orange oil. This material was used without purification.

### Example 118 N-[(2-chloro-4-fluorophenyl)methyl]-2-[3.5-dimethyl-1-(3-pyridinylmethyl)-1H pyrazol-4-yl]acetamide (E118)

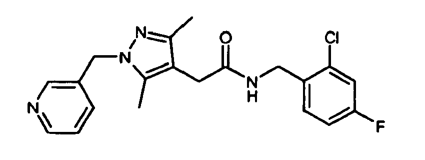

Crude (3*Z*)-3-acetyl-*N-*[(2-chloro-4-fluorophenyl)methyl]-4-{[(1,1-dimethylethyl)-(dimethyl)silyl]oxy}-3-pentenamide (-0.29 g, ~0.5 mmol, prepared as described above) was suspended in ethanol (1.5 ml) and treated with 3-(hydrazinomethyl)pyridine dihydrochloride (0.098 g, 0.5 mmol) and sodium acetate (0.082 g, 1.0 mmol). The mixture was heated in a sealed tube in a microwave reactor at 110°C for 45 minutes. The mixture was then cooled and the solvent evaporated. The residue was partitioned between water and a chloroform/isopropanol mixture and the organic layer was then separated and evaporated to give an orange oil. This was purified by mass-directed automated HPLC to give the title compound as product (0.056 g, 0.14 mmol) after concentration of the combined product fractions. LC/MS [M+H]⁺ = 387, retention time = 1.90 minutes.

### Examples 119 N-[(2-chloro-4-fluorophenyl)methyl]-2-(5-methyl-1-phenyl-1H-pyrazol-4-yl)acetamide trifluoroacetate (E119)

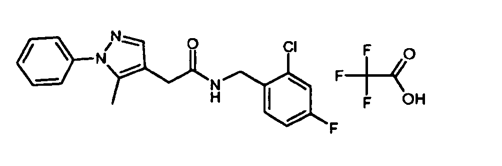

A 1:1 mixture of 3-(1-phenyl-1*H*-pyrazol-5-yl)propanoic acid and (5-methyl-1-phenyl-1*H-*pyrazol-4-yl)acetic acid (-0.30 g, ~1.4 mmol, prepared as described below) was dissolved in dimethylformamide (10 ml) and treated with water soluble carbodiimide (0.526 g, 2.78 mmol), 1-hydroxybenzotriazole (0.374 g, 2.78 mmol), diisopropylethylamine (1.0 ml, 5.56 mmol), and [(2-chloro-4-fluorophenyl)methyl]amine (0.447 g, 2.78 mmol). The mixture was heated at 45°C for 2 hrs. The mixture was then cooled and the solvent evaporated. The residue was partitioned between ethyl acetate and 2M aqueous hydrogen chloride. The organic layer was then separated and washed with saturated aqueous sodium hydrogen carbonate, and evaporated to give the crude product. This was purified by reverse phase to give the title compound as product (0.043 g) after concentration of the combined product fractions. LC/MS [M+H]⁺ = 358.09 retention time = 2.73 minutes.

The reverse phase purification of the crude product to give title compound as product was performed as follows:

| | |
|---|---|
| Column : | Phenomenex Luna C18 (2) |
| | (250 mm x 21.2 mm i.d.; 10 micron particle size) |
| Eluent : | A = Acetonitrile + 0.1% v/v trifluoroacetic acid |
| | B = Water + 0.1 % v/v trifluoroacetic acid |
| | A : B = 35:65 v/v; isocratic procedure |
| Flow-rate : | 20 mLmin-1 |
| Temperature: | Ambient |
| Detection : | U.V. Absorbance at 215 nm (band-width = 10 nm) |
| Injection volume : | VARIABLE (loading study performed) |
| Working concentration : | 100 mgmL-1 in dimethylformamide |

(5-methyl-1-phenyl-1H-pyrazol-4-yl)acetic acid used in the above procedure was prepared using the following sequence of reactions (see US 4,146,721 for analogous procedures):
(i) Methyl 4-oxopentanoate (2g, 15 mmol) was treated with dimethylformamide dimethyl acetal (3.7 g, 31.5 mmol) and glacial acetic acid (0.25 ml) and heated at 120°C for 18 hrs. The mixture was evaporated *in vacuo* to give a ~40:60 mixture of methyl 3-acetyl-4-(dimethylamino)-3-butenoate and methyl 6-(dimethylamino)-4-oxo-5-hexenoate respectively as a red oil (5.45 g). This mixture was used without further purification in the next step.
(ii) The mixture of methyl 3-acetyl-4-(dimethylamino)-3-butenoate and methyl 6-(dimethylamino)-4-oxo-5-hexenoate (5.45 g, ~29.4 mmol) obtained in step (i) was dissolved in ethanol (50 ml) and treated with phenyl hydrazine (2.9 ml, 29.4 mmol) and glacial acetic acid (1.7 ml, 29.4 mmol) and then heated at reflux for 2 hours. The mixture was evaporated *in vacuo* to give a red oil and this was partially purified by automated flash column chromatography (Flashmaster II) to give a mixture of mainly methyl (5-methyl-1-phenyl-1*H*-pyrazol-4-yl)acetate and methyl 3-(1-phenyl-1*H-*pyrazol-5-yl)propanoate by NMR as a red oil (1.05 g). This material was used in the next step without further purification.
(iii) The crude mixture of methyl (5-methyl-1-phenyl-1*H-*pyrazol-4-yl)acetate and methyl 3-(1-phenyl-1*H-*pyrazol-5-yl)propanoate (1.05 g, ~3.28 mmol) that was obtained in step (ii) was dissolved in methanol (8 ml) and treated with 2N aqueous sodium hydroxide (4 ml). The mixture was stirred overnight at room temperature and then evaporated *in vacuo.* The residue was taken up in water and acidified to pH 1 by the addition of 2M aqueous hydrogen chloride. The aqueous layer was then extracted with ethyl acetate and the organic phase was dried over anhydrous sodium sulphate and then filtered and evaporated to give an orange solid (0.595 g) comprising a 1:1 mixture of 3-(1-phenyl-1*H-*pyrazol-5-yl)propanoic acid and (5-methyl-1-phenyl-1*H-*pyrazol-4-yl)acetic acid. LC/MS [M+H]⁺ = 217.20 retention time = 1.91 minutes and [M-H]⁻ = 215.10, retention time = 215.10.

### Example 120 2-(3-butyl-5-methyl-1-phenyl-1H-pyrazol-4-yl)-N-[(2-chloro-4-fluorophenyl)methyl]acetamide (E120)

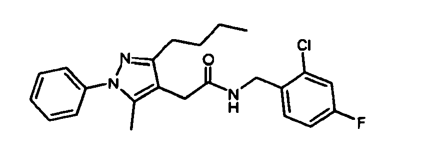

(3-Butyl-5-methyl-1-phenyl-1*H-*pyrazol-4-yl)acetic acid (0.100 g, 0.36 mmol, prepared as described below) was dissolved in dimethylformamide (5 ml) and treated with water soluble carbodiimide (0.138 g, 0.72 mmol), 1-hydroxybenzotriazole (0.099 g, 0.72 mmol), diisopropylethylamine (0.26 ml, 1.45 mmol), and [(2-chloro-4-fluorophenyl)methyl]amine (0.117 g, 0.72 mmol) and the mixture was stirred at room temperature for -48 hrs. The mixture was diluted with 2M aqueous hydrogen chloride and extracted with ethyl acetate. The combined organic layers were then washed with saturated aqueous sodium hydrogen carbonate, dried over anhydrous sodium sulphate, and evaporated to give the crude product. This was purified by mass-directed automated HPLC to give the title compound as product (0.087 g) after concentration of the combined product fractions and trituration with diethyl ether. LC/MS [M+H]⁺ = 414, retention time = 3.21 minutes.

(3-Butyl-5-methyl-1-phenyl-1*H-*pyrazol-4-yl)acetic acid used in the above procedure was prepared using the following sequence of reactions:
(i) 2,4-Octanedione (5g, 35.2 mmol) was dissolved in dimethylformamide (20 ml) and added dropwise to a suspension of 60% sodium hydride in oil (1.4 g, 35.2 mmol) in dimethylformamide (50 ml) and stirred at room temperature for 30 minutes under an atmosphere of argon. Ethyl bromoacetate (4.8 ml, 38.72 mmol) was then added and the mixture stirred for a further 4.5 hrs at room temperature. The solvents were removed *in-vacuo* and the remaining liquid was separated from any solids by filtration (washing the solid with acetone). Evaporation of the filtrate gave an oil which was further purified by automated flash column chromatography (Flashmaster II) to give ethyl 3-acetyl-4-oxooctanoate as a pale yellow oil (5.95 g).
(ii) Ethyl 3-acetyl-4-oxooctanoate (2.0 g, 8.7 mmol) obtained in step (i) was treated with phenyl hydrazine (0.86 ml, 8.7 mmol) and glacial acetic acid (0.5 ml, 8.7 mmol) and then heated at 100°C for 2 hours. The mixture was evaporated *in vacuo* and the residue was partially purified by automated flash column chromatography (Flashmaster II eluting with 0-10% ethyl acetate in hexane) to give an ~45:55 mixture of the starting material (Ethyl 3-acetyl-4-oxooctanoate)and ethyl (3-butyl-5-methyl-1-phenyl-1*H-*pyrazol-4-yl)acetate (0.407 g) as an oil. This material was used in the next step without further purification.
(iii) The crude mixture of (Ethyl 3-acetyl-4-oxooctanoate)and ethyl (3-butyl-5-methyl-1-phenyl-1*H-*pyrazol-4-yl)acetate (0.407 g, ~1.36 mmol) that was obtained in step (ii) was dissolved in ethanol (4 ml) and treated with 2N aqueous sodium hydroxide (4 ml). The mixture was stirred overnight at room temperature and then evaporated *in vacuo.* The residue was taken up in water and acidified to pH 5 by the addition of 2M aqueous hydrogen chloride. The aqueous layer was then extracted with ethyl acetate and the organic phase was dried over anhydrous sodium sulphate and then filtered and evaporated to give (3-butyl-5-methyl-1-phenyl-1*H-*pyrazol-4-yl)acetic acid as a yellow gum (0.260 g). LC/MS [M+H]⁺ = 273 retention time = 2.69 minutes.

### Example 121 N-[(2-chloro-4-fluorophenyl)methyl]-2-[3-methyl-5-(1-methylethyl)-1-phenyl-1H-pyrazol-4-yl]acetamide (E121)

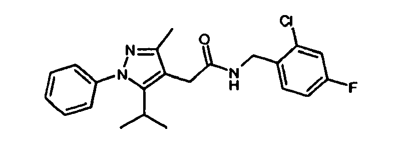

*N-*[(2-chloro-4-fluorophenyl)methyl]-2-[3-methyl-5-(1-methylethyl)-1-phenyl-1*H-*pyrazol-4-yl]acetamide was prepared in an analogous method to that described for the preparation of 2-(3-butyl-5-methyl-1-phenyl-1*H-*pyrazol-4-yl)-*N-*[(2-chloro-4-fluorophenyl)methyl]acetamide **(E120)** but using [3-Methyl-5-(1-methylethyl)-1-phenyl-1*H-*pyrazol-4-yl]acetic acid in the place of (3-Butyl-5-methyl-1-phenyl-1*H-*pyrazol-4-yl)acetic acid. LC/MS [M+H]⁺ = 400.25, retention time = 3.08 minutes.

[3-Methyl-5-(1-methylethyl)-1-phenyl-1*H-*pyrazol-4-yl]acetic acid was prepared in an analogous manner to that described above for the synthesis of (3-Butyl-5-methyl-1-phenyl-1*H-*pyrazol-4-yl)acetic acid but using 5-methyl-2,4-hexanedione in the place of 2,4-Octanedione.

### Examote 122 N-[(2-chloro-4-fluorophenyl)methyl]-2-[5-methyl-3-(1-methylethyl)-1-phenyl-1H-pyrazol-4-yl]acetamide (E122)

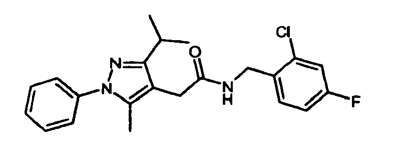

*N-*[(2-chloro-4-fluorophenyl)methyl]-2-[5-methyl-3-(1-methylethyl)-1-phenyl-1*H-*pyrazol-4-yl]acetamide was prepared in an analogous method to that described for the preparation of 2-(3-butyl-5-methyl-1-phenyl-1*H-*pyrazol-4-yl)-*N-*[(2-chloro-4-fluorophenyl)methyl]acetamide **(E120)** but using [5-methyl-3-(1-methylethyl)-1-phenyl-1*H-*pyrazol-4-yl]acetic acid in the place of (3-Butyl-5-methyl-1-phenyl-1*H-*pyrazol-4-yl)acetic acid. LC/MS [M+H]⁺ = 400.25 retention time = 3.06 minutes.

[5-methyl-3-(1-methylethyl)-1-phenyl-1*H-*pyrazol-4-yl]acetic acid was prepared in an analogous manner to that described above for the synthesis of (3-Butyl-5-methyl-1-phenyl-1*H-*pyrazol-4-yl)acetic acid but using 5-methyl-2,4-hexanedione in the place of 2,4-Octanedione.

### Example 123 N-[(2-chloro-4-fluorophenyl)methyl]-2-(3-methyl-1,5-diphenyl-1H-pyrazol-4-yl)acetamide (E123)

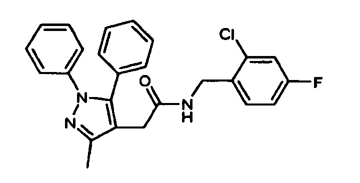

*N-*[(2-chloro-4-fluorophenyl)methyl]-2-(3-methyl-1,5-diphenyl-1*H-*pyrazol-4-yl)acetamide was prepared in an analogous method to that described for the preparation of 2-(3-butyl-5-methyl-1-phenyl-1*H-*pyrazol-4-yl)-*N-*[(2-chloro-4-fluorophenyl)methyl]acetamide **(E120)** but using (3-methyl-1,5-diphenyl-1*H-*pyrazol-4-yl)acetic acid in the place of (3-Butyl-5-methyl-1-phenyl-1*H-*pyrazol-4-yl)acetic acid. LC/MS [M+H]⁺ = 434.1 retention time = 3.12 minutes.

(3-methyl-1,5-diphenyl-1*H-*pyrazol-4-yl)acetic acid was prepared in an analogous manner to that described above for the synthesis of (3-Butyl-5-methyl-1-phenyl-1*H-*pyrazol-4-yl)acetic acid but using 1-phenyl-1,3-butanedione in the place of 2,4-Octanedione.

### Example 124 N-[(2-chloro-4-fluorophenyl)methyl]-2-(1-(4-fluorophenyl)-5-methyl-1H-pyrazol-4-yl]acetamide (E124)

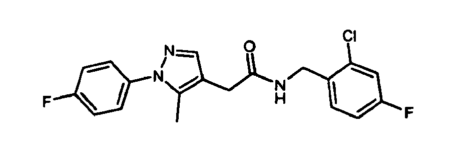

*N-*[(2-chloro-4-fluorophenyl)methyl]-2-[1-(4-fluorophenyl)-5-methyl-1*H-*pyrazol-4-yl]acetamide **(E124)** was prepared in an analogous manner to that described above for example 48 but using [1-(4-fluorophenyl)-5-methyl-1*H-*pyrazol-4-yl]acetic acid in the place of [1-(4-fluorophenyl)-3,5-dimethyl-1*H-*pyrazol-4-yl]acetic acid. LC/MS [M+H]⁺ = 376.18, retention time = 2.79 minutes.

The [1-(4-fluorophenyl)-5-methyl-1*H-*pyrazol-4-yl]acetic acid used above was prepared from [1-(4-fluorophenyl)-5-methyl-1*H-*pyrazol-4-yl]methanol by the procedure outlined in Scheme 5 and using analogous methodology to that described in J.Heterocyclic Chem., 30, 997 (1993).

### Example 125 Ethyl [4-(2-{[(2-chloro-4-fluorophenyl)methyl]amino}-2-oxoethyl)-3,5-dimethyl-1H-pyrazol-1-yl]acetate (E125)

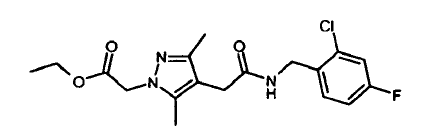

{1-[2-(ethyloxy)-2-oxoethyl]-3,5-dimethyl-1*H-*pyrazol-4-yl}acetic acid (0.945 g, prepared as described below) was dissolved in dimethylformamide (20 ml) and treated with water soluble carbodiimide (1.64 g, 8.58 mmol), 1-hydroxybenzotriazole (1.16 g, 8.59 mmol), diisopropylethylamine (2.3 ml, 13.2 mmol), and [(2-chloro-4-fluorophenyl)methyl]amine (0.755 g, 4.73 mmol) and the mixture was stirred at room temperature overnight. The mixture was diluted with ethyl acetate and washed with saturated aqueous sodium hydrogen carbonate. The combined organic layers were then washed with water and then with brine, filtered through a hydrophobic frit, and evaporated to give the crude product as a light brown solid. This was purified by flash column chromatography, eluting with a 1:1 mixture of dichloromethane and diethyl ether, to give the title compound as product (0.444 g) after concentration of the combined product fraction. LC/MS [M+H]⁺ = 382.2, retention time = 2.51 minutes.

{1-[2-(ethyloxy)-2-oxoethyl]-3,5-dimethyl-1*H-*pyrazol-4-yl}acetic acid used in the above procedure was prepared using the following sequence of reactions:
(i) 1,1-dimethylethyl 3-acetyl-4-oxopentanoate (see J.Org.Chem., 65(18), 2000, 5806-5816)(2.01 g, 9.38 mmol), ethyl hydrazinoacetate hydrochloride (1.45 g, 9.38 mmol), and sodium acetate (0.77 g, 9.38 mmol) were dissolved in ethanol (40 ml) and heated at reflux temperature for 3 hrs. The mixture was cooled and the ethanol evaporated *in vacuo.* The residue was taken up in dichloromethane and saturated aqueous sodium hydrogen carbonate and then the organic phase was separated, washed with brine, and filtered through a hydrophobic frit. Evaporation of the solvent gave the crude product (2.74 g), 1,1-dimethylethyl ethyl 2,2'-(3,5-dimethyl-1*H-*pyrazole-1,4-diyl)diacetate, which was used without further purification in the next step.
(ii) 1,1-dimethylethyl ethyl 2,2'-(3,5-dimethyl-1*H-*pyrazole-1,4-diyl)diacetate (2.74 g, 9.24 mmol) was dissolved in dry dichloromethane (25 ml) and treated with trifluoroacetic acid (5 ml). The mixture was left to stand for 18 hrs and then evaporated to give a light brown gum. The residue was treated with saturated sodium hydrogen carbonate until the pH had been adjusted to 3 and then the precipitated solid was collected by filtration, washed with water, and dried to give {1-[2-(ethyloxy)-2-oxoethyl]-3,5-dimethyl-1*H-*pyrazol-4-yl}acetic acid (1.92 g). LC/MS [M+H]⁺ = 241, retention time = 1.61 minutes.

### Example 126 [4-(2-{[(2-chloro-4-fluorophenyl)methyl]amino}-2-oxoethyl)-3,5-dimethyl-1H-pyrazol-1-yl]acetic acid (E126)

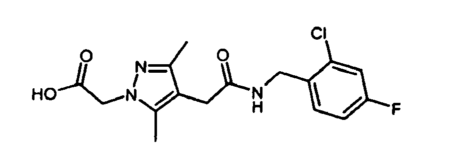

Ethyl [4-(2-{[(2-chloro-4-fluorophenyl)methyl]amino}-2-oxoethyl)-3,5-dimethyl-1*H-*pyrazol-1-yl]acetate **(E125)** (0.44 g, 1.15 mmol) was suspended in industrial methylated spirits (5 ml) and treated with 1 N aqueous sodium hydroxide. The mixture was stirred at room temperature for 1 hr and then the solvent was evaporated and the residue was dissolved in water (5 ml). The aqueous solution was acidified to pH=2 using concentrated hydrogen chloride and then the precipitated solid was collected by filtration, washing with water, and then dried to give [4-(2-{[(2-chloro-4-fluorophenyl)methyl]amino}-2-oxoethyl)-3,5-dimethyl-1*H-*pyrazol-1-yl]acetic acid (0.393 g) as a white solid. LC/MS [M+H]⁺ = 354.6, retention time = 2.02 minutes.

### Example 127 N-[(2-chloro-4-fluorophenyl)methyl]-2-{3,5-dimethyl-1-[2-(4-morpholinyl)-2-oxoethyl]-1H-pyrazol-4-yl}acetamide (E127)

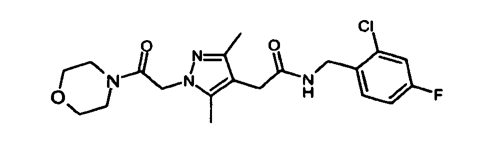

[4-(2-{[(2-chloro-4-fluorophenyl)methyl]amino}-2-oxoethyl)-3,5-dimethyl-1*H-*pyrazol-1-yl]acetic acid **(E126)** (0.053 g, 0.15 mmol, prepared as described above) was dissolved in dimethylformamide (2 ml) and treated with water soluble carbodiimide (0.058 g, 0.3 mmol), 1-hydroxybenzotriazole (0.041 g, 0.3 mmol), diisopropylethylamine (0.105 ml, 0.6 mmol), and morpholine (0.026 g, 0.3 mmol) and the mixture was stirred at room temperature overnight. The mixture was diluted with ethyl acetate and washed with saturated aqueous sodium hydrogen carbonate. The combined organic layers were then washed with water, filtered through a hydrophobic frit, and evaporated to give *N-*[(2-chloro-4-fluorophenyl)methyl]-2-{3,5-dimethyl-1-[2-(4-morpholinyl)-2-oxoethyl]-1*H-*pyrazol-4-yl}acetamide (0.030 g). LC/MS [M+H]⁺ = 423.5, retention time = 2.15 minutes.

### Examples 128-131

In a manner analogous to that described for Example 127 above the compounds tabulated below (Table 5) were prepared by substituting the appropriate amines, all of which are available from commercial sources or can be prepared using routes described previously in the chemical literature, for the morpholine used in the above procedure.

**Table 5**

| Example no. | Chemical name | [M+H]⁺ | Retention time (mins) |
|---|---|---|---|
| E128 | 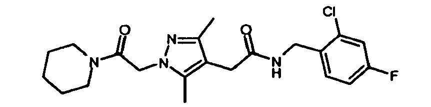 | 421 | 2.46 |
| | *N-*[(2-chloro-4-fluorophenyl)methyl]-2{3,5-dimethyl-1-[2-oxo-2-(1-piperidinyl)ethyl]-1*H-*pyrazol-4-yl}acetamide | | |
| E129 | 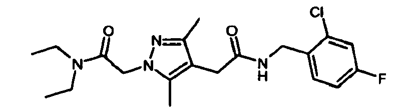 | 409 | 2.42 |
| | 2-[4-(2-{[(2-chloro-4-fluorophenyl)methyl)amino}-2-oxoethyl)-3,5-dimethyl-1*H-*pyrazol-1-yl]-*N,N-*diethylacetamide | | |
| E130 | 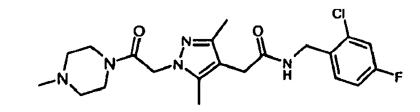 | 436 | 1.60 |
| | *N-*[(2-chloro-4-fluorophenyl)methyl]-2-{3,5-dimethyl-1-[2-(4-methyl-1-piperazinyl)-2-oxoethyl]-1*H-*pyrazol-4-yl}acetamide | | |
| E131 | 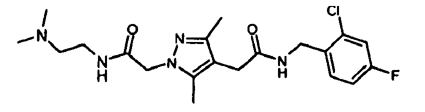 | 424 | 1.62 |
| | 2-[4-(2-{[(2-chloro-4-fluorophenyl)methyl]amino}-2-oxoethyl)-3,5-dimethyl-1*H-*pyrazol-1-yl]-*N-*[2-(dimethylamino)ethyl]acetamide | | |

### Example 132 N-[(2-chloro-4-fluorophenyl)methyl]-2-[1-(2-hydroxyethyl)3,5-dimethyl-1H-pyrazol-4-yl]acetamide (E132)

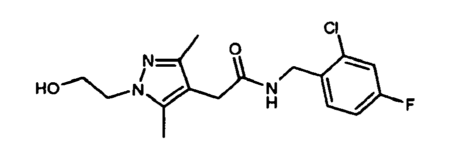

[4-(2-{[(2-chloro-4-fluorophenyl)methyl]amino}-2-oxoethyl)-3,5-dimethyl-1*H-*pyrazol-1-yl]acetic acid **(E126)** (0.982 g, 2.77 mmol) was dissolved in dry tetrahydrofuran (250 ml) by heating at reflux temperature for 30 minutes. The mixture was then cooled rapidly, with vigorous stirring, to -20°C to give a fine suspension. This was treated with isobutyl chloroformate (0.396 ml, 3.05 mmol) followed by *N-*methyl morpholine (0.336 ml, 3.05 mmol) over 2 minutes. Stirring at -20°C was continued for 30 minutes and then the mixture was filtered to remove the small amount of remaining solid. The filtrate was cooled to 0°C and then a solution of sodium borohydride (0.231 g, 6.11 mmol) in water (3 ml) was added dropwise over 5 minutes. The mixture was stirred between 0°C-5°C for 30 minutes and then 2M aqueous hydrogen chloride was added and stirring continued at 0°C for a further 30 minutes. The mixture was then warmed to 15°C over 30 minutes and the solvents were removed by evaporation *in vacuo.* Water (15 ml) was added to the mixture and this was then washed with ether (2 x 30 ml). The pH of the aqueous layer was then adjusted to -5 by the addition of 2N aqueous sodium hydroxide and extracted with dichloromethane. The combined organic extracts were filtered through a hydrophobic frit and evaporated to give *N-*[(2-chloro-4-fluorophenyl)methyl]-2-[1-(2-hydroxyethyl)-3,5-dimethyl-1*H-*pyrazol-4-yl]acetamide (0.892 g) as a white solid. LC/MS [M+H]⁺ = 340.2, retention time = 2.02 minutes.

### Example 133 N-[(2-chloro-4-fluorophenyl)methyl]-2-{3,5-dimethyl-1-[2-(4-morpholinyl)ethyl]-1H-pyrazol-4-yl}acetamide (E133)

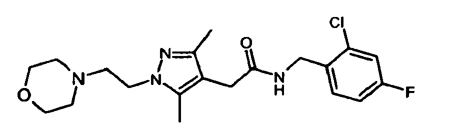

Crude 2-[4-(2-{[(2-chloro-4-fluorophenyl)methyl]amino}-2-oxoethyl)-3,5-dimethyl-1*H-*pyrazol-1-yl]ethyl trifluoromethanesulfonate (0.032 g, 0.068 mmol, prepared as described below) was dissolved in dichloromethane (2 ml) and added dropwise over 2 minutes to a solution of morpholine (0.030 g, 0.339 mmol) in dichloromethane (2 ml) which had been cooled to 0°C in an ice-bath. The mixture was warmed to room temperature and left to stand overnight. The mixture was then diluted with dichloromethane (5 ml) and washed with water (3 x 10 ml, saturated aqueous sodium chloride was added to the first two washes to improve seperation), then filtered through a hydrophobic frit and evaporated to give an oil. Purification of this residue by mass-directed automated HPLC gave *N-*[(2-chloro-4-fluorophenyl)methyl]-2-{3,5-dimethyl-1-[2-(4-morpholinyl)ethyl]-1*H-*pyrazol-4-yl}acetamide (0.013 g) after concentration of the combined product fractions. LC/MS [M+H]⁺ = 409.1, retention time = 1.66 minutes.

2-[4-(2-{[(2-Chloro-4-fluorophenyl)methyl]amino}-2-oxoethyl)-3,5-dimethyl-1*H-*pyrazol-1-yl]ethyl trifluoromethanesulfonate used in the procedure above was prepared by the following method:
*N-*[(2-chloro-4-fluorophenyl)methyl]-2-[1-(2-hydroxyethyl)-3,5-dimethyl-1*H-*pyrazol-4-yl]acetamide **(E132)** (0.228 g, 0.67 mmol) was dissolved (with warming) in dry dichloromethane (15 ml) and cooled to -78°C using an acetone/cardice bath. To this mixture was added diisopropylethylamine (0.350 ml, 2.0 mmol) followed by dropwise addition of triflic anhydride (0.17 ml, 1.0 mmol) over 1 minute. The mixture was stirred at -78°C for 45 minutes and then water (15 ml) was added and the mixture allowed to warm to room temperature. The dichloromethane layer was separated and washed with more water (3 x 15 ml) and then filtered through a hydrophobic frit and evaporated to give crude 2-[4-(2-{[(2-chloro-4-fluorophenyl)methyl]amino}-2-oxoethyl)-3,5-dimethyl-1*H-*pyrazol-1-yl]ethyl trifluoromethanesulfonate (0.308 g) as a light brown solid which was used without further purification. LC/MS [M+H]⁺ = 472.4, retention time = 2.90 minutes.

### Examples 134-143

In a manner analogous to that described for Example 133 above the compounds tabulated below (Table 6) were prepared by substituting the appropriate amines or alkoxides, all of which are available from commercial sources or can be prepared using routes described previously in the chemical literature, for the morpholine used in the above procedure.

**Table 6**

| Example no. | Chemical name | [M+H]⁺ | Retention time (mins) |
|---|---|---|---|
| E134 | 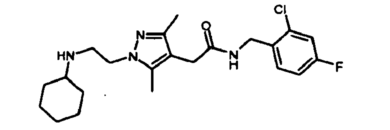 | 421 | 2.01 |
| | *N-*[(2-chloro-4-fluorophenyl)methyl]-2-{1-[2-(cyclohexylamino)ethyl]-3,5-dimethyl-1*H-*pyrazol-4-yl}acetamide | | |
| E135 | 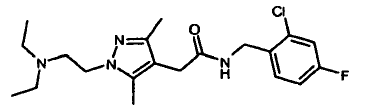 | 395 | 1.81 |
| | *N-*[(2-chloro-4-fluorophenyl)methyl]-2-{1-[2-(diethylamino)ethyl]-3,5-dimethyl-1*H-*pyrazol-4-yl}acetamide | | |
| E136 | 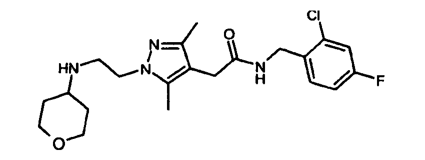 | 423 | 1.69 |
| | *N-*[(2-chloro-4-fluorophenyl)methyl]-2-{3,5-dimethyl-1-[2-(tetrahydro-2*H-*pyran-4-ylamino)ethyl]-1*H-*pyrazol-4-yl}acetamide | | |
| E137 | 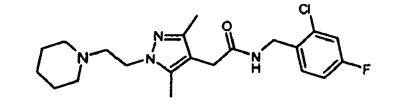 | 407 | 1.85 |
| | *N-*[(2-chloro-4-fluorophenyl)methyl]-2-{3,5-dimethyl-1-[2-(1-piperidinyl)ethyl]-1*H-*pyrazol-4-yl}acetamide | | |
| E138 | 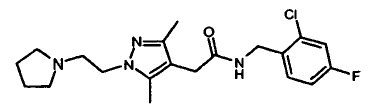 | 393 | 1.76 |
| | *N-*[(2-chloro-4-fluorophenyl)methyl]-2-{3,5-dimethyl-1-[2-(1-pyrrolidinyl)ethyl]-1*H-*pyrazol-4-yl}acetamide | | |
| E139 | 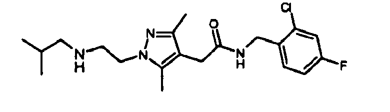 | 395 | 1.91 |
| | *N-*[(2-chloro-4-fluorophenyl)methyl]-2-(3,5-dimethyl-1-{2-[(2-methylpropyl)amino]ethyl}-1*H-*pyrazol-4-yl)acetamide | | |
| E140 | 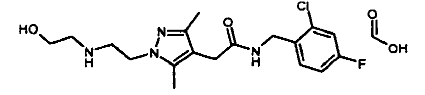 | 383 | 1.60 |
| | formic acid - *N-*[(2-chloro-4-fluorophenyl)methyl]-2-(1-{2-[(2-hydroxyethyl)amino]ethyl}-3,5-dimethyl-1*H-*pyrazol-4-yl)acetamide (1:1) | | |
| E141 | 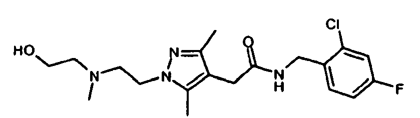 | 397 | 1.62 |
| | *N-*[(2-chloro-4-fluorophenyl)methyl]-2-(1-{2-[(2-hydroxyethyl)(methyl)amino]ethyl}-3,5-dimethyl-1*H*-pyrazol-4-yl)acetamide | | |
| E142 | 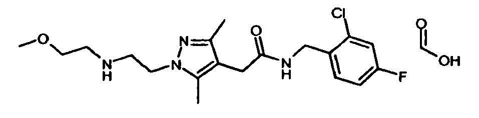 | 397 | 1.70 |
| | formic acid -*N-*[(2-chloro-4-fluorophenyl)methyl]-2-[3,5-dimethyl-1-(2-{[2-(methyloxy)ethyl]amino}ethyl)-1*H-*pyrazol-4-yl]acetamide (1:1) | | |
| E143 | 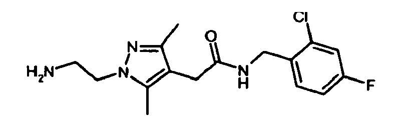 | 339 | 1.59 |
| | 2-[1-(2-aminoethyl)-3,5-dimethyl-1*H-*pyrazol-4-yl]-*N-*[(2-chloro-4-fluorophenyl)methyl]acetamide | | |

### Example 144 N-[(2-chloro-4-fluorophenyl)methyl]-2-{3,5-dimethyl-1-[2-(methyloxy)ethyl]-1H-pyrazol-4-yl}acetamide (E144)

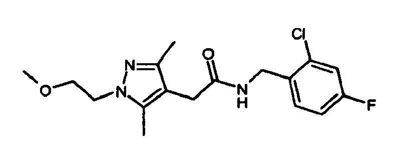

Crude 2-[4-(2-{[(2-chloro-4-fluorophenyl)methyl]amino}-2-oxoethyl)-3,5-dimethyl-1*H-*pyrazol-1-yl]ethyl trifluoromethanesulfonate (0.125 g, prepared as described above for E133) was dissolved in tetrahydrofuran (3 ml) and the mixture cooled to 0°C in an ice-bath. A 25% solution of sodium methoxide in methanol (1 ml) was then added to the mixture. The mixture was warmed to room temperature and stirred for 1 hr. The mixture was then diluted with ethyl acetate (5 ml) and washed sequentially with saturated aqueous sodium hydrogen carbonate (10 ml), water (10 ml), and saturated aqueous sodium chloride (10 ml), then dried over anhydrous magnesium sulphate and evaporated to give a gum. Purification of this residue by mass-directed automated HPLC gave *N-*[(2-chloro-4-fluorophenyl)methyl]-2-{3,5-dimethyl-1-[2-(methyloxy)ethyl]-1*H-*pyrazol-4-yl}acetamide (0.013 g) as a light brown solid after concentration of the combined product fractions and trituration with diethyl ether. LC/MS [M+H]⁺ = 354, retention time = 2.25 minutes.

### Example 145 N-[(2-chloro-4-fluorophenyl)methyl]-2-(3-methyl-1-phenyl-1H-pyrazol-4-yl)acetamide (E145)

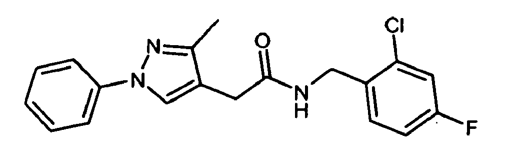

*N-*[(2-chloro-4-fluorophenyl)methyl]-2-(3-methyl-1-phenyl-1*H-*pyrazol-4-yl)acetamide **(E145)** was prepared in an analogous manner to that described above for example 48 but using (3-methyl-1-phenyl-1*H-*pyrazol-4-yl)acetic acid in the place of [1-(4-fluorophenyl)-3,5-dimethyl-1*H-*pyrazol-4-yl]acetic acid. LC/MS [M+H]⁺ = 358.2, retention time = 2.84 minutes.

The (3-methyl-1-phenyl-1*H-*pyrazol-4-yl)acetic acid used above was prepared from 3-methyl-1-phenyl-1*H-*pyrazole-4-carbaldehyde by the procedure outlined in Scheme 5 and using analogous methodology to that described in J.Heterocyclic Chem., 30, 997 (1993).

### Example 146 N-[(2-chloro-4-fluorophenyl)methyl]-2-[3,5-dimethyl-1-(2-methyl-4-pyridinyl)-1H-pyrazol-4-yl]acetamide (E146)

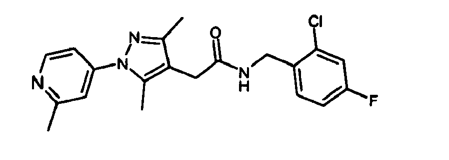

*N-*[(2-chloro-4-fluorophenyl)methyl]-2-[3,5-dimethyl-1-(2-methyl-4-pyridinyl)-1*H-*pyrazol-4-yl]acetamide **(E146)** was prepared in an analogous manner to that described above for example 48 but using [3,5-dimethyl-1-(2-methyl-4-pyridinyl)-1*H-*pyrazol-4-yl]acetic acid in the place of [1-(4-fluorophenyl)-3,5-dimethyl-1*H-*pyrazol-4-yl]acetic acid. LC/MS [M+H]⁺ = 387, retention time = 1.89 minutes.

The [3,5-dimethyl-1-(2-methyl-4-pyridinyl)-1*H-*pyrazol-4-yl]acetic acid used above was prepared by the following method:
(i) Palladium (II) acetate (0.022 g, 0.1 mmol) and Xantphos^{™} (0.064 g, 0.11 mmol) were suspended in toluene (1 ml) and the mixture was degassed with argon. Benzophenone hydrazone (0.393 g, 2.00 mmol), sodium t-butoxide (0.269 g, 2.80 mmol), and 4-Bromo-2-methylpyridine (0.344 g, 2.00 mmol) were then added to the mixture and the mixture was again degassed using argon. The mixture was heated at 80°C overnight under an atmosphere of argon, then cooled and loaded onto a 5g silica SPE cartridge. Elution with a 0-100% mixture of ethyl acetate in petroleum ether (60/80) followed by evaporation of solvent from the combined product-containing fractions gave diphenylmethanone (2-methyl-4-pyridinyl)hydrazone (0.505 g) which was used in the next step without further purification.
(ii) 1,1-dimethylethyl 3-acetyl-4-oxopentanoate (see J.Org.Chem., 65(18), 2000, 5806-5816)(0.313 g, 1.46 mmol), diphenylmethanone (2-methyl-4-pyridinyl)hydrazone (0.398 g, 1.39 mmol), and p-toluenesulphonic acid monohydrate (0.529 g, 2.78 mmol) were dissolved in ethanol and the mixture was heated at reflux temperature for -46 hrs. The mixture was allowed to cool and left to stand at room temperature for -48 hrs. The mixture was then diluted with dichloromethane (10 ml) and saturated aqueous sodium hydrogen carbonate (5 ml). The mixture was stirred for 5 minutes and then filtered through a hydrophobic frit washing the aqueous layer with a little more dichloromethane (∼2 ml). Evaporation of the solvent then gave the crude product as a yellow/brown oil. This was loaded onto a 10 g silica SPE cartridge using a minimum volume of dichloromethane and eluted with a 0-100% mixture of ethyl acetate in petroleum ether (60/80). Evaporation of the product-containing fractions then gave ethyl [3,5-dimethyl-1-(2-methyl-4-pyridinyl)-1*H-*pyrazol-4-yl]acetate (0.243 g) as a yellow oil.
(iii) ethyl [3,5-dimethyl-1-(2-methyl-4-pyridinyl)-1*H-*pyrazol-4-yl]acetate (0.243 g, 0.89 mmol) was dissolved in tetrahydrofuran (5 ml) and added to a solution of lithium hydroxide (0.064 g, 2.67 mmol) in water (2 ml). The mixture was heated at 50°C for 2.5 hrs and then cooled to room temperature. The mixture was then acidified to pH 1 by addition of 2N aqueous hydrogen chloride (1.4 ml) and then evaporated to give crude [3,5-dimethyl-1-(2-methyl-4-pyridinyl)-1*H-*pyrazol-4-yl]acetic acid as a yellow oil (0.390 g) which was used without further purification.

### Examples 147-151

In a manner analogous to that described for Example 146 above the compounds tabulated below (Table 7) were prepared by substituting the appropriate bromides, all of which are available from commercial sources or can be prepared using routes described previously in the chemical literature, for the 4-Bromo-2-methylpyridine used in the above procedure (step (i)).

**Table 7**

| Example no. | Chemical name | [M+H]⁺ | Retention time (mins) |
|---|---|---|---|
| E147 | 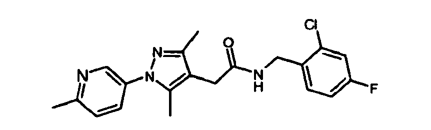 | 387 | 2.20 |
| | *N-*[(2-chloro-4-fluorophenyl)methyl]-2-[3,5-dimethyl-1-(6-methyl-3-pyridinyl)-1*H-*pyrazol-4-yl]acetamide | | |
| E148 | 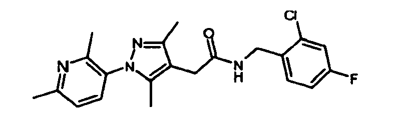 | 401 | 2.04 |
| | *N-*[(2-chloro-4-fluorophenyl)methyl]-2-[1-(2,6-dimethyl-3-pyridinyl)-3,5-dimethyl-1*H-*pyrazol-4-yl]acetamide | | |
| E149 | 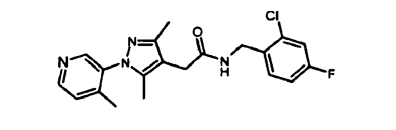 | 387 | 2.33 |
| | *N-*[(2-chloro-4-fluorophenyl)methyl]-2-[3,5-dimethyl-1-(4-methyl-3-pyridinyl)-1*H-*pyrazol-4-yl]acetamide | | |
| E150 | 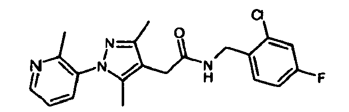 | 387 | 2.40 |
| | *N-*[(2-chloro-4-fluorophenyl)methyl]-2-[3,5-dimethyl-1-(2-methyl-3-pyridinyl)-1*H-*pyrazol-4-yl]acetamide | | |
| E151 | 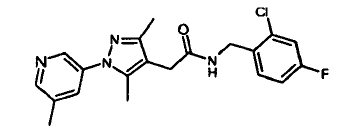 | 387 | 2.40 |
| | *N-*[(2-chloro-4-fluorophenyl)methyl]-2-[3,5-dimethyl-1-(5-methyl-3-pyridinyl)-1*H-*pyrazol-4-yl]acetamide | | |

### Example 152 N-[(2-chloro-4-fluorophenyl)methyl]-2-[3,5-dimethyl-1-(2-pyridinylmethyl)-1H-pyrazol-4-yl]acetamide (E152)

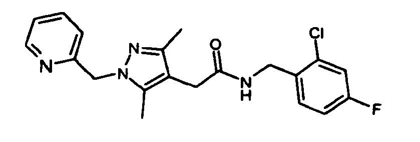

*N-*[(2-chloro-4-fluorophenyl)methyl]-2-[3,5-dimethyl-1-(2-pyridinylmethyl)-1*H-*pyrazol-4-yl]acetamide **(E152)** was prepared in an analogous manner to that described above for example 48 but using [3,5-dimethyl-1-(2-pyridinylmethyl)-1*H*-pyrazol-4-yl]acetic acid in the place of [1-(4-fluorophenyl)-3,5-dimethyl-1*H-*pyrazol-4-yl]acetic acid. LC/MS [M+H]⁺ = 387, retention time = 2.18 minutes.

The [3,5-dimethyl-1-(2-pyridinylmethyl)-1*H-*pyrazol-4-yl]acetic acid used above was prepared by the following method:
(i) 1,1-Dimethylethyl 3-acetyl-4-oxopentanoate (0.802 g, 3.75 mmol) was dissolved in ethanol (15 ml) and treated with hydrazine hydrate (0.218 ml, 4.5 mmol). The mixture was then stirred at room temperature for 24 hrs. The solvent was evaporated and the residue partitioned between dichloromethane (20 ml) and water (10 ml). The mixture was then filtered through a hydrophobic frit and then the organic phase was evaporated *in vacuo* to give 1,1-dimethylethyl (3,5-dimethyl-1*H-*pyrazol-4-yl)acetate (0.734 g) as a white waxy solid. This was used in the next step without further purification.
(ii) 1,1-Dimethylethyl (3,5-dimethyl-1*H-*pyrazol-4-yl)acetate (0.100 g, 0.478 mmol) was dissolved in dimethylformamide (5 ml) and treated with cesium carbonate (0.341 g, 1.05 mmol) and 2-(bromomethyl)pyridine hydrobromide (0.133 g, 0.526 mmol). The mixture was stirred at room temperature for 27 hrs and then treated with a further quantity of cesium carbonate (0.170 g, 0.526 mmol). Stirring was continued for a further 6 hrs at room temperature and then the mixture was evaporated *in vacuo.* The resulting residue was partitioned between ethyl acetate (40 ml) and water (30 ml). The aqueous layer was separated and extracted with ethyl acetate (2 x 30 ml) and then the combined organic layers were dried and the solvent evaporated to give a yellow gum. The gum was loaded onto a 10g silica SPE cartridge in a minimum of dichloromethane and then eluted with a 50-90% mixture of ethyl acetate in petroleum ether (40/60). The product-containing fractions were combined and evaporated to give partially purified 1,1-dimethylethyl [3,5-dimethyl-1-(2-pyridinylmethyl)-1-*H-*pyrazol-4-yl]acetate as a yellow gum (0.070 g). This was used in the next step without further purification.
(iii) 1,1-Dimethylethyl [3,5-dimethyl-1-(2-pyridinylmethyl)-1*H-*pyrazol-4-yl]acetate (0.070 g, 0.23 mmol) was dissolved in dichloromethane (2 ml) and treated with trifluoroacetic acid (0.7 ml). The mixture was stirred at room temperature for 4.5 hrs and then evaporated *in vacuo* to give 3,5-dimethyl-1-(2-pyridinylmethyl)-1*H-*pyrazol-4-yl]acetic acid as a yellow gum which was used without further purification. LC/MS [M+H]⁺ = 246, retention time = 1.36 minutes.

### Example 153 N-[(2-chloro-4-fluorophenyl)methyl]-2-{3,5-dimethyl-1-[4-(1-piperidinylcarbonyl)phenyl]-1H-pyrazol-4-yl}acetamide (E153)

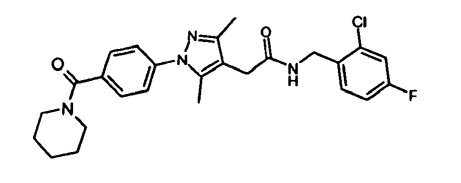

*N-*[(2-chloro-4-fluorophenyl)methyl]-2-{3,5-dimethyl-1-[4-(1-piperidinylcarbonyl)phenyl]-1*H-*pyrazol-4-yl}acetamide **(E153)** was prepared in an analogous manner to that described above for example 48 but using {3,5-dimethyl-1-[4-(1-piperidinylcarbonyl)phenyl]-1*H-*pyrazol-4-yl}acetic acid in the place of [1-(4-fluorophenyl)-3,5-dimethyl-1*H-*pyrazol-4-yl]acetic acid. LC/MS [M+H]⁺ = 483.3, retention time = 2.76 minutes.

The {3,5-dimethyl-1-[4-(1-piperidinylcarbonyl)phenyl]-1*H-*pyrazol-4-yl}acetic acid used above was prepared by the following method:
(i) Ethyl 3-acetyl-4-oxopentanoate (1.0 g, 5.37 mmol) was dissolved in ethanol (20 ml) and treated with 4-hydrazinobenzoic acid hydrochloride (1.01 g, 5.37 mmol) and sodium acetate (0.882 g, 10.74 mmol). The mixture was heated at reflux temperature for ~1.5 hrs then cooled to room temperature and evaporated. The residue was partitioned between 2N aqueous hydrogen chloride (20 ml) and ethyl acetate (20 ml) and the organic phase was separated and dried over anhydrous sodium sulphate. Filtration and evaporation of the solvents gave crude 4-{4-[2-(ethyloxy)-2-oxoethyl]-3,5-dimethyl-1*H-*pyrazol-1-yl}benzoic acid as a orange oil (1.84 g) which was used in the next step without further purification. LC/MS [M+H]⁺ = 302, retention time = 2.42 minutes.
(ii) Crude 4-{4-[2-(ethyloxy)-2-oxoethyl]-3,5-dimethyl-1*H*-pyrazol-1-yl}benzoic acid (0.100 g, 0.33 mmol) was dissolved in a mixture of dichloromethane (2 ml) and dimethylformamide (2 ml) and to this was added water soluble carbodiimide (0.082 g, 0.43 mmol), 1-hydroxybenzotriazole (0.058 g, 0.43 mmol), and *N-*ethyl morpholine (0.126 ml, 0.99 mmol). The mixture was stirred at room temperature for 15 minutes and then *N-*ethyl morpholine (0.126 ml, 0.99 mmol) and piperidine (0.034 g, 0.40 mmol) were added and the mixture was left to stir overnight at room temperature under an atmosphere of argon. Saturated aqueous sodium hydrogen carbonate (10 ml) was added to the mixture and stirring continued for 1 hr. Following this a further 10 ml of dichloromethane was added to the mixture and then the mixture was filtered through a phase separator and the organic phase was evaporated to give the crude product. The crude material was purified by flash column chromatography (eluting with a 7:3 mixture of ethyl acetate and petroleum ether (60/80)) to give the product, ethyl {3,5-dimethyl-1-[4-(1-piperidinylcarbonyl)phenyl]-1*H-*pyrazol-4-yl}acetate, as a yellow oil (0.097 g). LC/MS [M+H]⁺ = 370, retention time = 2.68 minutes.
(iii) Ethyl {3,5-dimethyl-1-[4-(1-piperidinylcarbonyl)phenyl]-1*H-*pyrazol-4-yl}acetate (0.097 g, 0.26 mmol) was dissolved in tetrahydrofuran (5 ml) and treated with a 0.2M solution of lithium hydroxide in water (3.9 ml). The mixture was stirred at 50°C for 2 hrs and then cooled to room temperature and acidified to pH 1 using 2M aqueous hydrogen chloride. The mixture was diluted with a 3:1 mixture of dichloromethane and isopropanol and then filtered through a hydrophobic frit. The organic phase was evaporated *in vacuo* and the residue triturated with acetonitrile to give {3,5-dimethyl-1-[4-(1-piperidinylcarbonyl)phenyl]-1*H-*pyrazol-4-yl}acetic acid (0.033 g) as a white solid after filtration and drying. This was used without further purification.

### Examples 154-162

In a manner analogous to that described for Example 153 above the compounds tabulated below (Table 8) were prepared by substituting the appropriate amines, all of which are available from commercial sources or can be prepared using routes described previously in the chemical literature, for the piperidine used in the above procedure (step (ii)).

**Table 8**

| Example no. | Chemical name | [M+H]⁺ | Retention time (mins) |
|---|---|---|---|
| E154 | 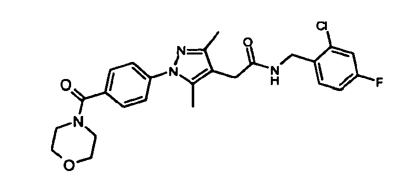 | 485.3 | 2.43 |
| | *N-*[(2-chloro-4-fluorophenyl)methyl]-2-{3,5-dimethyl-1-[4-(4-morpholinylcarbonyl)phenyl]-1*H-*pyrazol-4-yl}acetamide | | |
| E155 | 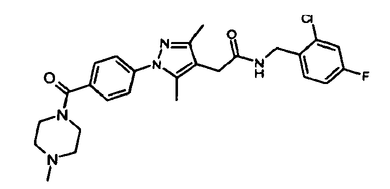 | 498.3 | 1.83 |
| | *N-*[(2-chloro-4-fluorophenyl)methyl]-2-(3,5-dimethyl-1-{4-[(4-methyl-1-piperazinyl)carbonyl]phenyl}-1*H-*pyrazol-4-yl)acetamide | | |
| E156 | 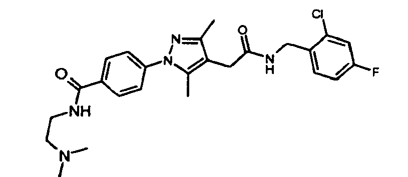 | 486.2 | 1.89 |
| | 4-[4-(2-{[(2-chloro-4-fluorophenyl)methyl]amino}-2-oxoethyl)-3,5-dimethyl-1*H-*pyrazol-1-yl]-*N-*[2-(dimethylamino)ethyl]benzamide | | |
| E157 | 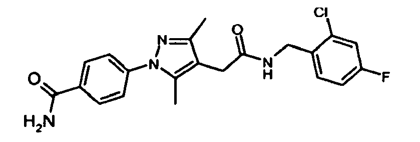 | 415 | 2.24 |
| | 4-[4-(2-{[(2-chloro-4-fluorophenyl)methyl]amino}-2-oxoethyl)-3,5-dimethyl-1*H-*pyrazol-1-yl]benzamide | | |
| E158 | 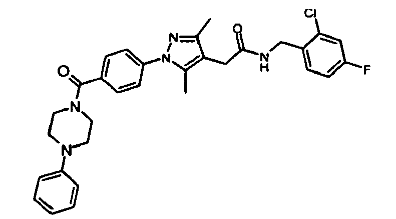 | 560.3 | 3.00 |
| | *N-*[(2-chloro-4-fluorophenyl)methyl]-2-(3,5-dimethyl-1-{4-[(4-phenyl-1-piperazinyl)carbonyl]phenyl}-1*H-*pyrazol-4-yl)acetamide | | |
| E159 | 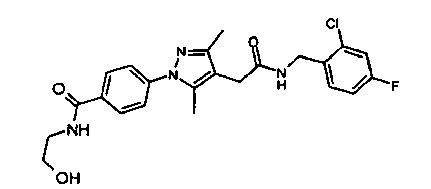 | 459.2 | 2.21 |
| | 4-[4-(2-{[(2-chloro-4-fluorophenyl)methyl]amino}-2-oxoethyl)-3,5-dimethyl-1*H-*pyrazol-1-yl]-*N-*(2-hydroxyethyl)benzamide | | |
| E160 | 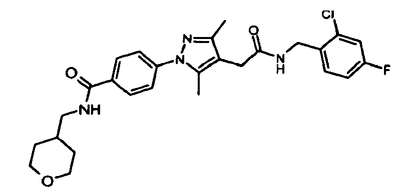 | 513.3 | 2.52 |
| | 4-[4-(2-{[(2-chloro-4-fluorophenyl)methyl]amino}-2-oxoethyl)-3,5-dimethyl-1*H-*pyrazol-1-yl]-*N-*(tetrahydro-2*H-*pyran-4-ylmethyl)benzamide | | |
| E161 | 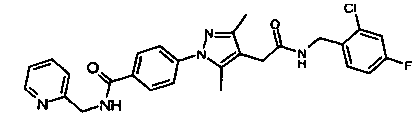 | 506.8 | 2.13 |
| | 4-[4-(2-{[(2-chloro-4-fluorophenyl)methyl]amino}-2-oxoethyl)-3,5-dimethyl-1*H-*pyrazol-1-yl]-*N-*(2-pyridinylmethyl)benzamide | | |
| E162 | 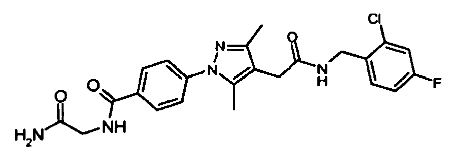 | 473.2 | 2.27 |
| | *N-*(2-amino-2-oxoethyl)-4-[4-(2-{[(2-chloro-4-fluorophenyl)methyl]amino}-2-oxoethyl)-3,5-dimethyl-1*H-*pyrazol-1-yl]benzamide | | |

### Example 163_N-[(3-chloro-2-methylphenyl)methyl]-2-[1-(2-hydroxyethyl)-3,5-dimethyl-1H-pyrazol-4-yl]acetamide (E163)

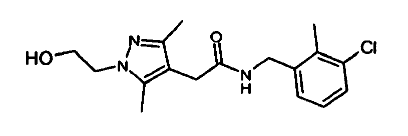

*N-*[(3-chloro-2-methylphenyl)methyl]-2-[1-(2-hydroxyethyl)-3,5-dimethyl-1*H-*pyrazol-4-yl]acetamide **(E163)** was prepared in an analogous manner to that described above for example 48 but using [1-(2-hydroxyethyl)-3,5-dimethyl-1*H-*pyrazol-4-yl]acetic acid in the place of [1-(4-fluorophenyl)-3,5-dimethyl-1*H-*pyrazol-4-yl]acetic acid. LC/MS [M+H]⁺ = 336, retention time = 2.11 minutes.

The [[1-(2-hydroxyethyl)-3,5-dimethyl-1*H-*pyrazol-4-yl]acetic acid used above was prepared by an analogous method to that described for E152 but using 2-hydrazinoethanol in the place of hydrazine hydrate.

### Example 164 N-[(3-chloro-2-methylphenyl)methyl]-2{3,5-dimethyl-1-[2-(4-morpholinyl)ethyl]-1H-pyrazol-4-yl}acetamide (E164)

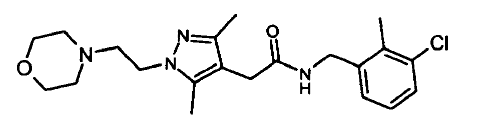

*N-*[(3-chloro-2-methylphenyl)methyl]-2-[1-(2-hydroxyethyl)-3,5-dimethyl-1*H-*pyrazol-4-yl]acetamide **(E163)** (0.050 g, 0.15 mmol) was dissolved in dry dichloromethane (5 ml), with a little warming, and treated with diisopropylethylamine (0.070 ml, 0.4 mmol). The mixture was cooled to -78°C using a cardice/acetone bath and then triflic anhydride (0.034 ml, 0.2 mmol) was added over 1 minute. The mixture was stirred for a further 15 minutes at -78°C and then treated with morpholine (0.035 ml, 0.4 mmol). After stirring at -78°C for a further 0.5 hr the mixture was warmed to room temperature and left to stand overnight. The mixture was diluted with dichloromethane (5 ml) and washed sequentially with 1 N aqueous sodium hydroxide (10 ml), water (2 x 10 ml) and saturated aqueous sodium chloride (10 ml). Filtration through a hydrophobic frit and evaporation gave a gum which was purified by mass-directed automated HPLC LC/MS. Evaporation of the collected product fractions and trituration of the residue with diethyl ether gave *N-*[(3-chloro-2-methylphenyl)methyl]-2-{3,5-dimethyl-1-[2-(4-morpholinyl)ethyl]-1*H*-pyrazol-4-yl}acetamide (0.031 g) as a white solid. [M+H]⁺ = 405, retention time = 1.75 minutes.

### Example 165 N-[(2-chloro-4-fluorophenyl)methyl]-2-[1-(2-hydroxy-2-methylpropyl)-3,5-dimethyl-1H-pyrazol-4-yl]acetamide (E165)

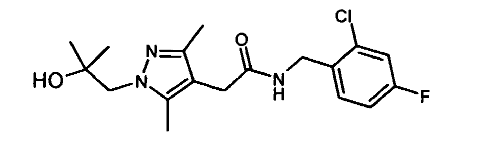

Crude [1-(2-hydroxy-2-methylpropyl)-3,5-dimethyl-1*H-*pyrazol-4-yl]acetic acid (0.040 g, 0.18 mmol, prepared as described below) was dissolved in dichloromethane (5 ml) and to this was added water soluble carbodiimide (0.044 g, 0.23 mmol), 1-hydroxybenzotriazole (0.031 g, 0.23 mmol), N-ethyl morpholine (0.068 ml, 0.53 mmol), and [(2-chloro-4-fluorophenyl)methyl]amine (0.029 ml, 0.23 mmol). The mixture was shaken in a shaker station for 18 hours and then diluted with dichloromethane and washed with saturated aqueous sodium hydrogen carbonate. The mixture was then filtered through a hydrophobic frit and the organic phase was evaporated to give the crude product. The crude material was purified by mass-directed automated HPLC to give the title compound as product as a white solid (0.021 g). LC/MS [M+H]⁺ = 368, retention time = 2.22 minutes.

[1-(2-hydroxy-2-methylpropyl)-3,5-dimethyl-1*H-*pyrazol-4-yl]acetic acid used in the procedure above was prepared by the following method:
(i) Acetyl acetone (5.0 g, 49.94 mmol) was dissolved in a mixture of dioxane (10 ml) and water (10 ml) and cooled in an ice-water bath. The mixture was then treated dropwise with a solution of potassium hydroxide (2.80 g, 49.94 mmol) in water (5 ml) over a 10 minute period to give a yellow solution. To this a solution of *tert*-butyl bromoacetate in dioxane (5 ml) was then added dropwise over a period of 20 minutes. The mixture was warmed to room temperature and then left to stir overnight under an atmosphere of argon. The organic layer was separated and the aqueous phase was acidified and further extracted with diethyl ether (2 x 20 ml). The combined organic layers were dried over anhydrous magnesium sulphate and the evaporated to give a yellow mobile oil (8.5 g). The oil was purified by flash column silica chromatography, eluting with 10-20% ethyl acetate in petrol, to give 5.0 g of 1,1-dimethylethyl 3-acetyl-4-oxopentanoate.
(ii) 1,1-dimethylethyl 3-acetyl-4-oxopentanoate (1.0 g, 4.7 mmol) was dissolved in ethanol (20 ml) and treated with hydrazine hydrate (0.273 ml, 5.6 mmol) and the mixture was stirred overnight at room temperature under an atmosphere of argon. The solvents were then evaporated and the residue partitioned between dichloromethane and water. The organic layer was collected by filtering through a hydrophobic frit and then evaporated to dryness to give 1,1-dimethylethyl (3,5-dimethyl-1*H-*pyrazol-4-yl)acetate (0.96 g) as a white crystalline solid. LC/MS [M+H]⁺ = 211, retention time = 1.93 minutes.
(iii) Isobutylene oxide (0.19 ml, 2.14 mmol) was added to a solution of 1,1-dimethylethyl (3,5-dimethyl-1*H*-pyrazol-4-yl)acetate (0.30 g, 1.43 mmol) and sodium hydride (60% in oil, 0.060 g, 1.5 mmol) in dimethylformamide (15 ml). The reaction mixture was heated at 50°C under argon for 2 hrs and then left stirring at room temperature overnight. The mixture was partitioned between ethyl acetate and water. The organic layer was separated, dried over anhydrous magnesium sulphate, filtered and then evaporated to dryness to give crude 1,1-dimethylethyl [1-(2-hydroxy-2-methylpropyl)-3,5-dimethyl-1*H-*pyrazol-4-yl]acetate (0.214 g) as a colourless oil which was used in the subsequent step without further purification. LC/MS [M+H]⁺ = 283, retention time = 2.35 minutes.
(iv) Trifluoroacetic acid (2 ml) was added to a solution of crude 1,1-dimethylethyl [1-(2-hydroxy-2-methylpropyl)-3,5-dimethyl-1*H-*pyrazol-4-yl]acetate (0.214 g, ~0.76 mmol) in dichloromethane (10 ml). The mixture was stirred at room temperature under argon for 18 hrs. The mixture was then evaporated to dryness to give crude [1-(2-hydroxy-2-methylpropyl)-3,5-dimethyl-1*H-*pyrazol-4-yl]acetic acid as a yellow oil/solid (0.383 g) which was used without further purification.

### Examples 166-168

In a manner analogous to that described for Example 165 above the compounds tabulated below (Table 9) were prepared by substituting the appropriate amines, all of which are available from commercial sources or can be prepared using routes described previously in the chemical literature, for the [(2-chloro-4-fluorophenyl)methyl]amine used in the above procedure.

**Table 9**

| Example no. | Chemical name | [M+H]⁺ | Retention time (mins) |
|---|---|---|---|
| E166 | 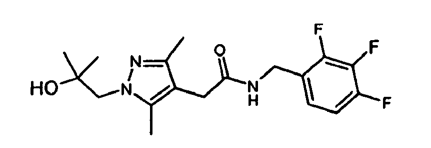 | 370 | 2.19 |
| | 2-[1-(2-hydroxy-2-methylpropyl)-3,5-dimethyl-1*H-*pyrazol-4-yl]-*N-*[(2,3,4-trifluorophenyl)methyl]acetamide | | |
| E167 | 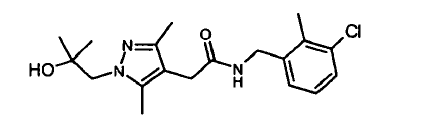 | 364 | 2.28 |
| | *N-*[(3-chloro-2-methylphenyl)methyl]-2-[1-(2-hydroxy-2-methylpropyl)-3,5-dimethyl-1*H-*pyrazol-4-yl]acetamide | | |
| E168 | 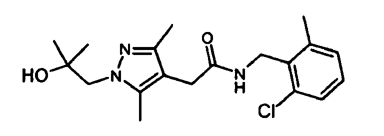 | 364 | 2.21 |
| | *N-*[(2-chloro-6-methylphenyl)methyl]-2-[1-(2-hydroxy-2-methylpropyl)-3,5-dimethyl-1*H-*pyrazol-4-yl]acetamide | | |

### Example 169 N-[(2-chloro-4-fluorophenyl)methyl]-2-{1-[6-(ethyloxy)-2-pyridinyl]-1H-pyrazol-4-yl}acetamide (E169)

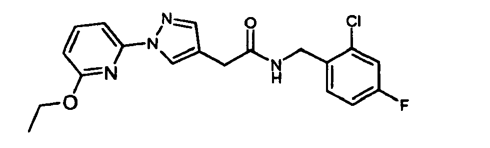

Crude *N-*[(2-chloro-4-fluorophenyl)methyl]-2-(1*H-*pyrazol-4-yl)acetamide (0.035 g, 0.131 mmol, prepared as described below) was dissolved in N-methyl pyrrolidinone (2 ml) and to this was added potassium carbonate (0.038 g, 0.27 mmol), *N,N*'-dimethyl-1,2-ethanediamine (0.003 ml, 0.026 mmol), 2-bromo-6-(ethyloxy)pyridine (0.079 g, 0.39 mmol), and finally copper iodide (0.0012 g, 0.0065 mmol). The mixture was heated at 180°C for 1 hr in a sealed tube in a microwave reactor. The mixture was loaded onto a 10 g C18 cartridge and the N-methyl pyrrolidinone was removed by washing with water. The cartridge was then eluted with methanol and the methanol was subsequently evaporated to give a dark brown gum. This crude material was purified by mass-directed automated HPLC to give the title compound as product as a beige solid (0.006 g). LC/MS [M+H]⁺ = 389, retention time =3.12 minutes.

*N-*[(2-chloro-4-fluorophenyl)methyl]-2-(1*H-*pyrazol-4-yl)acetamide used in the procedure above was prepared by the following method:
(i) [1-(Triphenylmethyl)-1*H-*pyrazol-4-yl]acetic acid (0.460 g, 1.25 mmol, which can be prepared as described in WO 95/07283) was dissolved in dichloromethane (30 ml) and to this was added water soluble carbodiimide (0.312 g, 1.63 mmol), 1-hydroxybenzotriazole (0.220 g, 1.63 mmol), N-ethyl morpholine (0.477 ml, 3.75 mmol), and [(2-chloro-4-fluorophenyl)methyl]amine (0.209 ml, 1.63 mmol). The mixture was stirred at room temperature under argon for 18 hours and then diluted with dichloromethane and washed with saturated aqueous sodium hydrogen carbonate. The mixture was then filtered through a hydrophobic frit and the organic phase was evaporated to give the crude product as a yellow oil. The crude material was purified by automated (Biotage SP4) flash-silica column chromatography, eluting with 0-80% ethyl acetate in hexane to give *N-*[(2-chloro-4-fluorophenyl)methyl]-2-[1-(triphenylmethyl)-1*H-*pyrazol-4-yl]acetamide as a white solid (0.330 g). LC/MS [M+H]⁺ = n.d., retention time = 3.64 minutes.
(ii) Trifluoroacetic acid (2 ml) was added to a solution of *N-*[(2-chloro-4-fluorophenyl)methyl]-2-[1-(triphenylmethyl)-1*H-*pyrazol-4-yl]acetamide (0.330 g, 0.65 mmol) in dichloromethane (15 ml). The reaction mixture was stirred at room temperature, under argon, for 18 hours. Water was then added to the mixture and the mixture was basified using solid potassium carbonate. The dichloromethane layer was separated and filtered through a hydrophobic frit. Evaporation of the solvent gave *N-*[(2-chloro-4-fluorophenyl)methyl]-2-(1*H-*pyrazol-4-yl)acetamide (0.265 g) as a white solid which was used without further purification. LC/MS [M+H]⁺ = 268, retention time = 1.99 minutes.

### Examples 170-172

In a manner analogous to that described for Example 146 above the compounds tabulated below (Table 10) were prepared by substituting the appropriate bromides, all of which are available from commercial sources or can be prepared using routes described previously in the chemical literature, for the 4-Bromo-2-methylpyridine used in the above procedure (Example 146, step (i)).

**Table 10**

| Example no. | Chemical name | [M+H]⁺ | Retention time (mins) |
|---|---|---|---|
| E170 | 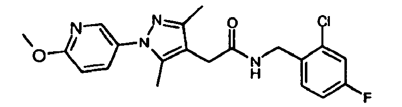 | 403 | 2.71 |
| | *N-*[(2-chloro-4-fluorophenyl)methyl]-2-{3,5-dimethyl-1-[6-(methyloxy)-3-pyridinyl]-1*H-*pyrazol-4-yl}acetamide | | |
| E171 | 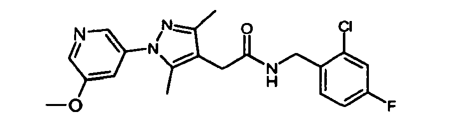 | 403 | 2.55 |
| | *N-*[(2-chloro-4-fluorophenyl)methyl]-2-{3,5-dimethyl-1-[5-(methyloxy)-3-pyridinyl]-1*H-*pyrazol-4-yl}acetamide | | |
| E172 | 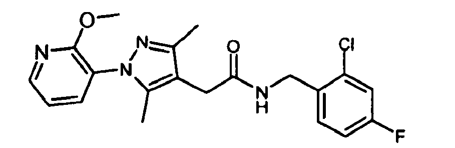 | 403 | 2.62 |
| | *N-*[(2-chloro-4-fluorophenyl)methyl]-2-{3,5-dimethyl-1-[2-(methyloxy)-3-pyridinyl]-1*H-*pyrazol-4-yl}acetamide | | |

### Example 173 N-[(2-chloro-4-fluorophenyl)methyl]-2-[1-(2-hydroxyethyl)-5-(trifluoromethyl)-1H-pyrazol-4-yl]acetamide (E173)

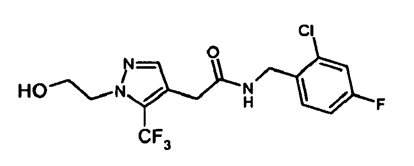

Crude [1-(2-hydroxyethyl)-5-(trifluoromethyl)-1*H-*pyrazol-4-yl]acetic acid (6.0 g, -6.52 mmol, prepared as described below) was suspended in dimethylformamide (8 ml) and to this was added water soluble carbodiimide (1.5 g, 7.82 mmol), 1-hydroxybenzotriazole (1.06 g, 7.82 mmol), N*-*ethyl morpholine (2.49 ml, 19.56 mmol), and [(2-chloro-4-fluorophenyl)methyl]amine (1.25 g, 7.82 mmol). The mixture was stirred at room temperature overnight and then partitioned between ethyl acetate (50 ml) and water (50 ml). The organic phase was separated and washed with more water (2 x 20 ml), dried over anhydrous sodium sulphate, filtered, and concentrated to an orange oil. This material was purified by automated flash-silica column chromatography (Biotage SP4), eluting with 0-100% ethyl acetate in hexane, to give *N-*[(2-chloro-4-fluorophenyl)methyl]-2-[1-(2-hydroxyethyl)-5-(trifluoromethyl)-1*H-*pyrazol-4-yl]acetamide as a sticky white solid (~1 g). LC/MS [M+H]⁺ = 381, retention time = 2.47 minutes.

[1-(2-hydroxyethyl)-5-(trifluoromethyl)-1*H-*pyrazol-4-yl]acetic acid used in the procedure above was prepared by the following method:
(i) Ethyl-3-(ethyloxy)-2-(trifluoroacetyl)-2-propenoate (2.0 g, 8.33 mmol) and 2-hydrazinoethanol (0.593 mL, 8.75 mmol) were dissolved in ethanol (25 ml) and heated for 2 hrs at 95°C. The mixture was then cooled to room temperature and left to stand overnight. The ethanol was evaporated and the resulting orange/red oil was purified by automated flash-silica column chromatography (Biotage SP4), eluting with 0-100% ethyl acetate in hexane, to give ethyl [1-(2-hydroxyethyl)-5-(trifluoromethyl)-1*H-*pyrazol-4-yl]acetate (0.685 g) and ethyl [1-(2-hydroxyethyl)-3-(trifluoromethyl)-1*H-*pyrazol-4-yl]acetate (0.845 g).
(ii) The [1-(2-hydroxyethyl)-5-(trifluoromethyl)-1*H-*pyrazol-4-yl]acetic acid used above was prepared from [1-(2-hydroxyethyl)-5-(trifluoromethyl)-1*H-*pyrazol-4-yl]acetate by the procedure outlined in Scheme 5 and using analogous methodology to that described in J.Heterocyclic Chem., 30, 997 (1993).

### Example 174 N-[(2-chloro-4-fluorophenyl)methyl]-2-[1-(2-hydroxyethyl)-3-(trifluoromethyl)-1 H-pyrazol-4-yl]acetamide (E174)

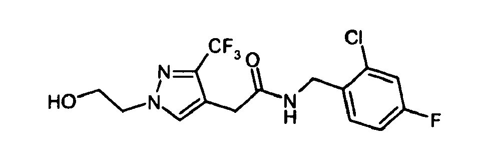

*N-*[(2-chloro-4-fluorophenyl)methyl]-2-[1-(2-hydroxyethyl)-3-(trifluoromethyl)-1*H-*pyrazol-4-yl]acetamide was prepared in an analogous method to that described for the preparation of *N-*[(2-chloro-4-fluorophenyl)methyl]-2-[1-(2-hydroxyethyl)-5-(trifluoromethyl)-1*H-*pyrazol-4-yl]acetamide **(E173)** but using [1-(2-hydroxyethyl)-3-(trifluoromethyl)-1 *H-*pyrazol-4-yl]acetic acid in the place of 1-(2-hydroxyethyl)-5-(trifluoromethyl)-1*H-*pyrazol-4-yl]acetic acid. LC/MS [M+H]⁺ = 380 retention time = 2.35 minutes.

[1-(2-hydroxyethyl)-3-(trifluoromethyl)-1*H-*pyrazol-4-yl]acetic acid was in turn prepared in an analogous manner to that described above for the synthesis of 1-(2-hydroxyethyl)-5-(trifluoromethyl)-1*H-*pyrazol-4-yl]acetic acid but using ethyl [1-(2-hydroxyethyl)-3-(trifluoromethyl)-1*H-*pyrazol-4-yl]acetate (synthesis described in Example 174, step (i)) in the place of [1-(2-hydroxyethyl)-5-(trifluoromethyl)-1*H-*pyrazol-4-yl]acetate.

### Example 175 N-[(2-chloro-4-fluorophenyl)methyl]-2-[1-{2-[(2-hydroxyethyl)amino]ethyl}-3-(trifluoromethyl)-1H-pyrazol-4-yl]acetamide (E175)

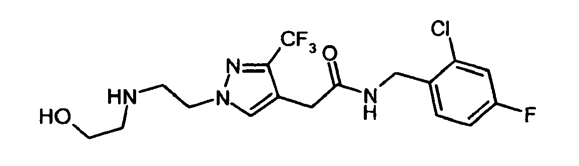

Crude 2-[4-(2-{[(2-chloro-4-fluorophenyl)methyl]amino}-2-oxoethyl)-3-(trifluoromethyl)-1*H-*pyrazol-1-yl]ethyl methanesulfonate (0.070 g, -0.13 mmol, prepared as described below) was dissolved in tetrahydrofuran (1 ml) and treated with 2-aminoethanol (0.012 ml, 0.19 mmol) and then heated at 100°C for 30 minutes in a sealed tube in a microwave reactor. A further aliquot of 2-aminoethanol was added (0.036 ml, 0.57 mmol) and heating at 110°C continued for 30 minutes. N-methyl pyrrolidinone (0.5 ml) was added to the mixture and heating continued for a further 30 mins at 160°C. The mixture was then cooled and the solvent evaporated and the crude residue was purified by mass-directed automated HPLC to give N-[(2-chloro-4-fluorophenyl)methyl]-2-[1-{2-[(2-hydroxyethyl)amino]ethyl}-3-(trifluoromethyl)-1H-pyrazol-4-yl]acetamide (0.029 g) after concentration of the combined product fractions. LC/MS [M+H]⁺ = 423, retention time = 1.74 minutes.

2-[4-(2-{[(2-chloro-4-fluorophenyl)methyl]amino}-2-oxoethyl)-3-(trifluoromethyl)-1*H-*pyrazol-1-yl]ethyl methanesulfonate used in the procedure above was prepared by the following method:
*N-*[(2-chloro-4-fluorophenyl)methyl]-2-[1-(2-hydroxyethyl)-3-(trifluoromethyl)-1*H-*pyrazol-4-yl]acetamide **(E174)** (0.100 g, 0.26 mmol) was dissolved in dry dichloromethane (9 ml) and cooled to 0°C using an ice bath. To this mixture was added diisopropylethylamine (0.131 ml, 0.78 mmol) followed by dropwise addition of a solution of mesyl chloride (0.030 ml, 0.39 mmol) in dichloromethane (1 ml). The mixture was stirred at 0°C for 10 minutes and then saturated aqueous sodium hydrogen carbonate (~5 ml) was added and the mixture allowed to warm to room temperature and stirred vigorously for 20 minutes. The dichloromethane layer was separated and washed with more water (∼5 ml) and then filtered through a hydrophobic frit and evaporated to give crude 2-[4-(2-{[(2-chloro-4-fluorophenyl)methyl]amino}-2-oxoethyl)-3-(trifluoromethyl)-1*H-*pyrazol-1-yl]ethyl methanesulfonate (0.142 g) as a yellow oil which was used without further purification.

### Example 176 N-[(2-chloro-4-fluorophenyl)methyl]-2-[1-[2-(tetrahydro-2H-pyran-4-ylamino)ethyl]-3-(trifluoromethyl)-1H-pyrazol-4-yl]acetamide (E176)

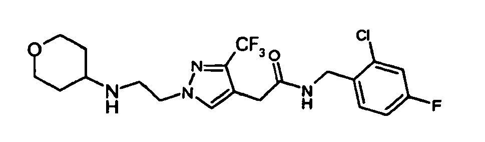

*N-*[(2-chloro-4-fluorophenyl)methyl]-2-[1-[2-(tetrahydro-2*H-*pyran-4-ylamino)ethyl]-3-(trifluoromethyl)-1*H-*pyrazol-4-yl]acetamide was prepared in an analogous method to that described for the preparation of *N-*[(2-chloro-4-fluorophenyl)methyl]-2-[1-(2-[(2-hydroxyethyl)amino]ethyl}-3-(trifluoromethyl)-1*H-*pyrazol-4-yl]acetamide **(E175)** but using tetrahydro-2*H-*pyran-4-ylamine in the place of 2-aminoethanol. LC/MS [M+H]⁺ = 463 retention time = 1.84 minutes.

### Example 177 N-[(2-chloro-4-fluorophenyl)methyl]-2-[1-[2-(dimethylamino)ethyl]-5-(trifluoromethyl)-1H-pyrazol-4-yl]acetamide hydrochloride (E177)

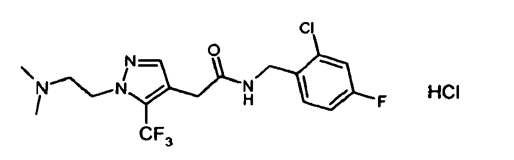

*N-*[(2-chloro-4-fluorophenyl)methyl]-2-[1-[2-(dimethylamino)ethyl]-5-(trifluoromethyl)-1*H-*pyrazol-4-yl]acetamide hydrochloride was prepared in an analogous method (with an additional hydrochloride salt forming step which involved treating the amine products with 1 M hydrogen chloride in ether and subsequent evaporation of the solvent) to that described for the preparation of *N-*[(2-chloro-4-fluorophenyl)methyl]-2-[1-{2-[(2-hydroxyethyl)amino]ethyl}-3-(trifluoromethyl)-1*H-*pyrazol-4-yl]acetamide **(E175)** but using dimethylamine (33% in ethanol) in the place of 2-aminoethanol and 2-[4-(2-{[(2-chloro-4-fluorophenyl)methyl]amino}-2-oxoethyl)-5-(trifluoromethyl)-1*H-*pyrazol-1-yl]ethyl methanesulfonate in the place of 2-[4-(2-{[(2-chloro-4-fluorophenyl)methyl]amino}-2-oxoethyl)-3-(trifluoromethyl)-1*H-*pyrazol-1-yl]ethyl methanesulfonate. LC/MS [M+H]⁺ = 407 retention time = 1.80 minutes.

2-[4-(2-{[(2-chloro-4-fluorophenyl)methyl]amino}-2-oxoethyl)-5-(trifluoromethyl)-1*H-*pyrazol-1-yl]ethyl methanesulfonate was in turn prepared in an analogous manner to that described above for the synthesis of 2-[4-(2-{[(2-chloro-4-fluorophenyl)methyl]amino}-2-oxoethyl)-3-(trifluoromethyl)-1*H-*pyrazol-1-yl]ethyl methanesulfonate but using *N-*[(2-chloro-4-fluorophenyl)methyl]-2-[1-(2-hydroxyethyl)-5-(trifluoromethyl)-1*H-*pyrazol-4-yl]acetamide **(E173)** in the place of *N-*[(2-chloro-4-fluorophenyl)methyl]-2-[1-(2-hydroxyethyl)-3-(trifluoromethyl)-1*H-*pyrazol-4-yl]acetamide **(E174).**

### Examples 178-180

In a manner analogous to that described for Example 177 above the compounds tabulated below (Table 11) were prepared by substituting the appropriate amines, all of which are available from commercial sources or can be prepared using routes described previously in the chemical literature, for the dimethylamine used in the above procedure.

**Table 11**

| Example no. | Chemical name | [M+H]⁺ | Retention time (mins) |
|---|---|---|---|
| E178 | 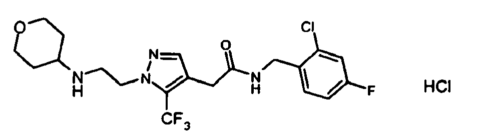 | 463 | 1.88 |
| | *N-*[(2-chloro-4-fluorophenyl)methyl]-2-[1-[2-(tetrahydro-2*H-*pyran-4-ylamino)ethyl]-5-(trifluoromethyl)-1*H-*pyrazol-4-yl]acetamide hydrochloride | | |
| E179 | 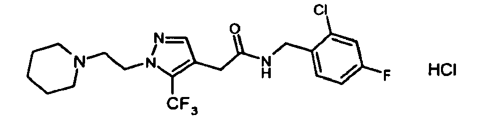 | 447 | 1.97 |
| | *N-*[(2-chloro-4-fluorophenyl)methyl]-2-[1-[2-(1-piperidinyl)ethyl]-5-(trifluoromethyl)-1*H-*pyrazol-4-yl]acetamide hydrochloride | | |
| E180 | 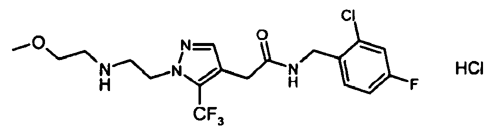 | 437 | 1.92 |
| | *N-*[(2-chloro-4-fluorophenyl)methyl]-2-[1-(2-{[2-(methyloxy)ethyl]amino}ethyl)-5-(trifluoromethyl)-1*H-*pyrazol-4-yl]acetamide hydrochloride | | |

### Example 181 2-[3,5-dimethyl-1-(2-pyrimidinyl)-1H-pyrazol-4-yl]-N-[(2,3,4-trifluorophenyl)methyl]acetamide (E181)

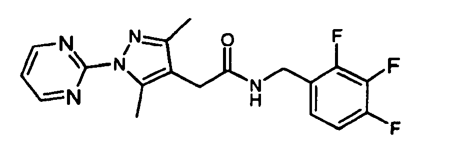

2-[3,5-dimethyl-1-(2-pyrimidinyl)-1*H-*pyrazol-4-yl]-*N-*[(2,3,4-trifluorophenyl)methyl]acetamide was prepared in an analogous method to that described for the preparation of *N-*[(2-chloro-4-fluorophenyl)methyl]-2-[1-(4-fluorophenyl)-3,5-dimethyl-1*H-*pyrazol-4-yl]acetamide (E48) but using [3,5-dimethyl-1-(2-pyrimidinyl)-1*H-*pyrazol-4-yl]acetic acid in the place of [1-(4-fluorophenyl)-3,5-dimethyl-1*H-*pyrazol-4-yl]acetic acid and [(2,3,4-trifluorophenyl)methyl]amine in the place of [(2-chloro-4-fluorophenyl)methyl]amine. LC/MS [M+H]⁺ = 376 retention time = 2.29 minutes.

[3,5-dimethyl-1-(2-pyrimidinyl)-1*H-*pyrazol-4-yl]acetic acid was obtained by hydrolysis of the corresponding ester ethyl [3,5-dimethyl-1-(2-pyrimidinyl)-1*H-*pyrazol-4-yl]acetate. Ethyl [3,5-dimethyl-1-(2-pyrimidinyl)-1*H-*pyrazol-4-yl]acetate was prepared by the reaction of ethyl 3-acetyl-4-oxopentanoate with 2(1H)-pyrimidinone hydrazone. Appropriate methodologies to prepare these or analgous compounds have been described previously (*e.g*. US 4, 146, 721).

### Example 182 2-[5-bromo-1-(4-fluorophenyl)-1H-pyrazol-4-yl]-N-[(2-chloro-4-fluorophenyl)methyl]acetamide (E182)

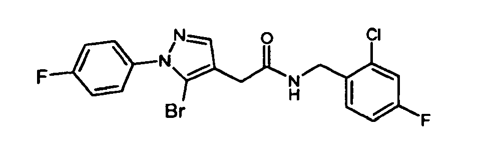

2-[5-bromo-1-(4-fluorophenyl)-1*H-*pyrazol-4-yl]-*N-*[(2-chloro-4-fluorophenyl)methyl]acetamide was prepared in an analogous method to that described for the preparation of *N-*[(2-chloro-4-fluorophenyl)methyl]-2-[1-(2-hydroxy-2-methylpropyl)-3,5-dimethyl-1*H-*pyrazol-4-yl]acetamide **(E165)** but using [5-bromo-1-(4-fluorophenyl)-1*H-*pyrazol-4-yl]acetic acid in the place of [1-(2-hydroxy-2-methylpropyl)-3,5-dimethyl-1*H-*pyrazol-4-yl]acetic acid.
LC/MS [M+H]⁺ = 442 retention time = 3.02 minutes.

[5-bromo-1-(4-fluorophenyl)-1*H-*pyrazol-4-yl]acetic acid used in the procedure above was prepared by the following method:
(i) Ethyl-2-cyano-3-(ethyloxy)-2-propenoate (5.0 g, 29.56 mmol), (4-fluorophenyl)hydrazine hydrochloride (4.81 g, 29.56 mmol) and sodium acetate (2.43 g, 29.56 mmol) were dissolved in ethanol (100 ml) and heated for 3.5 hrs at 95°C. The mixture was then cooled to room temperature and diluted with dichloromethane (∼150 ml) and water (50 ml). The organic layer was separated using a hydrophobic frit, dried over anhydrous sodium sulphate, filtered and to give ethyl 5-amino-1-(4-fluorophenyl)-1*H-*pyrazole-4-carboxylate (6.83 g) as an orange solid. This was used in the next step without further purification.
(ii) Ethyl 5-amino-1-(4-fluorophenyl)-1*H-*pyrazole-4-carboxylate (6.83 g, 27.40 mmol) was dissolved in chloroform (180 ml) and bromine (3.23 ml, 63.03 mmol) was added to give a dark red/brown solution. To this isoamyl nitrite (5.52 ml, 41.10 mmol) was added dropwise and the mixture was then stirred at room temperature for 1.5 hrs. The solvent was evaporated to give ethyl 5-bromo-1-(4-fluorophenyl)-1*H-*pyrazole-4-carboxylate (15.56 g) as an orange/brown oil which solidified on standing. This was used in the subsequent step without further purification.
(iii) The [5-bromo-1-(4-fluorophenyl)-1*H-*pyrazol-4-yl]acetic acid used above was then prepared from ethyl 5-bromo-1-(4-fluorophenyl)-1*H-*pyrazole-4-carboxylate by the procedure outlined in Scheme 4 and using analogous methodology to that described in J.Heterocyclic Chem., 30, 997 (1993).

### Example 183 N-[(2-chloro-4-fluorophenyl)methyl]-2-[5-ethyl-1-(4-fluorophenyl)-1H-pyrazol-4-yl]acetamide (E183)

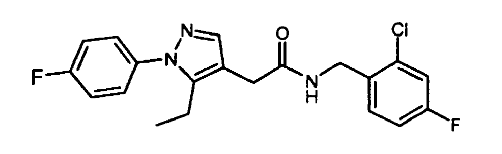

*N-*[(2-chloro-4-fluorophenyl)methyl]-2-[5-ethyl-1-(4-fluorophenyl)-1*H-*pyrazol-4-yl]acetamide was prepared in an analogous method to that described for the preparation of *N-*[(2-chloro-4-fluorophenyl)methyl]-2-[1-(2-hydroxy-2-methylpropyl)-3,5-dimethyl-1*H-*pyrazol-4-yl]acetamide **(E165)** but using [5-ethyl-1-(4-fluorophenyl)-1*H-*pyrazol-4-yl]acetic acid in the place of [1-(2-hydroxy-2-methylpropyl)-3,5-dimethyl-1 *H-*pyrazol-4-yl]acetic acid.
LC/MS [M+H]⁺ = 390 retention time = 2.93 minutes.

[5-ethyl-1-(4-fluorophenyl)-1*H-*pyrazol-4-yl]acetic acid used in the procedure above was prepared by the following method:
(i) [5-bromo-1-(4-fluorophenyl)-1*H-*pyrazol-4-yl]acetic acid (0.400 g, 13.37 mmol, prepared as described in example 182) was dissolved in ethanol (25 ml) and cooled to 0°C. Thionyl chloride (1.95 ml, 26.75 mmol) was then added slowly to the mixture. The mixture was warmed to room temperature and then heated at 60°C for -2 hrs. The solvent and thionyl chloride was evaporated to give a mobile red/orange oil. The crude oil was purified by automated flash-silica column chromatography (Biotage SP4), eluting with a 0-25% gradient of ethyl acetate in hexane, to give ethyl [5-bromo-1-(4-fluorophenyl)-1*H-*pyrazol-4-yl]acetate (0.292 g) as a pale yellow oil. This was used without further purification.
(ii) Ethyl [5-bromo-1-(4-fluorophenyl)-1*H-*pyrazol-4-yl]acetate (0.290 g, 0.89 mmol) was dissolved in dimethoxyethane (5 ml) and degassed with argon. Tetrakis(triphenylphosphine) palladium (0) (0.050 g, 0.04 mmol) was added to the mixture and the mixture was stirred for 10 minutes. A solution of vinylboronic anhydride pyridine complex (0.320 g, 1.33 mmol) in dimethoxyethane (5 ml) and water (3 ml) was then added to the mixture to give a dark red solution. Potassium carbonate (0.135 g, 0.98 mmol) was added and the mixture heated overnight under argon at 100°C (solution turned pale yellow). The mixture was partitioned between ethyl acetate (50 ml) and saturated aqueous sodium hydrogen carbonate (50 ml). The aqueous layer was collected and the pH was adjusted to -1 using 2N aqueous hydrogen chloride. The aqueous layer was then extracted with a mixture of chloroform and isopropanol (3:1 mixture, 3 x 30 ml). The combined organic layers were filtered through a hydrophobic frit and evaporated to give crude [5-ethenyl-1-(4-fluorophenyl)-1*H-*pyrazol-4-yl]acetic acid (0.192 g) as a colourless oil which was used in the subsequent step without further purification.
(iii) Crude [5-ethenyl-1-(4-fluorophenyl)-1*H-*pyrazol-4-yl]acetic acid (0.190 g, ~0.69 mmol) was dissolved in ethyl acetate (20 ml) and acetic acid (3 ml) and hydrogenated over 10% palladium on carbon (10%Pd/C cartridge, H-Cube, 1ml/min). Evaporation of the solvent gave crude [5-ethyl-1-(4-fluorophenyl)-1*H-*pyrazol-4-yl]acetic acid (0.170 g) as a pale yellow oil which was used without further purification.

### Microwave Reactor

Where indicated in the above examples, the microwave reactor used was a Biotage Initiator™. Reactions were carried out using normal power output unless specified otherwise.

### Mass-directed automated HPLC

Purification by mass-directed automated HPLC was carried out using the following apparatus and conditions:
Hardware
   Waters 2525 Binary Gradient Module
   Waters 515 Makeup Pump
   Waters Pump Control Module
   Waters 2767 Inject Collect
   Waters Column Fluidics Manager
   Waters 2996 Photodiode Array Detector
   Waters ZQ Mass Spectrometer
   Gilson 202 fraction collector
   Gilson Aspec waste collector
Software
   Waters MassLynx version 4 SP2
Column
   The columns used are Waters Atlantis, the dimensions of which are 19mm x 100mm (small scale) and 30mm x 100mm (large scale). The stationary phase particle size is 5µm.
Solvents
   A : Aqueous solvent = Water + 0.1% Formic Acid
   B : Organic solvent = Acetonitrile + 0.1 % Formic Acid
   Make up solvent = Methanol : Water 80:20
   Needle rinse solvent = Methanol
Methods

There are five methods used depending on the analytical retention time of the compound of interest. They have a 13.5-minute runtime, which comprises a 10-minute gradient followed by a 3.5 minute column flush and re-equilibration step.
Large/Small Scale 1.0-1.5 = 5-30% B
Large/Small Scale 1.5-2.2 = 15-55% B
Large/Small Scale 2.2-2.9 = 30-85% B
Large/Small Scale 2.9-3.6 = 50-99% B
Large/Small Scale 3.6-5.0 = 80-99% B (in 6 minutes followed by 7.5 minutes flush and re-equilibration)

### Flow rate

All of the above methods have a flow rate of either 20mls/min (Small Scale) or 40mls/min (Large Scale).

### Liquid Chromatography / Mass Spectrometry

Analysis of the above Examples by Liquid Chromatography / Mass Spectrometry (LC/MS) was carried out using the following apparatus and conditions:
Hardware
   Agilent 1100 Gradient Pump
   Agilent 1100 Autosampler
   Agilent 1100 DAD Detector
   Agilent 1100 Degasser
   Agilent 1100 Oven
   Agilent 1100 Controller
   Waters ZQ Mass Spectrometer
   Sedere Sedex 85
Software
   Waters MassLynx version 4.0 SP2
Column
   The column used is a Waters Atlantis, the dimensions of which are 4.6mm x 50mm. The stationary phase particle size is 3µm.
Solvents
   A : Aqueous solvent = Water + 0.05% Formic Acid
   B : Organic solvent = Acetonitrile + 0.05% Formic Acid

### Method

The generic method used has a 5 minute runtime.

| Time / min | %B |
|---|---|
| 0 | 3 |
| 0.1 | 3 |
| 4 | 97 |
| 4.8 | 97 |
| 4.9 | 3 |
| 5.0 | 3 |

The above method has a flow rate of 3ml/mins.

The injection volume for the generic method is 5ul.

The column temperature is 30deg.

The UV detection range is from 220 to 330nm.

### PHARMACOLOGICAL DATA

Compounds of the invention may be tested for *in vitro* biological activity at the P2X7 receptor in accordance with the following studies:

### Ethidium Accumulation Assay

Studies were performed using NaCl assay buffer of the following composition (in mM): 140mM NaCl, HEPES 10, *N-*methyl-D-glucamine 5, KCl 5.6, D-glucose 10, CaCl₂ 0.5 (pH 7.4). HEK293 cells, expressing human recombinant P2X7 receptors, were grown in poly-L-lysine pretreated 96 well plates for 18-24 h. (The cloning of the human P2X7 receptor is described in US 6,133,434). The cells were washed twice with 350µl of assay buffer before addition of 50µl of test compound. The cells were then incubated at room temperature (19-21°C) for 30 min before addition of ATP and ethidium (100µM final assay concentration). The ATP concentration was chosen to be close to the EC₈₀ for the receptor type and was 1 mM for studies on the human P2X7 receptor. Incubations were continued for 8 or 16 min and were terminated by addition of 25µl of 1.3M sucrose containing 5mM of the P2X7 receptor antagonist reactive black 5 (Aldrich). Cellular accumulation of ethidium was determined by measuring fluorescence (excitation wavelength of 530nm and emission wavelength of 620nm) from below the plate with a Canberra Packard Fluorocount (Pangbourne, UK) or a Flexstation.II (Molecular Devices). Antagonist plC₅₀ values for blocking ATP responses were determined using iterative curve fitting techniques.

### Fluorescent Imaging Plate Reader (FLIPR) Ca Assay

Studies were performed using NaCl assay buffer of the following composition (in mM) for human P2X7: 137 NaCl; 20 HEPES; 5.37 KCI; 4.17 NaHCO₃; 1 CaCl₂; 0.5 MgSO₄; and 1g/L of D-glucose (pH 7.4).

HEK293 cells, expressing human recombinant P2X7 receptors, were grown in poly-L-lysine pretreated 384 well plates for 42-48h. (The cloning of the human P2X7 receptor is described in US 6,133,434). The cells were washed three times with 80µl of assay buffer, loaded for 1 h at 37°C with 2µM Fluo4 (Teflabs), washed three times again, and left with 30µl buffer before the addition of 10 µl of 4x concentrated test compound. The cells were then incubated at room temperature for 30 mins before addition (online, by FLIPR384 or FLIPR3 instrument (Molecular Devices)) of Benzoylbenzoyl-ATP (BzATP) 60µM final assay concentration. The BzATP concentration was chosen to be close to the EC₈₀ for the receptor type. Incubations and reading were continued for 90sec, and intracellular calcium increase was determined by measuring fluorescence (excitation wavelength of 488nm and emission wavelength of 516nm) from below the plate, with FLIPR CCD camera. Antagonist plC₅₀ values for blocking BzATP responses were determined using iterative curve fitting techniques.

The compounds of Examples 1-183 were tested in the FLIPR Ca Assay and/or the Ethidium Accumulation Assay for human P2X7 receptor antagonist activity and found to have plC50 values > 4.7 in the FLIPR Ca Assay and/or pIC50 values > 5.5 in the Ethidium Accumulation Assay.

## Claims

1. A compound of formula (I), or a pharmaceutically acceptable salt thereof: 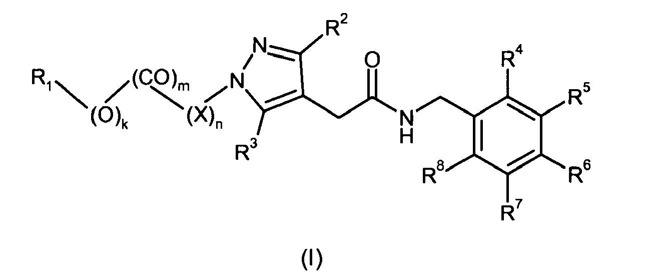 wherein:
R¹ represents NR⁹R¹⁰, C₁₋₆ alkyl, or trifluoromethyl; or may represent hydrogen when k is 1;
or R¹ represents a C₆₋₁₀ aryle, a 5 to 6 membered or an 8 to 10 membered heteroaryl,
a C₃₋₆ cycloalkyl or a 4 to 11 membered heterocyclyl; wherein any ring or ring system of said aryl, heteroaryl, cycloalkyl or heterocyclyl groups may be optionally substituted with 1 to 3 substituents, which may be the same or different, selected from the group consisting of halogen, hydroxyl, nitro, oxo, cyano, trifluoromethyl,
C₁₋₆ alkyl, C₁₋₃alkoxy, C₁₋₃ alkoxyC₁₋₃ alkyl-, -COC₁₋₃ alkyl, -CONR¹¹R¹² and -NR¹¹R¹²; or R¹ represents a group selected from formula (II), (III), (IV), (V), (VI) and (VII): 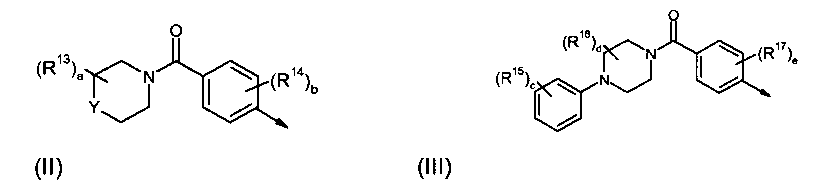 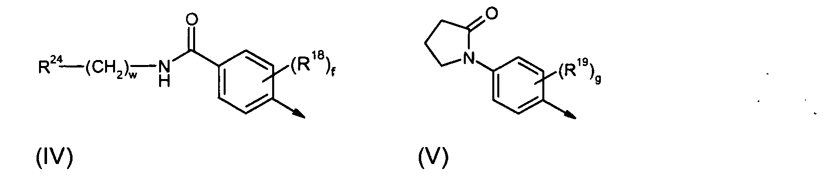 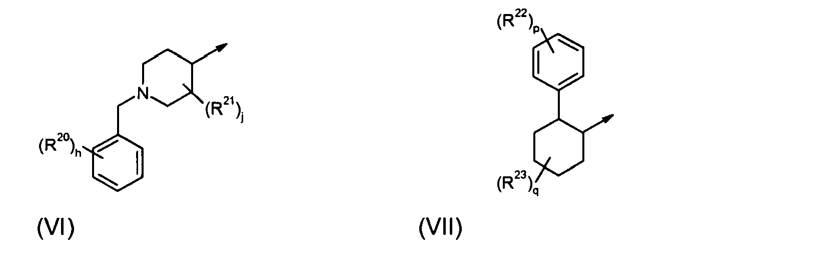
wherein:
Y represents CH₂, O or NR";
w represents 0, 1, 2 or 3;
a, b, c, d, e, f, g, h, j, p and q independently represent 0,1 or 2;
R¹³, R¹⁴, R¹⁵, R¹⁶, R¹⁷, R¹⁸, R¹⁹, R²⁰, R²¹, R²² and R²³ independently represent halogen, cyano, C₁₋₃ alkyl, C₁₋₃ alkoxy or trifluoromethyl;
R²⁴ represents NR¹¹R¹², -OH or-CONR¹¹R¹²;
or R²⁴ represents a C₃₋₆ cycloalkyl, a C₆₋₁₀ aryl, a 5 to 6 membered or an 8 to 10 membered heteroaryl, or a 4 to 11 membered heterocyclyl; wherein any ring or ring system of said aryl, heteroaryl, cycloalkyl or heterocyclyl groups may be optionally substituted with 1 to 3 substituents, which may be the same or different, selected from the group consisting of halogen, hydroxyl, nitro, oxo, cyano, trifluoromethyl, C₁₋₃ alkyl, C₁₋₃ alkoxy, C₁₋₃ alkoxyC₁₋₃ alkyl-, -COC₁₋₃ alkyl, -CONH₂ and -NR¹¹R¹²;
and wherein:
R² represents hydrogen, C₁₋₆ alkyl, C₁₋₄ alkoxy, trifluoromethyl, phenyl, a 5 to 6 membered monocyclic heteroaryl or a C₃₋₆ cycloalkyl;
R³ represents hydrogen, C₁₋₆ alkyl, C₁₋₄ alkoxy, trifluoromethyl, phenyl, a 5 to 6 membered monocyclic heteroaryl or C₃₋₆ cycloalkyl; or R³ represents halogen when R² is hydrogen;
R⁴ and R⁸ independently represent hydrogen, halogen, trifluoromethyl, C₁₋₆ alkyl, phenyl, phenyloxy, NO₂ or NH₂;
R⁵ and R⁷ independently represent hydrogen, halogen, trifluoromethyl, C₁₋₆ alkyl, phenyl, phenyloxy or NO₂;
R⁶ represents hydrogen, halogen, trifluoromethyl, trifluoromethoxy, C₁₋₆ alkyl,
C₃₋₆ cycloalkyl, phenyl, phenyloxy, NO₂ or cyano;
R⁹ and R¹⁰ independently represent hydrogen, C₁₋₄ alkyl, C₁₋₄ alkoxyC₁₋₄ alkyl-,
-C₁₋₄ alkylOH or-C₁₃ alkylNR¹¹R¹²;
or a C₃₋₆ cycloalkyl or a 6 membered heterocyclyl, either ring of which may be optionally substituted with 1 to 3 substituents, which may be the same or different, selected from the group consisting of halogen, cyano, C₁₋₃ alkyl, C₁₋₃ alkoxy and trifluoromethyl;
R¹¹ and R¹² independently represent hydrogen or C₁₋₃ alkyl;
k represents 0 or 1;
m represents 0 or 1;
n represents 0, 1, 2, 3 or 4;
X represents CR²⁵R²⁶; and
R²⁵ and R²⁶ at each occurrence independently represent hydrogen, fluorine or
C₁₋₆ alkyl;
with the proviso that the compound of formula (I) is other than 2-(3,5-dimethyl-1-phenyl-1*H*-pyrazol-4-yl)-*N*-(phenylmethyl)acetamide or 2-(3,5-dimethyl-1-phenyl-1*H* pyrazol-4-yl)-*N*-[(2-fluorophenyl)methyl]acetamide.

2. A compound or a pharmaceutically acceptable salt thereof, as claimed in claim 1, wherein R¹ represents NR⁹R¹⁰, C₁₋₆ alkyl or trifluoromethyl; or may represent hydrogen when k is 1; or R¹ represents a C₆₋₁₀ aryl, a 5 to 6 membered or an 8 to 10 membered heteroaryl, a C₃₋₆ cycloalkyl or a 4 to 11 membered heterocyclyl; wherein any ring or ring system of said aryl, heteroaryl, cycloalkyl or heterocyclyl groups may be optionally substituted with 1 to 3 substituents, which may be the same or different, selected from the group consisting of halogen, hydroxyl, nitro, oxo, cyano, trifluoromethyl, C₁₋₆ alkyl, C₁₋₃ alkoxy, C₁₋₃ alkoxyC₁₋₃ alkyl-, -COC₁₋₃ alkyl, -CONR¹¹R¹² and -NR¹¹R¹².

3. A compound or a pharmaceutically acceptable salt thereof, as claimed in claim 2, wherein R¹ represents NR⁹R¹⁰, C₁₋₆ alkyl or trifluoromethyl; or may represent hydrogen when k is 1; or R¹ represents a phenyl, a 5 to 6 membered or an 8 to 10 membered heteroaryl or a 4 to 11 membered heterocyclyl; wherein any ring or ring system of said phenyl, heteroaryl or heterocyclyl groups may be optionally substituted with 1 to 3 substituents, which may be the same or different, selected from the group consisting of halogen, C₁₋₆ alkyl, C₁₋₃ alkoxy, trifluoromethyl and - NR¹¹R¹².

4. A compound or a pharmaceutically acceptable salt thereof, as claimed in claim 2, wherein
R¹ represents NR⁹R¹⁰, C₁₋₆ alkyl or trifluoromethyl; or may represent hydrogen when k is 1; or
R¹ represents an optionally substituted group selected from phenyl, pyridinyl, pyrimidinyl, imidazolyl, pyrrolidinyl and pyrazolyl, wherein the optional substituents, which may be the same or different, are selected from the group consisting of halogen, C₁₋₆ alkyl, C₁₋₃ alkoxy, -CONH₂ and trifluoromethyl.

5. A compound or a pharmaceutically acceptable salt thereof, as claimed in claim 1, wherein R¹ represents a group selected from formula (II), (III), (IV), (V), (VI) and (VII), in which a, b, c, d, e, f, g, h, j, p and q represent 0.

6. A compound or a pharmaceutically acceptable salt thereof, as claimed in claim 1 or 5, wherein R²⁴ represents NH₂, -OH or -CONH₂; or an unsubstituted phenyl, pyridinyl or tetrahydropyranyl; and w represents 1 or 2.

7. A compound or a pharmaceutically acceptable salt thereof, as claimed in any one of the preceding claims, wherein R² and R³ independently represent hydrogen, C₁₋₆ alkyl or trifluoromethyl.

8. A compound or a pharmaceutically acceptable salt thereof, as claimed in claim 7, wherein both R² and R³ represent methyl.

9. A compound or a pharmaceutically acceptable salt thereof, as claimed in any one of the preceding claims, wherein R⁴, R⁵, R⁷ and R⁸ independently represent hydrogen, halogen or C₁₋₆ alkyl.

10. A compound or a pharmaceutically acceptable salt thereof, as claimed in any one of the preceding claims, wherein R⁶ represents hydrogen or halogen.

11. A compound or a pharmaceutically acceptable salt thereof, as claimed in any one of the preceding claims, wherein R⁴ and R⁸ independently represent hydrogen, halogen or methyl.

12. A compound or a pharmaceutically acceptable salt thereof, as claimed in claim 11, wherein one of R⁴ and R⁸ represents chlorine and the other represents hydrogen.

13. A compound or a pharmaceutically acceptable salt thereof, as claimed in claim 12, wherein one of R⁴ and R⁸ represents chlorine and the other represents hydrogen, and R⁶ represents fluorine.

14. A compound or a pharmaceutically acceptable salt thereof, as claimed in any one of the preceding claims, wherein R⁵ and R⁷ independently represent hydrogen, halogen or methyl.

15. A compound or a pharmaceutically acceptable salt thereof, as claimed in claim 14, wherein R⁵ and R⁷ represent hydrogen.

16. A compound or salt as claimed in any one of the preceding claims, which is a compound of formula (IA) or a pharmaceutically acceptable salt thereof: 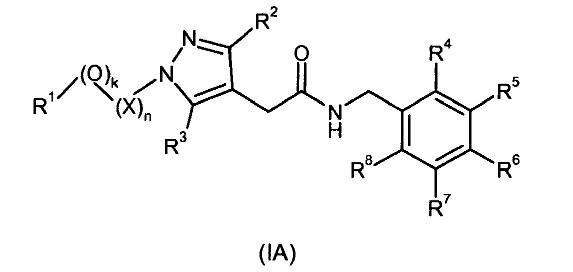 wherein:
R¹ represents NR⁹R¹⁰, C₁₋₆ alkyl or trifluoromethyl; or may represent hydrogen when k is 1;
or R¹ represents a C₆₋₁₀ aryl, a 5 to 6 membered or an 8 to 10 membered heteroaryl,
a C₃₋₆ cycloalkyl or a 4 to 11 membered heterocyclyl; wherein any ring or ring system of said aryl, heteroaryl, cycloalkyl or heterocyclyl groups may be optionally substituted with 1 to 3 substituents, which may be the same or different, selected from the group consisting of halogen, hydroxyl, nitro, oxo, cyano, trifluoromethyl, C₁₋₆ alkyl, C₁₋₃alkoxy, C₁₋₃ alkoxyC₁₋₃ alkyl-, -COC₁₋₃ alkyl, -CONR¹¹R¹² and -NR¹¹R¹²;
R² and R³ independently represent hydrogen, methyl or trifluoromethyl;
R⁴ and R⁸ independently represent hydrogen, halogen or methyl;
R⁵ and R⁷ represent hydrogen;
R⁶ represents halogen;
R⁹ and R¹⁰ independently represent hydrogen, C₁₋₄ alkyl, C₁₋₄ alkoxyC₁₋₄ alkyl- or -C₁₋₄ alkylOH;
R¹¹ and R¹² independently represent hydrogen or methyl;
k represents 0 or 1;
n represents 0, 1 or 2;
X represents CR²⁵R²⁶; and
R²⁵ and R²⁶ at each occurrence independently represent hydrogen or methyl;
with the proviso that the compound of formula (IA) is other than 2-(3,5-dimethyl-1-phenyl-1*H*-pyrazol-4-yl)-*N*-(phenylmethyl)acetamide or 2-(3,5-dimethyl-1-phenyl-1*H* pyrazol-4-yl)-*N*-[(2-fluorophenyl)methyl]acetamide.

17. A compound according to claim 1 or a pharmaceutically acceptable salt thereof, which is:
*N*-[(3,4-dichlorophenyl)methyl]-2-(3,5-dimethyl-1-phenyl-1*H*-pyrazol-4-yl)acetamide,
*N*-[(3,5-dichlorophenyl)methyl]-2-(3,5-dimethyl-1-phenyl-1*H*-pyrazol-4-yl)acetamide,
2-(3,5-dimethyl-1-phenyl-1*H*-pyrazol-4-yl)-*N*-({4-[(trifluoromethyl)oxy]phenyl}methyl)acetamide,
2-(3,5-dimethyl-1-phenyl-1*H*-pyrazol-4-yl)-*N*-{[2-fluoro-5-(trifluoromethyl)phenyl]methyl}acetamide,
2-(3,5-dimethyl-1-phenyl-1*H*-pyrazol-4-yl)-*N*-{[4-(trifluoromethyl)phenyl]methyl}acetamide,
2-(3,5-dimethy)-1-pheny)-1*H*-pyrazo)-4-yl)-*N*-{[4-fluoro-3-(trifluoromethyl)phenyl]methyl}acetamide,
*N*-[(3-chloro-4-fluorophenyl)methyl]-2-(3,5-dimethyl-1-phenyl-1*H*-pyrazol-4-yl)acetamide,
2-(3,5-dimethyl-1-phenyl-1*H*-pyrazol-4-yl)-*N*-{[3-fluoro-5-(trifluoromethyl)phenyl]methyl}acetamide,
*N*-[(2-chloro-4-fluorophenyl)methyl]-2-(3,5-dimethyl-1-phenyl-1*H*-pyrazol-4-yl)acetamide,
*N*-(2-biphenylylmethyl)-2-(3,5-dimethyl-1-phenyl-1*H*-pyrazol-4-yl)acetamide,
*N*-[(2,5-difluorophenyl)methyl]-2-(3,5-dimethyl-1-phenyl-1*H*-pyrazol-4-yl)acetamide,
*N*-[(3,4-difluorophenyl)methyl]-2-(3,5-dimethyl-1-phenyl-1*H*-pyrazol-4-yl)acetamide,
*N*-[(2-bromophenyl)methyl]-2-(3,5-dimethyl-1-phenyl-1*H*-pyrazol-4-yl)acetamide,
*N*-[(3-bromophenyl)methyl]-2-(3,5-dimethyl-1-phenyl-1*H*-pyrazol-4-yl)acetamide,
M-[(4-bromophenyl)methy)]-2-(3,5-dimethy)-1-phenyl-1*H*-pyrazol-4-yl)acetamide,
2-(3,5-dimethyl-1-phenyl-1*H*-pyrazol-4-yl)-*N*-[(2-nitrophenyl)methyl]acetamide,
2-(3,5-dimethyl-1-phenyl-1*H*-pyrazol-4-yl)-*N*-[(3-nitrophenyl)methyl]acetamide,
2-(3,5-dimethyl-1-phenyl-1*H*-pyrazol-4-yl)-*N*-[(4-nitrophenyl)methyl]acetamide,
*N*-[(3-chloro-2-methylphenyl)methyl]-2-(3,5-dimethyl-1-phenyl-1*H*-pyrazol-4-yl)acetamide,
2-(3,5-dimethyl-1-phenyl-1*H*-pyrazol-4-yl)-*N*-{[3-fluoro-4-(trifluoromethyl)phenyl]methyl}acetamide,
2-(3,5-dimethyl-1-phenyl-1*H*-pyrazol-4-yl)-*N*-[(2,3,4-trifluorophenyl)methyl]acetamide,
*N*-[(2,3-difluoro-4-methylphenyl)methyl]-2-(3,5-dimethyl-1-phenyl-1*H*-pyrazol-4-yl)acetamide,
*N*-[(2,5-dimethylphenyl)methyl]-2-(3,5-dimethyl-1-phenyl-1*H*-pyrazol-4-yl)acetamide,
*N*-[(5-chloro-2-methylphenyl)methyl]-2-(3,5-dimethyl-1-phenyl-1*H*-pyrazol-4-yl)acetamide,
*N*-[(2,6-difluorophenyl)methyl]-2-(3,5-dimethyl-1-phenyl-1*H*-pyrazol-4-yl)acetamide,
*N*-[(2-chloro-6-methylphenyl)methyl]-2-(3,5-dimethyl-1-phenyl-1*H*-pyrazol-4-yl)acetamide,
*N*-(4-biphenylylmethyl)-2-(3,5-dimethyl-1-phenyl-1*H*-pyrazol-4-yl)acetamide,
*N*-[(2,6-dimethylphenyl)methyl]-2-(3,5-dimethyl-1-phenyl-1*H*-pyrazol-4-yl)acetamide,
*N*-[(2-aminophenyl)methyl]-2-(3,5-dimethyl-1-phenyl-1-*H*-pyrazol-4-yl)acetamide,
*N*-(3-biphenylylmethyl)-2-(3,5-dimethyl-1-phenyl-1*H*-pyrazol-4-yl)acetamide, 2-(3,5-dimethyl-1-phenyl-1*H*-pyrazol-4-yl)-*N*-[(5-fluoro-2-methylphenyl)methyl]acetamide,
*N*-{[3,5-bis(trifluoromethyl)phenyl]methyl}-2-(3,5-dimethyl-1-phenyl-1*H*-pyrazol-4-yl)acetamide,
*N*-[(4-cyanophenyl)methyl]-2-(3,5-dimethyl-1-phenyl-1*H*-pyrazol-4-yl)acetamide,
*N-*[(2-chlorophenyl)methyl]-2-(3,5-dimethyl-1-phenyl-1*H*-pyrazol-4-yl)acetamide,
*N*-[(2,3-difluorophenyl)methyl]-2-(3,5-dimethyl-1-phenyl-1*H*-pyrazol-4-yl)acetamide,
*N*-{[2-chloro-6-(phenyloxy)phenyl]methyl}-2-(3,5-dimethyl-1-phenyl-1*H*-pyrazol-4-yl)acetamide,
*N*-[(2,3-dichlorophenyl)methyl]-2-(3,5-dimethyl-1-phenyl-1*H*-pyrazol-4-yl)acetamide,
2-(3,5-dimethyl-1-phenyl-1*H*-pyrazol-4-yl)-*N*-{[4-(phenyloxy)phenyl]methyl}acetamide,
*N*-[(2,4-dichlorophenyl)methyl]-2-(3,5-dimethyl-1-phenyl-1*H*-pyrazol-4-yl)acetamide,
*N-*[(2,3-dimethylphenyl)methyl]-2-(3,5-dimethyl-1-phenyl-1*H-*pyrazol-4-yl)acetamide,
*N*-[(2,6-dichlorophenyl)methyl]-2-(3,5-dimethyl-1-phenyl-1*H*-pyrazol-4-yl)acetamide,
*N*-[(2,5-dichlorophenyl)methyl]-2-(3,5-dimethyl-1-phenyl-1*H*-pyrazol-4-yl)acetamide,
*N*-[(2-chloro-6-fluorophenyl)methyl)-2-(3,5-dimethyl-1-phenyl-1*H*-pyrazol-4-yl)acetamide,
*N*-{[2-chloro-5-(trifluoromethyl)phenyl]methyl}-2-(3,5-dimethyl-1-phenyl-1*H*-pyrazol-4-yl)acetamide,
2-(3,5-dimethyl-1-phenyl-1*H*-pyrazol-4-yl)-*N*-{[5-fluoro-2-(trifluoromethyl)phenyl]methyl}acetamide,
2-(3,5-dimethyl-1-phenyl-1*H*-pyrazol-4-yl)-*N*-{[4-fluoro-2-(trifluoromethyl)phenyl]methyl}acetamide,
*N*-[(5-chloro-2-fluorophenyl)methyl]-2-(3,5-dimethyl-1-phenyl-1*H*-pyrazol-4-yl)acetamide,
*N*-[(2-chloro-4-fluorophenyl)methyl]-2-[1-(4-fluorophenyl)-3,5-dimethyl-1*H*-pyrazol-4-yl]acetamide,
*N*-[(2-chloro-4-fluorophenyl)methyl]-2-{1-[2-(dimethylamino)ethyl]-3,5-dimethyl-1*H-*pyrazol-4-yl}acetamide,
*N*-[(2-chloro-4-fluorophenyl)methyl]-2-[1-(1*H*-imidazol-4-ylmethyl)-3,5-dimethyl-1*H-*pyrazol-4-yl]acetamide,
*N*-[(2-chloro-4-fluorophenyl)methyl]-2-(1,3,5-trimethyl-1*H*-pyrazol-4-yl)acetamide, *N*-[(2-chloro-4-fluorophenyl)methyl]-2-[3,5-dimethyl-1-(phenylmethyl)-1*H*-pyrazol-4-yl]acetamide,
*N*-[(2-chloro-4-fluorophenyl)methyl]-2-[1-(2,4-difluorophenyl)-3,5-dimethyl-1*H* pyrazol-4-yl]acetamide,
*N*-[(2-chloro-4-fluorophenyl)methyl]-2-[1-(4-chlorophenyl)-3,5-dimethyl-1*H*-pyrazol-4-yl]acetamide,
*N*-[(2-chloro-4-fluorophenyl)methyl]-2-{3,5-dimethyl-1-[4-(methyloxy)phenyl]-1*H-*pyrazol-4-yl}acetamide,
*N*-[(2-chloro-4-fluorophenyl)methyl]-2-[1-(3-fluorophenyl)-3,5-dimethyl-1*H*-pyrazol-4-yl]acetamide,
*N* [(2-chloro-4-fluorophenyl)methyl]-2-[1-(3-chlorophenyl)-3,5-dimethyl-1*H* pyrazol-4-yl]acetamide,
*N-*[(2-chloro-4-fluorophenyl)methyl]-2-{3,5-dimethyl-1-[3-(methyloxy)phenyl]-1*H-*pyrazol-4-yl}acetamide,
*N*-[(2-chloro-4-fluorophenyl)methyl]-2-[1-(2-fluorophenyl)-3,5-dimethyl-1*H*-pyrazol-4-yl]acetamide,
*N*-((2-chloro-4-fluorophenyl)methyl]-2-[1-(2-chlorophenyl)-3,5-dimethyl-1*H*-pyrazol-4-yl]acetamide,
*N*-[(2-chloro-4-fluorophenyl)methyl]-2-{3,5-dimethyl-1-[2-(methyloxy)phenyl]-1*H-*pyrazol-4-yl}acetamide,
*N*-[(2-chloro-4-fluorophenyl)methyl]-2-[3,5-dimethyl-1-(4-methylphenyl)-1*H*-pyrazol-4-yl]acetamide,
*N*-[(2-chloro-4-fluorophenyl)methyl]-2-{1-[4-(ethyloxy)phenyl]-3,5-dimethyl-1*H-*pyrazol-4-yl}acetamide,
*N*-[(2-chloro-4-fluorophenyl)methyl]-2-[1-(5-cyano-2-pyridinyl)-3,5-dimethyl-1*H-*pyrazol-4-yl]acetamide,
*N*-[(2-chloro-4-fluorophenyl)methyl]-2-(1-(3,5-dichloro-2-pyridinyl)3,5-dimethyl-1*H-*pyrazol-4-yl]acetamide,
*N*-[(2-chloro-4-fluorophenyl)methyl]-2-[3,5-dimethyl-1-(4-pyridinyl)-1*H*-pyrazol-4-yl]acetamide,
*N*-[(2-chloro-4-fluorophenyl)methyl]-2-{3,5-dimethyl-1-(6-methyl-4-(trifluoromethyl)-2-pyridinyl]-1*H*-pyrazol-4-yl}acetamide,
2-[1-(5-bromo-2-pyridinyl)-3,5-dimethyl-1*H*-pyrazol-4-yl]-*N*-[(2-chloro-4-fluorophenyl)methyl]acetamide,
N-[(2-chloro-4-fluorophenyl)methyl]-2-[1-(5-chloro-2-pyridinyl)-3,5-dimethyl-1*H-*pyrazol-4-yl]acetamide,
*N*-[(2-chloro-4-fluorophenyl)methyl]-2-[3,5-dimethyl-1-(1,3-thiazol-2-yl)-1*H*-pyrazol-4-yl]acetamide,
*N*-[(2-chloro-4-fluorophenyl)methyl]-2-{3,5-dimethyl-1-[5-(trifluoromethyl)-2-pyridinyl]-1*H*-pyrazol-4-yl}acetamide,
*N*-[(2-chloro-4-fluorophenyl)methyl]-2-{3,5-dimethyl-1-[3-(trifluoromethyl)-2-pyridinyl]-1*H*-pyrazol-4-yl}acetamide,
*N*-[(2-chloro-4-fluorophenyl)methyl]-2-[3,5-dimethyl-1-(3-pyridinyl)-1*H*-pyrazol-4-yl]acetamide,
*N*-[(2-chloro-4-fluorophenyl)methyl]-2-[1-(1,1-dimethylethyl)-3,5-dimethyl-1*H*-pyrazol-4-yl]acetamide,
*N*-[(2-chloro-4-fluorophenyl)methyl]-2-[3,5-dimethyl-1-(2-pyridinyl)-1*H*-pyrazol-4-yl]acetamide,
*N*-[(2-chloro-4-fluorophenyl)methyl]-2-[1-(4-iodophenyl)-3,5-dimethyl-1*H*-pyrazol-4-yl]acetamide,
*N*-[(2-chloro-4-fluorophenyl)methyl]-2-[3,5-dimethyl-1-(4-pyridinylmethyl)-1*H*-pyrazol-4-yl]acetamide,
*N*-[(2-chloro-4-fluorophenyl)methyl]-2-[3,5-dimethyl-1-(6-methyl-3-pyridazinyl)-1*H-*pyrazol-4-yl]acetamide,
*N*-[(2-chloro-4-fluorophenyl)methyl]-2-[3,5-dimethyl-1-(2-pyrimidinyl)-1*H*-pyrazol-4-yl]acetamide,
*N*-[(2-chloro-4-fluorophenyl)methyl]-2-[1-(2,6-dimethyl-4-pyrimidinyl)-3,5-dimethyl-1*H-*pyrazol-4-yl]acetamide,
*N*-[(2-chloro-4-fluorophenyl)methyl]-2-{3,5-dimethyl-1-[4-(trifluoromethyl)-2-pyrimidinyl]-1*H*-pyrazol-4-yl}acetamide,
*N-[*(2-chloro-4-fluorophenyl)methyl]-2-[3,5-dimethyl-1-(2-phenylethyl)-1*H*-pyrazol-4-yl]acetamide,
*N*-[(2-chloro-4-fluorophenyl)methyl]-2-(1-cyclohexyl-3,5-dimethyl-1*H*-pyrazol-4-yl)acetamide,
*N*-[(2-chloro-4-fluorophenyl)methyl]-2-{1-[(2-chlorophenyl)methyl]-3,5-dimethyl-1*H-*pyrazol-4-yl}acetamide,
*N*-[(2-chloro-4-fluorophenyl)methyl]-2-[3,5-dimethyl-1-(2-methylpropyl)-1*H*-pyrazol-4-yl]acetamide,
*N*-[(2-chloro-4-fluorophenyl)methyl]-2-(1-cyclopentyl-3,5-dimethyl-1*H*-pyrazol-4-yl)acetamide,
*N*-[(2-chloro-4-fluorophenyl)methyl]-2-[3,5-dimethyl-1-(tetrahydro-2*H* pyran-4-yl)-1*H-*pyrazol-4-yl]acetamide,
*N*-[(2-chloro-4-fluorophenyl)methyl]-2-{1-[(2,4-difluorophenyl)methyl]-3,5-dimethyl-1*H*-pyrazol-4-yl}acetamide,
*N*-[(2-chloro-4-fluorophenyl)methyl]-2-{1-[(4-chlorophenyl)methyl]-3,5-dimethyl-1*H-*pyrazol-4-yl}acetamide,
*N*-[(2-chloro-4-fluorophenyl)methyl]-2-(3,5-dimethyl-1-{[4-(methyloxy)phenyl]methyl}-1*H*-pyrazol-4-yl)acetamide,
*N*-[(2-chloro-4-fluorophenyl)methyl]-2-f 3,5-dimethyl-1-[1-(phenylmethyl)-4-piperidinyl]-1*H*-pyrazol-4-yl}acetamide,
*N*-[(2-chloro-4-fluorophenyl)methyl]-2-(3,5-dimethyl-1-{[3-(methyloxy)phenyl]methyl}-1*H*-pyrazol-4-yl)acetamide,
*N*-[(2-chloro-4-fluorophenyl)methyl]-2-(3,5-dimethyl-1-{[2-(methyloxy)phenyl]methyl}-1 *H*-pyrazol-4-yl)acetamide,
*N*-[(2-chloro-4-fluorophenyl)methyl]-2-[1-(2,2-dimethylpropyl)-3,5-dimethyl-1*H-*pyrazol-4-yl]acetamide,
*N*-[(2-chloro-4-fluorophenyl)methyl]-2-(1-{[2-(dimethylamino)phenyl]methyl}-3,5-dimethyl-1*H*-pyrazol-4-yl)acetamide,
*N*-[(2-chloro-4-fluorophenyl)methyl]-2-[3,5-dimethyl-1-(1-methyl-4-piperidinyl)-1*H-*pyrazol-4-yl]acetamide,
2-[1-(1-azabicyclo[2.2.1]hept-3-yl)-3,5-dimethyl-1*H*-pyrazol-4-yl]-*N*-[(2-chloro-4-fluorophenyl)methyl]acetamide,
*N*-[(2-chloro-4-fluorophenyl)methyl]-2-(3,5-dimethyl-1-{1-m ethyl-2-[4-(methyloxy)phenyl]ethyl}-1*H*-pyrazol-4-yl)acetamide,
*N*-[(2-chloro-4-fluorophenyl)methyl]-2-[3,5-dimethyl-1-(2,2,2-trifluoroethyl)-1*H-*pyrazol-4-yl]acetamide,
*N*-[(2-chloro-4-fluorophenyl)methyl]-2-(1-{2-[(2,6-dimethylphenyl)oxy]ethyl}-3,5-dimethyl-1*H*-pyrazol-4-yl)acetamide,
2-[1-(1,3-benzothiazol-2-ylmethyl)-3,5-dimethyl-1*H*-pyrazol-4-yl]-*N*-[(2-chloro-4-fluorophenyl)methyl]acetamide,
*N*-[(2-chloro-4-fluorophenyl)methyl]-2-[3,5-dimethyl-1-(2-phenylcyclohexyl)-1*H-*pyrazol-4-yl]acetamide,
*N*-[(2-chloro-4-fluorophenyl)methyl]-2-{3,5-dimethyl-1-[(4-methylphenyl)methyl]-1*H* pyrazol-4-yl}acetamide,
2-(1-{[3,4-bis(methyloxy)phenyl]methyl}-3,5-dimethyl-1*H*-pyrazol-4-yl)-*N*-[(2-chloro-4-fluorophenyl)methyl]acetamide,
*N*-[(2-chloro-4-fluorophenyl)methyl]-2-[3,5-dimethyl-1-(1-methylethyl)-1*H*-pyrazol-4-yl]acetamide,
*N*-[(2-chloro-4-fluorophenyl)methyl]-2-{3,5-dimethyl-1-[3-(phenyloxy)propyl]-1*H-*pyrazol-4-yl}acetamide,
*N*-[(2-chloro-4-fluorophenyl)methyl]-2-[1-(cyclohexylmethyl)-3,5-dimethyl-1*H*-pyrazol-4-yl]acetamide,
*N*-[(2-chloro-4-fluorophenyl)methyl]-2-(1-ethyl-3,5-dimethyl-1*H*-pyrazol-4-yl)acetamide,
*N*-[(2-chloro-4-fluorophenyl)methyl]-2-{3,5-dimethyl-1-[2-(phenyloxy)ethyl]-1*H-*pyrazol-4-yl}acetamide,
*N*-[(2-chloro-4-fluorophenyl)methyl]-2-[3,5-dimethyl-1-(1-methyl-2-phenylethyl)-1*H-*pyrazol-4-yl]acetam ide,
*N*-[(2-chloro-4-fluorophenyl)methyl]-2-(1-{2-[(4-chloro-3-methylphenyl)oxy]ethyl}-3,5-dimethyl-1 *H*-pyrazol-4-yl)acetamide,
*N*-[(2-chloro-4-fluorophenyl)methyl]-2-{3,5-dimethyl-1-[4-(2-oxo-1-pyrrolidinyl)phenyl]-1*H*-pyrazol-4-yl}acetamide,
*N*-[(2-chloro-4-fluorophenyl)methyl]-2-[1-(4-fluorophenyl)-1*H*-pyrazol-4-yl]acetamide,
*N*-[(2-chloro-4-fluorophenyl)methyl]-2-[1-(4-fluorophenyl)-5-(trifluoromethyl)-1*H-*pyrazol-4-yl]acetamide,
*N*-[(2-chloro-4-fluorophenyl)methyl]-2-[1-(2-methylphenyl)-1*H*-pyrazol-4-yl]acetamide,
*N*-[(2-chloro-4-fluorophenyl)methyl]-2-[1-(2-ethylphenyl)-1*H*-pyrazol-4-yl]acetamide,
*N*-[(2-chloro-4-fluorophenyl)methyl]-2-{3,5-dimethyl-1-[4-(trifluoromethyl)phenyl]-1*H-*pyrazol-4-yl}acetamide,
*N*-[(2-chloro-4-fluorophenyl)methyl]-2-[3,5-dimethyl-1-(3-pyridinylmethyl)-1*H*-pyrazol-4-yl]acetamide,
*N*-[(2-chloro-4-fluorophenyl)methyl]-2-(5-methyl-1-phenyl-1*H*-pyrazol-4-yl)acetamide,
2-(3-butyl-5-methyl-1-phenyl-1*H*-pyrazol-4-yl)-*N*-[(2-chloro-4-fluorophenyl)methyl]acetamide,
*N*-[(2-chloro-4-fluorophenyl)methyl]-2-[3-methyl-5-(1-methylethyl)-1-phenyl-1*H-*pyrazol-4-yl]acetamide,
*N*-[(2-chloro-4-fluorophenyl)methyl]-2-[5-methyl-3-(1-methylethyl)-1-phenyl-1*H-*pyrazol-4-yl]acetamide,
*N*-[(2-chloro-4-fluorophenyl)methyl]-2-(3-methyl-1,5-diphenyl-1*H*-pyrazol-4-yl)acetamide,
*N*-[(2-chloro-4-fluorophenyl)methyl]-2-[1-(4-fluorophenyl)-5-methyl-1*H*-pyrazol-4-yl]acetamide,
Ethyl [4-(2-{[(2-chloro-4-fluorophenyl)methyl]amino}-2-oxoethyl)-3,5-dimethyl-1*H-*pyrazol-1-yl]acetate,
[4-(2-{[(2-chloro-4-fluorophenyl)methyl]amino}-2-oxoethyl)-3,5-dimethyl-1*H*-pyrazol-1-yl]acetic acid,
*N*-[(2-chloro-4-fluorophenyl)methyl]-2-{3,5-dimethyl-1-[2-(4-morpholinyl)-2-oxoethyl]-1*H*-pyrazol-4-yl}acetamide,
*N*-[(2-chloro-4-fluorophenyl)methyl]-2-{3,5-dimethyl-1-[2-oxo-2-(1-piperidinyl)ethyl]-1*H*-pyrazol-4-yl}acetamide,
2-[4-(2-{[(2-chloro-4-fluorophenyl)methyl]amino}-2-oxoethyl)-3,5-dimethyl-1*H*-pyrazol-1-yl]-*N*,*N*-diethylacetamide,
*N*-[(2-chloro-4-fluorophenyl)methyl]-2-{3,5-dimethyl-1-[2-(4-methyl-1-piperazinyl)-2-oxoethyl]-1*H*-pyrazol-4-yl}acetamide,
2-[4-(2-{[(2-chloro-4-fluorophenyl)methyl]amino}-2-oxoethylr3,5-dimethyl-1*H*-pyrazol-1-yl]-*N*-[2-(dimethylamino)ethyl]acetamide,
*N*-[(2-chloro-4-fluorophenyl)methyl]-2-[1-(2-hydroxyethyl)-3,5-dimethyl-1*H*-pyrazol-4-yl]acetamide,
*N*-[(2-chloro-4-fluorophenyl)methyl]-2-{3,5-dimethyl-1-[2-(4-morpholinyl)ethyl]-1*H-*pyrazol-4-yl}acetamide,
*N*-[(2-chloro-4-fluorophenyl)methyl]-2-{1-[2-(cyclohexylamino)ethyl]-3,5-dimethyl-1*H-*pyrazol-4-yl}acetamide,
*N*-[(2-chloro-4-fluorophenyl)methyl]-2-{1-[2-(diethylamino)ethyl]-3,5-dimethyl-1*H-*pyrazol-4-yl}acetamide,
*N*-[(2-chloro-4-fluorophenyl)methyl]-2-{3,5-dimethyl-1-[2-(tetrahydro-2*H*-pyran-4-ylamino)ethyl]-1*H*-pyrazol-4-yl}acetamide,
*N*-[(2-chloro-4-fluorophenyl)methyl]-2-{3,5-dimethyl-1-[2-(1-piperidinyl)ethyl]-1*H-*pyrazol-4-yl}acetamide,
*N*-[(2-chloro-4-fluorophenyl)methyl]-2-{3,5-dimethyl-1-[2-(1-pyrrolidinyl)ethyl]-1*H* pyrazol-4-yl}acetamide,
*N*-[(2-chloro-4-fluorophenyl)methyl]-2-(3,5-dimethyl-1-{2-[(2-methyl propyl)amino]ethyl}-1*H*-pyrazol-4-yl)acetamide,
*N*-[(2-chloro-4-fluorophenyl)methyl]-2-(1-{2-[(2-hydroxyethyl)amino]ethyl}-3,5-dimethyl-1*H*-pyrazol-4-yl)acetamide,
*N*-[(2-chloro-4-fluorophenyl)methyl]-2-(1-{2-[(2-hydroxyethyl)(methyl)a mino]ethyl}-3,5-dimethyl-1*H*-pyrazol-4-yl)acetamide,
*N*-[(2-chloro-4-fluorophenyl)methyl]-2-[3,5-dimethyl-1-(2-{[2-(methyloxy)ethyl]amino}ethyl)-1*H*-pyrazol-4-yl]acetamide,
2-[1-(2-aminoethyl)-3,5-dimethyl-1*H*-pyrazol-4-yl]-*N*-[(2-chloro-4-fluorophenyl)methyl]acetamide,
*N*-[(2-chloro-4-fluorophenyl)methyl]-2-{3,5-dimethyl-1-[2-(methyloxy)ethyl]-1*H-*pyrazol-4-yl}acetamide,
*N*-[(2-ch loro-4-fluorophenyl)methyl]-2-(3-methyl-1-phenyl-1*H*-pyrazol-4-yl)acetamide, *N*-[(2-chloro-4-fluorophenyl)methyl]-2-[3,5-dimethyl-1-(2-methyl-4-pyridinylyl-1*H-*pyrazol-4-yl]acetamide,
*N*-[(2-chloro-4-fluorophenyl)methyl]-2-[3,5-dimethyl-1-(6-methyl-3-pyridinyl)-1*H-*pyrazol-4-yl]acetamide,
*N*-[(2-chloro-4-fluorophenyl)methyl]-2-[1-(2,6-dimethyl-3-pyridinyl)-3,5-dimethyl-1*H-*pyrazol-4-yl]acetamide,
*N*-[(2-chloro-4-fluorophenyl)methyl]-2-[3,5-dimethyl-1-(4-methyl-3-pyridinyl)-1*H-*pyrazol-4-yl]acetamide,
*N*-[(2-chloro-4-fluorophenyl)methyl]-2-[3,5-dimethyl-1-(2-methyl-3-pyridinyl)-1*H-*pyrazol-4-yl]acetamide,
*N*-[(2-chloro-4-fluorophenyl)methyl]-2-[3,5-dimethyl-1-(5-methyl-3-pyridinyl)-1*H-*pyrazol-4-yl]acetamide,
*N*-[(2-chloro-4-fluorophenyl)methyl]-2-[3,5-dimethyl-1-(2-pyridinylmethyl)-1*H*-pyrazol-4-yl]acetamide,
*N*-[(2-chloro-4-fluorophenyl)methyl]-2-{3,5-dimethyl-1-[4-(1-piperidinylcarbonyl)phenyl]-1*H*-pyrazol-4-yl}acetamide,
*N*-[(2-chloro-4-fluorophenyl)methyl]-2-{3,5-dimethyl-1-[4-(4-morpholinylcarbonyl)phenyl]-1*H*-pyrazol-4-yl}acetamide,
*N*-[(2-chloro-4-fluorophenyl)methyl]-2-(3,5-dimethyl-1-{4-[(4-methyl-1-piperazinyl)carbonyl]phenyl}-1*H*-pyrazol-4-yl)acetamide,
4-[4-(2-{[(2-chloro-4-fluorophenyl)methyl]amino}-2-oxoethyl)-3,5-dimethyl-1*H*-pyrazol-1-yl]-*N*-[2-(dimethylamino)ethyl]benzamide,
4-[4-(2-f[(2-chloro-4-fluorophenyl)methyl]amino)-2-oxoethyl)-3,5-dimethyl1 *H*-pyrazol-1-yl]benzamide,
*N*-[(2-chloro-4-fluorophenyl)methyl]-2-(3,5-dimethyl-1-{4-[(4-phenyl-1-piperazinyl)carbonyl]phenyl}-1*H* pyrazol-4-yl)acetamide,
4-[4-(2-{[(2-chloro-4-fluorophenyl)methyl]amino}-2-oxoethyl)-3,5-dimethyl-1*H*-pyrazol-1-yl]-*N*-(2-hydroxyethyl)benzamide,
4-[4-(2-{[(2-chloro-4-fluorophenyl)methyl]amino}-2-oxoethyl)-3,5-dimethyl-1*H*-pyrazol-1-yl]-*N*-(tetrahydro-2*H*-pyran-4-ylmethyl)benzamide,
4-[4-(2-{[(2-chloro-4-fluorophenyl)methyl]amino}-2-oxoethyl)-3,5-dimethyl-1*H*-pyrazol-1-yl]-*N*-(2-pyridinylmethyl)benzamide,
*N*-(2-amino-2-oxoethyl)-4-[4-(2-{[(2-chloro-4-fluorophenyl)methyl]amino}-2-oxoethyl)-3,5-dimethyl-1*H*-pyrazol-1-yl]benzamide,
*N*-[(3-chloro-2-methylphenyl)methyl]-2-[1-(2-hydroxyethyl)3,5-dimethyl-1*H*-pyrazol-4-yl]acetamide,
*N*-[(3-chloro-2-methylphenyl)methyl]-2-{3,5-dimethyl-1-[2-(4-morpholinyl)ethyl]-1*H-*pyrazol-4-yl}acetamide,
*N*-[(2-chloro-4-fluorophenyl)methyl]-2-[1-(2-hydroxy-2-methylpropyl)-3,5-dimethyl-1*H-*pyrazol-4-yl]acetamide,
2-[1-(2-hydroxy-2-methyl propyl)-3,5-dimethyl-1*H*-pyrazol-4-yl]-*N*-[(2,3,4-trifluorophenyl)methyl]acetamide,
*N*-[(3-chloro-2-methylphenyl)methyl]-2-[1-(2-hydroxy-2-methylpropyl)-3,5-dimethyl-1*H* pyrazol-4-yl]acetamide,
*N*-[(2-chloro-6-methylphenyl)methyl]-2-[1-(2-hydroxy-2-methylpropyl)-3,5-dimethyl-1*H*-pyrazol-4-yl]acetamide,
*N*-[(2-chloro-4-fluorophenyl)methyl]-2-{1-[6-(ethyloxy)-2-pyridinyl]-1*H*-pyrazol-4-yl}acetamide,
*N-[*(2-chloro-4-fluorophenyl)methyl]-2-{3,5-dimethyl-1-[6-(methyloxy)-3-pyridinyl]-1*H-*pyrazol-4-yl}acetamide,
*N*-[(2-chloro-4-fluorophenyl)methyl]-2-{3,5-dimethyl-1-[5-(methyloxy)-3-pyridinyl]-1*H-*pyrazol-4-yl}acetamide,
*N*-[(2-chloro-4-fluorophenyl)methyl]-2-{3,5-dimethyl-1-[2-(methyloxy)-3-pyridinyl]-1*H-*pyrazol-4-yl}acetamide,
*N*-[(2-chloro-4-fluorophenyl)methyl]-2-(1-(2-hydroxyethyl)-5-(trifluoromethyl)-1*H-*pyrazol-4-yl]acetamide,
*N*-[(2-chloro-4-fluorophenyl)methyl]-2-[1-(2-hydroxyethyl)-3-(trifluoromethyl)-1*H-*pyrazol-4-yl]acetamide,
*N*-[(2-chloro-4-fluorophenyl)methyl]-2-[1-{2-[(2-hydroxyethyl)amino]ethyl}-3-(trifluoromethyl)-1*H*-pyrazol-4-yl]acetamide,
*N*-[(2-chloro-4-fluorophenyl)methyl]-2-[1-[2-(tetrahydro-2*H*-pyran-4-ylamino)ethyl]-3-(trifluoromethyl)-1*H*-pyrazol-4-yl]acetamide,
*N*-[(2-chloro-4-fluorophenyl)methyl]-2-[1-[2-(dimethylamino)ethyl]-5-(trifluoromethyl)-1*H*-pyrazol-4-yl]acetamide,
*N*-[(2-chloro-4-fluorophenyl)methyl]-2-[1-[2-(tetrahydro-2*H*-pyran-4-ylamino)ethyl]-5-(trifluoromethyl)-1*H*-pyrazol-4-yl]acetamide,
*N*-[(2-chloro-4-fluorophenyl)methyl]-2-[1-[2-(1-piperidinyl)ethyl]-5-(trifluoromethyl)-1*H-*pyrazol-4-yl]acetamide,
*N*-[(2-chloro-4-fluorophenyl)methyl]-2-[1-(2-{[2-(methyloxy)ethyl]amino}ethyl)-5-(triflu oromethyl)-1*H*-pyrazol-4-yl]acetam ide,
2-[3,5-dimethyl-1-(2-pyrimidinyl)-1*H*-pyrazol-4-yl]-*N*-[(2,3,4-trifluorophenyl)methyl]acetamide,
2-[5-bromo-1-(4-fluorophenyl)-1*H*-pyrazol-4-yl]-*N*-[(2-chloro-4-fluorophenyl)methyl]acetamide, or
*N*-[(2-chloro-4-fluorophenyl)methyl]-2-[5-ethyl-1-(4-fluorophenyl)-1*H*-pyrazol-4-yl]acetamide;
or a pharmaceutically acceptable salt thereof.

18. A pharmaceutical composition which comprises a compound or a pharmaceutically acceptable salt thereof, as defined in any one of the preceding claims, and a pharmaceutically acceptable carrier or excipient.

19. A compound, or a pharmaceutically acceptable salt thereof, as claimed in any one of claims 1 to 17, for use in therapy.

20. Use of a compound, or a pharmaceutically acceptable salt thereof, as defined in any one of claims 1 to 17, for the manufacture of a medicament for the treatment or prevention of pain, inflammation or a neurodegenerative disease.

## Patentansprüche

1. Eine Verbindung der Formel (I) oder ein pharmazeutisch verträgliches Salz davon: 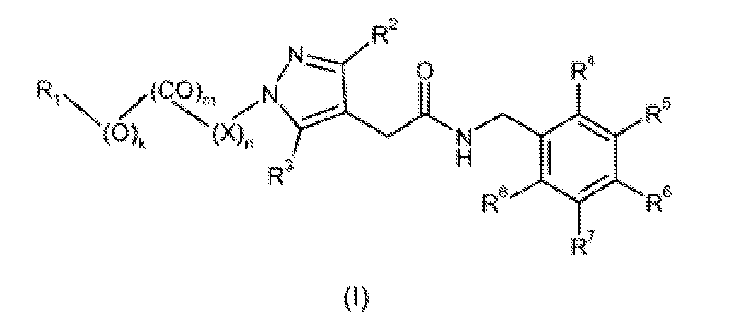 wobei:
R¹ für NR⁹R¹⁰, C₁₋₆-Alkyl oder Trifluormethyl steht; oder Wasserstoff darstellen kann, wenn k gleich 1 ist;
oder R¹ ein C₆₋₁₀-Aryl, ein 5- bis 6-gliedriges oder ein 8- bis 10-gliedriges Heteroaryl, ein C₃₋₆-Cycloalkyl oder ein 4- bis 11-gliedriges Heterocyclyl darstellt; wobei jeder Ring oder jedes Ringsystem der Aryl-, Heteroaryl-, Cycloalkyl- oder Heterocyclylreste gegebenenfalls mit 1 bis 3 Substituenten substituiert sein kann, welche gleich oder verschieden sein können, ausgewählt aus der Gruppe bestehend aus Halogen, Hydroxyl, Nitro, Oxo, Cyano, Trifluormethyl, C₁₋₆-Alkyl, C₁₋₃-Alkoxy, C₁₋₃-Alkoxy-C₁₋₃-alkyl-, -COC₁₋₃-Alkyl, -CONR¹¹R¹² und -NR¹¹R¹²
oder R¹ einen Rest darstellt, ausgewählt aus der Formel (II), (III), (IV), (V), (VI) und (VII): 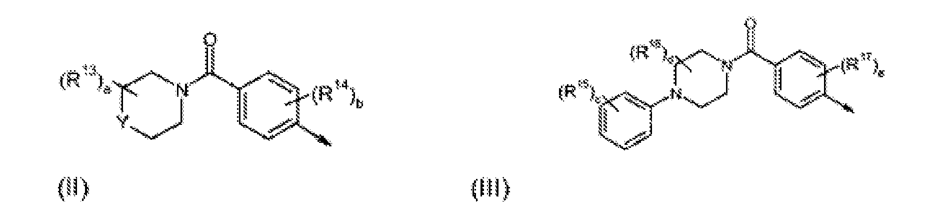 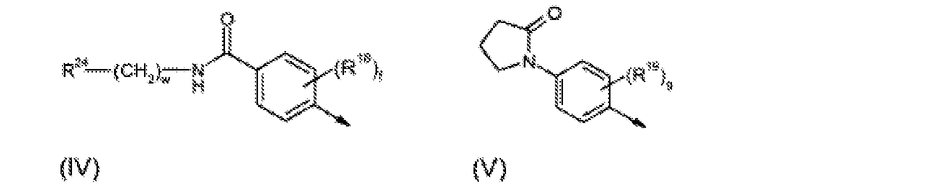 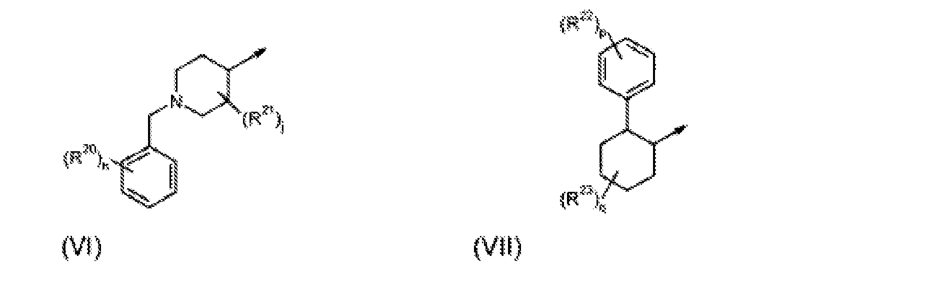 wobei:
Y für CH₂, O oder NR¹¹steht;
w für 0, 1, 2 oder 3 steht;
a, b, c, d, e, f, g, h, j, p und q unabhängig für 0, 1 oder 2 stehen;
R¹³, R¹⁴, R¹⁵, R¹⁶, R¹⁷ R¹⁸, R¹⁹, R²⁰, R²¹, R²² und R²³ unabhängig Halogen, Cyano, C₁₋₃-Alkyl, C₁₋₃-Alkoxy oder Trifluormethyl darstellen;
R²⁴ für NR¹¹R¹², -OH oder -CONR¹¹R¹² steht;
oder R²⁴ ein C₃₋₆-Cycloalkyl, ein C₆₋₁₀-Aryl, ein 5- bis 6-gliedriges oder ein 8- bis 10-gliedriges Heteroaryl oder ein 4- bis 11-gliedriges Heterocyclyl darstellt; wobei jeder Ring oder jedes Ringsystem der Aryl-, Heteroaryl-, Cycloalkyl- oder Heterocyclylreste gegebenenfalls mit 1 bis 3 Substituenten substituiert sein kann, welche gleich oder verschieden sein können, ausgewählt aus der Gruppe bestehend aus Halogen, Hydroxyl, Nitro, Oxo, Cyano, Trifluormethyl, C₁₋₃-Alkyl, C₁₋₃-Alkoxy, C₁₋₃-Alkoxy-C₁₋₃-alkyl-, -COC₁₋₃-Alkyl, -CONH₂ und -NR¹¹ R¹²;
und wobei:
R² Wasserstoff, C₁₋₆-Alkyl, C₁₋₄-Alkoxy, Trifluormethyl, Phenyl, ein 5- bis 6-gliedriges monocyclisches Heteroaryl oder ein C₃₋₆-Cycloalkyl darstellt;
R³ Wasserstoff, C₁₋₆-Alkyl, C₁₋₄-Alkoxy, Trifluormethyl, Phenyl, ein 5- bis 6-gliedriges monocyclisches Heteroaryl oder ein C₃₋₆-Cycloalkyl darstellt; oder R³ Halogen darstellt, wenn R² Wasserstoff ist;
R⁴ und R⁸ unabhängig Wasserstoff, Halogen, Trifluormethyl, C₁₋₆-Alkyl, Phenyl, Phenyloxy, NO₂ oder NH₂ darstellen;
R⁵ und R⁷ unabhängig Wasserstoff, Halogen, Trifluormethyl, C₁₋₆-Alkyl, Phenyl, Phenyloxy oder NO₂ darstellen;
R⁶ Wasserstoff, Halogen, Trifluormethyl, Trifluormethoxy, C₁₋₆-Alkyl, C₃₋₆-Cycloalkyl, Phenyl, Phenyloxy, NO₂ oder Cyano darstellt;
R⁹ und R¹⁰ unabhängig Wasserstoff, C₁₋₄-Alkyl, C₁₋₄-Alkoxy-C₁₋₄-alkyl-, -C₁₋₄-AlkylOH oder -C₁₋₃-AlkylNR¹¹R¹² darstellen;
oder ein C₃₋₆-Cycloalkyl oder ein 6-gliedriges Heterocyclyl darstellen, wobei jeder Ring davon gegebenenfalls mit 1 bis 3 Substituenten substituiert sein kann, welche gleich oder verschieden sein können, ausgewählt aus der Gruppe bestehend aus Halogen, Cyano, C₁₋₃-Alkyl, C₁₋₃-Alkoxyund Trifluormethyl;
R¹¹ und R¹² unabhängig Wasserstoff oder C₁₋₃-Alkyl darstellen;
k für 0 oder 1 steht;
m für 0 oder 1 steht;
n für 0, 1, 2, 3 oder 4 steht;
X für CR²⁵R²⁶ steht; und
R²⁵ und R²⁶ bei jedem Vorkommen unabhängig Wasserstoff, Fluor oder C₁₋₆-Alkyl darstellen;
mit der Maßgabe, dass die Verbindung der Formel (I) eine andere als 2-(3,5-Dimethyl-1-phenyl-1H-pyrazol-4-yl)-N-(phenylmethyl)acetamid oder 2-(3,5-Dimethyl-1-phenyl-1H-pyrazol-4-yl)-N-[(2-fluorphenyl)methyl]acetamid ist.

2. Eine Verbindung oder ein pharmazeutisch verträgliches Salz davon wie in Anspruch 1 beansprucht, wobei R¹ für NR⁹R¹⁰, C₁₋₆-Alkyl oder Trifluormethyl steht; oder Wasserstoff darstellen kann, wenn k gleich 1 ist; oder R¹ ein C₆₋₁₀-Aryl, ein 5- bis 6-gliedriges oder ein 8- bis 10-gliedriges Heteroaryl, ein C₃₋₆-Cycloalkyl oder ein 4- bis 11-gliedriges Heterocyclyl darstellt; wobei jeder Ring oder jedes Ringsystem der Aryl-, Heteroaryl-, Cycloalkyl- oder Heterocyclylreste gegebenenfalls mit 1 bis 3 Substituenten substituiert sein kann, welche gleich oder verschieden sein können, ausgewählt aus der Gruppe bestehend aus Halogen, Hydroxyl, Nitro, Oxo, Cyano, Trifluormethyl, C₁₋₆-Alkyl, C₁₋₃-Alkoxy, C₁₋₃-Alkoxy-C₁₋₃-alkyl-, -COC₁₋₃-Alkyl, -CONR¹¹R¹² und NR¹¹R¹².

3. Eine Verbindung oder ein pharmazeutisch verträgliches Salz davon wie in Anspruch 2 beansprucht, wobei R¹ für NR⁹R¹⁰, C₁₋₆-Alkyl oder Trifluormethyl steht; oder Wasserstoff darstellen kann, wenn k gleich 1 ist; oder R¹ ein Phenyl, ein 5- bis 6-gliedriges oder ein 8- bis 10-gliedriges Heteroaryl oder ein 4- bis 11-gliedriges Heterocyclyl darstellt; wobei jeder Ring oder jedes Ringsystem der Phenyl-, Heteroaryl-oder Heterocyclylreste gegebenenfalls mit 1 bis 3 Substituenten substituiert sein kann, welche gleich oder verschieden sein können, ausgewählt aus der Gruppe bestehend aus Halogen, C₁₋₆-Alkyl, C₁₋₃-Alkoxy, Trifluormethyl und-NR¹¹R¹².

4. Eine Verbindung oder ein pharmazeutisch verträgliches Salz davon wie in Anspruch 2 beansprucht, wobei
R¹ für NR⁹R¹⁰, C₁₋₆-Alkyl oder Trifluormethyl steht; oder Wasserstoff darstellen kann, wenn k gleich 1 ist; oder
R¹ einen gegebenenfalls substituierten Rest darstellt, ausgewählt aus Phenyl, Pyridinyl, Pyrimidinyl, Imidazolyl, Pyrrolidinyl und Pyrazolyl, wobei die optionalen Substituenten, welche gleich oder verschieden sein können, ausgewählt sind aus der Gruppe bestehend aus Halogen, C₁₋₆-Alkyl, C₁₋₃-Alkoxy, -CONH₂ und Trifluormethyl.

5. Eine Verbindung oder ein pharmazeutisch verträgliches Salz davon wie in Anspruch 1 beansprucht, wobei R¹ einen Rest darstellt, ausgewählt aus der Formel (II), (III), (IV), (V), (VI) und (VII), wobei a, b, c, d, e, f, g, h, j, p und q gleich 0 sind.

6. Eine Verbindung oder ein pharmazeutisch verträgliches Salz davon wie in Anspruch 1 oder 5 beansprucht, wobei R²⁴ NH₂, -OH oder -CONH₂, oder ein unsubstituiertes Phenyl, Pyridinyl oder Tetrahydropyranyl darstellt; und w für 1 oder 2 steht.

7. Eine Verbindung oder ein pharmazeutisch verträgliches Salz davon wie in einem der vorhergehenden Ansprüche beansprucht, wobei R² und R³ unabhängig Wasserstoff, C₁₋₆-Alkyl oder Trifluormethyl darstellen.

8. Eine Verbindung oder ein pharmazeutisch verträgliches Salz davon wie in Anspruch 7 beansprucht, wobei sowohl R² als auch R³ Methyl darstellen.

9. Eine Verbindung oder ein pharmazeutisch verträgliches Salz davon wie in einem der vorhergehenden Ansprüche beansprucht, wobei R⁴, R⁵, R⁷ und R⁸ unabhängig Wasserstoff, Halogen oder C₁₋₆-Alkyl darstellen.

10. Eine Verbindung oder ein pharmazeutisch verträgliches Salz davon wie in einem der vorhergehenden Ansprüche beansprucht, wobei R⁶ Wasserstoff oder Halogen darstellt.

11. Eine Verbindung oder ein pharmazeutisch verträgliches Salz davon wie in einem der vorhergehenden Ansprüche beansprucht, wobei R⁴ und R⁸ unabhängig Wasserstoff, Halogen oder Methyl darstellen.

12. Eine Verbindung oder ein pharmazeutisch verträgliches Salz davon wie in Anspruch 11 beansprucht, wobei einer der Reste R⁴ und R⁸ Chlor darstellt und der andere Wasserstoff darstellt.

13. Eine Verbindung oder ein pharmazeutisch verträgliches Salz davon wie in Anspruch 12 beansprucht, wobei einer der Reste R⁴ und R⁸ Chlor darstellt und der andere Wasserstoff darstellt und R⁶ Fluor darstellt.

14. Eine Verbindung oder ein pharmazeutisch verträgliches Salz davon wie in einem der vorhergehenden Ansprüche beansprucht, wobei R⁵ und R⁷ unabhängig Wasserstoff, Halogen oder Methyl darstellen.

15. Eine Verbindung oder ein pharmazeutisch verträgliches Salz davon wie in Anspruch 14 beansprucht, wobei R⁵ und R⁷ Wasserstoff darstellen.

16. Eine Verbindung oder ein Salz wie in einem der vorhergehenden Ansprüche beansprucht, welche eine Verbindung der Formel (IA) oder ein pharmazeutisch verträgliches Salz davon ist: 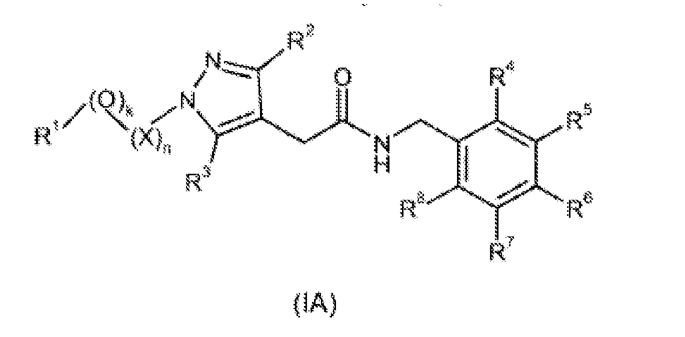 wobei:
R¹ für NR⁹R¹⁰, C₁₋₆-Alkyl oder Trifluormethyl steht; oder Wasserstoff darstellen kann, wenn k gleich 1 ist;
oder R¹ ein C₆₋₁₀-Aryl, ein 5- bis 6-gliedriges oder ein 8- bis 10-gliedriges Heteroaryl, ein C₃₋₆-Cycloalkyl oder ein 4- bis 11-gliedriges Heterocyclyl darstellt; wobei jeder Ring oder jedes Ringsystem der Aryl-, Heteroaryl-, Cycloalkyl- oder Heterocyclylreste gegebenenfalls mit 1 bis 3 Substituenten substituiert sein kann, welche gleich oder verschieden sein können, ausgewählt aus der Gruppe bestehend aus Halogen, Hydroxyl, Nitro, Oxo, Cyano, Trifluormethyl, C₁₋₆-Alkyl, C₁₋₃-Alkoxy, C₁₋₃-Alkoxy-C₁₋₃-alkyl-, -COC₁₋₃-Alkyl, -CONR¹¹R¹² und -NR¹¹R¹²;
R² und R³ unabhängig Wasserstoff, Methyl oder Trifluormethyl darstellen;
R⁴ und R⁸ unabhängig Wasserstoff, Halogen oder Methyl darstellen;
R⁵ und R⁷ Wasserstoff darstellen;
R⁶ Halogen darstellt;
R⁹ und R¹⁰ unabhängig Wasserstoff, C₁₋₄-Alkyl, C₁₋₄₋Alkoxy-C₁₋₄-alkyl- oder -C₁₋₄-AlkylOH darstellen;
R¹¹ und R¹² unabhängig Wasserstoff oder Methyl darstellen;
k gleich 0 oder 1 ist;
n gleich 0, 1 oder 2 ist;
X für CR²⁵R²⁶ steht; und
R²⁵ und R²⁶ bei jedem Vorkommen unabhängig Wasserstoff oder Methyl darstellen; mit der Maßgabe, dass die Verbindung der Formel (IA) eine andere als 2-(3,5-Dimethyl-1-phenyl-1H-pyrazol-4-yl)-N-(phenylmethyl)acetamid oder 2-(3,5-Dimethyl-1-phenyl-1H-pyrazol-4-yl)-N-[(2-fluorphenyl)methyl]acetamid ist.

17. Eine Verbindung gemäß Anspruch 1 oder ein pharmazeutisch verträgliches Salz davon, nämlich:
N-[(3,4-Dichlorphenyl)methyl]-2-(3,5-dimethyl-1-phenyl-1H-pyrazol-4-yl)acetamid,
N-[(3,5-Dichlorphenyl)methyl]-2-(3,5-dimethyl-1-phenyl-1H-pyrazol-4-yl)acetamid,
2-(3,5-Dimethyl-1-phenyl-1H-pyrazol-4-yl)-N-({4-[(trifluormethyl)oxy]phenyl} methyl)-acetamid,
2-(3,5-Dimethyl-1-phenyl-1H-pyrazol-4-yl)-N-{[2-fluor-5-(trifluormethyl)phenyl]-methyl}acetamid,
2-(3,5-Dimethyl-1-phenyl-1H-pyrazol-4-yl)-N-{[4-(trifluormethyl)phenyl]methyl}-acetamid,
2-(3,5 -Dimethyl-1-phenyl-1H-pyrazol-4-yl)-N-{[4-fluor-3-(trifluormethyl)phenyl]-methyl}acetamid,
N-[(3-Chlor-4-fluorphenyl)methyl]-2-(3,5-dimethyl-1-phenyl-1H-pyrazol-4-yl)acetamid, 2-(3,5-Dimethyl-1-phenyl-1H-pyrazol-4-yl)-N-{[3-fluor-5-(trifluormethyl)phenyl]-methyl}acetamid,
N-[(2-Chlor-4-fluorphenyl)methyl]-2-(3,5-dimethyl-1-phenyl-1H-pyrazol-4-yl)acetamid,
N-(2-Biphenylylmethyl)-2-(3,5-dimethyl-1-phenyl-1H-pyrazol-4-yl)acetamid,
N-[(2,5-Difluorphenyl)methyl]-2-(3,5-dimethyl-1-phenyl-1H-pyrazol-4-yl)acetamid,
N-[(3,4-Difluorphenyl)methyl]-2-(3,5-dimethyl-1-phenyl-1H-pyrazol-4-yl)acetamid,
N-[(2-Bromphenyl)methyl]-2-(3,5-dimethyl-1-phenyl-1H-pyrazol-4-yl)acetamid,
N-[(3-Bromphenyl)methyl]-2-(3,5-dimethyl-1-phenyl-1H-pyrazol-4-yl)acetamid,
N-[(4-Bromphenyl)methyl]-2-(3,5-dimethyl-1-phenyl-1H-pyrazol-4-yl)acetamid,
2-(3,5-Dimethyl-1-phenyl-1H-pyrazol-4-yl)-N-[(2-nitrophenyl)methyl] acetamid,
2-(3,5-Dimethyl-1-phenyl-1H-pyrazol-4-yl)-N-[(3-nitrophenyl)methyl] acetamid,
2-(3,5-Dimethyl-1-phenyl-1H-pyrazol-4-yl)-N-[(4-nitrophenyl)methyl] acetamid,
N-[(3-Chlor-2-methylphenyl)methyl]-2-(3,5-dimethyl-1-phenyl-1H-pyrazol-4-yl)-acetamid,
2-(3,5-Dimethyl-1-phenyl-1H-pyrazol-4-yl)-N-{[3-fluor-4-(trifluormethyl)phenyl]-methyl}acetamid,
2-(3,5-Dimethyl-1-phenyl-1H-pyrazol-4-yl)-N-[(2,3,4-trifluorphenyl)methyl] acetamid,
N-[(2,3-Difluor-4-methylphenyl)methyl]-2-(3,5-dimethyl-1-phenyl-1H-pyrazol-4-yl)-acetamid,
N-[(2,5-Dimethylphenyl)methyl]-2-(3,5-dimethyl-1-phenyl-1H-pyrazol-4-yl)acetamid,
N-[(5-Chlor-2-methylphenyl)methyl]-2-(3,5-dimethyl-1-phenyl-1H-pyrazol-4-yl)-acetamid,
N-[(2,6-Difluorphenyl)methyl]-2-(3,5-dimethyl-1-phenyl-1H-pyrazol-4-yl)acetamid,
N-[(2-Chlor-6-methylphenyl)methyl]-2-(3,5-dimethyl-1-phenyl-1H-pyrazol-4-yl)-acetamid,
N-(4-Biphenylylmethyl)-2-(3,5-dimethyl-1-phenyl-1H-pyrazol-4-yl)acetamid,
N-[(2,6-Dimethylphenyl)methyl]-2-(3,5-dimethyl-1-phenyl-1H-pyrazol-4-yl)acetamid,
N-[(2-Aminophenyl)methyl]-2-(3,5 -dimethyl-1-phenyl-1H-pyrazol-4-yl)acetamid,
N-(3-Biphenylylmethyl)-2-(3,5-dimethyl-1-phenyl-1H-pyrazol-4-yl)acetamid,
2-(3,5-Dimethyl-1-phenyl-1H-pyrazol-4-yl)-N-[(5-fluor-2-methylphenyl)methyl]-acetamid,
N-{[3,5-Bis(trifluormethyl)phenyl]methyl}-2-(3,5-dimethyl-1-phenyl-1H-pyrazol-4-yl)-acetamid,
N-[(4-Cyanophenyl)methyl]-2-(3,5-dimethyl-1-phenyl-1H-pyrazol-4-yl)acetamid,
N-[(2-Chlorphenyl)methyl]-2-(3,5 -dimethyl-1-phenyl-1H-pyrazol-4-yl)acetamid,
N-[(2,3-Difluorphenyl)methyl]-2-(3,5-dimethyl-1-phenyl-1H-pyrazol-4-yl)acetamid,
N-{[2-Chlor-6-(phenyloxy)phenyl]methyl}-2-(3,5-dimethyl-1-phenyl-1H-pyrazol-4-yl)-acetamid,
N-[(2,3-Dichlorphenyl)methyl]-2-(3,5-dimethyl-1-phenyl-1H-pyrazol-4-yl)acetamid,
2-(3,5-Dimethyl-1 -phenyl-1 H-pyrazol-4-yl)-N-{[4-(phenyloxy)phenyl]methyl} acetamid,
N-[(2,4-Dichlorphenyl)methyl]-2-(3,5-dimethyl-1-phenyl-1H-pyrazol-4-yl)acetamid,
N-[(2,3-Dimethylphenyl)methyl]-2-(3,5-dimethyl-1-phenyl-1H-pyrazol-4-yl)acetamid,
N-[(2,6-Dichlorphenyl)methyl]-2-(3,5-dimethyl-1-phenyl-1H-pyrazol-4-yl)acetamid,
N-[(2,5-Dichlorphenyl)methyl]-2-(3,5 -dimethyl-1-phenyl-1H-pyrazol-4-yl)acetamid,
N-[(2-Chlor-6-fluorphenyl)methyl]-2-(3,5-dimethyl-1-phenyl-1H-pyrazol-4-yl)acetamid,
N-{[2-Chlor-5-(trifluormethyl)phenyl]methyl}-2-(3,5-dimethyl-1-phenyl-1H-pyrazol-4-yl)acetamid,
2-(3,5-Dimethyl-1-phenyl-1H-pyrazol-4-yl)-N-{[5-fluor-2-(trifluormethyl)phenyl]-methyl}acetamid,
2-(3,5-Dimethyl-1-phenyl-1H-pyrazol-4-yl)-N-{[4-fluor-2-(trifluormethyl)phenyl]-methyl}acetamid,
N-[(5-Chlor-2-fluorphenyl)methyl]-2-(3,5-dimethyl-1-phenyl-1H-pyrazol-4-yl)acetamid, N-[(2-Chlor-4-fluorphenyl)methyl]-2-[1-(4-fluorphenyl)-3,5-dimethyl-1H-pyrazol-4-yl]-acetamid,
N-[(2-Chlor-4-fluorphenyl)methyl]-2-{1-[2-(dimethylamino)ethyl]-3,5-dimethyl-1H-pyrazol-4-yl}acetamid,
N-[(2-Chlor-4-fluorphenyl)methyl]-2-[1-(1H-imidazol-4-yl-methyl)-3,5-dimethyl-1H-pyrazol-4-yl]acetamid,
N-[(2-Chlor-4-fluorphenyl)methyl]-2-(1,3,5-trimethyl-1H-pyrazol-4-yl)acetamid,
N-[(2-Chlor-4-fluorphenyl)methyl]-2-[3,5-dimethyl-1-(phenylmethyl)-1H-pyrazol-4-yl]-acetamid,
N-[(2-Chlor-4-fluorphenyl)methyl]-2-[1-(2,4-difluorphenyl)-3,5-dimethyl-1H-pyrazol-4-yl] acetamid,
N-[(2-Chlor-4-fluorphenyl)methyl]-2-[1-(4-chlorphenyl)-3,5-dimethyl-1H-pyrazol-4-yl]-acetamid,
N-[(2-Chlor-4-fluorphenyl)methyl]-2-{3,5-dimethyl-1-[4-(methyloxy)phenyl]-1H-pyrazol-4-yl}acetamid,
N-[(2-Chlor-4-fluorphenyl)methyl]-2-[1-(3-fluorphenyl)-3,5-dimethyl-1H-pyrazol-4-yl]-acetamid,
N-[(2-Chlor-4-fluorphenyl)methyl]-2-[1-(3-chlorphenyl)-3,5-dimethyl-1H-pyrazol-4-yl]-acetamid,
N-[(2-Chlor-4-fluorphenyl)methyl]-2-{3,5-dimethyl-1-[3-(methyloxy)phenyl]-1H-pyrazol-4-yl}acetamid,
N-[(2-Chlor-4-fluorphenyl)methyl]-2-[1-(2-fluorphenyl)-3,5-dimethyl-1H-pyrazol-4-yl] - acetamid,
N-[(2-Chlor-4-fluorphenyl)methyl]-2-[1-(2-chlorphenyl)-3,5-dimethyl-1H-pyrazol-4-yl]-acetamid,
N-[(2-Chlor-4-fluorphenyl)methyl]-2-{3,5-dimethyl-1-[2-(methyloxy)phenyl]-1H-pyrazol-4-yl}acetamid,
N-[(2-Chlor-4-fluorphenyl)methyl]-2-[3,5-dimethyl-1-(4-methylphenyl)-1H-pyrazol-4-yl]acetamid,
N-[(2-Chlor-4-fluorphenyl)methyl]-2-{1-[4-(ethyloxy)phenyl]-3,5-dimethyl-1H-pyrazol-4-yl}acetamid,
N-[(2-Chlor-4-fluorphenyl)methyl]-2-[1-(5-cyano-2-pyridinyl)-3,5-dimethyl-1H-pyrazol-4-yl]acetamid,
N-[(2-Chlor-4-fluorphenyl)methyl]-2-[1-(3,5-dichlor-2-pyridinyl)-3,5-dimethyl-1H-pyrazol-4-yl]acetamid,
N-[(2-Chlor-4-fluorphenyl)methyl]-2-[3,5-dimethyl-1-(4-pyridinyl)-1H-pyrazol-4-yl]-acetamid,
N-[(2-Chlor-4-fluorphenyl)methyl]-2-{3,5-dimethyl-1-[6-methyl-4-(trifluormethyl)-2-pyridinyl]-1H-pyrazol-4-yl}acetamid,
2-[1-(5-Brom-2-pyridinyl)-3,5-dimethyl-1H-pyrazol-4-yl]-N-[(2-chlor-4-fluorphenyl)-methyl]acetamid,
N-[(2-Chlor-4-fluorphenyl)methyl]-2-[1-(5-chlor-2-pyridinyl)-3,5-dimethyl-1H-pyrazol-4-yl]acetamid,
N-[(2-Chlor-4-fluorphenyl)methyl]-2-[3,5-dimethyl-1-(1,3-thiazol-2-yl)-1H-pyrazol-4-yl]acetamid,
N-[(2-Chlor-4-fluorphenyl)methyl]-2-{3,5-dimethyl-1-[5-(trifluormethyl)-2-pyridinyl]-1H-pyrazol-4-yl}acetamid,
N-[(2-Chlor-4-fluorphenyl)methyl]-2-{3,5-dimethyl-1-[3-(trifluormethyl)-2-pyridinyl]-1H-pyrazol-4-yl}acetamid,
N-[(2-Chlor-4-fluorphenyl)methyl]-2-[3,5-dimethyl-1-(3-pyridinyl)-1H-pyrazol-4-yl]-acetamid,
N-[(2-Chlor-4-fluorphenyl)methyl]-2-[1-(1,1-dimethylethyl)-3,5-dimethyl-1H-pyrazol-4-yl]acetamid,
N-[(2-Chlor-4-fluorphenyl)methyl]-2-[3,5-dimethyl-1-(2-pyridinyl)-1H-pyrazol-4-yl]-acetamid,
N-[(2-Chlor-4-fluorphenyl)methyl]-2-[1-(4-iodphenyl)-3,5-dimethyl-1H-pyrazol-4-yl]-acetamid,
N-[(2-Chlor-4-fluorphenyl)methyl]-2-[3,5-dimethyl-1-(4-pyridinylmethyl)-1H-pyrazol-4-yl]acetamid,
N-[(2-Chlor-4-fluorphenyl)methyl]-2-[3,5-dimethyl-1-(6-methyl-3-pyridazinyl)-1H-pyrazol-4-yl]acetamid,
N-[(2-Chlor-4-fluorphenyl)methyl]-2-[3,5-dimethyl-1-(2-pyrimidinyl)-1H-pyrazol-4-yl]-acetamid,
N-[(2-Chlor-4-fluorphenyl)methyl]-2-[1-(2,6-dimethyl-4-pyrimidinyl)-3,5-dimethyl-1H-pyrazol-4-yl]acetamid,
N-[(2-Chlor-4-fluorphenyl)methyl]-2-{3,5-dimethyl-1-[4-(trifluormethyl)-2-pyrimidinyl]-1H-pyrazol-4-yl}acetamid,
N-[(2-Chlor-4-fluorphenyl)methyl]-2-[3,5-dimethyl-1-(2-phenylethyl)-1H-pyrazol-4-yl]-acetamid,
N-[(2-Chlor-4-fluorphenyl)methyl]-2-(1-cyclohexyl-3,5-dimethyl-1H-pyrazol-4-yl)-acetamid,
N-[(2-Chlor-4-fluorphenyl)methyl]-2-{1-[(2-chlorphenyl)methyl]-3,5-dimethyl-1H-pyrazol-4-yl}acetamid,
N-[(2-Chlor-4-fluorphenyl)methyl]-2-[3,5-dimethyl-1-(2-methylpropyl)-1H-pyrazol-4-yl]acetamid,
N-[(2-Chlor-4-fluorphenyl)methyl]-2-(1-cyclopentyl-3,5-dimethyl-1H-pyrazol-4-yl)-acetamid,
N-[(2-Chlor-4-fluorphenyl)methyl]-2-[3,5-dimethyl-1-(tetrahydro-2H-pyran-4-yl)-1H-pyrazol-4-yl]acetamid,
N-[(2-Chlor-4-fluorphenyl)methyl]-2-{1-[(2,4-difluorphenyl)methyl]-3,5-dimethyl-1H-pyrazol-4-yl}acetamid,
N- [(2-Chlor-4-fluorphenyl)methyl]-2-{1-[(4-chlorphenyl)methyl]-3,5-dimethyl-1H-pyrazol-4-yl}acetamid,
N-[(2-Chlor-4-fluorphenyl)methyl]-2-(3,5-dimethyl-1-{[4-(methyloxy)phenyl]methyl}-1H-pyrazol-4-yl)acetamid,
N-[(2-Chlor-4-fluorphenyl)methyl]-2-{3,5-dimethyl-1-[1-(phenylmethyl)-4-piperidinyl]-1H-pyrazol-4-yl}acetamid,
N-[(2-Chlor-4-fluorphenyl)methyl]-2-(3,5-dimethyl-1-{[3-(methyloxy)phenyl]methyl} - 1H-pyrazol-4-yl)acetamid,
N-[(2-Chlor-4-fluorphenyl)methyl]-2-(3,5-dimethyl-1-{[2-(methyloxy)phenyl]methyl} - 1 H-pyrazol-4-yl)acetamid,
N-[(2-Chlor-4-fluorphenyl)methyl]-2-[1-(2,2-dimethylpropyl)-3,5-dimethyl-1H-pyrazol-4-yl]acetamid,
N-[(2-Chlor-4-fluorphenyl)methyl]-2-(1-{[2-(dimethylamino)phenyl]methyl}-3,5-dimethyl-1H-pyrazol-4-yl)acetamid,
N-[(2-Chlor-4-fluorphenyl)methyl]-2-[3,5-dimethyl-1-(1-methyl-4-piperidinyl)-1H-pyrazol-4-yl]acetamid,
2-[1-(1-Azabicyclo[2.2.1]hept-3-yl)-3,5-dimethyl-1H-pyrazol-4-yl]-N-[(2-chlor-4-fluorphenyl)methyl]acetamid,
N-[(2-Chlor-4-fluorphenyl)methyl]-2-(3,5-dimethyl-1-{1-methyl-2-[4-(methyloxy)-phenyl]ethyl}-1H-pyrazol-4-yl)acetamid,
N-[(2-Chlor-4-fluorphenyl)methyl]-2-[3,5-dimethyl-1-(2,2,2-trifluorethyl)-1H-pyrazol-4-yl]acetamid,
N-[(2-Chlor-4-fluorphenyl)methyl]-2-(1-{2-[(2,6-dimethylphenyl)oxy]ethyl}-3,5-dimethyl-1H-pyrazol-4-yl)acetamid,
2-[1-(1,3-Benzothiazol-2-yl-methyl)-3,5-dimethyl-1H-pyrazol-4-yl]-N-[(2-chlor-4-fluorphenyl)methyl]acetamid,
N-[(2-Chlor-4-fluorphenyl)methyl]-2-[3,5-dimethyl-1-(2-phenylcyclohexyl)-1H-pyrazol-4-yl]acetamid,
N-[(2-Chlor-4-fluorphenyl)methyl]-2-{3,5-dimethyl-1-[(4-methylphenyl)methyl]-1H-pyrazol-4-yl}acetamid,
2-(1-{[3,4-Bis(methyloxy)phenyl]methyl}-3,5-dimethyl-1H-pyrazol-4-yl)-N-[(2-chlor-4-fluorphenyl)methyl]acetamid,
N-[(2-Chlor-4-fluorphenyl)methyl]-2-[3,5-dimethyl-1-(1-methylethyl)-1H-pyrazol-4-yl]-acetamid,
N-[(2-Chlor-4-fluorphenyl)methyl]-2-{3,5-dimethyl-1-[3-(phenyloxy)propyl]-1H-pyrazol-4-yl}acetamid,
N-[(2-Chlor-4-fluorphenyl)methyl]-2-[1-(cyclohexylmethyl)-3,5-dimethyl-1H-pyrazol-4-yl]acetamid,
N-[(2-Chlor-4-fluorphenyl)methyl]-2-(1-ethyl-3,5-dimethyl-1H-pyrazol-4-yl)acetamid,
N-[(2-Chlor-4-fluorphenyl)methyl]-2-{3,5-dimethyl-1-[2-(phenyloxy)ethyl]-1H-pyrazol-4-yl}acetamid,
N-[(2-Chlor-4-fluorphenyl)methyl]-2-[3,5-dimethyl-1-(1-methyl-2-phenylethyl)-1H-pyrazol-4-yl]acetamid,
N-[(2-Chlor-4-fluorphenyl)methyl]-2-(1-{2-[(4-chlor-3-methylphenyl)oxy]ethyl}-3,5-dimethyl-1H-pyrazol-4-yl)acetamid,
N-[(2-Chlor-4-fluorphenyl)methyl]-2-{3,5-dimethyl-1-[4-(2-oxo-1-pyrrolidinyl)phenyl]-1H-pyrazol-4-yl}acetamid,
N-[(2-Chlor-4-fluorphenyl)methyl]-2-[1-(4-fluorphenyl)-1H-pyrazol-4-yl]acetamid,
N-[(2-Chlor-4-fluorphenyl)methyl]-2-[1-(4-fluorphenyl)-5-(trifluormethyl)-1H-pyrazol-4-yl]acetamid,
N-[(2-Chlor-4-fluorphenyl)methyl]-2-[1-(2-methylphenyl)-1H-pyrazol-4-yl]acetamid,
N-[(2-Chlor-4-fluorphenyl)methyl]-2-[1-(2-ethylphenyl)-1H-pyrazol-4-yl]acetamid,
N-[(2-Chlor-4-fluorphenyl)methyl]-2-{3,5-dimethyl-1-[4-(trifluormethyl)phenyl]-1H-pyrazol-4-yl}acetamid,
N-[(2-Chlor-4-fluorphenyl)methyl]-2-[3,5-dimethyl-1-(3-pyridinylmethyl)-1H-pyrazol-4-yl]acetamid,
N-[(2-Chlor-4-fluorphenyl)methyl]-2-(5-methyl-1-phenyl-1H-pyrazol-4-yl)acetamid,
2-(3-Butyl-5-methyl-1-phenyl-1H-pyrazol-4-yl)-N-[(2-chlor-4-fluorphenyl)methyl]-acetamid,
N-[(2-Chlor-4-fluorphenyl)methyl]-2-[3-methyl-5-(1-methylethyl)-1-phenyl-1H-pyrazol-4-yl]acetamid,
N-[(2-Chlor-4-fluorphenyl)methyl]-2-[5-methyl-3-(1-methylethyl)-1-phenyl-1H-pyrazol-4-yl]acetamid,
N-[(2-Chlor-4-fluorphenyl)methyl]-2-(3-methyl-1,5-diphenyl-1H-pyrazol-4-yl)acetamid,
N-[(2-Chlor-4-fluorphenyl)methyl]-2-[1-(4-fluorphenyl)-5-methyl-1H-pyrazol-4-yl]-acetamid,
Ethyl-[4-(2-{[(2-chlor-4-fluorphenyl)methyl]amino}-2-oxoethyl)-3,5-dimethyl-1H-pyrazol-1-yl]acetat,
[4-(2-{[(2-Chlor-4-fluorphenyl)methyl]amino}-2-oxoethyl)-3,5-dimethyl-1H-pyrazol-1-yl]essigsäure,
N-[(2-Chlor-4-fluorphenyl)methyl]-2-{3,5-dimethyl-1-[2-(4-morpholinyl)-2-oxoethyl]-1H-pyrazol-4-yl}acetamid,
N-[(2-Chlor-4-fluorphenyl)methyl]-2-{3,5-dimethyl-1-[2-oxo-2-(1-piperidinyl)ethyl]-1H-pyrazol-4-yl}acetamid,
2-[4-(2-{[(2-Chlor-4-fluorphenyl)methyl]amino}-2-oxoethyl)-3,5-dimethyl-1H-pyrazol-1-yl]-N,N-diethylacetamid,
N-[(2-Chlor-4-fluorphenyl)methyl]-2-{3,5-dimethyl-1-[2-(4-methyl-1-piperazinyl)-2-oxoethyl]-1H-pyrazol-4-yl}acetamid,
2-[4-(2-{[(2-Chlor-4-fluorphenyl)methyl]amino}-2-oxoethyl)-3,5-dimethyl-1H-pyrazol-1-yl]-N-[2-(dimethylamino)ethyl]acetamid,
N-[(2-Chlor-4-fluorphenyl)methyl]-2-[1-(2-hydroxyethyl)-3,5-dimethyl-1H-pyrazol-4-yl]acetamid,
N-[(2-Chlor-4-fluorphenyl)methyl]-2-{3,5-dimethyl-1-[2-(4-morpholinyl)ethyl]-1H-pyrazol-4-yl}acetamid,
N-[(2-Chlor-4-fluorphenyl)methyl]-2-{1-[2-(cyclohexylamino)ethyl]-3,5-dimethyl-1H-pyrazol-4-yl}acetamid,
N-[(2-Chlor-4-fluorphenyl)methyl]-2-{1-[2-(diethylamino)ethyl]-3,5-dimethyl-1H-pyrazol-4-yl}acetamid,
N-[(2-Chlor-4-fluorphenyl)methyl]-2-{3,5-dimethyl-1-[2-(tetrahydro-2H-pyran-4-yl-amino)ethyl]-1H-pyrazol-4-yl}acetamid,
N-[(2-Chlor-4-fluorphenyl)methyl]-2-{3,5-dimethyl-1-[2-(1-piperidinyl)ethyl]-1H-pyrazol-4-yl}acetamid,
N-[(2-Chlor-4-fluorphenyl)methyl]-2-{3,5-dimethyl-1-[2-(1-pyrrolidinyl)ethyl]-1H-pyrazol-4-yl}acetamid,
N-[(2-Chlor-4-fluorphenyl)methyl]-2-(3,5-dimethyl-1-{2-[(2-methylpropyl)amino]-ethyl}-1H-pyrazol-4-yl)acetamid,
N-[(2-Chlor-4-fluorphenyl)methyl]-2-(1-{2-[(2-hydroxyethyl)amino]ethyl}-3,5-dimethyl-1H-pyrazol-4-yl)acetamid,
N-[(2-Chlor-4-fluorphenyl)methyl]-2-(1-{2-[(2-hydroxyethyl)(methyl)amino]ethyl}-3,5-dimethyl-1H-pyrazol-4-yl)acetamid,
N-[(2-Chlor-4-fluorphenyl)methyl]-2-[3,5-dimethyl-1-(2-{[2-(methyloxy)ethyl]amino}-ethyl)-1H-pyrazol-4-yl]acetamid,
2-[1-(2-Aminoethyl)-3,5-dimethyl-1H-pyrazol-4-yl]-N-[(2-chlor-4-fluorphenyl)methyl]-acetamid,
N-[(2-Chlor-4-fluorphenyl)methyl]-2-{3,5-dimethyl-1-[2-(methyloxy)ethyl]-1H-pyrazol-4-yl}acetamid,
N-[(2-Chlor-4-fluorphenyl)methyl]-2-(3-methyl-1-phenyl-1H-pyrazol-4-yl)acetamid,
N-[(2-Chlor-4-fluorphenyl)methyl]-2-[3,5-dimethyl-1-(2-methyl-4-pyridinyl)-1H-pyrazol-4-yl]acetamid,
N-[(2-Chlor-4-fluorphenyl)methyl]-2-[3,5-dimethyl-1-(6-methyl-3-pyridinyl)-1H-pyrazol-4-yl]acetamid,
N-[(2-Chlor-4-fluorphenyl)methyl]-2-[1-(2,6-dimethyl-3-pyridinyl)-3,5-dimethyl-1H-pyrazol-4-yl]acetamid,
N-[(2-Chlor-4-fluorphenyl)methyl]-2-[3,5-dimethyl-1-(4-methyl-3-pyridinyl)-1H-pyrazol-4-yl]acetamid,
N-[(2-Chlor-4-fluorphenyl)methyl]-2-[3,5-dimethyl-1-(2-methyl-3-pyridinyl)-1H-pyrazol-4-yl]acetamid,
N-[(2-Chlor-4-fluorphenyl)methyl]-2-[3,5-dimethyl-1-(5-methyl-3-pyridinyl)-1H-pyrazol-4-yl]acetamid,
N-[(2-Chlor-4-fluorphenyl)methyl]-2-[3,5-dimethyl-1-(2-pyridinylmethyl)-1H-pyrazol-4-yl]acetamid,
N-[(2-Chlor-4-fluorphenyl)methyl]-2-{3,5-dimethyl-1-[4-(1-piperidinylcarbonyl)-phenyl]-1H-pyrazol-4-yl}acetamid,
N-[(2-Chlor-4-fluorphenyl)methyl]-2-{3,5-dimethyl-1-[4-(4-morpholinylcarbonyl)-phenyl]-1H-pyrazol-4-yl}acetamid,
N-[(2-Chlor-4-fluorphenyl)methyl]-2-(3,5-dimethyl-1-{4-[(4-methyl-1-piperazinyl)-carbonyl]phenyl}-1H-pyrazol-4-yl)acetamid,
4-[4-(2-{[(2-Chlor-4-fluorphenyl)methyl]amino}-2-oxoethyl)-3,5-dimethyl-1H-pyrazol-1-yl]-N-[2-(dimethylamino)ethyl]benzamid,
4-[4-(2-{[(2-Chlor-4-fluorphenyl)methyl]amino}-2-oxoethyl)-3,5-dimethyl-1H-pyrazol-1-yl]benzamid,
N-[(2-Chlor-4-fluorphenyl)methyl]-2-(3,5-dimethyl-1-{4-[(4-phenyl-1-piperazinyl)-carbonyl]phenyl}-1H-pyrazol-4-yl)acetamid,
4-[4-(2-{[(2-Chlor-4-fluorphenyl)methyl]amino}-2-oxoethyl)-3,5-dimethyl-1H-pyrazol-1-yl]-N-(2-hydroxyethyl)benzamid,
4-[4-(2-{[(2-Chlor-4-fluorphenyl)methyl]amino}-2-oxoethyl)-3,5-dimethyl-1H-pyrazol-1-yl]-N-(tetrahydro-2H-pyran-4-yl-methyl)benzamid,
4-[4-(2-{[(2-Chlor-4-fluorphenyl)methyl]amino}-2-oxoethyl)-3,5-dimethyl-1H-pyrazol-1-yl]-N-(2-pyridinylmethyl)benzamid,
N-(2-Amino-2-oxoethyl)-4-[4-(2-{[(2-chlor-4-fluorphenyl)methyl]amino}-2-oxoethyl)-3,5-dimethyl-1H-pyrazol-1-yl]benzamid,
N-[(3-Chlor-2-methylphenyl)methyl]-2-[1-(2-hydroxyethyl)-3,5-dimethyl-1H-pyrazol-4-yl]acetamid,
N-[(3-Chlor-2-methylphenyl)methyl]-2-{3,5-dimethyl-1-[2-(4-morpholinyl)ethyl]-1H-pyrazol-4-yl}acetamid,
N-[(2-Chlor-4-fluorphenyl)methyl]-2-[1-(2-hydroxy-2-methylpropyl)-3,5-dimethyl-1H-pyrazol-4-yl]acetamid,
2-[1-(2-Hydroxy-2-methylpropyl)-3,5-dimethyl-1H-pyrazol-4-yl]-N-[(2,3,4-trifluorphenyl)methyl]acetamid,
N-[(3-Chlor-2-methylphenyl)methyl]-2-[1-(2-hydroxy-2-methylpropyl)-3,5-dimethyl-1H-pyrazol-4-yl]acetamid,
N-[(2-Chlor-6-methylphenyl)methyl]-2-[1-(2-hydroxy-2-methylpropyl)-3,5-dimethyl-1H-pyrazol-4-yl]acetamid,
N-[(2-Chlor-4-fluorphenyl)methyl]-2-{1-[6-(ethyloxy)-2-pyridinyl]-1H-pyrazol-4-yl}-acetamid,
N-[(2-Chlor-4-fluorphenyl)methyl]-2-{3,5-dimethyl-1-[6-(methyloxy)-3-pyridinyl]-1H-pyrazol-4-yl}acetamid,
N-[(2-Chlor-4-fluorphenyl)methyl]-2-{3,5-dimethyl-1-[5-(methyloxy)-3-pyridinyl]-1H-pyrazol-4-yl}acetamid,
N-[(2-Chlor-4-fluorphenyl)methyl]-2-{3,5-dimethyl-1-[2-(methyloxy)-3-pyridinyl]-1H-pyrazol-4-yl}acetamid,
N-[(2-Chlor-4-fluorphenyl)methyl]-2-[1-(2-hydroxyethyl)-5-(trifluormethyl)-1H-pyrazol-4-yl]acetamid,
N-[(2-Chlor-4-fluorphenyl)methyl]-2-[1-(2-hydroxyethyl)-3-(trifluormethyl)-1H-pyrazol-4-yl]acetamid,
N-[(2-Chlor-4-fluorphenyl)methyl]-2-[1-{2-[(2-hydroxyethyl)amino]ethyl}-3-(trifluormethyl)-1H-pyrazol-4-yl]acetamid,
N-[(2-Chlor-4-fluorphenyl)methyl]-2-[1-[2-(tetrahydro-2H-pyran-4-yl-amino)ethyl]-3-(trifluormethyl)-1H-pyrazol-4-yl]acetamid,
N-[(2-Chlor-4-fluorphenyl)methyl]-2-[1-[2-(dimethylamino)ethyl]-5-(trifluormethyl)-1 H-pyrazol-4-yl]acetamid,
N-[(2-Chlor-4-fluorphenyl)methyl]-2-[1-[2-(tetrahydro-2H-pyran-4-yl-amino)ethyl]-5-(trifluormethyl)-1H-pyrazol-4-yl]acetamid,
N-[(2-Chlor-4-fluorphenyl)methyl]-2-[1-[2-(1-piperidinyl)ethyl]-5-(trifluormethyl)-1H-pyrazol-4-yl]acetamid,
N-[(2-Chlor-4-fluorphenyl)methyl]-2-[1-(2-{[2-(methyloxy)ethyl]amino}ethyl)-5-(trifluormethyl)-1H-pyrazol-4-yl]acetamid,
2-[3,5-Dimethyl-1-(2-pyrimidinyl)-1H-pyrazol-4-yl]-N-[(2,3,4-trifluorphenyl)methyl]-acetamid,
2-[5-Brom-1-(4-fluorphenyl)-1H-pyrazol-4-yl]-N-[(2-chlor-4-fluorphenyl)methyl]-acetamid oder
N-[(2-Chlor-4-fluorphenyl)methyl]-2-[5-ethyl-1-(4-fluorphenyl)-1H-pyrazol-4-yl]-acetamid;
oder ein pharmazeutisch verträgliches Salz davon.

18. Ein Arzneimittel, umfassend eine Verbindung oder ein pharmazeutisch verträgliches Salz davon wie in einem der vorhergehenden Ansprüche definiert und einen pharmazeutisch verträglichen Träger oder Exzipienten.

19. Eine Verbindung oder ein pharmazeutisch verträgliches Salz davon wie in einem der Ansprüche 1 bis 17 beansprucht zur Verwendung in der Therapie.

20. Verwendung einer Verbindung oder eines pharmazeutisch verträglichen Salzes davon wie in einem der Ansprüche 1 bis 17 definiert zur Herstellung eines Medikaments zur Behandlung oder Vorbeugung von Schmerz, Entzündung oder einer neurodegenerativen Erkrankung.

## Revendications

1. Composé de formule (I), ou un de ses sels pharmaceutiquement acceptables : 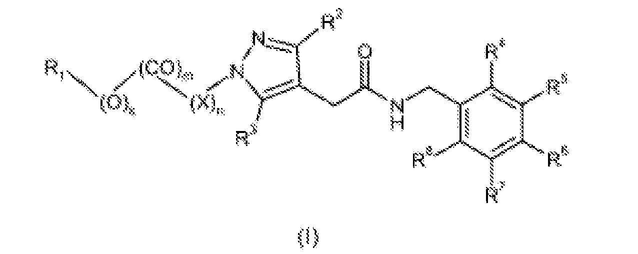 formule dans laquelle :
R¹ représente un groupe NR⁹R¹⁰, alkyle en C₁ à C₆ ou trifluorométhyle ; ou bien peut représenter un atome d'hydrogène lorsque k est égal à 1 ;
ou bien R¹ représente un groupe aryle en C₆ à C₁₀, un groupe hétéroaryle penta- ou hexagonal ou octo- à décagonal, un groupe cycloalkyle en C₃ à C₆ ou un groupe hétérocyclyle tétra- à undécagonal ; où n'importe quel noyau ou système cyclique desdits groupes aryle, hétéroaryle, cycloalkyle ou hétérocycliques peut être facultativement substitué avec 1 à 3 substituants, qui peuvent être identiques ou différents, choisis dans le groupe consistant en des substituants halogéno, hydroxyle, nitro, oxo, cyano, trifluorométhyle, alkyle en C₁ à C₆, alkoxy en C₁ à C₃, (alkoxy en C₁ à C₃) (alkyle en C₁ à C₃)-, -CO(alkyle en C₁ à C₃), -CONR¹¹R¹² et -NR¹¹R¹² ;
ou bien R¹ représente un groupe choisi parmi les groupes de formules (II), (III), (VI), (V), (VI) et (VII) : 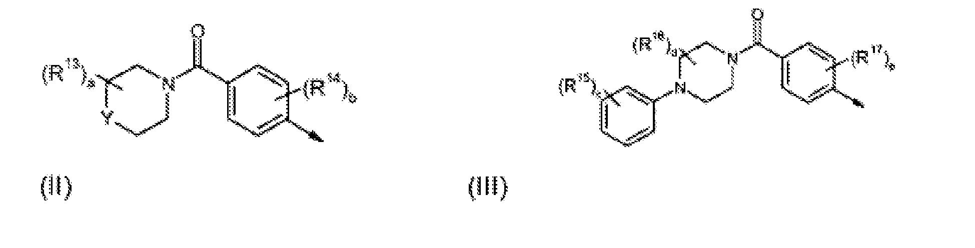 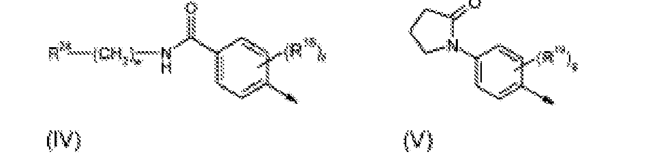 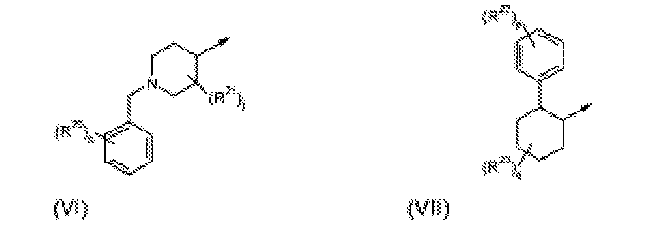
dans lesquelles :
Y représente un groupe CH₂, O ou NR¹¹ ;
w est égal à 0, 1, 2 ou 3 ;
a, b, c, d, e, f, g, h, j p et q sont égaux indépendamment à 0, 1 ou 2 ;
R¹³, R¹⁴, R¹⁵ R¹⁶, R¹⁷, R¹⁸, R¹⁹, R²⁰, R²¹, R²² et R²³ représentent indépendamment un groupe halogéno, cyano, alkyle en C₁ à C₃, alkoxy en C₁ à C₃ ou trifluorométhyle ;
R²⁴ représente un groupe NR¹¹R¹², -OH ou CONR¹¹R¹² ;
ou bien R²⁴ représente un groupe cycloalkyle en C₃ à C₆, un groupe aryle en C₆ à C₁₀, un groupe hétéroaryle penta- ou hexagonal ou octo- à décagonal, ou un groupe hétérocyclyle tétra- à undécagonal ; où n'importe quel noyau ou système cyclique desdits groupes aryle, hétéroaryle, cycloalkyle ou hétérocycliques peut être facultativement substitué avec 1 à 3 substituants, qui peuvent être identiques ou différents, choisis dans le groupe consistant en des substituants halogéno, hydroxyle, nitro, oxo, cyano, trifluorométhyle, alkyle en C₁ à C₃, alkoxy en C₁ à C₃, (alkoxy en C₁ à C₃)(alkyle en C₁ à C₃) -, -CO(alkyle en C₁ à C₃), -CONH₂ et -NR¹¹R¹² ;
et où :
R² représente un atome d'hydrogène, un groupe alkyle en C₁ à C₆, alkoxy en C₁ à C₄, trifluorométhyle, phényle, hétéroaryle monocyclique penta- ou hexagonal ou cycloalkyle en C₃ à C₆;
R³ représente un atome d'hydrogène, un groupe alkyle en C₁ à C₆, alkoxy en C₁ à C₄, trifluorométhyle, phényle, hétéroaryle monocyclique penta- ou hexagonal ou cycloalkyle en C₃ à C₆ ; ou bien R³ représente un groupe halogéno lorsque R² représente un atome d'hydrogène ;
R⁴ et R⁸ représentent indépendamment un atome d'hydrogène, un groupe halogéno, trifluorométhyle, alkyle en C₁ à C₆, phényle, phényloxy, NO₂ ou NH₂ ;
R⁵ et R⁷ représentent indépendamment un atome d'hydrogène, un groupe halogéno, trifluorométhyle, alkyle en C₁ à C₆, phényle, phényloxy ou NO₂;
R⁶ représente un atome d'hydrogène, un groupe halogéno, trifluorométhyle, trifluorométhoxy, alkyle en C₁ à C₆, cycloalkyle en C₃ à C₆, phényle, phényloxy, NO₂ ou cyano ;
R⁹ et R¹⁰ représentent indépendamment un atome d'hydrogène, un groupe alkyle en C₁ à C₄, (alkoxy en C₁ à C₄)(alkyle en C₁ à C₄)-, -(alkyle en C₁ à C₄)OH ou -(alkyle en C₁ à C₄)NR¹¹R¹² ;
ou un groupe cycloalkyle en C₃ à C₆ ou hétérocyclyle hexagonal, le noyau de l'un ou d'autre de ces groupes pouvant être facultativement substitué avec 1 à 3 substituants, qui peuvent être identiques ou différents, choisis dans le groupe consistant en des substituants halogéno, cyano, alkyle en C₁ à C₃, alkoxy en C₁ à C₃ et trifluorométhyle ;
R¹¹ et R¹² représentent indépendamment un atome d'hydrogène ou un groupe alkyle en C₁ à C₃.
k est égal à 0 ou 1 ;
m est égal à 0 ou 1 ;
n est égal à 0, 1, 2, 3 ou 4 ;
X représente un groupe CR²⁵R²⁶ ; et
R²⁵ et R²⁶, à chaque occurrence, représentent indépendamment un atome d'hydrogène ou de fluor ou un groupe alkyle en C₁ à C₆ ;
sous réserve que le composé de formule (I) soit autre que le 2-(3,5-diméthyl-1-phényl-1*H-*pyrazole-4-yl)-*N-*(phényl-méthyl)acétamide ou le 2-(3,5-diméthyl-1-phényl-1*H-*pyrazole-4-yl)-*N-*(2-fluorophényl)acétamide.

2. Composé ou un de ses sels pharmaceutiquement acceptables suivant la revendication 1, dans lequel R¹ représente un groupe NR⁹R¹⁰, alkyle en C₁ à C₆ ou trifluorométhyle ; ou bien peut représenter un atome d'hydrogène lorsque k est égal à 1 ; ou R¹ représente un groupe aryle en C₆ à C₁₀, hétéroaryle penta- ou hexagonal ou octo- à décagonal, cycloalkyle en C₃ à C₆ ou hétérocyclyle tétra- à undécagonal ; où n'importe quel noyau ou système cyclique desdits groupes aryle, hétéroaryle, cycloalkyle ou hétérocyclyle peut être facultativement substitué avec 1 à 3 substituants, qui peuvent être identiques ou différents, choisis dans le groupe consistant en des substituants halogéno, hydroxyle, nitro, oxo, cyano, trifluorométhyle, alkyle en C₁ à C₆, alkoxy en C₁ à C₃, (alkoxy en C₁ à C₃)(alkyle en C₁ à C₃)-, -CO(alkyle en C₁ à C₃), -CONR¹¹R¹² et -NR¹¹R¹².

3. Composé ou un de ses sels pharmaceutiquement acceptables suivant la revendication 2, dans lequel R¹ représente un groupe NR⁹R¹⁰, alkyle en C₁ à C₆ ou trifluorométhyle ; ou peut représenter un atome d'hydrogène lorsque k est égal à 1 ; ou bien R¹ représente un groupe phényle, hétéroaryle penta- ou hexagonal ou octo- à décagonal ou hétérocyclyle tétra- à undécagonal ; où n'importe quel noyau ou système cyclique desdits groupes phényle, hétéroaryle ou hétérocyclyle peut être facultativement substitué avec 1 à 3 substituants, qui peuvent être identiques ou différents, choisis dans le groupe consistant en des substituants halogéno, alkyle en C₁ à C₆, alkoxy en C₁ à C₃, trifluorométhyle et -NR¹¹R¹².

4. Composé ou un de ses sels pharmaceutiquement acceptables suivant la revendication 2, dans lequel
R¹ représente un groupe NR⁹R¹⁰, alkyle en C₁ à C₆ ou trifluorométhyle ; ou bien peut représenter un atome d'hydrogène lorsque k est égal à 1 ; ou
R¹ représente un groupe facultativement substitué choisi entre des groupes phényle, pyridinyle, pyrimidinyle, imidazolyle, pyrrolidinyle et pyrazolyle ; dans lequel les substituants facultatifs, qui peuvent être identiques ou différents, sont choisis dans le groupe consistant en des substituants halogéno, alkyle en C₁ à C₆, alkoxy en C₁ à C₃, -CONH₂ et trifluorométhyle.

5. Composé ou un de ses sels pharmaceutiquement acceptables suivant la revendication 1, dans lequel R¹ représente un groupe choisi parmi les groupes de formules (II), (III), (IV), (V), (VI) et (VII), dans lesquelles a, b, c, d, e, f, g, h, j, p et q sont égaux à 0.

6. Composé ou un de ses sels pharmaceutiquement acceptables suivant la revendication 1 ou 5, dans lequel R²⁴ représente un groupe NH₂, -OH ou -CONH₂ ; un groupe phényle, pyridinyle ou tétrahydropyrannyle non substitué ; et w est égal à 1 ou 2.

7. Composé ou un de ses sels pharmaceutiquement acceptables suivant l'une quelconque des revendications précédentes, dans lequel R² et R³ représentent indépendamment un atome d'hydrogène, un groupe alkyle en C₁ à C₆ ou trifluorométhyle.

8. Composé ou un de ses sels pharmaceutiquement acceptables suivant la revendication 7, dans lequel R² et R³ représentent tous deux un groupe méthyle.

9. Composé ou un de ses sels pharmaceutiquement acceptables suivant l'une quelconque des revendications précédentes, dans lequel R⁴, R⁵, R⁷ et R⁸ représentent indépendamment un atome d'hydrogène ou d'halogène ou un groupe alkyle en C₁ à C₆.

10. Composé ou un de ses sels pharmaceutiquement acceptables suivant l'une quelconque des revendications précédentes, dans lequel R⁶ représente un atome d'hydrogène ou d'halogène.

11. Composé ou un de ses sels pharmaceutiquement acceptables suivant l'une quelconque des revendications précédentes, dans lequel R⁴ et R⁸ représentent indépendamment un atome d'hydrogène ou d'halogène ou un groupe méthyle.

12. Composé ou un de ses sels pharmaceutiquement acceptables suivant la revendication 11, dans lequel un de R⁴ et R⁸ représente un atome de chlore et l'autre représente un atome d'hydrogène.

13. Composé ou un de ses sels pharmaceutiquement acceptables suivant la revendication 12, dans lequel un de R⁴ et R⁸ représente un atome de chlore et l'autre représente un atome d'hydrogène, et R⁶ représente un atome de fluor.

14. Composé ou un de ses sels pharmaceutiquement acceptables suivant l'une quelconque des revendications précédentes, dans lequel R⁵ et R⁷ représentent indépendamment un atome d'hydrogène ou d'halogène ou un groupe méthyle.

15. Composé ou un de ses sels pharmaceutiquement acceptables suivant la revendication 14, dans lequel R⁵ et R⁷ représentent des atomes d'hydrogène.

16. Composé ou sel suivant l'une quelconque des revendications précédentes, qui est un composé de formule (IA) ou un de ses sels pharmaceutiquement acceptables : 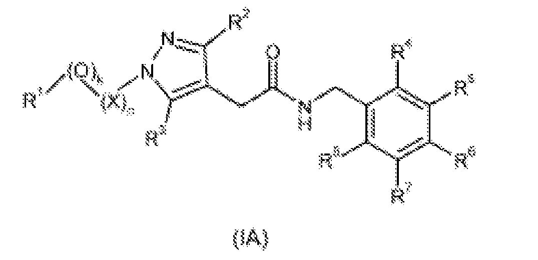 formule dans laquelle :
R¹ représente un groupe NR⁹R¹⁰, alkyle en C₁ à C₆ ou trifluorométhyle ; ou bien peut représenter un atome d'hydrogène lorsque k est égal à 1 ;
ou bien R¹ représente un groupe aryle en C₆ à C₁₀, un groupe hétéroaryle penta- ou hexagonal ou octo- à décagonal, un groupe cycloalkyle en C₃ à C₆ ou un groupe hétérocyclyle tétra- à undécagonal ; où n'importe quel noyau ou système cyclique desdits groupes aryle, hétéroaryle, cycloalkyle ou hétérocycliques peut être facultativement substitué avec 1 à 3 substituants, qui peuvent être identiques ou différents, choisis dans le groupe consistant en des substituants halogéno, hydroxyle, nitro, oxo, cyano, trifluorométhyle, alkyle en C₁ à C₆, alkoxy en C₁ à C₃, (alkoxy en C₁ à C₃)(alkyle en C₁ à C₃)-, -CO(alkyle en C₁ à C₃), -CONR¹¹R¹² et -NR¹¹R¹² ;
R² et R³ représentent indépendamment un atome d'hydrogène ou un groupe méthyle ou trifluorométhyle ;
R⁴ et R⁸ représentent indépendamment un atome d'hydrogène ou d'halogène ou un groupe méthyle ;
R⁵ et R⁷ représentent un atome d'hydrogène ;
R⁶ représente un atome d'halogène ;
R⁹ et R¹⁰ représentent indépendamment un atome d'hydrogène, un groupe alkyle en C₁ à C₄, (alkoxy en C₁ à C₄)(alkyle en C₁ à C₄)- ou -(alkyle en C₁ à C₄)OH ;
R¹¹ et R¹² représentent indépendamment un atome d'hydrogène ou un groupe méthyle ;
k est égal à 0 ou 1 ;
n est égal à 0, 1 ou 2 ;
X représente un groupe CR²⁵R²⁶ ; et
R²⁵ et R²⁶, à chaque occurrence, représentent indépendamment un atome d'hydrogène ou un groupe méthyle ;
sous réserve que le composé de formule (IA) soit autre que le 2-(3,5-diméthyl-1-phényl-1*H-*pyrazole-4-yl)-*N-*(phényl-méthyl)acétamide ou le 2-(3,5-diméthyl-1-phényl-1*H-*pyrazole-4-yl)-*N-*(2-fluorophényl)acétamide.

17. Composé suivant la revendication 1 ou un de ses sels pharmaceutiquement acceptables, qui est :
le *N-*[(3,4-dichlorophényl)méthyl]-2-(3,5-diméthyl-1-phényl-1*H-*pyrazole-4-yl)acétamide,
le *N-*[(3,5-dichlorophényl)méthyl]-2-(3,5-diméthyl-1-phényl-1*H-*pyrazole-4-yl)acétamide,
le 2-(3,5-diméthyl-1-phényl-1*H-*pyrazole-4-yl)-*N-*({4-[(trifluorométhyl)oxy]phényl}méthyl)acétamide,
le 2-(3,5-diméthyl-1-phényl-1*H-*pyrazole-4-yl)-*N-*{[2-fluoro-5-(trifluorométhyl)phényl]méthyl}acétamide,
le 2-(3,5-diméthyl-1-phényl-1*H-*pyrazole-4-yl)-*N-*{[4-(trifluorométhyl)phényl]méthyl}acétamide,
le 2-(3,5-diméthyl-1-phényl-1*H-*pyrazole-4-yl)-*N-*{[4-fluoro-3-(trifluorométhyl)phényl]méthyl}acétamide,
le *N*-[(3-chloro-4-fluorophényl)méthyl]-2-(3,5-diméthyl-1-phényl-1*H-*pyrazole-4-yl)acétamide,
le 2-(3,5-diméthyl-1-phényl-1*H-*pyrazole-4-yl)-*N-*{[3-fluoro-5-(trifluorométhyl)phényl]méthyl}acétamide,
le *N-*[(2-chloro-4-fluorophényl)méthyl]-2-(3,5-diméthyl-1-phényl-1*H-*pyrazole-4-yl)acétamide,
le *N-*(2-biphénylylméthyl)-2-(3,5-diméthyl-1-phényl-1*H-*pyrazole-4-yl)acétamide,
le *N-*[(2,5-difluorophényl)méthyl]-2-(3,5-diméthyl-1-phényl-1*H-*pyrazole-4-yl)acétamide,
le *N-*[(3,4-difluorophényl)méthyl]-2-(3,5-diméthyl-1-phényl-1*H-*pyrazole-4-yl)acétamide,
le *N-*[(2-bromophényl)méthyl]-2-(3,5-diméthyl-1-phényl-1*H-*pyrazole-4-yl)acétamide,
le *N-*[(3-bromophényl)méthyl]-2-(3,5-diméthyl-1-phényl-1*H-*pyrazole-4-yl)acétamide,
le *N-*[(4-bromophényl)méthyl]-2-(3,5-diméthyl-1-phényl-1*H-*pyrazole-4-yl)acétamide,
le 2-(3,5-diméthyl-1-phényl-1*H-*pyrazole-4-yl)-*N-*[(2-nitrophényl)méthyl]acétamide,
le 2-(3,5-diméthyl-1-phényl-1*H-*pyrazole-4-yl)-*N-*[(3-nitrophényl)méthyl]acétamide,
le 2-(3,5-diméthyl-1-phényl-1*H-*pyrazole-4-yl)-*N-*[(4-nitrophényl)méthyl]acétamide,
le *N-*[(3-chloro-2-méthylphényl)méthyl]-2-(3,5-diméthyl-1-phényl-1*H-*pyrazole-4-yl)acétamide,
le 2-(3,5-diméthyl-1-phényl-1*H-*pyrazole-4-yl)-*N-*{[3-fluoro-4-(trifluorométhyl)phényl]méthyl}acétamide,
le 2-(3,5-diméthyl-1-phényl-1*H-*pyrazole-4-yl)-*N-*[(2,3,4-trifluorophényl)méthyl]acétamide,
le *N-*[(2,3-difluoro-4-méthylphényl)méthyl]-2-(3,5-diméthyl-1-phényl-1*H-*pyrazole-4-yl)acétamide,
le *N-*[(2,5-diméthylphényl)méthyl]-2-(3,5-diméthyl-1-phényl-1*H-*pyrazole-4-yl)acétamide,
le *N-*[(5-chloro-2-méthylphényl)méthyl]-2-(3,5-diméthyl-1-phényl-1*H-*pyrazole-4-yl)acétamide,
le *N-*[(2,6-difluorophényl)méthyl]-2-(3,5-diméthyl-1-phényl-1*H-*pyrazole-4-yl)acétamide,
le *N-*[(2-chloro-6-méthylphényl)méthyl]-2-(3,5-diméthyl-1-phényl-1*H-*pyrazole-4-yl)acétamide,
le *N-*(4-biphénylylméthyl)-2-(3,5-diméthyl-1-phényl-1*H-*pyrazole-4-yl)acétamide,
le *N-*[(2,6-diméthylphényl)méthyl]-2-(3,5-diméthyl-1-phényl-1*H-*pyrazole-4-yl)acétamide,
le *N-*[(2-aminophényl)méthyl]-2-(3,5-diméthyl-1-phényl-1*H-*pyrazole-4-yl)acétamide,
le *N-*(3-biphénylylméthyl)-2-(3,5-diméthyl-1-phényl-1*H-*pyrazole-4-yl)acétamide,
le 2-(3,5-diméthyl-1-phényl-1*H-*pyrazole-4-yl)-*N-*[(5-fluoro-2-méthylphényl)méthyl]acétamide,
le *N-*{[3,5-bis(trifluorométhyl)phényl]méthyl}-2-(3,5-diméthyl-1-phényl-1*H-*pyrazole-4-yl)acétamide,
le *N-*[(4-cyanophényl)méthyl]-2-(3,5-diméthyl-1-phényl-1*H-*pyrazole-4-yl)acétamide,
le *N-*[(2-chlorophényl)méthyl]-2-(3,5-diméthyl-1-phényl-1*H-*pyrazole-4-yl)acétamide,
le *N-*[(2,3-difluorophényl)méthyl]-2-(3,5-diméthyl-1-phényl-1*H-*pyrazole-4-yl)acétamide,
le *N-*{[2-chloro-6-(phényloxy)phényl]méthyl}-2-(3,5-diméthyl-1-phényl-1*H-*pyrazole-4-yl)acétamide,
le *N-*[(2,3-dichlorophényl)méthyl]-2-(3,5-diméthyl-1-phényl-1*H-*pyrazole-4-yl)acétamide,
le 2-(3,5-diméthyl-1-phényl-1*H-*pyrazole-4-yl)-*N-*{[4-(phényloxy)phényl]méthyl}acétamide,
le *N-*[(2,4-dichlorophényl)méthyl]-2-(3,5-diméthyl-1-phényl-1*H-*pyrazole-4-yl)acétamide,
le *N-*[(2,3-diméthylphényl)méthyl]-2-(3,5-diméthyl-1-phényl-1*H-*pyrazole-4-yl)acétamide,
le *N-*[(2,6-dichlorophényl)méthyl]-2-(3,5-diméthyl-1-phényl-1*H-*pyrazole-4-yl)acétamide,
le *N-*[(2,5-dichlorophényl)méthyl]-2-(3,5-diméthyl-1-phényl-1*H-*pyrazole-4-yl)acétamide,
le *N-*[(2-chloro-6-fluorophényl)méthyl]-2-(3,5-diméthyl-1-phényl-1*H-*pyrazole-4-yl)acétamide,
le *N-*{[2-chloro-5-(trifluorométhyl)phényl]méthyl}-2-(3,5-diméthyl-1-phényl-1*H-*pyrazole-4-yl)acétamide,
le 2-(3,5-diméthyl-1-phényl-1*H*-pyrazole-4-yl)-*N-*{[5-fluoro-2-(trifluorométhyl)phényl]méthyl}acétamide,
le 2-(3,5-diméthyl-1-phényl-1*H-*pyrazole-4-yl)-*N-*{[4-fluoro-2-(trifluorométhyl)phényl]méthyl}acétamide,
le *N-*[(5-chloro-2-fluorophényl)méthyl]-2-(3,5-diméthyl-1-phényl-1*H*-pyrazole-4-yl)acétamide,
le *N-*[(2-chloro-4-fluorophényl)méthyl]-2-[1-(4-fluoro-phényl)-3,5-diméthyl-1*H-*pyrazole-4-yl]acétamide,
le *N-*[(2-chloro-4-fluorophényl)méthyl]-2-{1-[2-(diméthyl-amino)éthyl]-3,5-diméthyl-1*H-*pyrazole-4-yl]acétamide,
le *N-*[(2-chloro-4-fluorophényl)méthyl]-2-[1-(1*H-*imidazole-4-ylméthyl)-3,5-diméthyl-1*H-*pyrazole-4-yl]acétamide,
le *N-*[(2-chloro-4-fluorophényl)méthyl]-2-(1,3,5-triméthyl-1*H-*pyrazole-4-yl]acétamide,
le *N-*[(2-chloro-4-fluorophényl)méthyl]-2-[3,5-diméthyl-1-(phénylméthyl)-1*H-*pyrazole-4-yl]acétamide,
le *N-*[(2-chloro-4-fluorophényl)méthyl]-2-[1-2,4-difluoro-phényl)-3,5-diméthyl-1*H-*pyrazole-4-yl]acétamide,
le *N-*[(2-chloro-4-fluorophényl)méthyl]-2-[1-(4-chloro-phényl)-3,5-diméthyl-1*H-*pyrazole-4-yl]acétamide,
le *N-*[(2-chloro-4-fluorophényl)méthyl]-2-{3,5-diméthyl-1-[4-(méthyloxy)phényl]-1*H-*pyrazole-4-yl}acétamide,
le *N-*[(2-chloro-4-fluorophényl)méthyl]-2-[1-(3-fluoro-phényl)-3,5-diméthyl-1*H-*pyrazole-4-yl]acétamide,
le *N-*[(2-chloro-4-fluorophényl)méthyl]-2-[1-(3-chloro-phényl)-3,5-diméthyl-1*H-*pyrazole-4-yl]acétamide,
le *N-*[(2-chloro-4-fluorophényl)méthyl]-2-{3-(méthyloxy)-phény]-1*H-*pyrazole-4-yl}acétamide,
le *N-*[(2-chloro-4-fluorophényl)méthyl]-2-[1-(2-fluoro-phényl)-3,5-diméthyl-1*H-*pyrazole-4-yl]acétamide,
le *N-*[(2-chloro-4-fluorophényl)méthyl]-2-[1-(2-chloro-phényl)-3,5-diméthyl-1*H*-pyrazole-4-yl]acétamide,
le *N-*[(2-chloro-4-fluorophényl)méthyl]-2-{3,5-diméthyl-1-[2-(méthyloxy)phényl]-1*H-*pyrazole-4-yl}acétamide,
le *N-*[(2-chloro-4-fluorophényl)méthyl]-2-[3,5-diméthyl-1-(4-méthylphényl)-1*H-*pyrazole-4-yl]acétamide,
le *N-*[(2-chloro-4-fluorophényl)méthyl]-2-{1-[4-(éthyloxy)-phényl]-3,5-diméthyl-1*H-*pyrazole-4-yl}acétamide,
le *N-*[(2-chloro-4-fluorophényl)méthyl]-2-[1-(5-cyano-2-pyridinyl)-3,5-diméthyl-1*H-*pyrazole-4-yl]acétamide,
le *N-*[(2-chloro-4-fluorophényl)méthyl]-2-[1-(3,5-dichloro-2-pyridinyl)-3,5-diméthyl-1*H*-pyrazole-4-yl]acétamide,
le *N-*[(2-chloro-4-fluorophényl)méthyl]-2-[3,5-diméthyl-1-(4-pyridinyl)-1*H-*pyrazole-4-yl]acétamide,
le *N-*[(2-chloro-4-fluorophényl)méthyl]-2-{3,5-diméthyl-1-[6-méthyl-4-(trifluorométhyl)-2-pyridinyl]-1*H-*pyrazole-4-yl}acétamide,
le 2-[1-(5-bromo-2-pyridinyl)-3,5-diméthyl-1*H-*pyrazole-4-yl]-*N-*[(2-chloro-4-fluorophényl)méthyl]acétamide,
le *N-*[(2-chloro-4-fluorophényl)méthyl]-2-[1-(5-chloro-2-pyridinyl)-3,5-diméthyl-1*H-*pyrazole-4-yl]acétamide,
le *N-*[(2-chloro-4-fluorophényl)méthyl]-2-[3,5-diméthyl-1-(1,3-thiazole-2-yl)-1*H-*pyrazole-4-yl]acétamide,
le *N-*[(2-chloro-4-fluorophényl)méthyl]-2-{3,5-diméthyl-1-[5-(trifluorométhyl)-2-pyridinyl]-1*H-*pyrazole-4-yl}acétamide,
le *N-*[(2-chloro-4-fluorophényl)méthyl]-2-{3,5-diméthyl-1-[3-(trifluorométhyl)-2-pyridinyl]-1*H-*pyrazole-4-yl}acétamide,
le *N-*[(2-chloro-4-fluorophényl)méthyl]-2-[3,5-diméthyl-1-(3-pyridinyl)-1*H-*pyrazole-4-yl]acétamide,
le *N-*[(2-chloro-4-fluorophényl)méthyl]-2-[1-(1,1-diméthyl-éthyl)-3,5-diméthyl-1*H-*pyrazole-4-yl]acétamide,
le *N-*[(2-chloro-4-fluorophényl)méthyl]-2-[3,5-diméthyl-1-(2-pyridinyl)-1*H-*pyrazole-4-yl]acétamide,
le *N-*[(2-chloro-4-fluorophényl)méthyl]-2-[1-(4-iodo-phényl)-3,5-diméthyl-1*H-*pyrazole-4-yl]acétamide,
le *N-*[(2-chloro-4-fluorophényl)méthyl]-2-[3,5-diméthyl-1-(4-pyridinylméthyl)-1*H-*pyrazole-4-yl]acétamide,
le *N-*[(2-chloro-4-fluorophényl)méthyl]-2-[3,5-diméthyl-1-(6-méthyl-3-pyridazinyl)-1*H-*pyrazole-4-yl]acétamide,
le *N-*[(2-chloro-4-fluorophényl)méthyl]-2-[3,5-diméthyl-1-(2-pyrimidinyl)-1*H-*pyrazole-4-yl]acétamide,
le *N-*[(2-chloro-4-fluorophényl)méthyl]-2-[1-(2,6-diméthyl-4-pyrimidinyl)-3,5-diméthyl-1*H-*pyrazole-4-yl]acétamide,
le *N-*[(2-chloro-4-fluorophényl)méthyl]-2-{3,5-diméthyl-1-[4-(trifluorométhyl)-2-pyrimidinyl]-1*H-*pyrazole-4-yl}-acétamide,
le *N-*[(2-chloro-4-fluorophényl)méthyl]-2-[3,5-diméthyl-1-(2-phényléthyl)-1*H-*pyrazole-4-yl]acétamide,
le *N-*[(2-chloro-4-fluorophényl)méthyl]-2-(1-cyclohexyl-3,5-diméthyl-1*H-*pyrazole-4-yl)acétamide,
le *N-*[(2-chloro-4-fluorophényl)méthyl]-2-{1-[(2-chloro-phényl)méthyl]-3,5-diméthyl-1*H-*pyrazole-4-yl}acétamide,
le *N-*[(2-chloro-4-fluorophényl)méthyl]-2-[3,5-diméthyl-1-(2-méthylpropyl)-1*H-*pyrazole-4-yl]acétamide,
le *N-*[(2-chloro-4-fluorophényl)méthyl]-2-(1-cyclopentyl-3,5-diméthyl-1*H-*pyrazole-4-yl)acétamide,
le *N-*[(2-chloro-4-fluorophényl)méthyl]-2-[3,5-diméthyl-1-(tétrahydro-2*H-*pyranne)-1*H-*pyrazole-4-yl]acétamide,
le *N-*[(2-chloro-4-fluorophényl)méthyl]-2-{1-[(2,4-difluoro-phényl)méthyl]-3,5-diméthyl-1*H-*pyrazole-4-yl}acétamide,
le *N-*[(2-chloro-4-fluorophényl)méthyl]-2-{1-[(4-chloro-phényl)méthyl]-3,5-diméthyl-1*H-*pyrazole-4-yl}acétamide,
le *N-*[(2-chloro-4-fluorophényl)méthyl]-2-(3,5-diméthyl-1{[4-(méthyloxy)phényl]méthyl}-1*H-*pyrazole-4-yl)acétamide,
le *N-*[(2-chloro-4-fluorophényl)méthyl]-2-{3,5-diméthyl-1-[1-(phénylméthyl)-4-pipéridinyl]-1*H-*pyrazole-4-yl}acétamide,
le *N-*[(2-chloro-4-fluorophényl)méthyl]-2-(3,5-diméthyl-1{[3-(méthyloxy)phényl]méthyl}-1*H-*pyrazole-4-yl)acétamide,
le *N-*[(2-chloro-4-fluorophényl)méthyl]-2-(3,5-diméthyl-1-{[2-(méthyloxy)phényl]méthyl}-1*H-*pyrazole-4-yl)acétamide,
le *N-*[(2-chloro-4-fluorophényl)méthyl]-2-[1-(2,2-diméthyl-propyl)-3,5-diméthyl-1*H-*pyrazole-4-yl]acétamide,
le *N-*[(2-chloro-4-fluorophényl)méthyl]-2-(1-{[2-diméthyl-amino)phényl]méthyl}-3,5-diméthyl-1*H-*pyrazole-4-yl)acétamide,
le *N-*[(2-chloro-4-fluorophényl)méthyl]-2-[3,5-diméthyl-1-(1-méthyl-4-pipéridinyl)-1*H-*pyrazole-4-yl]acétamide,
le 2-[1-(1-azabicyclo[2.2.1]hept-3-yl)-3,5-diméthyl-1*H-*pyrazole-4-yl]-*N*-[(2-chloro-4-fluorophényl)méthyl]acétamide,
le *N-*[(2-chloro-4-fluorophényl)méthyl]-2-(3,5-diméthyl-1-{1-méthyl-2-[4-(méthyloxy)phényl]éthyl}-1*H-*pyrazole-4-yl)-acétamide,
le *N-*[(2-chloro-4-fluorophényl)méthyl]-2-[3,5-diméthyl-1-(2,2,2-trifluoréthyl)-1*H-*pyrazole-4-yl]acétamide,
le *N-*[(2-chloro-4-fluorophényl)méthyl]-2-(1-{2-[(2,6(diméthylphényl)oxy]éthyl}-3,5-diméthyl-1*H-*pyrazole-4-yl)-acétamide,
le 2-[1-(1,3-benzothiazole-2-ylméthyl)-3,5-diméthyl-1*H-*pyrazole-4-yl]-*N-*[(2-chloro-4-fluorophényl)méthyl]acétamide,
le *N-*[(2-chloro-4-fluorophényl)méthyl]-2-[3,5-diméthyl-1-(2-phénylcyclohexyl)-1*H-*pyrazole-4-yl]acétamide,
le *N-*[(2-chloro-4-fluorophényl)méthyl]-2-{3,5-diméthyl-1-[(4-méthylphényl)méthyl]-1*H-*pyrazole-4-yl}acétamide,
le 2-(1-{[3,4-bis(méthyloxy)phényl]méthyl}-3,5-diméthyl-1*H-*pyrazole-4-yl)-*N-*[(2-chloro-4-fluorophényl)méthyl]acétamide,
le *N-*[(2-chloro-4-fluorophényl)méthyl]-2-[3,5-diméthyl-1-(1-méthyléthyl)-1*H-*pyrazole-4-yl]acétamide,
le *N-*[(2-chloro-4-fluorophényl)méthyl]-2-{3,5-diméthyl-1-[3-(phényloxy)propyl]-1*H-*pyrazole-4-yl}acétamide,
le *N-*[(2-chloro-4-fluorophényl)méthyl]-2-[1-(cyclohexyl-méthyl)-3,5-diméthyl-1*H-*pyrazole-4-yl]acétamide,
le *N-*[(2-chloro-4-fluorophényl)méthyl]-2-(1-éthyl-3,5-diméthyl-1*H-*pyrazole-4-yl)acétamide,
le *N-*[(2-chloro-4-fluorophényl)méthyl]-2-{3,5-diméthyl-1-[2-(phényloxy)éthyl]-1*H-*pyrazole-4-yl}acétamide,
le *N-*[(2-chloro-4-fluorophényl)méthyl]-2-[3,5-diméthyl-1-(1-méthyl-2-phényléthyl)-1*H-*pyrazole-4-yl]acétamide,
le *N-*[(2-chloro-4-fluorophényl)méthyl]-2-(1-{2-[(4-chloro-3-méthylphényl)oxy]éthyl}-3,5-diméthyl-1*H*-pyrazole-4-yl)-acétamide,
le *N-*[(2-chloro-4-fluorophényl)méthyl]-2-{3,5-diméthyl-1-[4-(2-oxo-1-pyrrolidinyl)phényl]-1*H-*pyrazole-4-yl}acétamide,
le *N-*[(2-chloro-4-fluorophényl)méthyl]-2-[1-(4-fluoro-phényl)-1*H-*pyrazole-4-yl]acétamide,
le *N-*[(2-chloro-4-fluorophényl)méthyl]-2-[1-(4-fluoro-phényl)-5-(trifluorométhyl)-1*H-*pyrazole-4-yl]acétamide,
le *N-*[(2-chloro-4-fluorophényl)méthyl]-2-[1-(2-méthyl-phényl)-1*H-*pyrazole-4-yl]acétamide,
le *N-*[(2-chloro-4-fluorophényl)méthyl]-2-[1-(2-éthyl-phényl)-1*H*-pyrazole-4-yl]acétamide,
le *N-*[(2-chloro-4-fluorophényl)méthyl]-2-{3,5-diméthyl-1-[4-(trifluorométhyl)phényl]-1*H-*pyrazole-4-yl}acétamide,
le *N-*[(2-chloro-4-fluorophényl)méthyl]-2-[3,5-diméthyl-1-(3-pyridinylméthyl)-1*H-*pyrazole-4-yl]acétamide,
le *N-*[(2-chloro-4-fluorophényl)méthyl]-2-(5-méthyl-1-phényl-1*H-*pyrazole-4-yl)acétamide,
le 2-(3-butyl-5-méthyl-1-phényl-1*H-*pyrazole-4-yl)-*N-*[(2-chloro-4-fluorophényl)méthyl]acétamide,
le *N*-[(2-chloro-4-fluorophényl)méthyl]-2-[3-méthyl-5-(1-méthyléthyl)-1-phényl-1*H-*pyrazole-4-yl]acétamide,
le *N-*[(2-chloro-4-fluorophényl)méthyl]-2-[5-méthyl-3-(1-méthyléthyl)-1-phényl-1*H-*pyrazole-4-yl]acétamide,
le *N-*[(2-chloro-4-fluorophényl)méthyl]-2-(3-méthyl-1,5-diphényl-1*H-*pyrazole-4-yl)acétamide,
le *N-*[(2-chloro-4-fluorophényl)méthyl]-2-[1-(4-fluoro-phényl)-5-méthyl-1*H-*pyrazole-4-yl]acétamide,
le [4-(2-{[(2-chloro-4-fluorophényl)méthyl]amino}-2-oxo-éthyl)-3,5-diméthyl-1*H-*pyrazole-1-yl]acétate d'éthyle,
l'acide [4-(2-{[(2-chloro-4-fluorophényl)méthyl]amino}-2-oxoéthyl)-3,5-diméthyl-1*H-*pyrazole-1-yl]acétique,
le *N-*[(2-chloro-4-fluorophényl)méthyl]-2-{3,5-diméthyl-1-[2-(4-morpholinyl)2-oxoéthyl]-1*H-*pyrazole-4-yl}acétamide,
le *N-*[(2-chloro-4-fluorophényl)méthyl]-2-{3,5-diméthyl-1-[2-oxo-2-(1-pipéridinyl)éthyl]-1*H-*pyrazole-4-yl}acétamide,
le 2-[4-(2-{[(2-chloro-4-fluorophényl)méthyl]amino}-2-oxoéthyl)-3,5-diméthyl-1*H-*pyrazole-1-yl]-N,*N-*diéthylacétamide,
le *N-*[(2-chloro-4-fluorophényl)méthyl]-2-{3,5-diméthyl-1-[2-(4-méthyl-1-pipérazinyl)-2-oxoéthyl]-1*H-*pyrazole-4-yl}-acétamide,
le 2-[4-(2-{[(2-chloro-4-fluorophényl)méthyl]amino}-2-oxoéthyl)-3,5-diméthyl-1*H-*pyrazole-1-yl]-*N-*[2-(diméthylamino)-éthyl]acétamide,
le *N-*[(2-chloro-4-fluorophényl)méthyl]-2-[1-(2-hydroxy-éthyl)-3,5-diméthyl-1*H-*pyrazole-4-yl]acétamide,
le *N-*[(2-chloro-4-fluorophényl)méthyl]-2-{3,5-diméthyl-1-[2-(4-morpholinyl)-éthyl]-1*H-*pyrazole-4-yl}acétamide,
le *N-*[(2-chloro-4-fluorophényl)méthyl]-2-{1-[2-(cyclo-hexylamino)éthyl]-3,5-diméthyl-1*H-*pyrazole-4-yl}acétamide,
le *N-*[(2-chloro-4-fluorophényl)méthyl]-2-{1-[2-(diéthyl-amino)éthyl]-3,5-diméthyl-1*H-*pyrazole-4-yl}acétamide,
le *N-*[(2-chloro-4-fluorophényl)méthyl]-2-{3,5-diméthyl-1-[2-(tétrahydro-2*H-*pyranne-4-ylamino)éthyl]-1*H-*pyrazole-4-yl}acétamide,
le *N-*[(2-chloro-4-fluorophényl)méthyl]-2-{3,5-diméthyl-1-[2-(1-pipéridinyl)éthyl]-1*H-*pyrazole-4-yl}acétamide,
le *N-*[(2-chloro-4-fluorophényl)méthyl]-2-{3,5-diméthyl-1-[2-(1-pyrrolidinyl)éthyl]-1*H-*pyrazole-4-yl}acétamide,
le *N-*[(2-chloro-4-fluorophényl)méthyl]-2-(3,5-diméthyl-1-{2-[(2-méthylpropyl)amino]éthyl}-1*H-*pyrazole-4-yl)acétamide,
le *N-*[(2-chloro-4-fluorophényl)méthyl]-2-(1-{2-[(2-hydroxy-éthyl)amino]éthyl}-3,5-diméthyl-1*H-*pyrazole-4-yl)acétamide,
le *N-*[(2-chloro-4-fluorophényl)méthyl]-2-(1-{2-[(2-hydroxy-éthyl)(méthyl)amino]éthyl}-3,5-diméthyl-1*H*-pyrazole-4-yl)-acétamide,
le *N-*[(2-chloro-4-fluorophényl)méthyl]-2-[3,5-diméthyl-1-(2-{[2-(méthyloxy)éthyl]amino}éthyl)-1*H-*pyrazole-4-yl]-acétamide,
le 2-[1-(2-aminoéthyl)-3,5-diméthyl-1*H-*pyrazole-4-yl]-*N-*[(2-chloro-4-fluorophényl)méthyl]acétamide,
le *N-*[(2-chloro-4-fluorophényl)méthyl]-2-{3,5-diméthyl-1-[2-(méthyloxy)éthyl]-1*H-*pyrazole-4-yl}acétamide,
le *N-*[(2-chloro-4-fluorophényl)méthyl]-2-(3-méthyl-1-phényl-1*H-*pyrazole-4-yl)acétamide,
le *N-*[(2-chloro-4-fluorophényl)méthyl]-2-[3,5-diméthyl-1-(2-méthyl-4-pyridinyl)-1*H-*pyrazole-4-yl]-acétamide,
le *N-*[(2-chloro-4-fluorophényl)méthyl]-2-[3,5-diméthyl-1-(6-méthyl-3-pyridinyl)-1*H-*pyrazole-4-yl]-acétamide,
le *N-*[(2-chloro-4-fluorophényl)méthyl]-2-[1-(2,6-diméthyl-3-pyridinyl)-3,5-diméthyl-1*H-*pyrazole-4-yl]-acétamide,
le *N-*[(2-chloro-4-fluorophényl)méthyl]-2-[3,5-diméthyl-1-(4-méthyl-3-pyridinyl)-1*H-*pyrazole-4-yl]-acétamide,
le *N-*[(2-chloro-4-fluorophényl)méthyl]-2-[3,5-diméthyl-1-(2-méthyl-3-pyridinyl)-1*H-*pyrazole-4-yl]-acétamide,
le *N-*[(2-chloro-4-fluorophényl)méthyl]-2-[3,5-diméthyl-1-(5-méthyl-3-pyridinyl)-1*H-*pyrazole-4-yl]-acétamide,
le *N-*[(2-chloro-4-fluorophényl)méthyl]-2-[3,5-diméthyl-1-(2-pyridinylméthyl)-1*H-*pyrazole-4-yl]-acétamide,
le *N-*[(2-chloro-4-fluorophényl)méthyl]-2-{3,5-diméthyl-1-[4-(1-pipéridinylcarbonyl)phényl]-1*H-*pyrazole-4-yl}acétamide,
le *N-*[(2-chloro-4-fluorophényl)méthyl]-2-{3,5-diméthyl-1-[4-(4-morpholinylcarbonyl)phényl]-1*H-*pyrazole-4-yl}acétamide,
le *N-*[(2-chloro-4-fluorophényl)méthyl]-2-(3,5-diméthyl-1-(4-[(4-méthyl-1-pipérazinyl) carbonyl]phényl)-1*H-*pyrazole-4-yl)acétamide,
le 4-[4-(2-{[(2-chloro-4-fluorophényl)méthyl]amino}-2-oxoéthyl)-3,5-diméthyl-1*H-*pyrazole-1-yl]-*N-*[2-(diméthylamino)-éthyl]benzamide,
le 4-[4-(2-{[(2-chloro-4-fluorophényl)méthyl]amino}-2-oxoéthyl)-3,5-diméthyl-1*H-*pyrazole-1-yl]-benzamide,
le *N-*[(2-chloro-4-fluorophényl)méthyl]-2-(3,5-diméthyl-1-{4-[(4-phényl-1-pipérazinyl)carbonyl]phényl}-1*H-*pyrazole-4-yl)acétamide,
le 4-[4-(2-{[(2-chloro-4-fluorophényl)méthyl]amino}-2-oxoéthyl)-3,5-diméthyl-1*H*-pyrazole-1-yl]-*N*-(2-hydroxyéthyl)-benzamide,
le 4-[4-(2-{[(2-chloro-4-fluorophényl)méthyl]amino}-2-oxoéthyl)-3,5-diméthyl-1*H*-pyrazole-1-yl]-*N*-(tétrahydro-2*H-*pyranne-4-ylméthyl)benzamide,
le 4-[4-(2-{[(2-chloro-4-fluorophényl)méthyl]amino}-2-oxoéthyl)-3,5-diméthyl-1*H*-pyrazole-1-yl]-*N*-(2-pyridinylméthyl)-benzamide,
le *N*-(2-amino-2-oxoéthyl)-4-[4-(2-{[(2-chloro-4-fluoro-phényl)méthyl]amino}-2-oxoéthyl)-3,5-diméthyl-1*H-*pyrazole-1-yl]benzamide,
le *N*-[(3-chloro-2-méthylphényl)méthyl]-2-[1-(2-hydroxy-éthyl)-3,5-diméthyl-1*H*-pyrazole-4-yl]acétamide,
le *N*-[(3-chloro-2-méthylphényl)méthyl]-2-{3,5-diméthyl-1-[2-(4-morpholinyl)éthyl]-1*H*-pyrazole-4-yl}acétamide,
le *N*-[(2-chloro-4-fluorophényl)méthyl]-2-[1-(2-hydroxy-2-méthylpropyl)-3,5-diméthyl-1*H*-pyrazole-4-yl]acétamide,
le 2-[1-(2-hydroxy-2-méthylpropyl)-3,5-diméthyl-1*H*-pyrazole-4-yl]-*N*-[(2,3,4-trifluorophényl)méthyl]acétamide,
le *N*-[(3-chloro-4-méthylphényl)méthyl]-2-[1-(2-hydroxy-2-méthylpropyl)-3,5-diméthyl-1*H*-pyrazole-4-yl]acétamide,
le *N*-[(2-chloro-6-méthylphényl)méthyl]-2-[1-(2-hydroxy-2-méthylpropyl)-3,5-diméthyl-1*H*-pyrazole-4-yl]acétamide,
le *N*-[(2-chloro-4-fluorophényl)méthyl]-2-{1-[6-(éthyloxy)-2-pyridinyl]-1*H*-pyrazole-4-yl}acétamide,
le *N*-[(2-chloro-4-fluorophényl)méthyl]-2-{3,5-diméthyl-1-[6-(méthyloxy)-3-pyridinyl]-1*H*-pyrazole-4-yl}acétamide,
le *N*-[(2-chloro-4-fluorophényl)méthyl]-2-{3,5-diméthyl-1-[5-(méthyloxy)-3-pyridinyl]-1*H*-pyrazole-4-yl}acétamide,
le *N*-[(2-chloro-4-fluorophényl)méthyl]-2-{3,5-diméthyl-1-[2-(méthyloxy)-3-pyridinyl]-1*H*-pyrazole-4-yl}acétamide,
le *N*-[(2-chloro-4-fluorophényl)méthyl]-2-[1-(2-hydroxy-éthyl)-5-(trifluorométhyl)-1*H*-pyrazole-4-yl}acétamide,
le *N*-[(2-chloro-4-fluorophényl)méthyl]-2-[1-(2-hydroxy-éthyl)-3-(trifluorométhyl)-1*H*-pyrazole-4-yl}acétamide,
le *N*-[(2-chloro-4-fluorophényl)méthyl]-2-[1-{2-[(2-hydroxy-éthyl)amino]éthyl}-3-(trifluorométhyl)-1*H*-pyrazole-4-yl]-acétamide,
le *N*-[(2-chloro-4-fluorophényl)méthyl]-2-[1-[2-(tétrahydro-2H-pyranne-4-ylamino)éthyl]-3-(trifluorométhyl)-1H-pyrazole-4-yl]acétamide,
le *N*-[(2-chloro-4-fluorophényl)méthyl]-2-[1-[2-(diméthyl-amino)éthyl]-5-(trifluorométhyl)-1*H-*pyrazole-4-yl]acétamide,
le N-[(2-chloro-4-fluorophényl)méthyl]-2-[1-[2-(tétrahydro-2H-pyranne-4-ylamino)éthyl]-5-(trifluorométhyl)-1H-pyrazole-4-yl]acétamide,
le *N*-[(2-chloro-4-fluorophényl)méthyl]-2-[1-[2-(1-pipéridinyl)éthyl]-5-(trifluorométhyl)-1*H-*pyrazole-4-yl]-acétamide,
le *N*-[(2-chloro-4-fluorophényl)méthyl]-2-[1-(2-{[2-(méthyloxy)éthyl]amino}éthyl)-5-(trifluorométhyl)-1*H-*pyrazole-4-yl]acétamide,
le 2-[3,5-diméthyl-1-(2-pyrimidinyl)-1*H*-pyrazole-4-yl]-*N*-[(2,3,4-trifluorophényl)méthyl]acétamide,
le 2-[5-bromo-1-(4-fluorophényl)-1*H*-pyrazole-4-yl]-*N-*[(2-chloro-4-fluorophényl)méthyl]acétamide, ou
le *N*-[(2-chloro-4-fluorophényl)méthyl]-2-[5-éthyl-1-(4-fluorophényl)-1*H*-pyrazole-4-yl]acétamide ;
ou un de ses sels pharmaceutiquement acceptables.

18. Composition pharmaceutique qui comprend un composé ou un de ses sels pharmaceutiquement acceptables, tel que défini dans l'une quelconque des revendications précédentes, et un support ou excipient pharmaceutiquement acceptable.

19. Composé ou un de ses sels pharmaceutiquement acceptables, tel que défini dans l'une quelconque des revendications 1 à 17, destiné à être utilisé en thérapie.

20. Utilisation d'un composé ou d'un de ses sels pharmaceutiquement acceptables, tel que défini dans l'une quelconque des revendications 1 à 17, pour la production d'un médicament destiné au traitement ou à la prévention de la douleur, de l'inflammation ou d'une maladie neurodégénérative.
